# EUROPEAN PATENT SPECIFICATION

(11) **EP 3 983 020 B1**
(45) Date of publication and mention of the grant of the patent: **26.06.2024**
(21) Application number: 20743893.8
(22) Date of filing: 17.06.2020
(51) Int. Cl.: A61K 51/10, A61P 35/02, C07K 16/32

(54) **AGENTS FOR CLEAVING LABELS FROM BIOMOLECULES IN VIVO**
MITTEL ZUM ABSPALTEN VON LABELN VON BIOMOLEKÜLEN IN VIVO
AGENTS DE SÉPARATION DE MARQUEURS DE BIOMOLÉCULES IN VIVO

(30) Priority: 17.06.2019 EP 19180694
(43) Date of publication of application: 20.04.2022
(60) Divisional application: 24174729.4
(73) Proprietor: Tagworks Pharmaceuticals B.V., 6525 ED Nijmegen (NL)
(72) Inventor: ROSSIN, Raffaella, 6525 ED Nijmegen (NL); ROBILLARD, Marc Stefan, 6525 ED Nijmegen (NL); KLEIJN, Laurens Henri Johan, 6525 ED Nijmegen (NL)
(74) Representative: V.O.
(86) International application number: PCT/NL2020/050387
(87) International publication number: WO 2020/256545

(56) References cited:
- WO-A1-2014/081303

## Description

### Field of the invention

The invention relates to compounds, combinations, and kits for the cleaving of imaging or radiotherapy labels from (bio)molecules in a subject, such as (bio)molecules that are administered to said subject for purposes such as targeted imaging and targeted radiotherapy.

### Background of the invention

In many areas of medical diagnostics, imaging and radiotherapy, it is desired to selectively deliver an agent, such as a radiotherapeutic agent or a diagnostic (e.g. imaging) agent, to a specific site, or a confined region, in the body of a subject such as a patient. Targeting of an organ or a tissue is typically achieved by the conjugation of the desired imaging or radiotherapy label (i.e. a radionuclide) to a targeting agent, which binds to cell surfaces or promotes cellular uptake at or near the target site of interest. The targeting agents used to target such labels are typically constructs that have affinity for cell surface targets (e.g., membrane receptors), structural proteins (e.g., amyloid plaques), or intracellular targets (e.g., RNA, DNA, enzymes, cell signaling pathways). These targeting agents can be antibodies (and fragments), proteins, aptamers, oligopeptides, oligonucleotides, oligosaccharides, as well as peptides, peptoids and organic drug compounds known to accumulate at a particular disease or malfunction. Alternatively, an imaging or radiotherapy agent may target a metabolic pathway, which is upregulated during a disease (like infection or cancer) such as DNA, protein, and membrane synthesis and carbohydrate uptake. In diseased tissues, abovementioned markers can discriminate diseased cells from healthy tissue and offer unique possibilities for early detection, specific diagnosis and (targeted) therapy.

As radio-imaging and radiotherapeutic agents (i.e. nuclear imaging and therapy agents) comprise radionuclides, which are radioactive, it is desired to quickly, efficiently, and/or conveniently reduce the amount of radionuclides in a patient once its therapeutic and/or imaging purposes are fulfilled. For example, this will enable reducing the dose of potentially harmful radiation given to the whole body. Also, in the context of imaging, clearing the radionuclides from the patient as quickly as possible after the imaging procedure, allows starting another imaging procedure in the same patient of the same or a different imaging target (i.e. image cycling). Natural clearance, however, is very slow.

Furthermore, an important criterion for successful imaging/therapy agents in general and nuclear imaging/therapy agents in particular is that they exhibit a high target uptake while showing an efficient clearance (through renal and/or hepatobiliary systems) from non-target tissues and from the blood. However, this is often problematic, especially when using antibodies. For example, imaging studies in humans have shown that the maximum concentration of a radiolabeled antibody at the tumor site is attainable within 24 h but several more days are required before the concentration of the labeled antibody in circulation decreases to levels low enough for successful imaging to take place. In the context of radioimmunotherapy (RIT), the slow antibody clearance from blood results in high radiation doses to *e.g.* the bone marrow limiting the amount of radioactivity that can be safely administered, limiting the therapeutic effect.

Further, targeted imaging (e.g. optical or nuclear) or radiotherapy can be hampered by circulating fractions of the receptor that is being targeted, and which can capture the imaging or radiotherapy agents before they can reach the target receptor at the target cell surface, negatively impacting target-background ratios.

In addition, off target uptake of imaging agents (e.g. in the liver) can obscure the target uptake.

Effectively, the targeting process can be divided into three processes:
(I) the administration process, in which the compound comprising a targeting agent is administered to a subject, and a fraction of said compound binds to the target;
(II) the clearance process, wherein the fraction of the compound comprising a targeting agent that circulates in the blood (rather than being bound to the target) is cleared (i.e. removed by excretion) from the blood and other non-target tissues;
(III) the imaging/therapy process, wherein the compound comprising a targeting agent present in the subject is used for imaging or therapy purposes.

It will be understood that the tumor-to-blood (T/B) ratios are increased in process (I) due to targeting, viz. the labelled compound accumulates at the targeted site, in this case a tumor.

For process (III), the TIE ratio should be sufficiently high, so that the fraction of circulating administered compound or compound bound to non-target tissues does not interfere with the imaging/therapy. Typically, this is achieved by waiting for an undesired long time during process (II), for reasons given above.

In general, it is desired to have methods to increase target-non target ratio of imaging or radiotherapy agents in the abovementioned process (II) more quickly, and to have temporal and spatial control over the action of those agents.

The poor T/B ratios for antibodies have led to pre-targeting approaches to improve image quality in radioimmunoimaging and to increase the therapeutic index in RIT. The long-circulating monoclonal antibody (mAb) is administered first, allowed to bind the tumor and slowly clear from circulation, after which a small radiolabeled probe is injected. This probe binds the tumor-bound antibody or otherwise rapidly clears from circulation, leading to improved TIE ratios.

Typically, a clearing agent is administered prior to injection of the probe, to clear any freely circulating antibody from blood, resulting in further improved TIE ratios [F.C. van de Watering et al., Front. Med. 2014, 1, 44]. However, pretargeting can typically only be used with non-internalizing receptors, and is relatively complex, requiring the optimization of dosing and timing for three agents.

An alternative approach is to administer a clearing agent to remove a radiolabeled antibody from circulation after sufficient amounts have bound the target, but this has not worked well in the clinic and gives high radiation doses to the liver [R.H.J. Begent et al., Br. J. Cancer 1989, 60, p. 406-412].

Another approach is to cleave the radiolabel from freely circulating antibody after sufficient amounts of radiolabeled antibody have bound and internalized in the target cells by of administering an enzyme designed to cleave the bond between antibody and label. However, the enzymatic cleavage method was rather slow and inefficient, giving only 3-fold improvements in TIE ratios [Q. Ren et al., Mol. Pharm. 2019, 16, p. 1065-1073]. In addition, three injections with the enzyme were typically required, which makes it an inconvenient method.

Another background reference is WO 2014/081303 A1 which discloses conjugates comprising trans-cyclooctene-derived groups which can be cleaved by reaction with a diene. Z

It is a desire to provide compounds and/or a combination of compounds that enables to quickly reduce the amount of radionuclides in a patient, e.g. after radioimaging is completed. It is also desired that a compound and/or a combination of compounds be provided that enables an increase of the target-non target ratio of imaging or radiotherapy agents in the clearance process. It is also desired that this increase is obtained faster than in known methods. Furthermore, it is desired to provide a compound and/or a combination of compounds that enables temporal and spatial control over the action of those agents. In addition, it is desired that this compound and/or a combination of compounds is versatile, rapid, efficient, and/or convenient.

### Summary of the invention

The invention, in one aspect, pertains to a compound satisfying Formula (1): 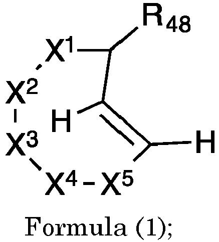 and pharmaceutically acceptable salts thereof, wherein
the compound of Formula (1) comprises at least one Label and at least one Administration Agent;
the Label is a moiety comprising a radionuclide;
the Administration Agent is an antibody, wherein the term "antibody" refers to an antibody, an antibody fragment which is at least a portion of the variable region of the immunoglobulin that binds to its target, or an antibody mimetic;
each X¹, X², X³, X⁴ is independently selected from the group consisting of -C(R₄₇)₂-, -NR₃₇-, -C(O)-, -O-, such that at most two of X¹, X², X³, X⁴ are not -C(R₄₇)₂-, and with the proviso that no sets consisting of adjacent atoms are present selected from the group consisting of -O-O-, -O-N-, -C(O)-O-, N-N-, and -C(O)-C(O)-;
X⁵ is -C(R₄₇)₂- or -CHR₄₈, preferably X⁵ is -C(R₄₇)₂-;
each R₄₈ is independently selected from the group consisting of -L^{B}, and -L^{A}; preferably R₄₈ is -L^{B};
R₄₈ is bound to the remainder of the compound of Formula (1) via a part of R₄₈ that is -O-, -S-, -OC(O)-, -OC(S)-, -SC(O)-, or -SC(S)-;
L^{B} is a moiety satisfying Formula (2): 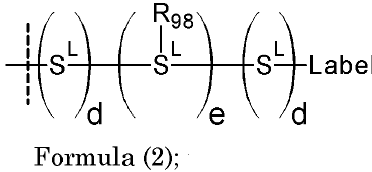 wherein
   the dashed line denotes a bond to the remainder of the compound of Formula (1); S^{L} is a linker, which optionally is a self-immolative linker L^{C};
   each R₉₈ individually is the Label or a clearance-directing group, wherein a clearance-directing group is a moiety that directs a compound to an excretory organ;
   each d independently is 0 or 1;
   e is an integer in a range of from 0 to 4, preferably e is 0;
   L^{A} is a moiety satisfying Formula (3): 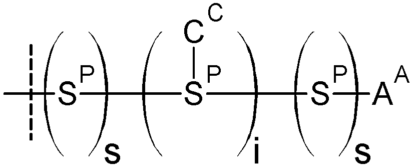 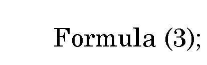 wherein
      the dashed line denotes a bond to the remainder of the compound of Formula (1); each s is independently 0 or 1; preferably each s is 0;
      i is an integer in a range of from 0 to 4, preferably 0 or 1, most preferably 0;
      each S^{P} independently is a spacer, which optionally is a self-immolative linker L^{C}; A^{A} denotes the Administration Agent;
      C^{C} denotes a Construct-C, wherein each Construct-C is independently selected from the group consisting of a Label and an Administration Agent; preferably the compound of Formula (1) comprises at most one C^{C};
      provided that L^{A} only comprises both the Label and the Administration Agent when L^{A} is R₄₈;
      provided that if L^{A} being R₄₈ comprises both the Label and the Administration Agent, then the S^{P} linked to said Label and said Administration Agent is a self-immolative linker, wherein said Label and said Administration Agent will be uncoupled after reaction of the compound of Formula (1) with a Cleaving Agent due to the self-immolative character of the linker, wherein the Cleaving Agent is a diene;
      each R₄₇ is independently selected from the group consisting of hydrogen, -L^{B}, -L^{A}, -(S^{P})ᵢ-C^{C}, -F, -Cl, -Br, -I, -OR₃₇, -N(R₃₇)₂, -SO₃, -PO₃-, -NO₂, - CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, -S(O)₂R₃₇, NR₃₇S(O)₂R₃₇, -ON(R₃₇)₂, .NR₃₇OR₃₇, C₁-C₂₄ alkyl groups, C₂-C₂₄ alkenyl groups, C₂-C₂₄ alkynyl groups, C₆-C₂₄ aryl groups, C₂-C₂₄ heteroaryl groups, C₃-C₂₄ cycloalkyl groups, C₅-C₂₄ cycloalkenyl groups, C₁₂-C₂₄ cycloalkynyl groups, C₃-C₂₄ (cyclo)alkyl(hetero)aryl groups, C₃-C₂₄ (hetero)aryl(cyclo)alkyl, C₄-C₂₄ (cyclo)alkenyl(hetero)aryl groups, C₄-C₂₄ (hetero)aryl(cyclo)alkenyl groups, C₄-C₂₄ (cyclo)alkynyl(hetero)aryl groups, C₄-C₂₄ (hetero)aryl(cyclo)alkynyl groups, C₄-C₂₄ alkylcycloalkyl groups, and C₄-C₂₄ cycloalkylalkyl groups; wherein i is an integer in a range of from 0 to 4, preferably i is an integer ranging from 0 to 1,
         wherein the alkyl groups, alkenyl groups, alkynyl groups, aryl, heteroaryl, cycloalkyl groups, cycloalkenyl groups, cycloalkynyl groups, (cyclo)alkyl(hetero)aryl groups, (hetero)aryl(cyclo)alkyl groups, (cyclo)alkenyl(hetero)aryl groups, (hetero)aryl(cyclo)alkenyl groups, (cyclo)alkynyl(hetero)aryl groups, (hetero)aryl(cyclo)alkynyl groups, alkylcycloalkyl groups, cycloalkylalkyl groups are optionally substituted with a moiety selected from the group consisting of -Cl, -F, -Br, -I, -OR₃₇, -N(R₃₇)₂, - SO₃R₃₇, -PO₃(R₃₇)₂, -PO₄(R₃₇)₂, -NO₂, -CF₃, =O, =NR₃₇, and -SR₃₇, and optionally contain one or more heteroatoms selected from the group consisting of O, S, NR₃₇, P, and Si, wherein the N, S, and P atoms are optionally oxidized,
         wherein the N atoms are optionally quaternized;
      two R₄₇ and/or R₃₇ are optionally comprised in a ring,
      two R₄₇ and/or R₃₇ are optionally comprised in a ring so as to form a ring fused to the eight membered trans-ring of Formula (1);
      each R₃₇ is independently selected from the group consisting of hydrogen, -L^{B}, - L^{A}, -(S^{P})ᵢ-C^{C}, C₁-C₂₄ alkyl groups, C₂-C₂₄ alkenyl groups, C₂-C₂₄ alkynyl groups, C₆-C₂₄ aryl groups, C₂-C₂₄ heteroaryl groups, C₃-C₂₄ cycloalkyl groups, C₅-C₂₄ cycloalkenyl groups, C₁₂-C₂₄ cycloalkynyl groups, C₃-C₂₄ (cyclo)alkyl(hetero)aryl groups, C₃-C₂₄ (hetero)aryl(cyclo)alkyl, C₄-C₂₄ (cyclo)alkenyl(hetero)aryl groups, C₄-C₂₄ (hetero)aryl(cyclo)alkenyl groups, C₄-C₂₄ (cyclo)alkynyl(hetero)aryl groups, C₄-C₂₄ (hetero)aryl(cyclo)alkynyl groups, C₄-C₂₄ alkylcycloalkyl groups, and C₄-C₂₄ cycloalkylalkyl groups; wherein i is an integer in a range of from 0 to 4, preferably i is 1;
      the R₃₇ groups not being hydrogen are optionally substituted with a moiety selected from the group consisting of -Cl, -F, -Br, -I, -OH, -NH₂, -SO₃H, -PO₃H, - PO₄H₂, -NO₂, -CF₃, =O, =NH, and -SH, and optionally contain one or more heteroatoms selected from the group consisting of O, S, NH, P, and Si, wherein the N, S, and P atoms are optionally oxidized, wherein the N atoms are optionally quaternized.

In another aspect, the invention relates to a combination comprising the compound according to Formula (1), and a Cleaving Agent, with the proviso that when at least one R₄₈ in Formula (1) comprises a Label, then the Cleaving Agent does not comprise the same Label as R₄₈; with the proviso that when at least one R₄₈ in Formula (1) comprises an Administration Agent, then the Cleaving Agent does not comprise the same Administration Agent as R₄₈;
wherein the Cleaving Agent is a diene.

In yet another aspect, the invention pertains to the compound according to Formula (1), or the combination according to the invention for use as a medicament.

In yet another aspect, the invention relates to the compound according to Formula (1), or the combination according to the invention, for use in the treatment of a disease, preferably cancer, in a subject, preferably a human, wherein the treatment is radiotherapy.

In a further aspect still, the invention relates to the compound according to Formula (1), or the combination according to the invention, for use in a diagnostic method comprising the steps of
(a) administering a compound according to Formula (1) as defined herein, to a subject, preferably a human;
(b) administering a Cleaving Agent as defined herein, to said subject;
(c) imaging the compound according to Formula (1) present in the subject to collect data;
(d) comparing said data to standard values;
(e) finding a significant deviation from said standard values during comparison;
(f) attributing the significant deviation to a particular clinical picture, preferably to cancer.

In yet another aspect, the invention pertains to a non-therapeutic method for imaging a compound according to Formula (1) in a subject, preferably a human wherein the following steps have been performed on the subject:
(a) administering a compound according to Formula (1) as defined herein, to the subject;
(b) administering a Cleaving Agent as defined herein, to said subject;
wherein the non-therapeutic method comprises the step of:
(c) imaging the compound according to Formula (1) present in the subject.

In yet a further aspect, the invention relates to a use of a compound according to Formula (1), or a combination according to the invention, for imaging in a subject, preferably a human.

### Brief Description of the Figures

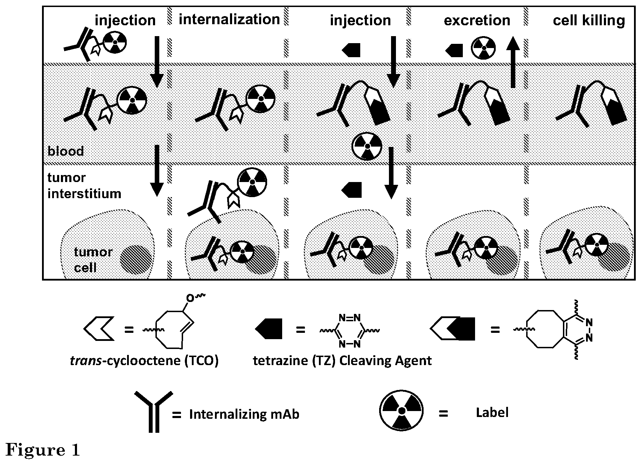

**Figure 1****.** General scheme depicting the use of the invention in radioimmunotherapy. A radiolabelled antibody is administered, allowed to circulate and bind an internalizing cancer receptor, and after sufficient internalization has occurred a Cleaving Agent is administered that cleaves the radiolabel (e.g. a moiety comprising a radiometal-chelate complex) from the antibody, resulting in rapid renal clearance of the radioactivity from blood and non-target tissues, but not of the tumor cell-internalized radioactivity.

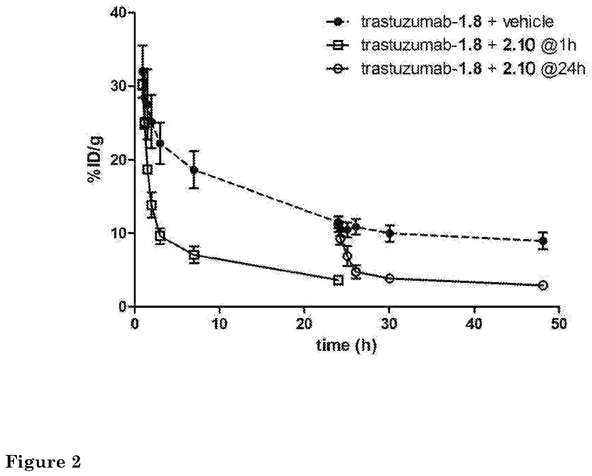

Figure 2. Radioactivity profiles in blood in mice injected with a mAb-trigger-Label conjugate followed by a Cleaving Agent or by vehicle. The Cleaving Agent is administered one hour or 24 hours post-mAb injection. The figure shows rapid clearance of the Label from blood upon trigger reaction with the Cleaving Agent in vivo.

### Detailed Description of the Invention

In a broad sense, the invention pertains to the judicious recognition that compounds according to Formula (1), and combinations and kits as defined herein, better address one or more of the abovementioned desires. The compounds, combinations, and kits of the invention can be used to quickly lower the amount of radionuclides in a subject. In particular, the compounds, combinations, and kits of the invention can be used to increase the target-non target ratio of imaging or radiotherapy agents in the clearance process, and more particularly to reach such an increase more rapidly.

The invention, in one aspect, presents the concept of administering a compound according to Formula (1) comprising a label (a moiety comprising a radionuclide) and an Administration Agent, and subsequently administering a Cleaving Agent comprising a diene in the clearance phase, said dienophile and diene being capable of undergoing a bio-orthogonal reaction with each other, resulting in the decoupling of the label from the Administration Agent, preferably at specifically the non-targeted site *(e.g.* the blood), and the efficient clearance of the released label from circulation and other non-target tissues, and/or the body as a whole. An Administration Agent is to be understood as any antibody, in particular those of which it is desired to image its biodistribution or target binding in vivo, or which is used as a targeting agent for therapeutic radiation.

This bio-orthogonal reaction is very fast, and readily results in good release of the moiety comprising a radionuclide. Typically, high release yields are obtained after only one administration of the Cleaving Agent, making the method efficient, and convenient for both the patient and the medical practitioner.

Thus, preferably the cleavage of the label from the compound of Formula (1) pertains to the fraction present in, or bound to non-target tissues while the portion bound to target tissues is not cleaved or does not lead to accelerated clearance.

The fact that the inverse electron demand Diels-Alder (IEDDA) reaction can be used in the abovementioned applications is surprising. Firstly, the IEDDA reaction has never been used to lower the amount of radionuclides in a subject. Instead, the IEDDA reaction has typically been used to specifically release a drug at a target site, after which the drug entered a cell, a tumor, and the like. Thus, this does not relate to radionuclides, and speaks against ways to quickly remove the released agent from the body. In addition, previously the IEDDA reaction was used to specifically release a drug at the target site specifically after the clearance process. Moreover, the IEDDA reaction was used in applications wherein no release occurs, but a clearance-directing group is used to remove the Administration Agent from circulation. By contrast, the present invention is based on the judicious insight that with the compounds and combinations of the invention, decoupling of a label and the Administration Agent occurs, preferably in the blood and other non-target sites, after which specifically the label is rapidly cleared (Figure 1).

Without wishing to be bound by theory, the inventors believe that an antibody comprised in the compounds of Formula (1) has a relatively slow clearance rate due large size and/or the binding to biomolecules and much larger structures, such as cells, which greatly reduces the clearance rate of the compound of Formula (1). By addition of a Cleaving Agent, the moiety comprising a radionuclide is released. Said moiety is by definition smaller than the compound of Formula (1), and has no specific affinity for any biomolecule and thus typically does not bind to such a biomolecule. The inventors believe that for at least these reasons, the released moiety is cleared much faster than the compound of Formula (1).

Specifically, when the Administration Agent is an intact IgG antibody, this antibody will clear very slowly from blood due to FcRn-mediated recycling. In addition, or alternatively, Administration Agents may bind to a Primary Target present in blood (e.g. on a blood cell or a shed receptor from a tumor) or other biomolecules (e.g. serum albumin) and tissues, target tissues and non-target tissues, and as a result clear slowly from the body as a whole, from the target tissues or from the non-target tissues. Also, proteins in general, including antibodies, can clear slowly due to relatively large size. On the contrary, the released moiety comprising a radionuclide is generally cleared fast, as it is much smaller and typically has low affinity to biomolecules and tissues. Reference is made to [Orcutt et al, Mol Imaging Biol. 2011, 215-221] demonstrating the fast clearance of radiolabeled chelate derivatives.

Thus, in some embodiments, a compound of Formula (1) comprising a label, particularly a radiolabeled chelate, and an Administration Agent, will be administered for the purpose of being targeted to and internalized by a certain tissue in the body, e.g. a tumor cell receptor or a brain target (Figure 1). Subsequent injection of Cleaving Agent, preferably a non-internalizing (i.e. not cell permeable) Cleaving Agent, results in release of the radiolabeled chelate from Administration Agent in circulation and its rapid excretion, while any released chelate inside the target cell will not be excreted, leading to increased target-non-target (T-NT) ratios, that in particular are achieved more rapidly than with known methods.

In other embodiments, the compound of Formula (1) targets a non-internalizing receptor in a tissue. Then, preferably the Cleaving Agent is chosen such that it does not significantly extravasate from blood into other tissues, to enable specific release of the Label in the blood, *i.e.* off-target.

The compound of Formula (1) can be specifically designed as an imaging agent of a particular target or process in vivo, such as in the case of radioimmunoimaging. It can also be specifically designed to deliver therapeutic radiation to a particular target in vivo, such as in the case of radioimmunotherapy.

A Cleaving Agent is an agent or compound that is administered to a subject for the purpose of cleaving the label from the Administration Agent resulting in a different biodistribution and pharmacokinetics of the released label compared to the label when bound to the Administration Agent. The released label has a much faster blood clearance than the compound of Formula (1) and/or the released label has a much lower retention in one or more non-target tissues such as epithelial cells, fat, muscle, and kidney and RES organs such as liver and spleen, than the compound of Formula (1).

The concept of cleaving a compound of Formula (1) in vivo is of general relevance in targeted radiotherapy and imaging.

### Definitions

The present invention will further be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where an indefinite or definite article is used when referring to a singular noun e.g. "a" or "an", "the", this includes a plural of that noun unless something else is specifically stated. The verb "to comprise", and its conjugations, as used in this description and in the claims is used in its non-limiting sense to mean that items following the word are included, but items not specifically mentioned are not excluded. Thus, the scope of the expression "a device comprising means A and B" should not be limited to devices consisting only of components A and B. It means that with respect to the present invention, the only relevant components of the device are A and B.

In addition, reference to an element by the indefinite article "a" or "an" does not exclude the possibility that more than one of the element is present, unless the context clearly requires that there is one and only one of the elements. The indefinite article "a" or "an" thus usually means "at least one".

The compounds disclosed in this description and in the claims may comprise one or more asymmetric centres, and different diastereomers and/or enantiomers may exist of the compounds. The description of any compound in this description and in the claims is meant to include all diastereomers, and mixtures thereof, unless stated otherwise. In addition, the description of any compound in this description and in the claims is meant to include both the individual enantiomers, as well as any mixture, racemic or otherwise, of the enantiomers, unless stated otherwise. When the structure of a compound is depicted as a specific enantiomer, it is to be understood that the invention of the present application is not limited to that specific enantiomer, unless stated otherwise. When the structure of a compound is depicted as a specific diastereomer, it is to be understood that the invention of the present application is not limited to that specific diastereomer, unless stated otherwise.

The compounds may occur in different tautomeric forms. The compounds according to the invention are meant to include all tautomeric forms, unless stated otherwise. When the structure of a compound is depicted as a specific tautomer, it is to be understood that the invention of the present application is not limited to that specific tautomer, unless stated otherwise.

The compounds disclosed in this description and in the claims may further exist as exo and endo diastereomers. Unless stated otherwise, the description of any compound in the description and in the claims is meant to include both the individual exo and the individual endo diastereomers of a compound, as well as mixtures thereof. When the structure of a compound is depicted as a specific endo or exo diastereomer, it is to be understood that the invention of the present application is not limited to that specific endo or exo diastereomer, unless stated otherwise.

Unless stated otherwise, the compounds of the invention and/or groups thereof may be protonated or deprotonated. It will be understood that it is possible that a compound may bear multiple charges which may be of opposite sign. For example, in a compound containing an amine and a carboxylic acid, the amine may be protonated while simultaneously the carboxylic acid is deprotonated.

The present invention also provides a combination of a compound of Formula (1) and a Cleaving Agent. The term "combination" is to be understood in broad sense, not limited to a kit comprising both components. Thus, the compound of Formula (1) and the Cleaving Agent can be provided totally separately of each other. It will be understood that the function of the Cleaving Agent in particular is to act in combination with a compound of Formula (1).

In several formulae, groups or substituents are indicated with reference to letters such as "A", "B", "X", "Y", and various (numbered) "R" groups. In addition, the number of repeating units may be referred to with a letter, e.g. n in -(CH₂)ₙ-. The definitions of these letters are to be read with reference to each formula, i.e. in different formulae these letters, each independently, can have different meanings unless indicated otherwise.

In several chemical formulae and texts below reference is made to "alkyl", "heteroalkyl", "aryl", "heteroaryl", "alkenyl", "alkynyl", "alkylene", "alkenylene", "alkynylene", "arylene", "cycloalkyl", "cycloalkenyl", "cycloakynyl", arenetriyl, and the like. The number of carbon atoms that these groups have, excluding the carbon atoms comprised in any optional substituents as defined herein, can be indicated by a designation preceding such terms (e.g. "C₁-C₈ alkyl" means that said alkyl may have from 1 to 8 carbon atoms). For the avoidance of doubt, a butyl group substituted with a -OCH₃ group is designated as a C₄ alkyl, because the carbon atom in the substituent is not included in the carbon count.

Unsubstituted alkyl groups have the general formula CₙH₂ₙ₊ᵢ and may be linear or branched. Optionally, the alkyl groups are substituted by one or more substituents further specified in this document. Examples of alkyl groups include methyl, ethyl, propyl, 2-propyl, t-butyl, 1-hexyl, 1-dodecyl, etc. Unless stated otherwise, an alkyl group optionally contains one or more heteroatoms independently selected from the group consisting of O, NR₃₆, S, P, and Si, wherein the N, S, and P atoms are optionally oxidized and the N atoms are optionally quaternized. In preferred embodiments, up to two heteroatoms may be consecutive, such as in for example -CH₂-NH-OCH₃ and -CH₂-O-Si(CH₃)₃. In some preferred embodiments the heteroatoms are not directly bound to one another. Examples of heteroalkyls include -CH₂CH₂-O-CH₃, -CH₂CH₂-NH-CH₃, - CH₂CH₂-S(O)-CH₃, -CH-CH-O-CH₃, -Si(CH₃)₃. In preferred embodiments, a C₁-C₄ alkyl contains at most 2 heteroatoms.

A cycloalkyl group is a cyclic alkyl group. Unsubstituted cycloalkyl groups comprise at least three carbon atoms and have the general formula CₙH₂ₙ₋₁ Optionally, the cycloalkyl groups are substituted by one or more substituents further specified in this document. Examples of cycloalkyl groups include cyclopropyl, cyclobutyl, cyclopentyl and cyclohexyl. Unless stated otherwise, a cycloalkyl group optionally contains one or more heteroatoms independently selected from the group consisting of O, NR₃₆, S, P, and Si, wherein the N, S, and P atoms are optionally oxidized and the N atoms are optionally quaternized.

An alkenyl group comprises one or more carbon-carbon double bonds, and may be linear or branched. Unsubstituted alkenyl groups comprising one C-C double bond have the general formula CₙH₂ₙ₋₁ Unsubstituted alkenyl groups comprising two C-C double bonds have the general formula CₙH₂ₙ₋₃. An alkenyl group may comprise a terminal carbon-carbon double bond and/or an internal carbon-carbon double bond. A terminal alkenyl group is an alkenyl group wherein a carbon-carbon double bond is located at a terminal position of a carbon chain. An alkenyl group may also comprise two or more carbon-carbon double bonds. Examples of an alkenyl group include ethenyl, propenyl, isopropenyl, t-butenyl, 1,3-butadienyl, 1,3-pentadienyl, etc. Unless stated otherwise, an alkenyl group may optionally be substituted with one or more, independently selected, substituents as defined herein. Unless stated otherwise, an alkenyl group optionally contains one or more heteroatoms independently selected from the group consisting of O, NR₃₆, S, P, and Si, wherein the N, S, and P atoms are optionally oxidized and the N atoms are optionally quaternized.

An alkynyl group comprises one or more carbon-carbon triple bonds, and may be linear or branched. Unsubstituted alkynyl groups comprising one C-C triple bond have the general formula CₙH₂ₙ₋₃. An alkynyl group may comprise a terminal carbon-carbon triple bond and/or an internal carbon-carbon triple bond. A terminal alkynyl group is an alkynyl group wherein a carbon-carbon triple bond is located at a terminal position of a carbon chain. An alkynyl group may also comprise two or more carbon-carbon triple bonds. Unless stated otherwise, an alkynyl group may optionally be substituted with one or more, independently selected, substituents as defined herein. Examples of an alkynyl group include ethynyl, propynyl, isopropynyl, t-butynyl, etc. Unless stated otherwise, an alkynyl group optionally contains one or more heteroatoms independently selected from the group consisting of O, NR₃₆, S, P, and Si, wherein the N, S, and P atoms are optionally oxidized and the N atoms are optionally quaternized.

An aryl group refers to an aromatic hydrocarbon ring system that comprises six to twenty-four carbon atoms, more preferably six to twelve carbon atoms, and may include monocyclic and polycyclic structures. When the aryl group is a polycyclic structure, it is preferably a bicyclic structure. Optionally, the aryl group may be substituted by one or more substituents further specified in this document. Examples of aryl groups are phenyl and naphthyl.

Arylalkyl groups and alkylaryl groups comprise at least seven carbon atoms and may include monocyclic and bicyclic structures. Optionally, the arylalkyl groups and alkylaryl may be substituted by one or more substituents further specified in this document. An arylalkyl group is for example benzyl. An alkylaryl group is for example 4-tert-butylphenyl.

Heteroaryl groups comprise at least two carbon atoms (i.e. at least C₂) and one or more heteroatoms N, O, P or S. A heteroaryl group may have a monocyclic or a bicyclic structure. Optionally, the heteroaryl group may be substituted by one or more substituents further specified in this document. Examples of suitable heteroaryl groups include pyridinyl, quinolinyl, pyrimidinyl, pyrazinyl, pyrazolyl, imidazolyl, thiazolyl, pyrrolyl, furanyl, triazolyl, benzofuranyl, indolyl, purinyl, benzoxazolyl, thienyl, phospholyl and oxazolyl. Heteroaryl groups preferably comprise five to sixteen carbon atoms and contain between one to five heteroatoms.

Heteroarylalkyl groups and alkylheteroaryl groups comprise at least three carbon atoms (i.e. at least C₃) and may include monocyclic and bicyclic structures. Optionally, the heteroaryl groups may be substituted by one or more substituents further specified in this document.

Where an aryl group is denoted as a (hetero)aryl group, the notation is meant to include an aryl group and a heteroaryl group. Similarly, an alkyl(hetero)aryl group is meant to include an alkylaryl group and an alkylheteroaryl group, and (hetero)arylalkyl is meant to include an arylalkyl group and a heteroarylalkyl group. A C₂-C₂₄ (hetero)aryl group is thus to be interpreted as including a C₂-C₂₄ heteroaryl group and a C₆-C₂₄ aryl group. Similarly, a C₃-C₂₄ alkyl(hetero)aryl group is meant to include a C₇-C₂₄ alkylaryl group and a C₃-C₂₄ alkylheteroaryl group, and a C₃-C₂₄ (hetero)arylalkyl is meant to include a C₇-C₂₄ arylalkyl group and a C₃-C₂₄ heteroarylalkyl group.

A cycloalkenyl group is a cyclic alkenyl group. An unsubstituted cycloalkenyl group comprising one double bond has the general formula CₙH₂ₙ₋₃. Optionally, a cycloalkenyl group is substituted by one or more substituents further specified in this document. An example of a cycloalkenyl group is cyclopentenyl. Unless stated otherwise, a cycloalkenyl group optionally contains one or more heteroatoms independently selected from the group consisting of O, NR₃₆, S, P, and Si, wherein the N, S, and P atoms are optionally oxidized and the N atoms are optionally quaternized.

A cycloalkynyl group is a cyclic alkynyl group. An unsubstituted cycloalkynyl group comprising one triple bond has the general formula CₙH₂ₙ₋₅. Optionally, a cycloalkynyl group is substituted by one or more substituents further specified in this document. An example of a cycloalkynyl group is cyclooctynyl. Unless stated otherwise, a cycloalkynyl group optionally contains one or more heteroatoms independently selected from the group consisting of O, NR₃₆, S, P, and Si, wherein the N, S, and P atoms are optionally oxidized and the N atoms are optionally quaternized.

In general, when (hetero) is placed before a group, it refers to both the variant of the group without the prefix hetero- as well as the group with the prefix hetero-. Herein, the prefix hetero- denotes that the group contains one or more heteroatoms selected from the group consisting of O, N, S, P, and Si. It will be understood that groups with the prefix hetero- by definition contain heteroatoms. Hence, it will be understood that if a group with the prefix hetero-is part of a list of groups that is defined as optionally containing heteroatoms, that for the groups with the prefix hetero- it is not optional to contain heteroatoms, but is the case by definition.

Herein, it will be understood that when the prefix hetero- is used for combinations of groups, the prefix hetero- only refers to the one group before it is directly placed. For example, heteroarylalkyl denotes the combination of a heteroaryl group and an alkyl group, not the combination of a heteroaryl and a heteroalkyl group. As such, it will be understood that when the prefix hetero- is used for a combination of groups that is part of a list of groups that are indicated to optionally contain heteroatoms, it is only optional for the group within the combination without the prefix hetero- to contain a heteroatom, as it is not optional for the group within the combination with the prefix hetero- by definition (see above). For example, if heteroarylalkyl is part of a list of groups indicated to optionally contain heteroatoms, the heteroaryl part is considered to contain heteroatoms by definition, while for the alkyl part it is optional to contain heteroatoms.

Herein, the prefix cyclo- denotes that groups are cyclic. It will be understood that when the prefix cyclo- is used for combinations of groups, the prefix cyclo- only refers to the one group before it is directly placed. For example, cycloalkylalkenylene denotes the combination of a cycloalkylene group (see the definition of the suffix -ene below) and an alkenylene group, not the combination of a cycloalkylene and a cycloalkenylene group.

In general, when (cyclo) is placed before a group, it refers to both the variant of the group without the prefix cyclo- as well as the group with the prefix cyclo-.

Herein, the suffix -ene denotes divalent groups, i.e. that the group is linked to at least two other moieties. An example of an alkylene is propylene (-CH₂-CH₂-CH₂-), which is linked to another moiety at both termini. It is understood that if a group with the suffix -ene is substituted at one position with -H, then this group is identical to a group without the suffix. For example, an alkylene substituted with -H is identical to an alkyl group. I.e. propylene, -CH₂-CH₂-CH₂-, substituted with -H at one terminus, -CH₂-CH₂-CH₂-H, is logically identical to propyl, -CH₂-CH₂-CH₃.

Herein, when combinations of groups are listed with the suffix -ene, it refers to a divalent group, i.e. that the group is linked to at least two other moieties, wherein each group of the combination contains one linkage to one of these two moieties. As such, for example alkylarylene is understood as a combination of an arylene group and an alkylene group. An example of an alkylarylene group is -phenyl-CH₂-, and an example of an arylalkylene group is -CH₂-phenyl-.

Herein, the suffix -triyl denotes trivalent groups, i.e. that the group is linked to at least three other moieties. An example of an arenetriyl is depicted below: 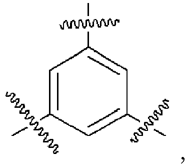 wherein the wiggly lines denote bonds to different groups of the main compound.

It is understood that if a group with the suffix -triyl is substituted at one position with -H, then this group is identical to a divalent group with the suffix -ene. For example, an arenetriyl substituted with -H is identical to an arylene group. Similarly, it is understood that if a group with the suffix -triyl is substituted at two positions with -H, then this group is identical to a monovalent group. For example, an arenetriyl substituted with two -H is identical to an aryl group.

It is understood that if a group, for example an alkyl group, contains a heteroatom, then this group is identical to a hetero-variant of this group. For example, if an alkyl group contains a heteroatom, this group is identical to a heteroalkyl group. Similarly, if an aryl group contains a heteroatom, this group is identical to a heteroaryl group. It is understood that "contain" and its conjugations mean herein that when a group contains a heteroatom, this heteroatom is part of the backbone of the group. For example, a C₂ alkylene containing an N refers to -NH-CH₂-CH₂-, -CH₂-NH-CH₂-, and -CH₂-CH₂-NH-.

Unless indicated otherwise, a group may contain a heteroatom at non-terminal positions or at one or more terminal positions. In this case, "terminal" refers to the terminal position within the group, and not necessarily to the terminal position of the entire compound. For example, if an ethylene group contains a nitrogen atom, this may refer to -NH-CH₂-CH₂-, -CH₂-NH-CH₂-, and -CH₂-CH₂-NH-. For example, if an ethyl group contains a nitrogen atom, this may refer to -NH-CH₂-CH₃, -CH₂-NH-CH₃, and -CH₂-CH₂-NH₂.

Herein, it is understood that cyclic compounds (i.e. aryl, cycloalkyl, cycloalkenyl, etc.) are understood to be monocyclic, polycyclic or branched. It is understood that the number of carbon atoms for cyclic compounds not only refers to the number of carbon atoms in one ring, but that the carbon atoms may be comprised in multiple rings. These rings may be fused to the main ring or substituted onto the main ring. For example, C₁₀ aryl optionally containing heteroatoms may refer to *inter alia* a naphthyl group (fused rings) or to *e.g.* a bipyridyl group (substituted rings, both containing an N atom).

Unless stated otherwise, (hetero)alkyl groups, (hetero)alkenyl groups, (hetero)alkynyl groups, (hetero)cycloalkyl groups, (hetero)cycloalkenyl groups, (hetero)cycloalkynyl groups, (hetero)alkylcycloalkyl groups, (hetero)alkylcycloalkenyl groups, (hetero)alkylcycloalkynyl groups, (hetero)cycloalkylalkyl groups, (hetero)cycloalkenylalkyl groups, (hetero)cycloalkynylalkyl groups, (hetero)alkenylcycloalkyl groups, (hetero)alkenylcycloalkenyl groups, (hetero)alkenylcycloalkynyl groups, (hetero)cycloalkylalkenyl groups, (hetero)cycloalkenylalkenyl groups, (hetero)cycloalkynylalkenyl groups, (hetero)alkynylcycloalkyl groups, (hetero)alkynylcycloalkenyl groups, (hetero)alkynylcycloalkynyl groups, (hetero)cycloalkylalkynyl groups, (hetero)cycloalkenylalkynyl groups, (hetero)cycloalkynylalkynyl groups, (hetero)aryl groups, (hetero)arylalkyl groups, (hetero)arylalkenyl groups, (hetero)arylalkynyl groups, alkyl(hetero)aryl groups, alkenyl(hetero)aryl groups, alkynyl(hetero)aryl groups, cycloalkyl(hetero)aryl groups, cycloalkenyl(hetero)aryl groups, cycloalkynyl(hetero)aryl groups, (hetero)arylcycloalkyl groups, (hetero)arylcycloalkenyl groups, (hetero)arylcycloalkynyl groups, (hetero)alkylene groups, (hetero)alkenylene groups, (hetero)alkynylene groups, (hetero)cycloalkylene groups, (hetero)cycloalkenylene groups, (hetero)cycloalkynylene groups, (hetero)arylene groups, alkyl(hetero)arylene groups, (hetero)arylalkylene groups, (hetero)arylalkenylene groups, (hetero)arylalkynylene groups, alkenyl(hetero)arylene, alkynyl(hetero)arylene, (hetero)arenetriyl groups, (hetero)cycloalkanetriyl groups, (hetero)cycloalkenetriyl and (hetero)cycloalkynetriyl groups are optionally substituted with one or more substituents independently selected from the group consisting of -Cl, -F, -Br, -I, - OH, -NH₂, -SO₃H, -PO₃H, -PO₄H₂, -NO₂, -CF₃, =O, =NR₃₆, -SR₃₆, C₁-C₂₄ alkyl groups, C₂-C₂₄ alkenyl groups, C₂-C₂₄ alkynyl groups, C₆-C₂₄ aryl groups, C₂-C₂₄ heteroaryl groups, C₃-C₂₄ cycloalkyl groups, C₅-C₂₄ cycloalkenyl groups, C₁₂-C₂₄ cycloalkynyl groups, C₃-C₂₄ alkyl(hetero)aryl groups, C₃-C₂₄ (hetero)arylalkyl groups, C₄-C₂₄ (hetero)arylalkenyl groups, C₄-C₂₄ (hetero)arylalkynyl groups, C₄-C₂₄ alkenyl(hetero)aryl groups, C₄-C₂₄ alkynyl(hetero)aryl groups, C₄-C₂₄ alkylcycloalkyl groups, C₆-C₂₄ alkylcycloalkenyl groups, C₁₃-C₂₄ alkylcycloalkynyl groups, C₄-C₂₄ cycloalkylalkyl groups, C₆-C₂₄ cycloalkenylalkyl groups, C₁₃-C₂₄ cycloalkynylalkyl groups, C₅-C₂₄ alkenylcycloalkyl groups, C₇-C₂₄ alkenylcycloalkenyl groups, C₁₄-C₂₄ alkenylcycloalkynyl groups, C₅-C₂₄ cycloalkylalkenyl groups, C₇-C₂₄ cycloalkenylalkenyl groups, C₁₄-C₂₄ cycloalkynylalkenyl groups, C₅-C₂₄ alkynylcycloalkyl groups, C₇-C₂₄ alkynylcycloalkenyl groups, C₁₄-C₂₄ alkynylcycloalkynyl groups, C₅-C₂₄ cycloalkylalkynyl groups, C₇-C₂₄ cycloalkenylalkynyl groups, C₁₄-C₂₄ cycloalkynylalkynyl groups, C₅-C₂₄ cycloalkyl(hetero)aryl groups, C₇-C₂₄ cycloalkenyl(hetero)aryl groups, C₁₄-C₂₄ cycloalkynyl(hetero)aryl groups, C₅-C₂₄ (hetero)arylcycloalkyl groups, C₇-C₂₄ (hetero)arylcycloalkenyl groups, and C₁₄-C₂₄ (hetero)arylcycloalkynyl groups. Unless stated otherwise, the substituents disclosed herein optionally contain one or more heteroatoms selected from the group consisting of O, S, NR₃₆, P, and Si, wherein the N, S, and P atoms are optionally oxidized, wherein the N atoms are optionally quaternized. Preferably, these substituents optionally contain one or more heteroatoms selected from the group consisting of O, S, and NR₃₆.

In preferred embodiments, the substituents are selected from the group consisting of -Cl, -F, -Br, -I, -OH, -NH₂, -SO₃H, -PO₃H, -PO₄H₂, -NO₂, -CF₃, =O, =NR₃₆, -SR₃₆, C₁-C₁₂ alkyl groups, C₂-C₁₂ alkenyl groups, C₂-C₁₂ alkynyl groups, C₆-C₁₂ aryl groups, C₂-C₁₂ heteroaryl groups, C₃-C₁₂ cycloalkyl groups, C₅-C₁₂ cycloalkenyl groups, C₁₂ cycloalkynyl groups, C₃-C₁₂ alkyl(hetero)aryl groups, C₃-C₁₂ (hetero)arylalkyl groups, C₄-C₁₂ (hetero)arylalkenyl groups, C₄-C₁₂ (hetero)arylalkynyl groups, C₄-C₁₂ alkenyl(hetero)aryl groups, C₄-C₁₂ alkynyl(hetero)aryl groups, C₄-C₁₂ alkylcycloalkyl groups, C₆-C₁₂ alkylcycloalkenyl groups, C₁₃-C₁₆ alkylcycloalkynyl groups, C₄-C₁₂ cycloalkylalkyl groups, C₆-C₁₂ cycloalkenylalkyl groups, C₁₃-C₁₆ cycloalkynylalkyl groups, C₅-C₁₂ alkenylcycloalkyl groups, C₇-C₁₂ alkenylcycloalkenyl groups, C₁₄-C₁₆ alkenylcycloalkynyl groups, C₅-C₁₂ cycloalkylalkenyl groups, C₇-C₁₂ cycloalkenylalkenyl groups, C₁₄-C₁₆ cycloalkynylalkenyl groups, C₅-C₁₂ alkynylcycloalkyl groups, C₇-C₁₂ alkynylcycloalkenyl groups, C₁₄-C₁₆ alkynylcycloalkynyl groups, C₅-C₁₂ cycloalkylalkynyl groups, C₇-C₁₂ cycloalkenylalkynyl groups, C₁₄-C₁₆ cycloalkynylalkynyl groups, C₅-C₁₂ cycloalkyl(hetero)aryl groups, C₇-C₁₂ cycloalkenyl(hetero)aryl groups, C₁₄-C₁₆ cycloalkynyl(hetero)aryl groups, C₅-C₁₂ (hetero)arylcycloalkyl groups, C₇-C₁₂ (hetero)arylcycloalkenyl groups, and C₁₄-C₁₆ (hetero)arylcycloalkynyl groups.

In preferred embodiments, the substituents are selected from the group consisting of -Cl, -F, -Br, -I, -OH, -NH₂, -SO₃H, -PO₃H, -PO₄H₂, -NO₂, -CF₃, =O, =NR₃₆, -SR₃₆, C₁-C₇ alkyl groups, C₂-C₇ alkenyl groups, C₂-C₇ alkynyl groups, C₆-C₇ aryl groups, C₂-C₇ heteroaryl groups, C₃-C₇ cycloalkyl groups, C₅-C₇ cycloalkenyl groups, C₁₂ cycloalkynyl groups, C₃-C₇ alkyl(hetero)aryl groups, C₃-C₇ (hetero)arylalkyl groups, C₄-C₈ (hetero)arylalkenyl groups, C₄-C₈ (hetero)arylalkynyl groups, C₄-C₈ alkenyl(hetero)aryl groups, C₄-C₈ alkynyl(hetero)aryl groups, C₄-C₇ alkylcycloalkyl groups, C₆-C₇ alkylcycloalkenyl groups, C₁₃-C₁₆ alkylcycloalkynyl groups, C₄-C₇ cycloalkylalkyl groups, C₆-C₇ cycloalkenylalkyl groups, C₁₃-C₁₆ cycloalkynylalkyl groups, C₅-C₇ alkenylcycloalkyl groups, C₇-C₇ alkenylcycloalkenyl groups, C₁₄-C₁₆ alkenylcycloalkynyl groups, C₅-C₇ cycloalkylalkenyl groups, C₇-C₈ cycloalkenylalkenyl groups, C₁₄-C₁₆ cycloalkynylalkenyl groups, C₅-C₇ alkynylcycloalkyl groups, C₇-C₈ alkynylcycloalkenyl groups, C₁₄-C₁₆ alkynylcycloalkynyl groups, C₅-C₇ cycloalkylalkynyl groups, C₇-C₈ cycloalkenylalkynyl groups, C₁₄-C₁₆ cycloalkynylalkynyl groups, C₅-C₉ cycloalkyl(hetero)aryl groups, C₇-C₁₁ cycloalkenyl(hetero)aryl groups, C₁₄-C₁₆ cycloalkynyl(hetero)aryl groups, C₅-C₉ (hetero)arylcycloalkyl groups, C₇-C₁₁ (hetero)arylcycloalkenyl groups, and C₁₄-C₁₆ (hetero)arylcycloalkynyl groups.

Unless stated otherwise, any group disclosed herein that is not cyclic is understood to be linear or branched. In particular, (hetero)alkyl groups, (hetero)alkenyl groups, (hetero)alkynyl groups, (hetero)alkylene groups, (hetero)alkenylene groups, (hetero)alkynylene groups, and the like are linear or branched, unless stated otherwise.

The general term "sugar" is herein used to indicate a monosaccharide, for example glucose (Glc), galactose (Gal), mannose (Man) and fucose (Fuc). The term "sugar derivative" is herein used to indicate a derivative of a monosaccharide sugar, i.e. a monosaccharide sugar comprising substituents and/or functional groups. Examples of a sugar derivative include amino sugars and sugar acids, e.g. glucosamine (GlcNH₂), galactosamine (GalNH₂), N-acetylglucosamine (GlcNAc), N-acetylgalactosamine (GalNAc), sialic acid (Sia) which is also referred to as N-acetylneuraminic acid (NeuNAc), and N-acetylmuramic acid (MurNAc), glucuronic acid (GlcA) and iduronic acid (ldoA).

A sugar may be without further substitution, and then it is understood to be a monosaccharide. A sugar may be further substituted with at one or more of its hydroxyl groups, and then it is understood to be a disaccharide or an oligosaccharide. A disaccharide contains two monosaccharide moieties linked together. An oligosaccharide chain may be linear or branched, and may contain from 3 to 10 monosaccharide moieties.

The term "protein" is herein used in its normal scientific meaning. Herein, polypeptides comprising about 10 or more amino acids are considered proteins. A protein may comprise natural, but also unnatural amino acids. The term "protein" herein is understood to comprise antibodies and antibody fragments.

The term "peptide" is herein used in its normal scientific meaning. Herein, peptides are considered to comprise a number of amino acids in a range of from 2 to 9.

The term "peptoids" is herein used in its normal scientific meaning.

An antibody is a protein, typically generated by the immune system that is capable of recognizing and binding to a specific antigen. While antibodies or immunoglobulins derived from IgG antibodies are particularly well-suited for use in this invention, immunoglobulins from any of the classes or subclasses may be selected, e.g. IgG, IgA, IgM, IgD and IgE. Suitably, the immunoglobulin is of the class IgG including but not limited to IgG subclasses (IgG1, 2, 3 and 4) or class IgM which is able to specifically bind to a specific epitope on an antigen. Antibodies can be intact immunoglobulins derived from natural sources or from recombinant sources and can be immunoreactive portions of intact immunoglobulins. Antibodies may exist in a variety of forms including, for example, polyclonal antibodies, monoclonal antibodies, camelized single domain antibodies, recombinant antibodies, anti-idiotype antibodies, multispecific antibodies, antibody fragments, such as, Fv, VHH, Fab, F(ab)₂, Fab', Fab'-SH, F(ab')₂, single chain variable fragment antibodies (scFv), tandem/bis-scFv, Fc, pFc', scFv-Fc, disulfide Fv (dsFv), bispecific antibodies (bc-scFv) such as BiTE antibodies, trispecific antibody derivatives such as tribodies, camelid antibodies, minibodies, nanobodies, resurfaced antibodies, humanized antibodies, fully human antibodies, single domain antibodies (sdAb, also known as Nanobody^{™}), chimeric antibodies, antibody fusions, chimeric antibodies comprising at least one human constant region, dual-affinity antibodies such as dual-affinity retargeting proteins (DART^{™}), and multimers and derivatives thereof, such as divalent or multivalent single-chain variable fragments (e.g. di-scFvs, tri-scFvs) including but not limited to minibodies, diabodies, triabodies, tribodies, tetrabodies, and the like, and multivalent antibodies. Reference is made to [Trends in Biotechnology 2015, 33, 2, 65], [Trends Biotechnol. 2012, 30, 575-582], and [Canc. Gen. Prot. 2013 10, 1-18], and [BioDrugs 2014, 28, 331-343]. "Antibody fragment" refers to at least a portion of the variable region of the immunoglobulin that binds to its target, i.e. the antigen-binding region. Other embodiments use antibody mimetics, such as but not limited to Affimers, Anticalins, Avimers, Alphabodies, Affibodies, DARPins, and multimers and derivatives thereof; reference is made to [Trends in Biotechnology 2015, 33, 2, 65]. "Antibody" as used herein also refers to antibodies with further functionalities, such as labelled antibodies, particularly radiolabeled antibodies, and antibody-drug conjugates. For the avoidance of doubt, in the context of this invention the term "antibody" is meant to encompass all of the antibody variations, fragments, derivatives, fusions, analogs and mimetics outlined in this paragraph, unless specified otherwise.

A linker is herein defined as a moiety that connects two or more elements of a compound. For example in a bioconjugate, a biomolecule and another moiety, e.g. a label, are covalently connected to each other via a linker.

A biomolecule is herein defined as any molecule that can be isolated from nature or any molecule composed of smaller molecular building blocks that are the constituents of macromolecular structures derived from nature, in particular nucleic acids, proteins, glycans and lipids. Examples of a biomolecule include an enzyme, a (non-catalytic) protein, a polypeptide, a peptide, an amino acid, an oligonucleotide, a monosaccharide, an oligosaccharide, a polysaccharide, a glycan, a lipid and a hormone.

As used herein, an organic molecule is defined as a molecule comprising a C-H bond. Organic compound and organic molecule are used synonymously. It will be understood that "organic molecule" as used herein includes biomolecules, such as nucleic acids (oligonucleotides, polynucleotides, DNA, RNA), peptides, proteins (in particular antibodies), carbohydrates (monosaccharides, oligosaccharides, and polysaccharides), aptamers, hormones, toxins, steroids, cytokines, and lipids; small organic molecules as defined herein; polymers (in particular polyethylene glycol); LNA and PNA; amino acids; peptoids; molecules comprising a radionuclide; fluorescent dyes; drugs; resins (in particular polystyrene and agarose); beads; particles (in particular polymersomes, liposomes, and beads); gels; surfaces; organometallic compounds; cells; and combinations thereof.

As used herein, an inorganic molecule is defined as any molecule not being an organic molecule, *i.e.* not comprising a C-H bond. It will be understood that "inorganic molecule" as used herein includes surfaces (in particular chips, wafers, gold, metal, silica-based surfaces such as glass); particles such as beads (in particular magnetic beads, gold beads), silica-based particles, polymer-based materials, iron oxide particles; caron nanotubes; allotropes of carbon (in particular fullerenes such as Buckminsterfullerene; graphite, graphene, diamond, Lonsdaleite, Q-carbon, linearn acetylenic carbon, amorphous carbon, and carbon nanotubes); drugs (in particular cisplatin); and combinations thereof.

As used herein, "particle" is preferably defined as a microparticle or a nanoparticle.

The term "salt thereof' means a compound formed when an acidic proton, typically a proton of an acid, is replaced by a cation, such as a metal cation or an organic cation and the like. The term "salt thereof' also means a compound formed when an amine is protonated. Where applicable, the salt is a pharmaceutically acceptable salt, although this is not required for salts that are not intended for administration to a patient. For example, in a salt of a compound the compound may be protonated by an inorganic or organic acid to form a cation, with the conjugate base of the inorganic or organic acid as the anionic component of the salt.

The term "pharmaceutically accepted" salt means a salt that is acceptable for administration to a patient, such as a mammal (salts with counterions having acceptable mammalian safety for a given dosage regime). Such salts may be derived from pharmaceutically acceptable inorganic or organic bases and from pharmaceutically acceptable inorganic or organic acids.

"Pharmaceutically acceptable salt" refers to pharmaceutically acceptable salts of a compound, which salts are derived from a variety of organic and inorganic counter ions known in the art and include, for example, sodium, potassium, calcium, magnesium, ammonium, tetraalkylammonium, etc., and when the molecule contains a basic functionality, salts of organic or inorganic acids, such as hydrochloride, hydrobromide, formate, tartrate, besylate, mesylate, acetate, maleate, oxalate, etc.

In preferred embodiments, in relation to the invention "particle" is defined as a nanoparticle or a microparticle.

The logarithm of the partition-coefficient, i.e. Log P, is herein used as a measure of the hydrophobicity of a compound. Typically, the Log P is defined as 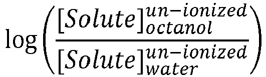 The skilled person is aware of methods to determine the partition-coefficient of compounds without undue experimentation. Alternatively, the skilled person knows that software is available to reliably estimate the Log P value, for example as a function within ChemDraw^{®} software or online available tools.

The unified atomic mass unit or Dalton is herein abbreviated to Da. The skilled person is aware that Dalton is a regular unit for molecular weight and that 1 Da is equivalent to 1 g/mol (grams per mole).

It will be understood that herein, the terms "moiety" and "group" are used interchangeably when referring to a part of a molecule.

It will be understood that when a heteroatom is denoted as -X(R')₂-, wherein X is the heteroatom and R' is a certain moiety, then this denotes that two moieties R' are attached to the heteroatom.

It will be understood that when a group is denoted as, for example, -((R₅₁)₂-R₅₂)₂- or a similar notation, in which R₅₁ and R₅₂ are certain moieties, then this denotes that first, it should be written as -R₅₁-R₅₁-R₅₂-R₅₁-R₅₁-R₅₂-before the individual R₅₁ and R₅₂ moieties are selected, rather than first selecting moieties R₅₁ and R₅₂ and then writing out the formula.

### The Inverse Electron-Demand Diels-Alder reaction (IEDDA)

The established IEDDA conjugation chemistry generally involves a pair of reactants that comprise, as one reactant (i.e. one Bio-orthogonal Reactive Group), a suitable diene, such as a derivative of tetrazine (TZ), e.g. an electron-deficient tetrazine and, as the other reactant (i.e. the other Bio-orthogonal Reactive Group), a suitable dienophile, such as a *trans*-cyclooctene (TCO). The exceptionally fast reaction of (substituted) tetrazines, in particular electron-deficient tetrazines, with a TCO moiety results in an intermediate that rearranges to a dihydropyridazine Diels-Alder adduct by eliminating N₂ as the sole by-product. The initially formed 4,5-dihydropyridazine product may tautomerize to a 1,4- or a 2,5-dihydropyridazine product, especially in aqueous environments. Below a reaction scheme is given for a [4+2] IEDDA reaction between (3,6)-di-(2-pyridyl)-s-tetrazine diene and a *trans*-cyclooctene dienophile, followed by a retro Diels Alder reaction in which the product and dinitrogen is formed. Because the *trans*-cyclooctene derivative does not contain electron withdrawing groups as in the classical Diels Alder reaction, this type of Diels Alder reaction is distinguished from the classical one, and frequently referred to as an "inverse-electron-demand Diels Alder (IEDDA) reaction". In the following text the sequence of both reaction steps, i.e. the initial Diels-Alder cyclo-addition (typically an inverse electron-demand Diels Alder cyclo-addition) and the subsequent retro Diels Alder reaction will be referred to in shorthand as the "inverse electron-demand Diels Alder reaction" or "inverse electron-demand Diels Alder conjugation" or "IEDDA". The product of the reaction is then the IEDDA adduct or conjugate. This is illustrated in Scheme 1 below.

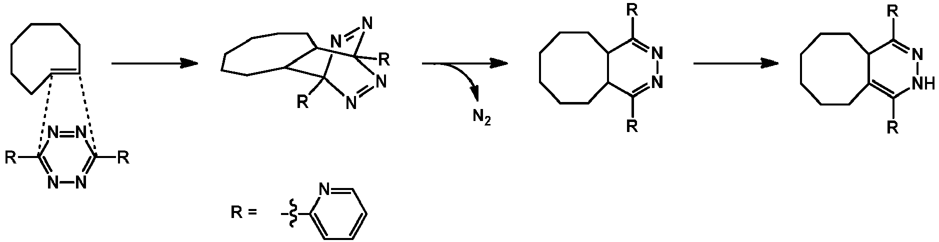

### Scheme 1: the IEDDA conjugation reaction

The two reactive species are abiotic and do not undergo fast metabolism or side reactions in vitro or in vivo. They are bio-orthogonal, e.g. they selectively react with each other in physiologic media. Thus, the compounds and the method of the invention can be used in a living organism. Moreover, the reactive groups are relatively small and can be introduced in biological samples or living organisms without significantly altering the size of biomolecules therein. References on the inverse electron demand Diels Alder reaction, and the behavior of the pair of reactive species include: [Thalhammer et al., Tetrahedron Lett., 1990, 31, 47, 6851-6854], [Wijnen et al., J. Org. Chem., 1996, 61, 2001-2005], [Blackman et al., J. Am. Chem. Soc., 2008, 130, 41, 13518-19], [Rossin et al., Angew. Chem. Int. Ed. 2010, 49, 3375], [Devaraj et al., Angew. Chem. Int. Ed. 2009, 48, 7013], [Devaraj et al., Angew. Chem. Int. Ed., 2009, 48, 1-5].

### The IEDDA Pyridazine Elimination Reaction

Below, the dienophile, a TCO, that may be comprised in kits of the invention may be referred to as a "Trigger" (T^{R}). The dienophile is connected at the allylic position to a Construct-A. Moreover, tetrazines that are used in the IEDDA pyridazine elimination reaction may be referred to as "Cleaving Agents". For ease of reference, the term Construct-A in this invention is used to indicate an Administration Agent or a Label, of which it is desired to have it first in a bound state, and being able to provoke release from that state.

Without wishing to be bound by theory, the inventors believe that the Cleaving Agent provokes Construct-A release via a cascade mechanism within the IEDDA adduct, i.e. the dihydropyridazine. The cascade mechanism can be a simple one step reaction, or it can be comprised in multiple steps that involves one or more intermediate structures. These intermediates may be stable for some time or may immediately degrade to the thermodynamic end-product or to the next intermediate structure. In any case, whether it be a simple or a multistep process, the result of the cascade mechanism is that the Construct-A gets released from the IEDDA adduct. Without wishing to be bound by theory, the design of the diene is such that the distribution of electrons within the IEDDA adduct is unfavorable, so that a rearrangement of these electrons must occur. This situation initiates the cascade mechanism, and it therefore induces the release of the Construct-A. Specifically, and without wishing to be bound by theory, the inventors believe that the NH moiety comprised in the various dihydropyridazine tautomers, such as the 1,4-dihydropyridazine tautomer, of the IEDDA adduct can initiate an electron cascade reaction, a concerted or consecutive shift of electrons over several bonds, leading to release of the Construct-A. Occurrence of the cascade reaction in and /or Construct-A release from the Trigger is not efficient or cannot take place prior to the IEDDA reaction, as the Trigger-Construct-A conjugate itself is relatively stable as such. The cascade can only take place after the Cleaving Agent and the Trigger-Construct conjugate have reacted and have been assembled in the IEDDA adduct.

With reference to Scheme 2 below, and without wishing to be bound by theory, the inventors believe that the pyridazine elimination occurs from the 1,4-dihydropyridazine tautomer 4. Upon formation of the 4,5-dihydropyridazine **3,** tautomerization affords intermediates **4** and **7,** of which the 2,5-dihydropyridazine **7** cannot eliminate the Construct-A (C^{A}). Instead it can slowly convert into aromatic **8,** which also cannot eliminate C^{A} or it can tautomerize back to intermediate **3.** Upon formation of **4** the C^{A} is eliminated near instantaenously, affording free C^{A} 8 as an amine, and pyridazine elimination products **5** and **6.** This elimination reaction has been shown to work equally well in the cleavage of carbamates, carbonates, esters and ethers from the TCO trigger [Versteegen et al., Angew. Chem. Int. Ed., 2018, 57, 10494]. The Trigger in Scheme 2 is also optionally bound to a Construct-B (C^{B}) , which in this case cannot release from the Trigger. Thereby Construct A can be seperated from Construct B by means of the IEDDA pyridazine elimination.

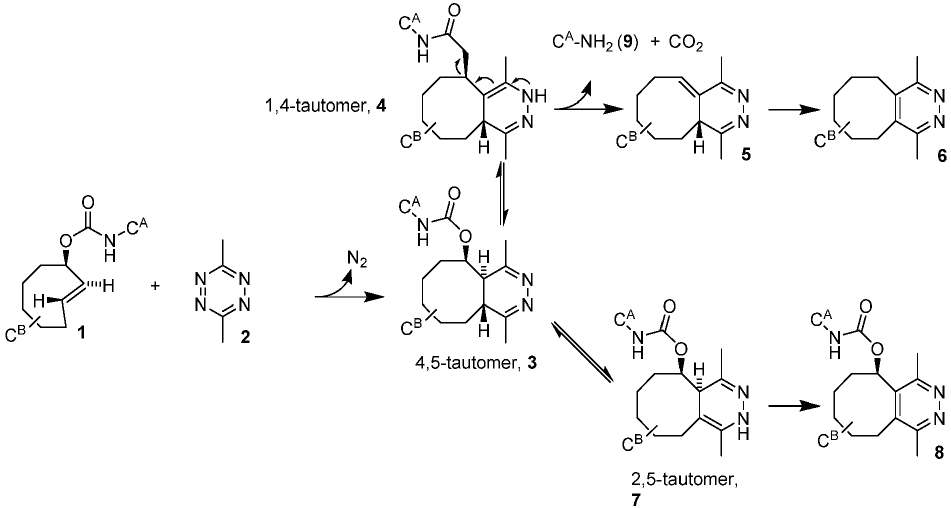

### Scheme 2. IEDDA pyridazine elimination mechanism.

In preferred embodiments, the dienophile trigger moiety used in the present invention comprises a *trans*-cyclooctene ring. Herein, this eight-membered ring moiety will be defined as a *trans*-cyclooctene moiety, for the sake of legibility, or abbreviated as "TCO" moiety. It will be understood that the essence resides in the possibility of the eight-membered ring to act as a dienophile and to be released from its conjugated Construct-A upon reaction.

The tetrazines of the kits of the invention and dienophiles are capable of reacting in an inverse electron-demand Diels-Alder reaction (IEDDA). IEDDA reaction of the Trigger with the Cleaving Agent leads to release of the Construct-A through an electron-cascade-based elimination, termed the "pyridazine elimination". When a Cleaving Agent reacts with a Trigger capable of eliminating Construct-A, the combined proces of reaction and Construct-A elimination is termed the "IEDDA pyridazine elimination".

This invention provides a Cleaving Agent that reacts with a Construct-A-conjugated Trigger, resulting in the cleavage of the Trigger from the Construct-A. In one prominent embodiment this results in the cleavage of Construct-A from Construct-B. In another embodiment the Trigger cleavage results in cleavage of one Construct A from another Construct A, as the dienophile Trigger of Formula (1) can comprise two allylic positioned Constructs-A, wherein one or both can release from the Trigger upon reaction with a diene. In another embodiment, Trigger cleavage results in the cleavage of one or more Construct-A from one or more Construct-B. Construct-B is the Construct, *i.e.* either Administration Agent or Label depending on the nature of Construct-A, that is bound to the dienophile, and cannot be released from the dienophile, unless it is bound to the allylic position via a spacer or self-imolative linker that also binds Construct-A . In preferred embodiments, the Trigger is used as a reversible covalent bond between two molecular species.

Scheme 3a below is a general scheme of Construct release according to a preferred embodiment of this invention, wherein the Construct being released is termed Construct-A (C^{A}), and wherein another Construct, Construct-B (C^{B}) is bound to the dienophile but not via the allylic position, wherein Construct-B cannot be released from the dienophile, and wherein either Construct A or B is the Administration Agent and the other is the Label.

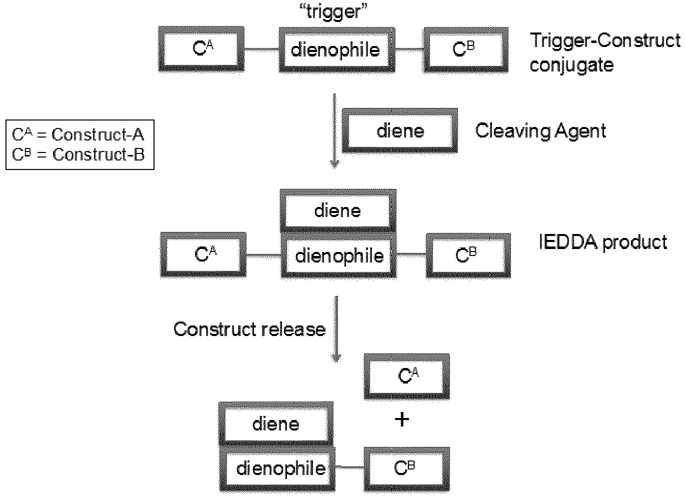

### Scheme 3a: General scheme of IEDDA pyridazine elimination reaction for the release of Construct-A from Construct-B according to a preferred embodiment of this invention

Scheme 3b below is a general scheme of Construct release according to another embodiment of this invention, wherein Construct-B (C^{B}) is bound to the dienophile via a spacer or self-imolative linker that also binds Construct-A and, wherein when the spacer or self-immolative linker is released from the allylic position then Construct-B and Construct A are released from the Trigger and from each other.

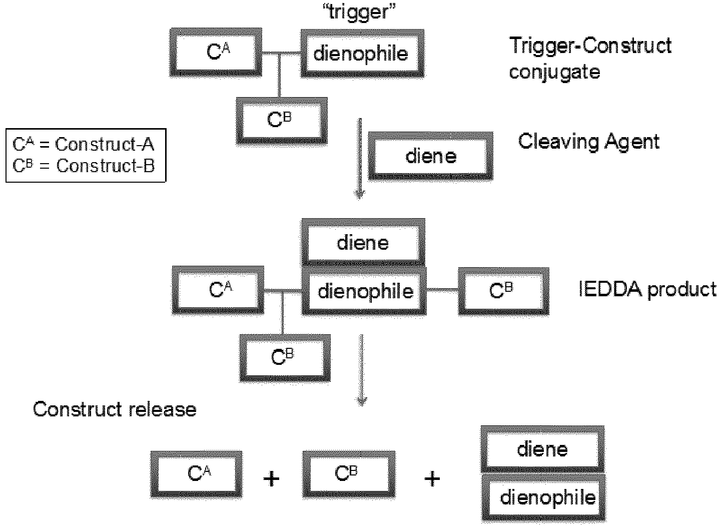

### Scheme 3b: General scheme of IEDDA pyridazine elimination reaction for the release of Construct-A from Construct-B according to a another embodiment of this invention

Scheme 3c below is a general scheme of Construct release according to another embodiment of this invention, wherein the Trigger is linked to two allylic positioned Construct-A's, and wherein one or both Constructs-A's can be released from the Trigger, in any case resulting in cleavage of one Construct-A from the other Construct-A, and and wherein one Construct-A is the Administration Agent and the other Construct-A is the Label.

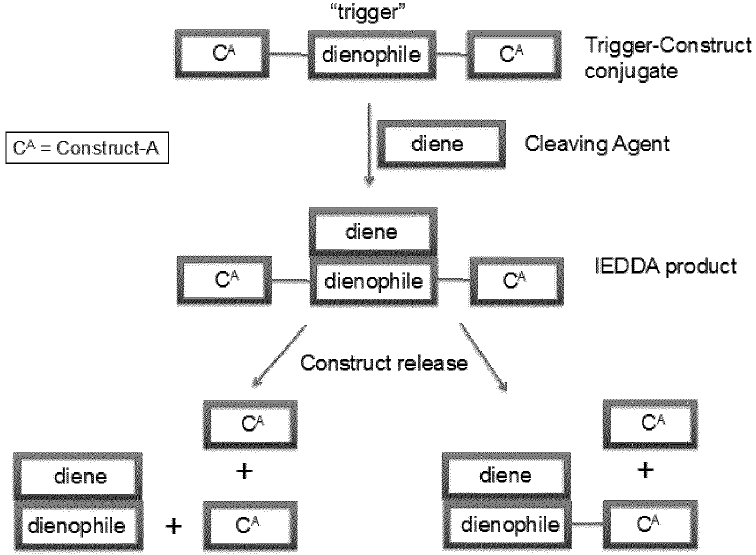

### Scheme 3c: General scheme of IEDDA pyridazine elimination reaction for the release of Construct-A from another Construct-A according to a preferred embodiment of this invention

The Construct release occurs through a powerful, abiotic, bio-orthogonal reaction of the dienenophile (Trigger) with the diene (Cleaving Agent), viz. the aforementioned IEDDA. The bound Construct is a Construct-dienenophile conjugate. Possibly the Construct-A is linked to one or more additional Constructs A linked via a spacer, for example a self-immolative linker. It will be understood that in Scheme 3a, 3b and 3c in the IEDDA adduct as well as in the end product after release, the indicated dienophile group and the indicated diene group are the residues of, respectively, the dienophile and diene groups after these groups have been converted in the IEDDA reaction.

The difference between C^{A} and C^{B} is that the bond between C^{B} and the moiety holding C^{B} is not broken upon reaction of the Trigger with the diene, whereas the bond between C^{A} and the moiety holding C^{A} is broken upon reaction of the Trigger with the diene. A person skilled in the art will understand that the moiety holding C^{A} and C^{B} refers to the Trigger, or a self immolative linker L^{C} bound to the Trigger. For the sake of clarity, when C^{B} is bound to a L^{C} that is bound to the Trigger, the L^{C} holding C^{B} will release from the Trigger upon reaction with the diene but the C^{B} will not release from the released L^{C}. Likewise if C^{B} is bound directly to the Trigger, C^{B} will not release from the Trigger upon. reaction with the diene. A person skilled in the art will understand that when it is required to seperate one Label from one Administration Agent, that preferably one of the following requirements is met:
1) one C^{A} comprises the Label and another C^{A} comprises the Adminstration Agent
2) either Label or Adminstration Agent is C^{A} and the other is C^{B}
3) Label and Adminstration Agent are both C^{B}, provided that one C^{B} is not part of R₄₈ and the other is bound to a L^{C} which is part of R₄₈, or provided that one C^{B} moiety is bound to a different L^{C} moiety than the other C^{B} moiety.

Other than is the case with e.g. medicinally active substances, where the *in vitro* or *in vivo* action is often changed with minor structural changes, the present invention first and foremost requires the right chemical reactivity combined with sufficient stability for the intended application. Thus, the possible structures extend to those of which the skilled person is familiar with that these are reactive as dienes or dienophiles.

### Targeting

A "primary target" as used in the present invention, both in relation to the Administration Agent and the Cleaving Agent, relates to a target to be detected in a diagnostic and/or imaging method, and/or to be modulated, bound, or otherwise addressed by a pharmaceutically active compound, or other therapeutic modality. The primary target can be selected from any suitable target within the human or animal body or on a pathogen or parasite. For example, a primary target can be any molecule or tissue, which is present in an organism, tissue or cell. Targets include cells components such as cell membranes and cell walls, cell surface targets, e.g. receptors, glycoproteins; structural proteins, e.g. amyloid plaques; extracellular targets such as stroma targets, tumor microenvironment targets, extracellular matrix targets such as growth factors, and proteases; intracellular targets, e.g. surfaces of Golgi bodies, surfaces of mitochondria, RNA, DNA, enzymes, components of cell signaling pathways; and/or foreign bodies, e.g. pathogens such as viruses, viral particles, bacteria, fungi, yeast or parts thereof. Examples of primary targets include compounds such as proteins of which the presence or expression level is correlated with a certain tissue or cell type or of which the expression level is up regulated or down-regulated in a certain disorder. According to a particular embodiment of the present invention, the primary target is a protein such as a (internalizing or non-internalizing) receptor. According to a preferred embodiment of the present invention, the primary target is an internalizing receptor. According to another preferred embodiment of the present invention, the primary target is a non-internalizing receptor.

In preferred embodiments the Primary Target can be a system in the body, such as blood circulation, lymphatic system, the nervous system, the digestion system, RES system, or organs such as the heart or kidney. For example, there are imaging agents that visualize blood flow, the liver, or identify the sentinel lymph node of a tumor.

Alternatively, the primary target may be a metabolic pathway, which is upregulated during a disease, e.g. infection or cancer, such as DNA synthesis, protein synthesis, membrane synthesis and carbohydrate uptake. In diseased tissues, above-mentioned markers can differ from healthy tissue and offer unique possibilities for early detection, specific diagnosis and therapy, especially targeted therapy.

Other non-limiting examples of Primary Targets include antibodies, proteins, carbohydrates, monosacharides, polysaccharides, cytokines, hormones, steroids, somatostatin receptor, monoamine oxidase, muscarinic receptors, myocardial sympatic nerve system, leukotriene receptors, e.g. on leukocytes, urokinase plasminogen activator receptor (uPAR), folate receptor, apoptosis marker, (anti-angiogenesis marker, gastrin receptor, dopaminergic system, serotonergic system, GABAergic system, adrenergic system, cholinergic system, opoid receptors, GPIIb/IIIa receptor and other thrombus related receptors, fibrin, calcitonin receptor, tuftsin receptor, integrin receptor, fibronectin, VEGF/EGF and VEGF/EGF receptors, TAG72, CEA, CD19, CD20,CD22, CD40, CD45, CD74, CD79, CD105, CD138, CD174, CD227, CD326, CD340, MUC1, MUC16, GPNMB, PSMA, Cripto, Tenascin C, Melanocortin-1 receptor, CD44v6, G250, HLA DR, ED-A, ED-B, TMEFF2 , EphB2, EphA2, FAP, Mesothelin, GD2, CAIX, 5T4, matrix metalloproteinase (MMP), P/E/L-selectin receptor, LDL receptor, P-glycoprotein, neurotensin receptors, neuropeptide receptors, substance P receptors, NK receptor, CCK receptors, sigma receptors, interleukin receptors, herpes simplex virus tyrosine kinase, human tyrosine kinase, MSR1, FAP, CXCR, tumor endothelial marker (TEM), cMET, IGFR, FGFR, GPA33, hCG, According to a further particular embodiment of the invention, the primary target and targeting agent are selected so as to result in the specific or increased targeting of a tissue or disease, such as cancer, an inflammation, an infection, a cardiovascular disease, e.g. thrombus, atherosclerotic lesion, hypoxic site, e.g. stroke, tumor, cardiovascular disorder, brain disorder, apoptosis, angiogenesis, an organ, and reporter gene/enzyme. This can be achieved by selecting primary targets with tissue-, cell- or disease- specific expression. For example, membrane folic acid receptors mediate intracellular accumulation of folate and its analogs, such as methotrexate. Expression is limited in normal tissues, but receptors are overexpressed in various tumor cell types.

In preferred embodiments the Primary Target equals a therapeutic target.

Preferred internalizing targets are:
Transferrin receptor, urokinase plasminogen activator receptor (uPAR), folate receptor, gastrin receptor, GPIIb/IIIa receptor, calcitonin receptor, tuftsin receptor, integrin receptor, VEGF/EGF receptors, CD19, CD20, CD22, CD25, CD30, CD33, CD40, CD45, CD56, CD70, CD74, CD79, CD105, CD123, CD138, CD163, CD174, CD184, CD227, CD269, CD326, CD340, CD352, MUC1, MUC16, GPNMB, PSMA, Cripto, Melanocortin-1 receptor, HLA DR, TMEFF2 , EphB2, EphA2, FAP, Mesothelin, GD2, CAIX, 5T4, matrix metalloproteinases (MMP), ADAM-9, P/E/L-selectin receptor, LDL receptor, P-glycoprotein, neurotensin receptors, neuropeptide receptors, substance P receptors, NK receptor, CCK receptors, CXCR, tumor endothelial marker (TEM), cMET, IGFR, FGFR, GPA33, hCG, platelet-derived growth factor (PDGF), HER2, HER3, CA19, TAM, LGALS3BP, nectin-4, IFGR, cMET, PD1, PDL1, GPNMB, AGS-5, AGS-16, endosialin, ETBR, TM4SF1, BCMA, GPC2, TROP-2, AXL, HLA-DR, B7-H3, MTX3, MTX5, EFNA4, NOTCH, tissue factor (TF), PDGFR, GITR, OX40, RIG, MDA-5, NLRP1, NLRP3, AIM2, IDO, MEK, cGAS, NKG2A.

Preferred non-internalizing targets are Fibronectin ED-A, Fibronectin ED-B, VEGF, EGF , TAG72, CEA, CD20, CD25, MUC1, MUC16, Tenascin C, CD44v6, CAIX, matrix metalloproteinase (e.g. MMP2), A33, mesothelin, LGALS3BP, hCG, nectin-4, CD45, G250, GPA33 , GD2, PD1, PDL1, GITR, CD3, CD28, CTLA4, OX40, RIG, MDA-5, NLRP1, NLRP3, AIM2, IDO, MEK, cGAS, NKG2A.

The person skilled in the art will understand that the preceding classification is relative, as most targets are not completely internalizing or non-internalizing, but instead can be classified as relatively internalizing or relatively non-internalizing, as compared to other targets, such as TAG72 (non-internalizing) and HER2 (fast internalizing). The person skilled in the art will understand that internalization rate is an important factor in this classification, and that the nature of the Administration Agent or Targeting Agent T^{T} can affect this rate.

### Compounds of Formula (1)

The invention pertains to compounds of Formula (1) as disclosed herein. It will be understood that the compounds of Formula (1) comprise an eight-membered trans-ring, preferably a trans-cyclooctene, formed by -CHR₄₈, -C=C-, and X¹-X⁵.

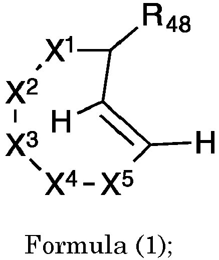

R₄₈ is selected from the group consisting of -L^{B}, and -L^{A}.

In a preferred embodiment, R₄₈ is -L^{L}.

In a preferred embodiment, R₄₈ is -L^{A}.

As is clear from the definitions of -L^{B} and -L^{A} herein, both -L^{B} and -L^{A} may comprise a moiety L^{C}, which is an optional self-immolative linker, which may consist of multiple units arranged linearly and/or branched and may release one or more moieties that are denoted as C^{A} moieties, while moieties that are not released from the L^{C} may be denoted as C^{B}. It will be understood that if L^{C} comprises more than one C^{A} moiety, these C^{A} moieties can independently be Label or Administration Agent, optionally linked to L^{C} via a spacer S^{P}, since both are released due to the self-immolative character of L^{C}. Preferably, if L^{C} comprises one C^{B}, then said C^{B} moiety may be either Administration Agent or Label. Preferably, if L^{C} comprises more than one C^{B}, then said C^{B} moieties are all either Administration Agent or Label. It will be understood that in such a way, one R₄₈ group may comprise both a Label and an Administration Agent, that are uncoupled after reaction of the compound of Formula (1) with a Cleaving Agent due to the self-immolative character of the linker L^{C}.

By way of further clarification, if R₄₈ is L^{B} and all d and e in Formula (2) are 0, the Label directly constitutes the leaving group of the release reaction, and if one of d or e in Formula (2) is 1, S^{L}, which may be a self-immolative linker L^{C}, constitutes the leaving group of the release reaction.

In Formula (1), R₄₈ is bound to the remainder of the compound of Formula (1) via a part of R₄₈ that is -O-, -S-, -OC(O)-, -OC(S)-, -SC(O)-, or -SC(S)-; preferably -O-, or -OC(O)-, most preferably -OC(O)N- (*i.e.* a carbamate). Preferably, the -O- or -S- is directly coupled to an aromatic moiety that is also part of R₄₈. Preferably, the -OC(O)-, -OC(S)-, -SC(O)-, or -SC(S)- are directly coupled to a carbon, oxygen, nitrogen, or sulphur atom that is also part of R₄₈, preferably to a secondary or tertiary amine. In a preferred embodiment, in Formula (1), R₄₈ is bound to the remainder of the compound of Formula (1) via a part of R₄₈ that is -O-R₉₇-, -S-R₉₇-, -OC(O)-R₉₇-, -OC(S)-R₉₇-, -SC(O)-R₉₇-, or -SC(S)-R₉₇-. In a preferred embodiment, in Formula (1), R₄₈ is bound to the remainder of the compound of Formula (1) via a part of R₄₈ that is -O-, -S-R₉₉-, - OC(O)-, -OC(O)-R₉₉-, -OC(S)-R₉₉-, -SC(O)-R₉₉-, or -SC(S)-R₉₉-.

It will be understood, that preferably this notation indicates that in e.g. -OC(O)-R₉₉-, the first O is attached to the eight-membered ring of Formula (1), and R₉₉ is connected to the remainder of R₄₈. It will be understood that -O- denotes an ether, and can be an aromatic ether or an aliphatic ether. Likewise, -OC(O)-denotes an ester, that can be an aromatic ester or an aliphatic ester, preferably an aromatic ester. It will be understood that the part of R₄₈ that is -O-, -OC(O)-, or -OC(S)-, could be part of a spacer S^{P} or S^{L}, if present, or of the Administration Agent or the Label, if no spacer S^{P} or S^{L} is present.

In Formula (1), when R₄₈ is L^{B} and does not comprise an Administration Agent, then R₄₈ being L^{B} preferably has a molecular weight of at most 50 kDa, and preferably of at most 5 kDa, even more preferably at most 2 kDa, even more preferably at most 1 kDa, even more preferably at most 500 Da.

In Formula (1), when R₄₈ is L^{A} and does not comprise a Label then the compound of Formula (1) without R₄₈ preferably has a molecular weight of at most 50 kDa, and more preferably of at most 5 kDa, even more preferably at most 2 kDa, even more preferably at most 1 kDa. Preferably, when Formula (1) comprises one R₄₈ moiety that is L^{A} and does not comprise a Label, then the compound of Formula (1) without R₄₈ being L^{A} has a molecular weight of at most 50 kDa, and more preferably of at most 5 kDa, even more preferably at most 2 kDa, even more preferably at most 1 kDa. Preferably, when Formula (1) comprises two R₄₈ moieties that are L^{A} and do not comprise a Label, then the compound of Formula (1) without both R₄₈ has a molecular weight of at most 50 kDa, and more preferably of at most 5 kDa, even more preferably at most 2 kDa, even more preferably at most 1 kDa.

The compound of Formula (1) comprises at least one Label and at least one Administration Agent. Preferably, the compound of Formula (1) comprises at most one Label and at most one Administration Agent.

Preferably, when at least one R₄₈ is L^{B}, then X¹, X², X³, and X⁴ do not comprise the same Label as comprised in R₄₈ being L^{B}. Preferably, when at least one R₄₈ is L^{A}, then X¹, X², X³, and X⁴ do not comprise an L^{A}.

In particularly favorable embodiments, one or both R₄₈ is/are in the axial position.

Preferably, if X⁵ is -C(R₄₇)₂-, R₄₈ comprises a Label, and R₄₈ does not comprise an Administration Agent, then X¹-X⁵ do not comprise the same Label as comprised in R₄₈.

Preferably, if X⁵ is -C(R₄₇)₂-, R₄₈ comprises an Administration Agent, and R₄₈ does not comprise a Label, then X¹-X⁵ do not comprise the same Administration Agent as comprised in R₄₈.

Preferably, if X⁵ is -C(R₄₇)₂-, and R₄₈ comprises at least one Administration Agent and at least one Label, then X¹-X⁵ optionally comprise either the same Administration Agent as comprised in R₄₈ or the same Label as comprised in R₄₈.

In Formula (1), L^{A} only comprises both the Label and the Administration Agent when L^{A} is R₄₈.

In Formula (1), if L^{A} being R₄₈ comprises both the Label and the Administration Agent, then the S^{P} linked to said Label and said Administration Agent is a self-immolative linker, preferably with the proviso that at least one of Label and Administration Agent is C^{A}in the definition of L^{C} or the proviso that the Label and Administration Agent are bound to different L^{C} moieties within the same R₄₈.

### Formula (2)

In Formula (1), the moiety L^{B} satisfies Formula (2):

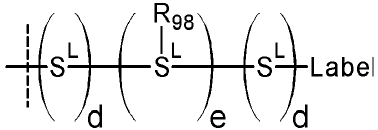

Formula (2); wherein the dashed line denotes a bond to the remainder of the compound of Formula (1).

In Formula (2) each d independently is 0 or 1. In a preferred embodiment, at least one d is 1. In another preferred embodiment, both d are 1. In yet another preferred embodiment, both d are 0.

In Formula (2) e is an integer in a range of from 0 to 4, preferably e is at most 3, more preferably at most 2, most preferably e is at most 1. In a preferred embodiment, e is 1. In another preferred embodiment, e is 0.

In Formula (2), the Label is preferably as defined herein. -L^{B} does not comprise an Administration Agent.

Preferably, Formula (2) is -S^{L}-Label, wherein S^{L} is a polyethyleneglycol (PEG), more preferably S^{L} is PEG₄.

### Linker S^{L}

In Formula (2), S^{L} is a linker, which may be a self-immolative linker L^{C} as defined herein.

Preferably, S^{L} is defined as S^{P} as defined herein, wherein it will be understood that when S^{L} is attached to an R₉₈, in some embodiments S^{L} is a trivalent radical, and the suffix -ene in a preferred definition of S^{P} is replaced with -triyl.

In preferred embodiments, S^{L} being S^{P} comprises at least one moiety selected from the group consisting of a residue of R₃₂, a moiety C^{M2}, and a moiety C^{X}; all as described herein. In preferred embodiments, said C^{M2}, C^{X} or a residue of R₃₂ connects the S^{P} to a Label, L^{C}, S^{P}, R₉₈, or T^{R}.

In a preferred embodiment, S^{L} is L^{C}. Preferably, when S^{L} is L^{C}, then e is 0, one d is 0, and one d is 1. In another preferred embodiment, S^{L} is not L^{C} when S^{L} is comprised in any one of X¹-X⁴. In another preferred embodiment, S^{L} is not L^{C}.

### R98

In Formula (2), each R₉₈ individually is a Label or a clearance-directing group. In a preferred embodiment, R₉₈ is a Label. The Label of R₉₈ is preferably as defined herein, and may be the same Label as the one comprised in L^{B} that is not R₉₈, or a different Label.

### R99

R₉₉ is selected from the group consisting of -O-, -C(R₃₆)₂-, -S-, and -NR₃₆-.

### Clearance-directing group

A clearance-directing group is a moiety that directs a compound to an excretory organ, such as the kidneys or the liver. In that way, it ensures faster clearance of a compound from the blood in a subject and / or it controls which excretory organ will or will not process the compound to improve imaging procedures or non-target tissue dosimetry. In relation to the invention, it will be understood that the increased clearance rate of the Label after being separated from the Administration Agent is mainly due to this separation, and that the clearance-directing group merely further enhances the clearance rate of the Label and / or controls the clearance pathway.

In a preferred embodiment, the clearance-directing group is hexose-based. Hexose-based clearance-directing groups incorporate one or more hexoses (six carbon sugar moieties) recognized by Ashwell receptors or other receptors such as the mannose/N-acetylglucosamine receptor which are associated with hepatocytes, endothelial cells and/or Kupffer cells of the liver or the mannose 6-phosphate receptor.

Exemplary hexoses are galactose, mannose, mannose 6-phosphate, N-acetylglucosamine, pentamannosylphosphate, and the like. Other moieties recognized by Ash well receptors, including glucose, N-galactosamine, N-acetylgalactosamine, pentamannosyl phosphate, thioglycosides of galactose and, generally, D-galactosides and glucosides or the like may also be used in the practice of the present invention.

In a preferred embodiment, the clearance-directing group is galactose.

Galactose thioglycoside conjugation is preferably accomplished following a procedure largely similar to the teachings of Lee et al., "2-Imino-2-methoxyethyl 1- 20 Thioglycosides: New Reagents for Attaching Sugars to Proteins," Biochemistry, (18):3956, 1976. Another useful galactose thioglycoside conjugation method is set forth in Drantz et al, "Attachment of Thioglycosides to Proteins: Enhancement of Liver Membrane Binding," Biochemistry, 15(18): 3963, 1976.

### Formula (3)

In Formula (1), L^{A} is a moiety satisfying Formula (3): 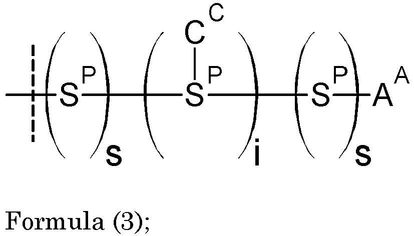 wherein the dashed line denotes a bond to the remainder of the compound of Formula (1).

In Formula (3) each s independently is 0 or 1. In a preferred embodiment, at least one s is 1. In another preferred embodiment, both s are 1. In yet another preferred embodiment, both s are 0.

In Formula (3) i is an integer in a range of from 0 to 4, preferably i is at most 3, more preferably at most 2, most preferably i is at most 1. In a preferred embodiment, i is 1. In another preferred embodiment, i is 0.

In Formula (3), A^{A} denotes an Administration Agent that is an antibody.

In a preferred embodiment, Formula (3) is -S^{P}-A^{A}, wherein S^{P} is a polyethylene glycol (PEG), more preferably S^{P} is PEG₈.

In a preferred embodiment, moiety L^{A} has a molecular weight of at most 500 kDa, more preferably at most 200 kDa, most preferably at most 160 kDa.

In a preferred embodiment, moiety L^{A} has a molecular weight of at least 0.1 kDa, more preferably at least 1 kDa, more preferably at least 10 kDa, most preferably at least 140 kDa.

In Formula (3), each S^{P} independently is a spacer, which optionally is a self-immolative linker L^{C} as defined herein. It will be understood that if S^{P} is linked to C^{C}, then S^{P} is preferably a trivalent radical and the suffix -ene in the definition of S^{L} is to be replaced with the suffix -triyl. Preferably, S^{P} is a spacer as defined herein. Preferably, when S^{P} is L^{C}, then i in Formula (3) is 0, one s is 0, and one s is 1.

In Formula (3) C^{C} denotes a Construct-C, wherein each Construct-C is independently selected from the group consisting of a Label, and an additional Administration Agent. The additional Administration Agent may be the same Administration Agent as comprised in L^{A} not being C^{C}, or it may be a different Administration Agent.

In a preferred embodiment, the compound of Formula (1) comprises at most one C^{C}. Preferably, when i in Formula (3) is at least 1, then in -S^{P}-C^{C}, the S^{P} is an L^{C}.

In preferred embodiments, S^{P} in Formula (3) comprises at least one moiety selected from the group consisting of a residue of R₃₂, a moiety C^{M2}, and a moiety C^{X}; all as described herein. In preferred embodiments, said C^{M2}, C^{X} or a residue of R₃₂ connects the S^{P} to a Label, an Administration Agent, L^{C}, S^{P}, C^{C}, or T^{R}. In a preferred embodiment, L^{A} does not comprise a Label. In a preferred embodiment, X⁵ does not comprise C^{C}, L^{A}, or L^{B}.

### Spacers S^{P}

It will be understood that when herein, it is stated that "each individual S^{P} is linked at all ends to the remainder of the structure" this refers to the fact that the spacer S^{P} connects multiple moieties within a structure, and therefore the spacer has multiple ends by defintion. The spacer S^{P} may be linked to each individual moiety via different or identical moieties that may be each individually selected. Typically, these linking moieties are to be seen to be part of spacer S^{P} itself. In case the spacer S^{P} links two moieties within a structure, "all ends" should be interpreted as "both ends". As an example, if the spacer connects a trans-cyclooctene moiety to a Construct A, then "the remainder of the molecule" refers to the trans-cylooctene moiety and Construct A, while the connecting moieties between the spacer and the trans-cyclooctene moiety and Construct A (i.e. at both ends) may be individually selected.

Spacers S^{P} may consist of one or multiple Spacer Units S^{U} arranged linearly and/or branched and may be connected to one or more C^{A} or C^{B} moieties and / or one or more L^{C} or T^{R} moieties. The Spacer may be used to connect C^{B} to one T^{R} (Example A) or more T^{R} (Example B and C), but it can also be used to modulate the properties, e.g. pharmacokinetic properties, of the C^{B}-T^{R}-C^{A} conjugate (Example D). Thus a Spacer does not necessarily connect two entities together, it may also be bound to only one component, e.g. the T^{R} or L^{C}. Alternatively, the Spacer may comprise a Spacer Unit linking C^{A} or C^{B} to T^{R} and in addition may comprise another Spacer Unit that is only bound to the Spacer and serves to modulate the properties of the conjugate (Example F). The Spacer may also consist of two different types of S^{U} constructs, e.g. a PEG linked to a peptide, or a PEG linked to an alkylene moiety (Example E). For the sake of clarity, Example B depicts a S^{U} that is branched by using a multivalent branched S^{U}. Example C depicts a S^{U} that is branched by using a linear S^{U} polymer, such as a peptide, whose side chain residues serve as conjugation groups.

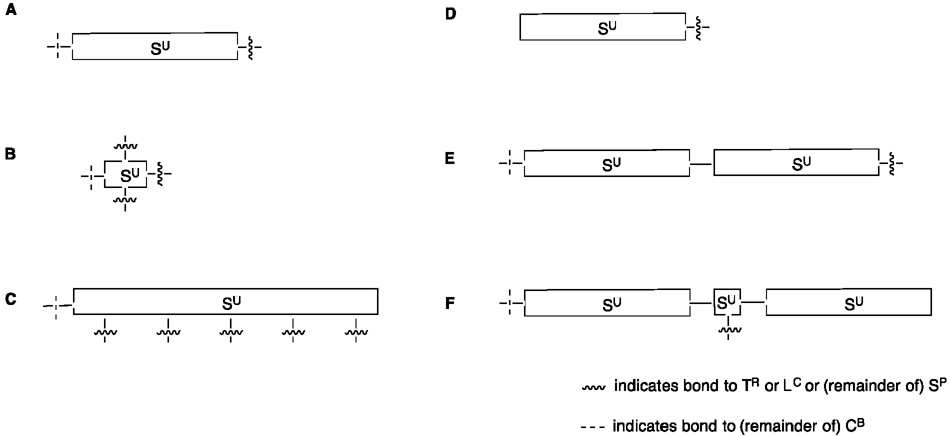

The Spacer may be bound to the Cleaving Agent in similar designs such as depicted in above examples A- F.

The Spacer Units include but are not limited to amino acids, nucleosides, nucleotides, and biopolymer fragments, such as oligo- or polypeptides, oligo- or polypeptoids, or oligo- or polylactides, or oligo- or poly-carbohydrates, varying from 2 to 200, particularly 2 to 113, preferably 2 to 50, more preferably 2 to 24 and more preferably 2 to 12 repeating units. Exemplary preferred biopolymer S^{U} are peptides.

Yet other examples are alkyl, alkylene, alkenyl, alkenylene, alkynyl, alkynylene, cycloalkyl, cycloalkylene, cycloalkenyl, cycloalkenylene, cycloalkynyl, cycloalkynylene, aryl, arylene, alkylaryl, alkylarylene, arylalkyl, arylalkylene, arylalkenyl, arylalkenylene, arylalkynyl, arylalkynylene , polyethyleneamino, polyamine, which may be substituted or unsubstituted, linear or branched, may contain further cyclic moieties and / or heteroatoms, preferably O, N, and S, more preferably O; wherein in some embodiments these example S^{U} comprise at most 50 carbon atoms, more preferably at most 25 carbon atoms, more preferably at most 10 carbon atoms. In preferred embodiments the S^{U} is independently selected from the group consisting of (CH₂)ᵣ, (C₃-C₈ carbocyclo), O-(CH₂)ᵣ, arylene, (CH₂)ᵣ-arylene, arylene-(CH₂)ᵣ, (CH₂)ᵣ -(C₃-C₈ carbocyclo), (C₃-C₈ carbocyclo)-(CH₂)ᵣ, (C₃-C₈ heterocyclo, (CH₂)ᵣ -(C₃-C₈ heterocyclo), (C₃-C₈ heterocyclo)-(CH₂)ᵣ, - (CH₂)ᵣC(O)NR₄(CH₂)ᵣ, (CH₂CH₂O)ᵣ, (CH₂CH₂O)ᵣCH₂,(CH₂)ᵣC(O)NR₄(CH₂ CH₂O)ᵣ, (CH₂)ᵣC(O)NR₄(CH₂CH₂O)ᵣCH₂, (CH₂CH₂O)ᵣ C(O)NR₄(CH₂CH₂O)ᵣ, (CH₂CH₂O)ᵣ C(O)NR₄(CH₂CH₂O)ᵣCH₂, (CH₂CH₂O)ᵣC(O)NR₄CH₂, -(CH₂)ᵣC(O)NR₃₇(CH₂)ᵣ, (CH₂CH₂O)ᵣ, (CH₂CH₂O)ᵣCH₂,(CH₂)ᵣC(O)NR₃₇(CH₂ CH₂O)ᵣ, (CH₂)ᵣC(O)NR₃₇(CH₂CH₂O)ᵣCH₂, (CH₂CH₂O)ᵣ C(O)NR₃₇(CH₂CH₂O)ᵣ , (CH₂CH₂O)ᵣ C(O)NR₃₇(CH₂CH₂O)ᵣCH₂, (CH₂CH₂O)ᵣC(O)NR₃₇CH₂; wherein r is independently an integer from 1 -10, R₄ is as defined herein, and R₃₇ is as defined herein.

Other examples of Spacer Units S^{U} are linear or branched polyalkylene glycols such as polyethylene glycol (PEG) or polypropylene glycol (PPG) chains varying from 2 to 200, particularly 2 to 113, preferably 2 to 50, more preferably 2 to 24 and more preferably 2 to 12 repeating units. It is preferred that when polyalkylene glycols such as PEG and PPG polymers are only bound via one end of the polymer chain, that the other end is terminated with -OCH₃, -OCH₂CH₃, - OCH₂CH₂CO₂H.

Other polymeric Spacer Units are polymers and copolymers such as poly-(2-oxazoline), poly(N-(2-hydroxypropyl)methacrylamide) (HPMA), polylactic acid (PLA), polylactic-glycolic acid (PLGA), polyglutamic acid (PG), dextran, polyvinylpyrrolidone (PVP), poly(1-hydroxymethylethylene hydroxymethyl-formal (PHF). Other exemplary polymers are polysaccharides, glycopolysaccharides, glycolipids, polyglycoside, polyacetals, polyketals, polyamides, polyethers, polyesters. Examples of naturally occurring polysaccharides that can be used as S^{U} are cellulose, amylose, dextran, dextrin, levan, fucoidan, carraginan, inulin, pectin, amylopectin, glycogen, lixenan, agarose, hyaluronan, chondroitinsulfate, dermatansulfate, keratansulfate, alginic acid and heparin. In yet other exemplary embodiments, the polymeric S^{U} comprises a copolymer of a polyacetal/polyketal and a hydrophilic polymer selected from the group consisting of polyacrylates, polyvinyl polymers, polyesters, polyorthoesters, polyamides, oligopeptides, polypeptides and derivatives thereof. Exemplary preferred polymeric S^{U} are PEG, HPMA, PLA, PLGA, PVP, PHF, dextran, oligopeptides, and polypeptides.

In some aspects of the invention polymers used in a S^{U} have a molecular weight ranging from 2 to 200 kDa, from 2 to 100 kDa, from 2 to 80 kDa, from 2 to 60 kDa, from 2 to 40 kDa, from 2 to 20 kDa, from 3 to 15 kDa, from 5 to 10 kDa, from 500 dalton to 5 kDa.

Other exemplary S^{U} are dendrimers, such as poly(propylene imine) (PPI) dendrimers, PAMAM dendrimers, and glycol based dendrimers.

The S^{U} of the invention expressly include but are not limited to conjugates prepared with commercially available cross-linker reagents such as BMPEO, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, sulfo-SMPB, and SVSB, DTME, BMB, BMDB, BMH, BMOE, BM(PEO)₃ and BM(PEO)₄.

To construct a branching Spacer one may use a S^{U} based on one or several natural or non-natural amino acids, amino alcohol, aminoaldehyde, or polyamine residues or combinations thereof that collectively provide the required functionality for branching. For example serine has three functional groups, i.e. acid, amino and hydroxyl groups and may be viewed as a combined amino acid an aminoalcohol residue for purpose of acting as a branching S^{U}. Other exemplary amino acids are lysine and tyrosine.

In preferred embodiments, the Spacer consist of one Spacer Unit, therefore in those cases S^{P} equals S^{U}. In preferred embodiments the Spacer consist of two, three or four Spacer Units.

In some aspects of the invention S^{P} has a molecular weight ranging from 2 to 200 kDa, from 2 to 100 kDa, from 2 to 80 kDa, from 2 to 60 kDa, from 2 to 40 kDa, from 2 to 20 kDa, from 3 to 15 kDa, from 5 to 10 kDa, from 500 dalton to 5 kDa. In some aspects of the invention, the S^{P} has a mass of no more than 5000 daltons, no more than 4000 daltons, no more than 3000 daltons, no more than 2000 daltons, no more than 1000 daltons, no more than 800 daltons, no more than 500 daltons, no more than 300 daltons, no more than 200 daltons. In some aspects the S^{P} has a mass from 100 daltons, from 200 daltons, from 300 daltons to 5000 daltons. In some aspects of the S^{P} has a mass from 30, 50, or 100 daltons to 1000 daltons, from 30, 50, or 100 daltons to 500 daltons.

Preferably, each S^{P} is independently selected from the group consisting of R₉₇, -O-, -OC(O)-, -OC(O)-R₉₉-, -OC(S)-R₉₉-, -OR₉₇-, -OC(O)-R₉₇-, -OC(O)-R₉₉-R₉₇-, -OC(S)-R₉₉-R₉₇-, and L^{C}. In another preferred embodiment, S^{P} is not L^{C} when S^{P} is comprised in any one of X¹-X⁴. In a preferred embodiment, S^{P} is not L^{C}.

In preferred embodiments, S^{P} comprises a residue of R₃₂, a moiety C^{M2} or a moiety C^{X}; all as described herein. In preferred embodiments, said C^{M2}, C^{X} or a residue of R₃₂ connects the S^{P} to a Label, an Administration Agent, L^{C}, S^{P}, C^{C}, R₉₈, or T^{R}.

### R₉₇

In a preferred embodiment, each R₉₇ is independently selected from the group consisting of C₁-C₂₄ alkylene groups, C₂-C₂₄ alkenylene groups, C₂-C₂₄ alkynylene groups, C₆-C₂₄ arylene, C₂-C₂₄ heteroarylene, C₃-C₂₄ cycloalkylene groups, C₅-C₂₄ cycloalkenylene groups, and C₁₂-C₂₄ cycloalkynylene groups, which are optionally further substituted with one or more substituents selected from the group consisting of -Cl, -F, -Br, -I, -OH, -NH₂, -SO₃H, -PO₃H, -PO₄H₂, -NO₂, - CF₃, =O, =NR₃₆, -SR₃₆, C₁-C₂₄ alkyl groups, C₂-C₂₄ alkenyl groups, C₂-C₂₄ alkynyl groups, C₆-C₂₄ aryl groups, C₂-C₂₄ heteroaryl groups, C₃-C₂₄ cycloalkyl groups, C₅-C₂₄ cycloalkenyl groups, C₁₂-C₂₄ cycloalkynyl groups, C₃-C₂₄ alkyl(hetero)aryl groups, C₃-C₂₄ (hetero)arylalkyl groups, C₄-C₂₄ (hetero)arylalkenyl groups, C₄-C₂₄ (hetero)arylalkynyl groups, C₄-C₂₄ alkenyl(hetero)aryl groups, C₄-C₂₄ alkynyl(hetero)aryl groups, C₄-C₂₄ alkylcycloalkyl groups, C₆-C₂₄ alkylcycloalkenyl groups, C₁₃-C₂₄ alkylcycloalkynyl groups, C₄-C₂₄ cycloalkylalkyl groups, C₆-C₂₄ cycloalkenylalkyl groups, C₁₃-C₂₄ cycloalkynylalkyl groups, C₅-C₂₄ alkenylcycloalkyl groups, C₇-C₂₄ alkenylcycloalkenyl groups, C₁₄-C₂₄ alkenylcycloalkynyl groups, C₅-C₂₄ cycloalkylalkenyl groups, C₇-C₂₄ cycloalkenylalkenyl groups, C₁₄-C₂₄ cycloalkynylalkenyl groups, C₅-C₂₄ alkynylcycloalkyl groups, C₇-C₂₄ alkynylcycloalkenyl groups, C₁₄-C₂₄ alkynylcycloalkynyl groups, C₅-C₂₄ cycloalkylalkynyl groups, C₇-C₂₄ cycloalkenylalkynyl groups, C₁₄-C₂₄ cycloalkynylalkynyl groups, C₅-C₂₄ cycloalkyl(hetero)aryl groups, C₇-C₂₄ cycloalkenyl(hetero)aryl groups, C₁₄-C₂₄ cycloalkynyl(hetero)aryl groups, C₅-C₂₄ (hetero)arylcycloalkyl groups, C₇-C₂₄ (hetero)arylcycloalkenyl groups, and C₁₄-C₂₄ (hetero)arylcycloalkynyl groups, wherein the substituents optionally contain one or more heteroatoms selected from the group consisting of O, S, NR₃₆, P, and Si, wherein the N, S, and P atoms are optionally oxidized, wherein the N atoms are optionally quaternized; and wherein preferably the alkylene groups, alkenylene groups, alkynylene groups, cycloalkylene groups, cycloalkenylene groups, and cycloalkynylene groups optionally contain one or more heteroatoms selected from the group consisting of O, S, NR₃₆, P, and Si, wherein the N, S, and P atoms are optionally oxidized, wherein the N atoms are optionally quaternized.

In a preferred embodiment, each R₉₇ is independently selected from the group consisting of C₁-C₁₂ alkylene groups, C₂-C₁₂ alkenylene groups, C₂-C₁₂ alkynylene groups, C₆-C₁₂ arylene, C₂-C₁₂ heteroarylene, C₃-C₁₂ cycloalkylene groups, C₅-C₁₂ cycloalkenylene groups, and C₁₂ cycloalkynylene groups; and wherein preferably the alkylene groups, alkenylene groups, alkynylene groups, cycloalkylene groups, cycloalkenylene groups, and cycloalkynylene groups optionally contain one or more heteroatoms selected from the group consisting of O, S, NR₃₆, P, and Si, wherein the N, S, and P atoms are optionally oxidized, wherein the N atoms are optionally quaternized.

In a preferred embodiment, each R₉₇ is independently selected from the group consisting of C₁-C₆ alkylene groups, C₂-C₆ alkenylene groups, C₂-C₆ alkynylene groups, C₆-C₆ arylene, C₂-C₆ heteroarylene, C₃-C₆ cycloalkylene groups, and C₅-C₆ cycloalkenylene groups; and wherein preferably the alkylene groups, alkenylene groups, alkynylene groups, cycloalkylene groups, cycloalkenylene groups, and cycloalkynylene groups optionally contain one or more heteroatoms selected from the group consisting of O, S, NR₃₆, P, and Si, wherein the N, S, and P atoms are optionally oxidized, wherein the N atoms are optionally quaternized.

In a preferred embodiment, the R₉₇ groups are optionally further substituted with one or more substituents selected from the group consisting of - Cl, -F, -Br, -I, -OH, -NH₂, -SO₃H, -PO₃H, -PO₄H₂, -NO₂, -CF₃, =O, =NR₃₆, -SR₃₆, C₁-C₁₂ alkyl groups, C₂-C₁₂ alkenyl groups, C₂-C₁₂ alkynyl groups, C₆-C₁₂ aryl groups, C₂-C₁₂ heteroaryl groups, C₃-C₁₂ cycloalkyl groups, C₅-C₁₂ cycloalkenyl groups, C₁₂ cycloalkynyl groups, C₃-C₁₂ alkyl(hetero)aryl groups, C₃-C₁₂ (hetero)arylalkyl groups, C₄-C₁₂ (hetero)arylalkenyl groups, C₄-C₁₂ (hetero)arylalkynyl groups, C₄-C₁₂ alkenyl(hetero)aryl groups, C₄-C₁₂ alkynyl(hetero)aryl groups, C₄-C₁₂ alkylcycloalkyl groups, C₆-C₁₂ alkylcycloalkenyl groups, C₁₃-C₁₈ alkylcycloalkynyl groups, C₄-C₁₂ cycloalkylalkyl groups, C₆-C₁₂ cycloalkenylalkyl groups, C₁₃-C₁₈ cycloalkynylalkyl groups, C₅-C₁₂ alkenylcycloalkyl groups, C₇-C₁₂ alkenylcycloalkenyl groups, C₁₄-C₁₆ alkenylcycloalkynyl groups, C₅-C₁₂ cycloalkylalkenyl groups, C₇-C₁₂ cycloalkenylalkenyl groups, C₁₄-C₁₆ cycloalkynylalkenyl groups, C₅-C₁₂ alkynylcycloalkyl groups, C₇-C₁₂ alkynylcycloalkenyl groups, C₁₄-C₁₆ alkynylcycloalkynyl groups, C₅-C₁₂ cycloalkylalkynyl groups, C₇-C₁₂ cycloalkenylalkynyl groups, C₁₄-C₁₆ cycloalkynylalkynyl groups, C₅-C₁₂ cycloalkyl(hetero)aryl groups, C₇-C₁₂ cycloalkenyl(hetero)aryl groups, C₁₄-C₁₆ cycloalkynyl(hetero)aryl groups, C₅-C₁₂ (hetero)arylcycloalkyl groups, C₇-C₁₂ (hetero)arylcycloalkenyl groups, and C₁₄-C₁₆ (hetero)arylcycloalkynyl groups, wherein the substituents optionally contain one or more heteroatoms selected from the group consisting of O, S, NR₃₆, P, and Si, wherein the N, S, and P atoms are optionally oxidized, wherein the N atoms are optionally quaternized.

In preferred embodiments, the R₉₇ groups are optionally further substituted with one or more substituents selected from the group consisting of - Cl, -F, -Br, -I, -OH, -NH₂, -SO₃H, -PO₃H, -PO₄H₂, -NO₂, -CF₃, =O, =NR₃₆, -SR₃₆, C₁-C₆ alkyl groups, C₂-C₆ alkenyl groups, C₂-C₆ alkynyl groups, C₆ aryl groups, C₂-C₆ heteroaryl groups, C₃-C₆ cycloalkyl groups, C₅-C₆ cycloalkenyl groups, C₃-C₆ alkyl(hetero)aryl groups, C₃-C₆ (hetero)arylalkyl groups, C₄-C₆ (hetero)arylalkenyl groups, C₄-C₆ (hetero)arylalkynyl groups, C₄-C₆ alkenyl(hetero)aryl groups, C₄-C₆ alkynyl(hetero)aryl groups, C₄-C₆ alkylcycloalkyl groups, C₆ alkylcycloalkenyl groups, C₄-C₆ cycloalkylalkyl groups, C₆ cycloalkenylalkyl groups, C₅-C₆ alkenylcycloalkyl groups, C₇ alkenylcycloalkenyl groups, C₅-C₆ cycloalkylalkenyl groups, C₇ cycloalkenylalkenyl groups, C₅-C₆ alkynylcycloalkyl groups, C₇ alkynylcycloalkenyl groups, C₅-C₆ cycloalkylalkynyl groups, C₅-C₆ cycloalkyl(hetero)aryl groups, and C₅-C₆ (hetero)arylcycloalkyl groups, wherein the substituents optionally contain one or more heteroatoms selected from the group consisting of O, S, NR₃₆, P, and Si, wherein the N, S, and P atoms are optionally oxidized, wherein the N atoms are optionally quaternized.

In preferred embodiments, the R₉₇ groups are optionally further substituted with one or more substituents selected from the group consisting of - Cl, -F, -Br, -I, -OH, -NH₂, -SO₃H, -PO₃H, -PO₄H₂, -NO₂, -CF₃, =O, =NR₃₆, -SR₃₆, C₁-C₆ alkyl groups, C₂-C₆ alkenyl groups, C₂-C₆ alkynyl groups, C₆ aryl groups, C₂-C₆ heteroaryl groups, C₃-C₆ cycloalkyl groups, C₅-C₆ cycloalkenyl groups, C₃-C₇ alkyl(hetero)aryl groups, C₃-C₇ (hetero)arylalkyl groups, C₄-C₈ (hetero)arylalkenyl groups, C₄-C₈ (hetero)arylalkynyl groups, C₄-C₈ alkenyl(hetero)aryl groups, C₄-C₈ alkynyl(hetero)aryl groups, C₄-C₆ alkylcycloalkyl groups, C₆₋C₇ alkylcycloalkenyl groups, C₄-C₆ cycloalkylalkyl groups, C₆₋C₇ cycloalkenylalkyl groups, C₅-C₆ alkenylcycloalkyl groups, C₇₋C₈ alkenylcycloalkenyl groups, C₅-C₆ cycloalkylalkenyl groups, C₇₋C₈ cycloalkenylalkenyl groups, C₅-C₆ alkynylcycloalkyl groups, C₇₋C₈ alkynylcycloalkenyl groups, C₅-C₆ cycloalkylalkynyl groups, C₅-C₉ cycloalkyl(hetero)aryl groups, and C₅-C₉ (hetero)arylcycloalkyl groups, wherein the substituents optionally contain one or more heteroatoms selected from the group consisting of O, S, NR₃₆, P, and Si, wherein the N, S, and P atoms are optionally oxidized, wherein the N atoms are optionally quaternized.

In a preferred embodiment, the R₉₇ groups are not substituted. In a preferred embodiment, the R₉₇ groups do not contain a heteroatom.

### Linker L^{C}

L^{C} is an optional self-immolative linker, which may consist of multiple units arranged linearly and/or branched and may release one or more C^{A} moieties.

It will be understood that if L^{C} comprises more than one C^{A} moiety, these C^{A} moieties can independently be Label or Administration Agent, optionally linked to L^{C} via a spacer S^{P}, since both are released due to the self-immolative character of L^{C}. Preferably, if L^{C} comprises more than one C^{B}, then said C^{B} moieties are all either Administration Agent or Label.

By way of further clarification, if R₄₈ is L^{B} and all d and e in Formula (2) are 0, the Label directly constitutes the leaving group of the release reaction, and if one of d or e in Formula (2) is 1, S^{L}, which may be a self-immolative linker L^{C}, constitutes the leaving group of the release reaction. The possible L^{C} structures, their use, position and ways of attachment of linkers L^{C} , constructs C^{A} and C^{B} , and the T^{R} are known to the skilled person, see for example [Papot et al., Anticancer Agents Med. Chem., 2008, 8, 618-637]. Nevertheless, typical but nonlimiting examples of self-immolative linkers L^{C} are benzyl-derivatives, such as those drawn below. There are two main self-immolation mechanisms: electron cascade elimination and cyclization-mediated elimination. The preferred example below on the left functions by means of the cascade mechanism, wherein the bond to the Y^{C} between Trigger and L^{C}, here termed Y^{C1}, is cleaved, and an electron pair of Y^{C1}, for example an electron pair of NR⁶, shifts into the benzyl moiety resulting in an electron cascade and the formation of 4-hydroxybenzyl alcohol, CO₂ and the liberated C^{A} also comprising an Y^{C}, here termed Y^{C2}. The preferred example in the middle functions by means of the cyclization mechanism, wherein cleavage of the bond to the amine of Y^{C1} leads to nucleophilic attack of the amine on the carbonyl, forming a 5-ring 1,3-dimethylimidazolidin-2-one and liberating the C^{A} including Y^{C2}. The preferred example on the right combines both mechanisms, this linker will degrade not only into CO₂ and one unit of 4-hydroxybenzyl alcohol (when Y^{C1} is O), but also into one 1,3-dimethylimidazolidin-2-one unit.

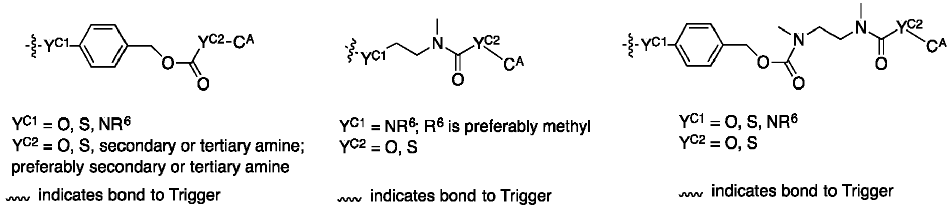

Herein, C^{A} is optionally linked to the remainder of L^{C} via a spacer S^{P}.

By substituting the benzyl groups of aforementioned self-immolative linkers L^{C}, it is possible to tune the rate of release of the construct C^{A}, caused by either steric and/or electronic effects on the cyclization and/or cascade release. Synthetic procedures to prepare such substituted benzyl-derivatives are known to the skilled person (see for example [Greenwald et al, J. Med. Chem., 1999, 42, 3657-3667] and [Thornthwaite et al, Polym. Chem., 2011, 2, 773-790]. Preferred substituted benzyl-derivatives with different release rates are drawn below.

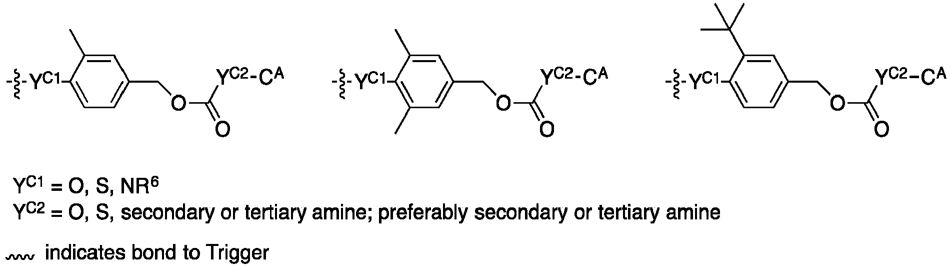

Herein, C^{A} is optionally linked to the remainder of L^{C} via a spacer S^{P}.

In preferred embodiments the L^{C} satisfies one of the following Formulae 23a-c 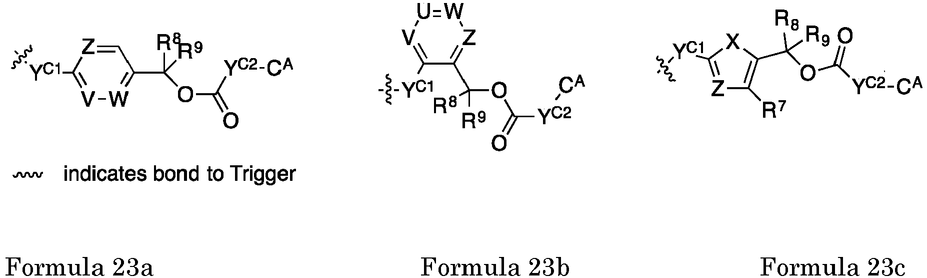 wherein Y^{C1} is O, S or NR⁶; V, U, W, Z are each independently CR⁷ or N; Y^{C2} is O, S, secondary amine or tertiary amine, wherein these Y^{C2} moieties are part of C^{A}; with R⁶, R⁷, R⁸, R⁹ as defined herein, and wherein, C^{A} is optionally linked to the remainder of L^{C} via a spacer S^{P}.

In preferred embodiments it is preferred that R⁶ is H or methyl, R⁷ is H, R⁸ is H or methyl and R⁹ is H. In preferred embodiments the R⁷ comprised in Formula 23c is CF₃ and Z is N.

In preferred embodiments the L^{C} satisfies the following Formula 23d 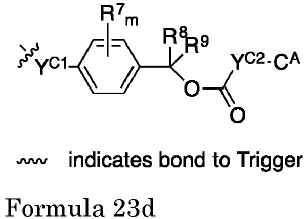 wherein Y^{C1} is O, S or NR⁶; Y^{C2} is O, S, secondary amine or tertiary amine, wherein these Y^{C2} moieties are part of C^{A}; with R⁶, R⁷, R⁸, R⁹ as defined herein; preferably R⁷ is C₁-C₈ alkyl, C₆-C₁₂ aryl, C₁-C₈ O-alkyl, C₆-C₁₂ O-aryl , NO₂, F, Cl, Br, I, CN, with m being an integer from 0 to 4; preferably each R⁸ and R⁹ are independently H, C₁-C₈ alkyl, C₆-C₁₂ aryl, C₁-C₈ O-alkyl, C₆-C₁₂ O-aryl , NO₂, F, Cl, Br, I, CN; and wherein, C^{A} is optionally linked to the remainder of L^{C} via a spacer S^{P}. Preferably R⁷ is electron donating and preferably m is an integer between 0 and 2, more preferably m is 0. Preferably R⁸ is H and R⁹ is H or methyl.

Self-immolative linkers that undergo cyclization include but are not limited to substituted and unsubstituted aminobutyric acid amide, appropriately substituted bicyclo[2.2.1] and bicyclo[2.2.2] ring system, 2-aminophenylpropionic acid amides, and trimethyl lock-based linkers, see e.g. [Chem. Biol. 1995, 2, 223], [J. Am. Chem. Soc. 1972, 94, 5815], [J. Org. Chem. 1990, 55, 5867]. Preferably, with an L^{C} that releases C^{A} by means of cyclization, the remainder of C^{A} is bound to L^{C} via Y^{C1} being an aromatic oxygen of sulfur. It will be understood that e.g. aromatic oxygen means an oxygen that is directly attached to an aromatic group.

In preferred embodiments such cyclization L^{C} satisfies one of the following Formulae 24a-f.

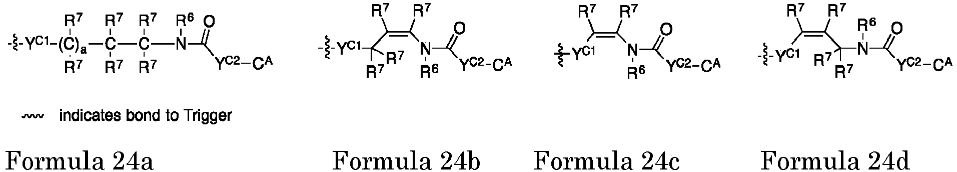

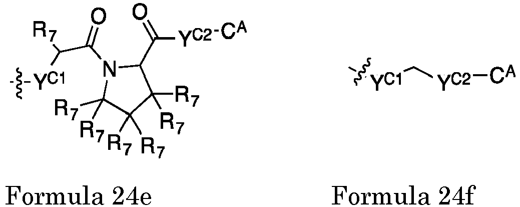

Wherein Y^{C1} is NR⁶; Y^{C2} is O or S, wherein these Y^{C2} moieties are part of C^{A}; a is independently 0 or 1; R⁶ and R⁷ are as defined herein, and wherein, C^{A} is optionally linked to the remainder of L^{C} via a spacer S^{P}. Preferably R⁶ and R⁷ are H, unsubstituted C₁-C₈ alkyl, C₆ aryl, more preferably R⁶ is H or methyl and R⁷ is H.

In a preferred embodiment, the remainder of C^{A} in Formula 24a, 24b, 24c, 24d and 24e is bound to Y^{C2} via an aromatic moiety, said moiety being a part of C^{A}. In a preferred embodiment, the remainder of C^{A} in Formula 24f is bound to Y^{C2} via an aliphatic moiety, said moiety being a part of C^{A}.

Several preferred structures of L^{C} are shown below. In these examples C^{A} is preferably bound to L^{C} via an Y^{C2} that is O or S, wherein O or S is part of C^{A}. For the avoidance of doubt, in these examples Y^{C1} is not denoted as such but is embodied by the relevant NH, NR⁶, S, O groups.

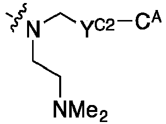

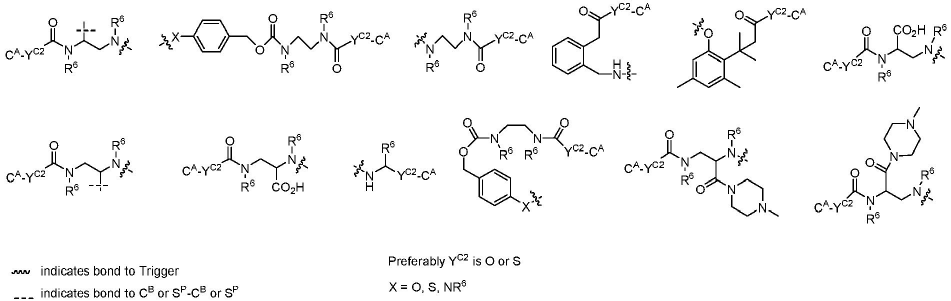

Herein, C^{A} is optionally linked to the remainder of L^{C} via a spacer S^{P}; and R⁶ is as defined herein

Several other preferred structures of L^{C} are shown below. In these examples C^{A} is preferably bound to L^{C} via an Y^{C2} that is a secondary or primary amine, and wherein said Y^{C2} is part of C^{A}. For the avoidance of doubt, in these examples Y^{C1} is not denoted as such but is embodied by the relevant NH, NR⁶, S, O groups, and C^{A} is optionally linked to the remainder of L^{C} via a spacer S^{P}.

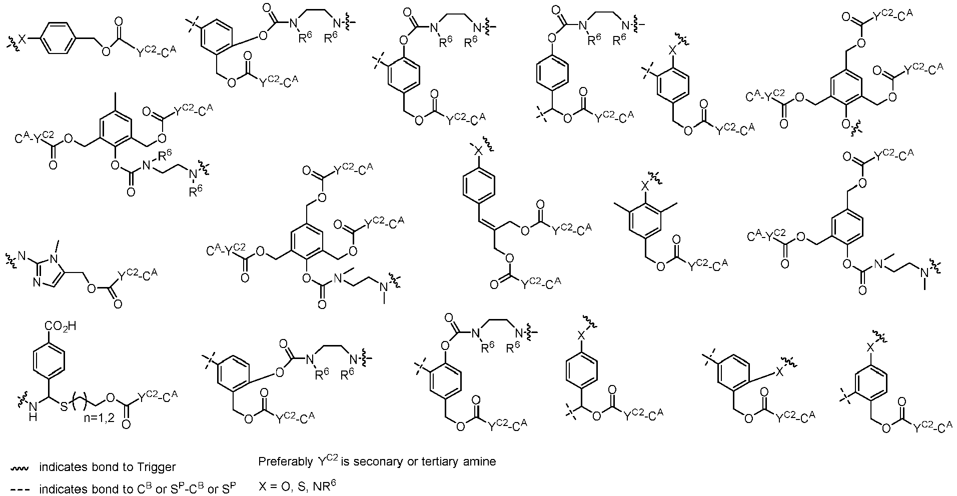

Herein, R⁶ is as defined herein.

Further preferred structures of L^{C} can be found in WO2009017394(A1), US7375078, WO2015038426A1, WO2004043493, Angew. Chem. Int. Ed. 2015, 54, 7492 - 7509.

In preferred embodiments of the invention the L^{C} has a mass of no more than 1000 daltons, no more than 500 daltons, no more than 400 daltons, no more than 300 daltons, or from 10, 50 or 100 to 1000 daltons, from 10, 50, 100 to 400 daltons, from 10, 50, 100 to 300 daltons, from 10, 50, 100 to 200 daltons, e.g., 10-1000 daltons, such as 50-500 daltons, such as 100 to 400 daltons.

A person skilled in the art will know that one L^{C} may be connected to another L^{C} that is bound to C^{A}, wherein upon reaction of the Cleaving Agent with the Trigger T^{R}, L^{C}-L^{C}-C^{A} is released from the T^{R}, leading to self-immolative release of both L^{C} moieties and the C^{A} moiety. With respect to the L^{C} formulas disclosed herein, the L^{C} linking the T^{R} to the other L^{C} then does not release C^{A} but an L^{C} that is bound via Y^{C1} and further links to a C^{A}.

R⁶, R⁷, R⁸, R⁹ are as defined herein.

In a preferred embodiment, L^{C} is selected from the group consisting of linkers according to Group I, Group II, and Group III,
wherein linkers according to Group I are 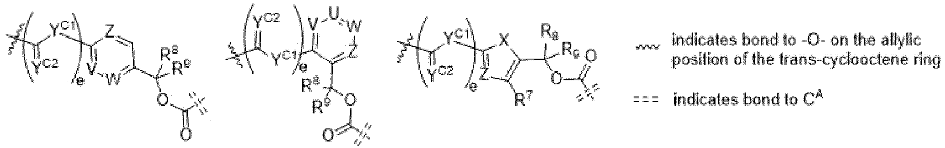 , wherein U, V, W, Z are each independently selected from the group consisting of -CR⁷-, and -N-. Herein, e is either 0 or 1, and X is selected from the group consisting of -O-, -S- and -NR⁶-. Preferably, each R⁸ and R⁹ are independently selected from the group consisting of hydrogen, C₁-C₄ alkyl groups, C₂-C₄ alkenyl groups, and C₄₋₆ (hetero)aryl groups, wherein for R⁸ and R⁹ the alkyl groups, alkenyl groups, and (hetero)aryl groups are optionally substituted with a moiety selected from the group consisting of -Cl, -F, -Br, -I, -OH, -NH₂, =O, -SH, -SO₃H, - PO₃H, -PO₄H₂ and -NO₂ and optionally contain at most two heteroatoms selected from the group consisting of -O-, -S-, -NH-, -P-, and -Si-, wherein the N, S, and P atoms are optionally oxidized.

For linkers according to Group I C^{A} is linked to L^{C} via a moiety selected from the group consisting of -O-, -N-, -C-, and -S-, preferably from the group consisting of secondary amines and tertiary amines, wherein said moieties are part of C^{A}.

The linker according to Group II is 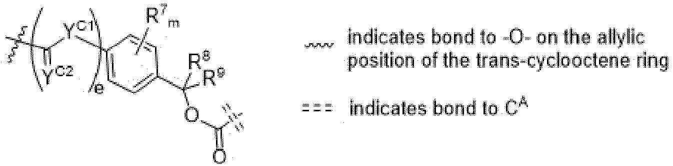 , wherein m is an integer between 0 and 2, preferably m is 0, and wherein e is either 0 or 1. For linkers according to Group II C^{A} is linked to L^{C} via a moiety selected from the group consisting of -O-, -N-, -C-, and -S-, preferably from the group consisting of secondary amines and tertiary amines, wherein said moieties are part of C^{A}.

Linkers according to Group III are 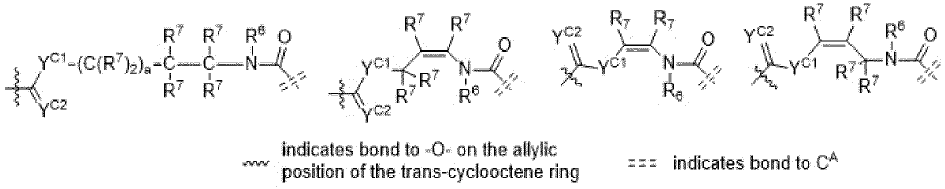 wherein for linkers according to Group III C^{A} is linked to L^{C} via a moiety selected from the group consisting of -O- and -S-, preferably -O- or -S- bound to a C₄₋₆ (hetero)aryl group, wherein said moieties are part of C^{A}.

For linkers according to Groups I-III preferably each R⁶ is independently selected from the group consisting of hydrogen, C₁-C₄ alkyl groups, C₂-C₄ alkenyl groups, and C₄₋₆ (hetero)aryl groups, wherein for R⁶ the alkyl groups, alkenyl groups, and (hetero)aryl groups are optionally substituted with a moiety selected from the group consisting of -Cl, -F, -Br, -I, -OH, -NH₂, =O, -SH, -SO₃H, -PO₃H, - PO₄H₂ and -NO₂ and optionally contain at most two heteroatoms selected from the group consisting of -O-, -S-, -NH-, -P-, and -Si-, wherein the N, S, and P atoms are optionally oxidized.

For linkers of Groups I-III preferably each R⁷ is independently selected from the group consisting of hydrogen and C₁-C₃ alkyl groups, C₂-C₃ alkenyl groups, and C₄₋₆ (hetero)aryl groups, wherein for R⁷ the alkyl groups, alkenyl groups, and (hetero)aryl groups are optionally substituted with a moiety selected from the group consisting of -Cl, -F, -Br, -I, -OH, -NH₂, =O, =NH, -N(CH₃)₂, - S(O)₂CH₃, and -SH, and are optionally interrupted by at most one heteroatom selected from the group consisting of -O-, -S-, -NH-, -P-, and -Si-, wherein the N, S, and P atoms are optionally oxidized, wherein the N atoms are optionally quaternized, wherein R⁷ is preferably selected from the group consisting of hydrogen, methyl, -CH₂-CH₂-N(CH₃)₂, and -CH₂-CH₂-S(O)₂-CH₃.

R⁶, R⁷, R⁸, R⁹ comprised in said Group I, II and III, can optionally also be - (S^{P})ᵢ-C^{B}.

For all linkers according to Group I and Group II Y^{C1} is selected from the group consisting of -O-, -S-, and -NR⁶-, preferably -NR⁶-. For all linkers according to Group III, Y^{C1} is -NR⁶-. For all linkers according to Group I, Group II, and Group III, Y^{C2} is selected from the group consisting of O and S, preferably O.

Preferably, in Groups I-III when two L^{C} are linked to each other, then the L^{C} attached to the -O- or -S- at the allylic position of the trans-cyclooctene is selected from the group consisting of linkers according to Group I and Group II, and the L^{C} between the L^{C} attached to the -O- or -S- at the allylic position of the trans-cyclooctene and C^{A} is selected from Group III, and that the wiggly line in the structures of Group III then denotes a bond to the L^{C} attached to the -O- or -S- at the allylic position of the trans-cyclooctene instead of a bond to the allylic -O- or -S- on the trans-cyclooctene ring, and that the double dashed line in the structures of Groups I and II then denotes a bond to the L^{C} between the L^{C} attached to the -O- or -S- at the allylic position of the trans-cyclooctene and the C^{A} instead of a bond to C^{A}.

In preferred embodiments, L^{C} is selected from the group consisting of linkers according to Group IV, Group V, Group VI, and Group VII.

Linkers according to Group IV are 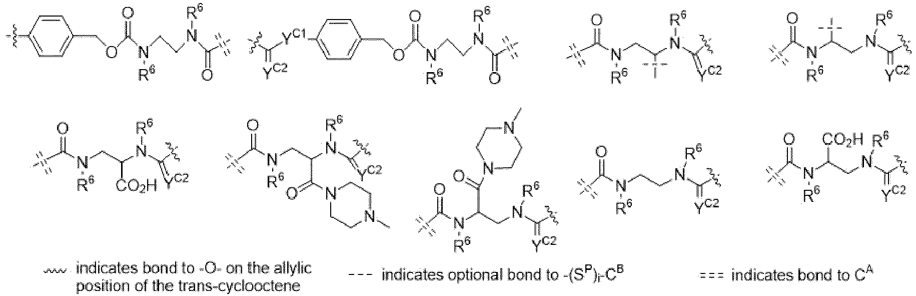 , wherein C^{A} is linked to L^{C} via a moiety selected from the group consisting of -O- and -S-, preferably from the group consisting of -O-C₅₋₈-arylene- and -S-C₅₋₈-arylene-, wherein said moieties are part of C^{A}.

Linkers according to Group V are 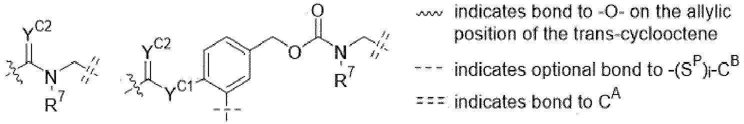 , wherein C^{A} is linked to L^{C} via a moiety selected from the group consisting of -O- and -S-, wherein said moieties are part of C^{A}.

Linkers according to Group VI are 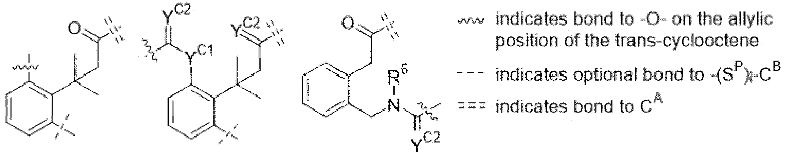 , wherein C^{A} is linked to L^{C} via a moiety selected from the group consisting of -O-, -N-, and -S-, preferably a secondary or a tertiary amine, wherein said moieties are part of C^{A}.

Linkers according to Group VII are 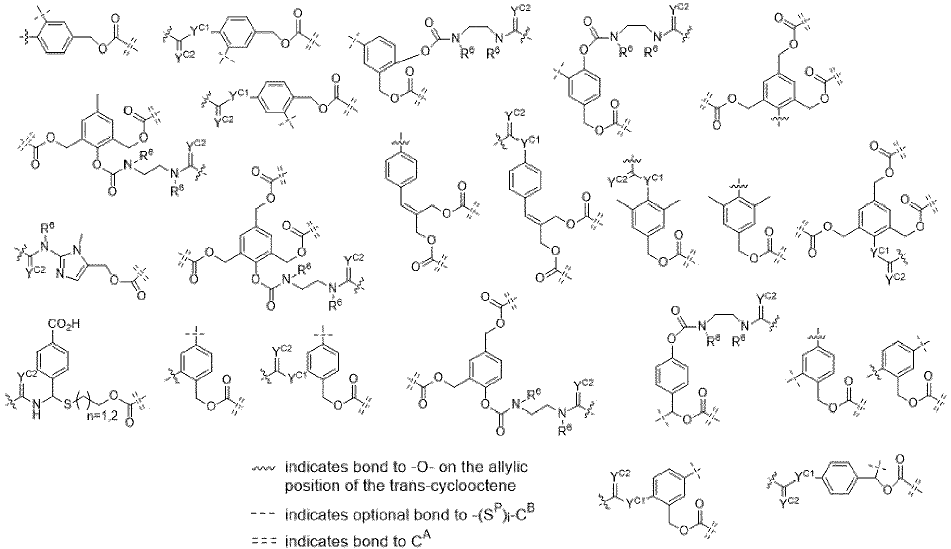 , wherein C^{A} is linked to L^{C} via a moiety selected from the group consisting of -O-, -N-, and -S-, preferably from the group consisting of secondary amines and tertiary amines, wherein said moieties are part of C^{A}, wherein when multiple double dashed lines are shown within one L^{C}, each C^{A} moiety is independently selected.

For all linkers according to Group IV, Group V, Group VI, and Group VII, Y^{C1} is selected from the group consisting of -O-, -S-, and -NR⁶-. For all linkers according to Groups IV-VII R⁶ and R⁷ are as defined herein; i is an integer in a range of from 0 to 4, preferably 0 or 1; and j is 0 or 1.

In preferred embodiments, R⁶, R⁷, R⁸ and R⁹ in L^{C} formulas, in particular in any one of Groups I-VII, are hydrogen.

### R⁶

Preferably, each R⁶ is independently selected from the group consisting of hydrogen, -(S^{P})ᵢ-C^{C}, C₁-C₂₄ alkyl groups, C₂-C₂₄ alkenyl groups, C₂-C₂₄ alkynyl groups, C₆-C₂₄ aryl groups, C₂-C₂₄ heteroaryl groups, C₃-C₂₄ cycloalkyl groups, C₅-C₂₄ cycloalkenyl groups, C₁₂-C₂₄ cycloalkynyl groups, C₃-C₂₄ (cyclo)alkyl(hetero)aryl groups, C₃-C₂₄ (hetero)aryl(cyclo)alkyl, C₄-C₂₄ (cyclo)alkenyl(hetero)aryl groups, C₄-C₂₄ (hetero)aryl(cyclo)alkenyl groups, C₄-C₂₄ (cyclo)alkynyl(hetero)aryl groups, C₄-C₂₄ (hetero)aryl(cyclo)alkynyl groups, C₄-C₂₄ alkylcycloalkyl groups, and C₄-C₂₄ cycloalkylalkyl groups; wherein i is an integer in a range of from 0 to 4, preferably i is 1;
the R⁶ groups not being hydrogen are optionally substituted with a moiety selected from the group consisting of -Cl, -F, -Br, -I, -OH, -NH₂, -SO₃H, -PO₃H, - PO₄H₂, -NO₂, -CF₃, =O, =NH, and -SH, and optionally contain one or more heteroatoms selected from the group consisting of O, S, NH, P, and Si, wherein the N, S, and P atoms are optionally oxidized, wherein the N atoms are optionally quaternized.

In preferred embodiments, each R⁶ is individually selected from the group consisting of hydrogen, C₁-C₈ alkyl groups, C₂-C₈ alkenyl groups, C₂-C₈ alkynyl groups, C₆-C₁₂ aryl, C₂-C₁₂ heteroaryl, C₃-C₈ cycloalkyl groups, C₅-C₈ cycloalkenyl groups, C₃-C₁₂ alkyl(hetero)aryl groups, C₃-C₁₂ (hetero)arylalkyl groups, C₄-C₁₂ alkylcycloalkyl groups, C₄-C₁₂ cycloalkylalkyl groups, C₅-C₁₂ cycloalkyl(hetero)aryl groups and C₅-C₁₂ (hetero)arylcycloalkyl groups, wherein the R⁶ groups not being hydrogen are optionally substituted with a moiety selected from the group consisting of -Cl, -F, -Br, -I, -OH, -NH₂, -SO₃H, -PO₃H, - PO₄H₂, -NO₂, -CF₃, =O, =NH, and -SH, and optionally contain one or more heteroatoms selected from the group consisting of O, S, NH, P, and Si, wherein the N, S, and P atoms are optionally oxidized, wherein the N atoms are optionally quaternized.

In preferred embodiments, R⁶ is selected from the group consisting of hydrogen, C₁-C₄ alkyl groups, C₂-C₄ alkenyl groups, C₂-C₄ alkynyl groups, C₆-C₈ aryl, C₂-C₈ heteroaryl, C₃-C₆ cycloalkyl groups, C₅-C₆ cycloalkenyl groups, C₃-C₁₀ alkyl(hetero)aryl groups, C₃-C₁₀ (hetero)arylalkyl groups, C₄-C₈ alkylcycloalkyl groups, C₄-C₈ cycloalkylalkyl groups, C₅-C₁₀ cycloalkyl(hetero)aryl groups and C₅-C₁₀ (hetero)arylcycloalkyl groups, wherein the R⁶ groups not being hydrogen are optionally substituted with a moiety selected from the group consisting of -Cl, -F, -Br, -I, -OH, -NH₂, -SO₃H, -PO₃H, -PO₄H₂, -NO₂, -CF₃, =O, =NH, and -SH, and optionally contain one or more heteroatoms selected from the group consisting of O, S, NH, P, and Si, wherein the N, S, and P atoms are optionally oxidized, wherein the N atoms are optionally quaternized.

In preferred embodiments, R⁶ is selected from the group consisting of hydrogen, C₁-C₄ alkyl groups, C₂-C₄ alkenyl groups, and C₄₋₆ (hetero)aryl groups, wherein for R⁶ the alkyl groups, alkenyl groups, and (hetero)aryl groups are optionally substituted with a moiety selected from the group consisting of -Cl, -F, -Br, -I, -OH, -NH₂, =O, -SH, -SO₃H, -PO₃H, -PO₄H₂ and -NO₂ and optionally contain at most two heteroatoms selected from the group consisting of -O-, -S-, - NH-, -P-, and -Si-, wherein the N, S, and P atoms are optionally oxidized.

In preferred embodiments, R⁶ is selected from the group consisting of hydrogen, C₁-C₃ alkyl groups, C₂-C₃ alkenyl groups, and C₄₋₆ (hetero)aryl groups, wherein for R⁶ the alkyl groups, alkenyl groups, and (hetero)aryl groups are optionally substituted with a moiety selected from the group consisting of -Cl, -F, -Br, -I, -OH, -NH₂, =O, -SH, -SO₃H, -PO₃H, -PO₄H₂ and -NO₂ and optionally contain at most two heteroatoms selected from the group consisting of -O-, -S-, - NH-, -P-, and -Si-, wherein the N, S, and P atoms are optionally oxidized.

In preferred embodiments, the R⁶ groups not being hydrogen are not substituted. In preferred embodiments, the R⁶ groups not being hydrogen do not contain heteroatoms. In preferred embodiments, the R⁶ groups are hydrogen.

### R⁷

In preferred embodiments, each R⁷ is independently selected from the group consisting of hydrogen, -(S^{P})ᵢ-C^{C} , -F, -Cl, -Br, -I, -OR₃₇, -N(R₃₇)₂, -SO₃, -PO₃⁻, -NO₂, -CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, -S(O)₂R₃₇, NR₃₇S(O)₂R₃₇, -ON(R₃₇)₂, ₋NR₃₇OR₃₇, C₁-C₂₄ alkyl groups, C₂-C₂₄ alkenyl groups, C₂-C₂₄ alkynyl groups, C₆-C₂₄ aryl groups, C₂-C₂₄ heteroaryl groups, C₃-C₂₄ cycloalkyl groups, C₅-C₂₄ cycloalkenyl groups, C₁₂-C₂₄ cycloalkynyl groups, C₃-C₂₄ (cyclo)alkyl(hetero)aryl groups, C₃-C₂₄ (hetero)aryl(cyclo)alkyl, C₄-C₂₄ (cyclo)alkenyl(hetero)aryl groups, C₄-C₂₄ (hetero)aryl(cyclo)alkenyl groups, C₄-C₂₄ (cyclo)alkynyl(hetero)aryl groups, C₄-C₂₄ (hetero)aryl(cyclo)alkynyl groups, C₄-C₂₄ alkylcycloalkyl groups, and C₄-C₂₄ cycloalkylalkyl groups; wherein i is an integer in a range of from 0 to 4, preferably i is 1, wherein the alkyl groups, alkenyl groups, alkynyl groups, aryl, heteroaryl, cycloalkyl groups, cycloalkenyl groups, cycloalkynyl groups, (cyclo)alkyl(hetero)aryl groups, (hetero)aryl(cyclo)alkyl groups, (cyclo)alkenyl(hetero)aryl groups, (hetero)aryl(cyclo)alkenyl groups, (cyclo)alkynyl(hetero)aryl groups, (hetero)aryl(cyclo)alkynyl groups, alkylcycloalkyl groups, cycloalkylalkyl groups are optionally substituted with a moiety selected from the group consisting of -Cl, -F, -Br, -I, -OR₃₇, -N(R₃₇)₂, - SO₃R₃₇, -PO₃(R₃₇)₂, -PO₄(R₃₇)₂, -NO₂, -CF₃, =O, =NR₃₇, and -SR₃₇, and optionally contain one or more heteroatoms selected from the group consisting of O, S, NR₃₇, P, and Si, wherein the N, S, and P atoms are optionally oxidized, wherein the N atoms are optionally quaternized.

In preferred embodiments, each R⁷ is independently selected from the group consisting hydrogen, -F, -Cl, -Br, -I, -OR₃₇, -N(R₃₇)₂, -SO₃, -PO₃⁻, -NO₂, -CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, - S(O)₂R₃₇, NR₃₇S(O)₂R₃₇, -ON(R₃₇)₂, ₋NR₃₇OR₃₇, C₁-C₈ alkyl groups, C₂-C₈ alkenyl groups, C₂-C₈ alkynyl groups, C₆-C₁₂ aryl groups, C₂-C₁₂ heteroaryl groups, C₃-C₈ cycloalkyl groups, C₅-C₈ cycloalkenyl groups, C₃-C₁₂ alkyl(hetero)aryl groups, C₃-C₁₂ (hetero)arylalkyl groups, C₄-C₁₂ alkylcycloalkyl groups, C₄-C₁₂ cycloalkylalkyl groups, C₅-C₁₂ cycloalkyl(hetero)aryl groups and C₅-C₁₂ (hetero)arylcycloalkyl groups, wherein the alkyl groups, alkenyl groups, alkynyl groups, aryl, heteroaryl, cycloalkyl groups, cycloalkenyl groups, alkyl(hetero)aryl groups, (hetero)arylalkyl groups, alkylcycloalkyl groups, cycloalkylalkyl groups, cycloalkyl(hetero)aryl groups and (hetero)arylcycloalkyl groups are optionally substituted with a moiety selected from the group consisting of -Cl, -F, -Br, -I, - OR₃₇, -N(R₃₇)₂, -SO₃R₃₇, -PO₃(R₃₇)₂, -PO₄(R₃₇)₂, -NO₂, -CF₃, =O, =NR₃₇, and -SR₃₇, and optionally contain one or more heteroatoms selected from the group consisting of O, S, NR₃₇, P, and Si, wherein the N, S, and P atoms are optionally oxidized, wherein the N atoms are optionally quaternized.

In preferred embodiments, each R⁷ is independently selected from the group consisting of hydrogen, -F, -Cl, -Br, -I, -OR₃₇, -N(R₃₇)₂, -SO₃, -PO₃⁻, -NO₂, - CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, -S(O)₂R₃₇, NR₃₇S(O)₂R₃₇, -ON(R₃₇)₂, ₋NR₃₇OR₃₇, C₁-C₄ alkyl groups, C₂-C₄ alkenyl groups, C₂-C₄ alkynyl groups, C₆-C₈ aryl groups, C₂-C₈ heteroaryl groups, C₃-C₆ cycloalkyl groups, C₅-C₆ cycloalkenyl groups, C₃-C₁₀ alkyl(hetero)aryl groups, C₃-C₁₀ (hetero)arylalkyl groups, C₄-C₁₀ alkylcycloalkyl groups, C₄-C₁₀ cycloalkylalkyl groups, C₅-C₁₀ cycloalkyl(hetero)aryl groups and C₅-C₁₀ (hetero)arylcycloalkyl groups, wherein the alkyl groups, alkenyl groups, alkynyl groups, aryl, heteroaryl, cycloalkyl groups, cycloalkenyl groups, alkyl(hetero)aryl groups, (hetero)arylalkyl groups, alkylcycloalkyl groups, cycloalkylalkyl groups, cycloalkyl(hetero)aryl groups and (hetero)arylcycloalkyl groups are optionally substituted with a moiety selected from the group consisting of -Cl, -F, -Br, -I, - OR₃₇, -N(R₃₇)₂, -SO₃R₃₇, -PO₃(R₃₇)₂, -PO₄(R₃₇)₂, -NO₂, -CF₃, =O, =NR₃₇, and -SR₃₇, and optionally contain one or more heteroatoms selected from the group consisting of O, S, NR₃₇, P, and Si, wherein the N, S, and P atoms are optionally oxidized, wherein the N atoms are optionally quaternized.

In preferred embodiments, each R⁷ is independently selected from the group consisting of hydrogen and C₁-C₃ alkyl groups, C₂-C₃ alkenyl groups, and C₄₋₆ (hetero)aryl groups, wherein the alkyl groups, alkenyl groups, and (hetero)aryl groups are optionally substituted with a moiety selected from the group consisting of -Cl, -F, -Br, -I, -OH, -NH₂, =O, =NH, -N(CH₃)₂, -S(O)₂CH₃, and -SH, and are optionally interrupted by at most one heteroatom selected from the group consisting of -O-, -S-, -NH-, -P-, and -Si-, wherein the N, S, and P atoms are optionally oxidized, wherein the N atoms are optionally quaternized.

In preferred embodiments, R⁷ is preferably selected from the group consisting of hydrogen, methyl, -CH₂-CH₂-N(CH₃)₂, and -CH₂-CH₂-S(O)₂-CH₃. In preferred embodiments, the R⁷ groups not being hydrogen are not substituted. In preferred embodiments, the R⁷ groups not being hydrogen do not contain heteroatoms. In preferred embodiments, the R⁷ groups are hydrogen.

### R⁸ and R⁹

R⁸ and R⁹ are as defined for R⁷. In preferred embodiments, at least one or all R⁸ are -H. In preferred embodiments, at least one or all R⁸ are -CH₃. In preferred embodiments, at least one or all R⁹ are -H. In preferred embodiments, at least one or all R⁹ are -CH₃. In preferred embodiments, the R₈ and R₉ groups not being hydrogen are not substituted. In preferred embodiments, the R₈ and R₉ groups not being hydrogen do not contain heteroatoms. In preferred embodiments, the R⁸ and R⁹ groups are hydrogen.

### X¹, X², X³, X⁴, and X⁵

In Formula (1), each X¹, X², X³, X⁴ is independently selected from the group consisting of -C(R₄₇)₂-, -NR₃₇-, -C(O)-, -O-, such that at most two of X¹, X², X³, X⁴ are not -C(R₄₇)₂-, and with the proviso that no sets consisting of adjacent atoms are present selected from the group consisting of -O-O-, -O-N-, -C(O)-O-, N-N-, and -C(O)-C(O)-.

In a preferred embodiment, X¹, X², X³, and X⁴ are -C(R₄₇)₂-, and preferably at most four R₄₇, more preferably at most two R₄₇, most preferably at most one R₄₇, are not H. In Formula (1) X⁵ is -C(R₄₇)₂- or -CHR₄₈. In a preferred embodiment, X⁵ is -C(R₄₇)₂-; more preferably X⁵ is -CHR₄₇, and most preferably X⁵ is -CH₂. In a preferred embodiment X⁵ is CHR₄₈.

### R₃₆

In a preferred embodiment, R₃₆ is as defined for R₃₇.

In Formula (1) R₃₆ is preferably selected from the group consisting of hydrogen, C₁-C₈ alkyl groups, C₂-C₈ alkenyl groups, and C₄₋₆ (hetero)aryl groups, wherein the alkyl groups, alkenyl groups, and (hetero)aryl groups are optionally substituted with a moiety selected from the group consisting of -Cl, -F, -Br, -I, - OH, -NH₂, =O, -SH, -SO₃H, -PO₃H, -PO₄H₂ and -NO₂ and optionally contain at most two heteroatoms selected from the group consisting of -O-, -S-, -NH-, -P-, and -Si-, wherein the N, S, and P atoms are optionally oxidized. In preferred embodiments, the R₃₆ groups not being hydrogen are not substituted. In preferred embodiments, the R₃₆ groups not being hydrogen do not contain heteroatoms. In some preferred embodiments, R₃₆ is hydrogen.

### R₄₇

In Formula (1) each R₄₇ is independently selected from the group consisting of hydrogen, -L^{B}, -L^{A}, -(S^{P})ᵢ-C^{C}, -F, -Cl, -Br, -I, -OR₃₇, -N(R₃₇)₂, -SO₃, -PO₃⁻, -NO₂, - CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, -S(O)₂R₃₇, NR₃₇S(O)₂R₃₇, -ON(R₃₇)₂, ₋NR₃₇OR₃₇, C₁-C₂₄ alkyl groups, C₂-C₂₄ alkenyl groups, C₂-C₂₄ alkynyl groups, C₆-C₂₄ aryl groups, C₂-C₂₄ heteroaryl groups, C₃-C₂₄ cycloalkyl groups, C₅-C₂₄ cycloalkenyl groups, C₁₂-C₂₄ cycloalkynyl groups, C₃-C₂₄ (cyclo)alkyl(hetero)aryl groups, C₃-C₂₄ (hetero)aryl(cyclo)alkyl, C₄-C₂₄ (cyclo)alkenyl(hetero)aryl groups, C₄-C₂₄ (hetero)aryl(cyclo)alkenyl groups, C₄-C₂₄ (cyclo)alkynyl(hetero)aryl groups, C₄-C₂₄ (hetero)aryl(cyclo)alkynyl groups, C₄-C₂₄ alkylcycloalkyl groups, and C₄-C₂₄ cycloalkylalkyl groups; wherein i is an integer in a range of from 0 to 4, preferably i is an integer ranging from 0 to 1, wherein the alkyl groups, alkenyl groups, alkynyl groups, aryl, heteroaryl, cycloalkyl groups, cycloalkenyl groups, cycloalkynyl groups, (cyclo)alkyl(hetero)aryl groups, (hetero)aryl(cyclo)alkyl groups, (cyclo)alkenyl(hetero)aryl groups, (hetero)aryl(cyclo)alkenyl groups, (cyclo)alkynyl(hetero)aryl groups, (hetero)aryl(cyclo)alkynyl groups, alkylcycloalkyl groups, cycloalkylalkyl groups are optionally substituted with a moiety selected from the group consisting of -Cl, -F, -Br, -I, -OR₃₇, -N(R₃₇)₂, - SO₃R₃₇, -PO₃(R₃₇)₂, -PO₄(R₃₇)₂, -NO₂, -CF₃, =O, =NR₃₇, and -SR₃₇, and optionally contain one or more heteroatoms selected from the group consisting of O, S, NR₃₇, P, and Si, wherein the N, S, and P atoms are optionally oxidized, wherein the N atoms are optionally quaternized.

In preferred embodiments, each R₄₇ is independently selected from the group consisting of hydrogen, -L^{B}, -L^{A}, -(S^{P})ᵢ-C^{C}, -F, -Cl, -Br, -I, -OR₃₇, -N(R₃₇)₂, - SO₃, -PO₃⁻, -NO₂, -CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, -S(O)₂R₃₇, NR₃₇S(O)₂R₃₇, -ON(R₃₇)₂, ₋NR₃₇OR₃₇, C₁-C₈ alkyl groups, C₂-C₈ alkenyl groups, C₂-C₈ alkynyl groups, C₆-C₁₂ aryl groups, C₂-C₁₂ heteroaryl groups, C₃-C₈ cycloalkyl groups, C₅-C₈ cycloalkenyl groups, C₃-C₁₂ alkyl(hetero)aryl groups, C₃-C₁₂ (hetero)arylalkyl groups, C₄-C₁₂ alkylcycloalkyl groups, C₄-C₁₂ cycloalkylalkyl groups, C₅-C₁₂ cycloalkyl(hetero)aryl groups and C₅-C₁₂ (hetero)arylcycloalkyl groups, wherein the alkyl groups, alkenyl groups, alkynyl groups, aryl, heteroaryl, cycloalkyl groups, cycloalkenyl groups, alkyl(hetero)aryl groups, (hetero)arylalkyl groups, alkylcycloalkyl groups, cycloalkylalkyl groups, cycloalkyl(hetero)aryl groups and (hetero)arylcycloalkyl groups are optionally substituted with a moiety selected from the group consisting of -Cl, -F, -Br, -I, -OR₃₇, -N(R₃₇)₂, -SO₃R₃₇, -PO₃(R₃₇)₂, -PO₄(R₃₇)₂, -NO₂, - CF₃, =O, =NR₃₇, and -SR₃₇, and optionally contain one or more heteroatoms selected from the group consisting of O, S, NR₃₇, P, and Si, wherein the N, S, and P atoms are optionally oxidized, wherein the N atoms are optionally quaternized.

In preferred embodiments, each R₄₇ is independently selected from the group consisting of hydrogen, -L^{B}, -L^{A}, -(S^{P})ᵢ-C^{C}, -F, -Cl, -Br, -I, -OR₃₇, -N(R₃₇)₂, - SO₃, -PO₃⁻, -NO₂, -CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, -S(O)₂R₃₇, NR₃₇S(O)₂R₃₇, -ON(R₃₇)₂, ₋NR₃₇OR₃₇, C₁-C₄ alkyl groups, C₂-C₄ alkenyl groups, C₂-C₄ alkynyl groups, C₆-C₈ aryl groups, C₂-C₈ heteroaryl groups, C₃-C₆ cycloalkyl groups, C₅-C₆ cycloalkenyl groups, C₃-C₁₀ alkyl(hetero)aryl groups, C₃-C₁₀ (hetero)arylalkyl groups, C₄-C₁₀ alkylcycloalkyl groups, C₄-C₁₀ cycloalkylalkyl groups, C₅-C₁₀ cycloalkyl(hetero)aryl groups and C₅-C₁₀ (hetero)arylcycloalkyl groups, wherein the alkyl groups, alkenyl groups, alkynyl groups, aryl, heteroaryl, cycloalkyl groups, cycloalkenyl groups, alkyl(hetero)aryl groups, (hetero)arylalkyl groups, alkylcycloalkyl groups, cycloalkylalkyl groups, cycloalkyl(hetero)aryl groups and (hetero)arylcycloalkyl groups are optionally substituted with a moiety selected from the group consisting of -Cl, -F, -Br, -I, - OR₃₇, -N(R₃₇)₂, -SO₃R₃₇, -PO₃(R₃₇)₂, -PO₄(R₃₇)₂, -NO₂, -CF₃, =O, =NR₃₇, and -SR₃₇, and optionally contain one or more heteroatoms selected from the group consisting of O, S, NR₃₇, P, and Si, wherein the N, S, and P atoms are optionally oxidized, wherein the N atoms are optionally quaternized.

### R37

In Formula (1) each R₃₇ is independently selected from the group consisting of hydrogen, -L^{B}, -L^{A}, -(S^{P})ᵢ-C^{C}, C₁-C₂₄ alkyl groups, C₂-C₂₄ alkenyl groups, C₂-C₂₄ alkynyl groups, C₆-C₂₄ aryl groups, C₂-C₂₄ heteroaryl groups, C₃-C₂₄ cycloalkyl groups, C₅-C₂₄ cycloalkenyl groups, C₁₂-C₂₄ cycloalkynyl groups, C₃-C₂₄ (cyclo)alkyl(hetero)aryl groups, C₃-C₂₄ (hetero)aryl(cyclo)alkyl, C₄-C₂₄ (cyclo)alkenyl(hetero)aryl groups, C₄-C₂₄ (hetero)aryl(cyclo)alkenyl groups, C₄-C₂₄ (cyclo)alkynyl(hetero)aryl groups, C₄-C₂₄ (hetero)aryl(cyclo)alkynyl groups, C₄-C₂₄ alkylcycloalkyl groups, and C₄-C₂₄ cycloalkylalkyl groups; wherein i is an integer in a range of from 0 to 4, preferably i is 1; the R₃₇ groups not being hydrogen are optionally substituted with a moiety selected from the group consisting of -Cl, -F, -Br, -I, -OH, -NH₂, -SO₃H, -PO₃H, -PO₄H₂, -NO₂, -CF₃, =O, =NH, and -SH, and optionally contain one or more heteroatoms selected from the group consisting of O, S, NH, P, and Si, wherein the N, S, and P atoms are optionally oxidized, wherein the N atoms are optionally quaternized.

In preferred embodiments, R₃₇ is selected from the group consisting of hydrogen, -L^{B}, -L^{A}, -(S^{P})ᵢ-C^{C}, C₁-C₈ alkyl groups, C₂-C₈ alkenyl groups, C₂-C₈ alkynyl groups, C₆-C₁₂ aryl, C₂-C₁₂ heteroaryl, C₃-C₈ cycloalkyl groups, C₅-C₈ cycloalkenyl groups, C₃-C₁₂ alkyl(hetero)aryl groups, C₃-C₁₂ (hetero)arylalkyl groups, C₄-C₁₂ alkylcycloalkyl groups, C₄-C₁₂ cycloalkylalkyl groups, C₅-C₁₂ cycloalkyl(hetero)aryl groups and C₅-C₁₂ (hetero)arylcycloalkyl groups, wherein the R₃₇ groups not being hydrogen are optionally substituted with a moiety selected from the group consisting of -Cl, -F, -Br, -I, -OH, -NH₂, -SO₃H, -PO₃H,-PO₄H₂, -NO₂, -CF₃, =O, =NH, and -SH, and optionally contain one or more heteroatoms selected from the group consisting of O, S, NH, P, and Si, wherein the N, S, and P atoms are optionally oxidized, wherein the N atoms are optionally quaternized.

In preferred embodiments, R₃₇ is selected from the group consisting of hydrogen, -L^{B}, -L^{A}, -(S^{P})ᵢ-C^{C}, C₁-C₄ alkyl groups, C₂-C₄ alkenyl groups, C₂-C₄ alkynyl groups, C₆-C₈ aryl, C₂-C₈ heteroaryl, C₃-C₆ cycloalkyl groups, C₅-C₆ cycloalkenyl groups, C₃-C₁₀ alkyl(hetero)aryl groups, C₃-C₁₀ (hetero)arylalkyl groups, C₄-C₈ alkylcycloalkyl groups, C₄-C₈ cycloalkylalkyl groups, C₅-C₁₀ cycloalkyl(hetero)aryl groups and C₅-C₁₀ (hetero)arylcycloalkyl groups, wherein the R₃₇ groups not being hydrogen are optionally substituted with a moiety selected from the group consisting of -Cl, -F, -Br, -I, -OH, -NH₂, -SO₃H, -PO₃H,-PO₄H₂, -NO₂, -CF₃, =O, =NH, and -SH, and optionally contain one or more heteroatoms selected from the group consisting of O, S, NH, P, and Si, wherein the N, S, and P atoms are optionally oxidized, wherein the N atoms are optionally quaternized.

In preferred embodiments, the R₃₇ groups not being hydrogen are not substituted. In preferred embodiments, the R₃₇ groups not being hydrogen do not contain heteroatoms. In some preferred embodiments, R₃₇ is hydrogen.

S^{P} is a spacer as defined herein and C^{C} is a Construct-C as defined herein.

In Formula (1) two R₄₇ and /or R₃₇ are optionally comprised in a ring.

In Formula (1) two R₄₇ and /or R₃₇ are optionally comprised in a ring so as to form a ring fused to the eight-membered trans-ring of Formula (1). It is preferred that when two R₄₇ and /or R₃₇ groups are comprised in a ring so as to form a ring fused to the eight-membered trans-ring, that these rings fused to the eight-membered trans-ring are C₃-C₇ cycloalkylene groups and C₄-C₇ cycloalkenylene groups, optionally substituted and containing heteroatoms as described for R₄₇.

### Label

The compound of Formula (1) comprises a Label that is capable of providing the desired diagnostic, imaging, and/or radiotherapeutic effect.

The Label is a moiety comprising a radionuclide.

Especially for imaging applications, it is preferred that the Label is a detectable label. A "detectable label" as used herein relates to the part of the compound of Formula (1) which allows detection of the compound of Formula (1) when present in a cell, tissue or organism. One type of detectable label envisaged within the context of the present invention is a contrast providing label. Different types of detectable labels are envisaged within the context of the present invention and are described hereinbelow.

Thus, according to a particular embodiment of the present invention, the compounds, combinations, kits, and methods of the present invention are used in imaging, especially medical imaging. In order to identify the Primary Target and/or to evaluate the biodistribution of the compound of Formula (1), use is made of a detectable Label.

Preferred detectable labels for imaging are contrast-providing moieties used in traditional imaging systems.

Preferably, the radionuclide comprised in a Label for imaging is an isotope selected from the group consisting of ³H, ¹¹C, ¹³N, ¹⁵O, ¹⁸F, ¹⁹F, ⁴⁴Sc, ⁵¹Cr, ⁵²Fe, ⁵²Mn, ⁵⁵Co, ⁶⁰Cu, ⁶¹Cu, ⁶²Zn, ⁶²Cu, ⁶³Zn, ⁶⁴Cu, ⁶⁶Ga, ⁶⁷Ga, ⁶⁸Ga, ⁷⁰As, ⁷¹As, ⁷²As, ⁷⁴As, ⁷⁵Se, ⁷⁵Br, ⁷⁶Br, ⁷⁷Br, ^{8O}Br, ⁸²Br, ⁸²Rb, ⁸⁶Y, ⁸⁸Y, ⁸⁹Sr, ⁸⁹Zr, ⁹⁷Ru, ^{99m}Tc, ¹¹⁰In, ¹¹¹In ¹¹³In, ¹¹⁴In, ¹¹⁷Sn, ¹²⁰I ¹²²Xe, ¹²³I, ¹²⁴I, ¹²⁵I, ¹⁶⁶Ho, ¹⁶⁷Tm, ¹⁶⁹Yb, ¹⁹³Pt, ¹⁹⁵Pt, ²⁰¹Tl, and ²⁰³Pb.

More preferably, the radionuclide comprised in a Label for imaging is an isotope selected from the group consisting of ¹⁸F, ⁴⁴Sc, ⁶⁴Cu, ⁶⁸Ga, ⁸⁹Zr, ^{99m}Tc, ¹¹¹In, ¹²³I, and ¹²⁴I.

Other elements and isotopes, such as being used for therapy may also be applied for imaging in certain applications.

In one embodiment the detectable labels comprise small size organic PET and SPECT radioisotopes, such as ¹⁸F, ¹¹C , ¹²³I or ¹²⁴I. Due to their small size, organic PET or SPECT radioisotopes are ideally suited for monitoring intracellular events as they do not greatly affect the properties of the Administration Agent in general and its membrane transport in particular.

In preferred embodiments, especially when the compound of Formula (1) is used in therapeutic applications, the Label is a therapeutic Label, said Label comprising a radioactive isotope for radiation therapy. A radionuclide used for therapy is preferably an isotope selected from the group consisting of ²⁴Na, ³²P, ³³P, ⁴⁷Sc, ⁵⁹Fe, ⁶⁷Cu, ⁷⁶As, ⁷⁷As, ⁸⁰Br, ⁸²Br, ⁸⁹Sr, ⁹⁰Nb, ⁹⁰Y, ¹⁰³Ru, ¹⁰⁵Rh, ¹⁰⁹Pd, ¹¹¹Ag ¹¹¹In ¹²¹Sn, ¹²⁷Te, ¹³¹I, ¹⁴⁰La, ¹⁴¹Ce, ¹⁴²Pr, ¹⁴³Pr, ¹⁴⁴Pr, ¹⁴⁹Pm, ¹⁴⁹Tb, ¹⁵¹Pm, ¹⁵³Sm, ¹⁵⁹Gd, ¹⁶¹Tb, ¹⁶⁵Dy, ¹⁶⁶Dy, ¹⁶⁶Ho, ¹⁶⁹Er, ¹⁷²Tm, ¹⁷⁵Yb, ¹⁷⁷Lu, ¹⁸⁶Re, ¹⁸⁸Re, ¹⁹⁸Au, ¹⁹⁹Au, ²¹¹At, ²¹¹Bi, ²¹²Bi, ²¹²Pb, ²¹³Bi, ²¹⁴Bi, ²²³Ra, ²²⁴Ra, ²²⁵Ac, and ²²⁷Th.

More preferably, the radionuclide comprised in a Label for therapy is an isotope selected from the group consisting of ⁹⁰Y, ¹¹¹In, ¹³¹I, ¹⁷⁷Lu, ¹⁸⁶Re, ¹⁸⁸Re, ²¹¹At, ²¹²Pb, ²¹³Bi ²²⁵Ac, and ²²⁷Th.

When the Label is intended to comprise a metal, such as ¹¹¹In for SPECT imaging, such is preferably provided in the form of a chelate. In such a case the Label preferably comprises a structural moiety capable of forming a coordination complex with such a metal. A good example hereof are macrocylic lanthanide(III) chelates derived from 1,4,7,10-tetraazacyclododecane-1,4,7,10-tetraacetic acid (H₄dota).

In preferred embodiments, the Label is selected from the group consisting of -OR₃₇, -N(R₃₇)₂, -CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, -S(O)₂R₃₇, NR₃₇S(O)₂R₃₇, -ON(R₃₇)₂, ₋NR₃₇OR₃₇, C₁-C₂₄ alkyl groups, C₂-C₂₄ alkenyl groups, C₂-C₂₄ alkynyl groups, C₆-C₂₄ aryl groups, C₂-C₂₄ heteroaryl groups, C₃-C₂₄ cycloalkyl groups, C₅-C₂₄ cycloalkenyl groups, C₁₂-C₂₄ cycloalkynyl groups, C₃-C₂₄ (cyclo)alkyl(hetero)aryl groups, C₃-C₂₄ (hetero)aryl(cyclo)alkyl, C₄-C₂₄ (cyclo)alkenyl(hetero)aryl groups, C₄-C₂₄ (hetero)aryl(cyclo)alkenyl groups, C₄-C₂₄ (cyclo)alkynyl(hetero)aryl groups, C₄-C₂₄ (hetero)aryl(cyclo)alkynyl groups, C₄-C₂₄ alkylcycloalkyl groups, and C₄-C₂₄ cycloalkylalkyl groups;
the R₃₇ groups, alkyl groups, alkenyl groups, alkynyl groups, aryl, heteroaryl, cycloalkyl groups, cycloalkenyl groups, cycloalkynyl groups, (cyclo)alkyl(hetero)aryl groups, (hetero)aryl(cyclo)alkyl groups, (cyclo)alkenyl(hetero)aryl groups, (hetero)aryl(cyclo)alkenyl groups, (cyclo)alkynyl(hetero)aryl groups, (hetero)aryl(cyclo)alkynyl groups, alkylcycloalkyl groups, cycloalkylalkyl groups comprise, are substituted with, and/or chelating at least one isotope selected from the group consisting of ³H, ¹¹C, ¹³N, ¹⁵O, ¹⁸F, ¹⁹F, ⁵¹Cr, ⁵²Fe, ⁵²Mn, ⁵⁵Co, ⁶⁰Cu, ⁶¹Cu, ⁶²Zn, ⁶²Cu, ⁶³Zn, ⁶⁴Cu, ⁶⁶Ga, ⁶⁷Ga, ⁶⁸Ga, ⁷⁰As, ⁷¹As, ⁷²As, ⁷⁴As, ⁷⁵Se, ⁷⁵Br, ⁷⁶Br, ⁷⁷Br, ^{8O}Br, ⁸²Br, ⁸²Rb, ⁸⁶Y, ⁸⁸Y, ⁹⁰Y, ⁸⁹Sr, ⁸⁹Zr, ⁹⁷Ru, ^{99m}Tc, ¹¹⁰In, ¹¹¹In ¹¹³In, ¹¹⁴In, ¹¹⁷Sn, ¹²⁰I, ¹²²Xe, ¹²³I, ¹²⁴I, ¹²⁵I, ¹⁶⁶Ho, ¹⁶⁷Tm, ¹⁶⁹Yb, ¹⁹³Pt, ¹⁹⁵Pt, ²⁰¹Tl, ²⁰³Pb, ²⁴Na, ³²P, ³³P, ⁴⁷Sc, ⁵⁹Fe, ⁶⁷Cu, ⁷⁶As, ⁷⁷As, ⁹⁰Nb, ¹⁰³Ru, ¹⁰⁵Rh, ¹⁰⁹Pd, ¹¹¹Ag, ¹²¹Sn, ¹²⁷Te, ¹³¹I, ¹⁴⁰La, ¹⁴¹Ce, ¹⁴²Pr, ¹⁴³Pr ¹⁴⁴Pr, ¹⁴⁹Pm, ¹⁴⁹Tb, ¹⁵¹Pm, ¹⁵³Sm, ¹⁵⁹Gd, ¹⁶¹Tb, ¹⁶⁵Dy, ¹⁶⁶Dy, ¹⁶⁶Ho, ¹⁶⁹Er, ¹⁷²Tm, ¹⁷⁵Yb, ¹⁷⁷Lu, ¹⁸⁶Re, ¹⁸⁸Re, ¹⁹⁸Au, ¹⁹⁹Au, ²¹¹At, ²¹¹Bi, ²¹²Bi, ²¹²Pb, ²¹³Bi, ²¹⁴Bi, ²²³Ra, ²²⁴Ra, ²²⁵Ac and ²²⁷Th,
and are optionally further substituted with a moiety selected from the group consisting of -Cl, -F, -Br, -I, -OR₃₇, -N(R₃₇)₂, -SO₃R₃₇, -PO₃(R₃₇)₂, -PO₄(R₃₇)₂, -NO₂,-CF₃, =O, =NR₃₇, and -SR₃₇, and optionally contain one or more heteroatoms selected from the group consisting of O, S, NR₃₇, P, and Si, wherein the N, S, and P atoms are optionally oxidized, wherein the N atoms are optionally quaternized.

In another preferred embodiment, the Label is selected from the group consisting of -OR₃₇, -N(R₃₇)₂, -CF₃, -SR₃₇, S(=O)₂N(R₃₇)₂, OC(=O)R₃₇, SC(=O)R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, C₁-C₁₂ alkyl groups, C₂-C₁₂ alkenyl groups, C₂-C₁₂ alkynyl groups, C₆-C₁₂ aryl groups, C₂-C₁₂ heteroaryl groups, C₃-C₁₂ cycloalkyl groups, C₅-C₁₂ cycloalkenyl groups, C₁₂-C₁₂ cycloalkynyl groups, C₃-C₁₂ (cyclo)alkyl(hetero)aryl groups, C₃-C₁₂ (hetero)aryl(cyclo)alkyl, C₄-C₁₂ (cyclo)alkenyl(hetero)aryl groups, C₄-C₁₂ (hetero)aryl(cyclo)alkenyl groups, C₄-C₁₂ (cyclo)alkynyl(hetero)aryl groups, C₄-C₁₂ (hetero)aryl(cyclo)alkynyl groups, C₄-C₁₂ alkylcycloalkyl groups, and C₄-C₁₂ cycloalkylalkyl groups;
the R₃₇ groups, alkyl groups, alkenyl groups, alkynyl groups, aryl, heteroaryl, cycloalkyl groups, cycloalkenyl groups, cycloalkynyl groups, (cyclo)alkyl(hetero)aryl groups, (hetero)aryl(cyclo)alkyl groups, (cyclo)alkenyl(hetero)aryl groups, (hetero)aryl(cyclo)alkenyl groups, (cyclo)alkynyl(hetero)aryl groups, (hetero)aryl(cyclo)alkynyl groups, alkylcycloalkyl groups, cycloalkylalkyl groups comprise, are substituted with, and/or chelating at least one isotope selected from the group consisting of ³H, ¹¹C, ¹³N, ¹⁵O, ¹⁸F, ¹⁹F, ⁴⁴Sc, ⁵¹Cr, ⁵²Fe, ⁵²Mn, ⁵⁵Co, ⁶⁰Cu, ⁶¹Cu, ⁶²Zn, ⁶²Cu, ⁶³Zn, ⁶⁴Cu, ⁶⁶Ga, ⁶⁷Ga, ⁶⁸Ga, ⁷⁰As, ⁷¹As, ⁷²As, ⁷⁴As, ⁷⁵Se, ⁷⁵Br, ⁷⁶Br, ⁷⁷Br, ^{8O}Br, ⁸²Br, ⁸²Rb, ⁸⁶Y, ⁸⁸Y, ⁹⁰Y, ⁸⁹Sr, ⁸⁹Zr, ⁹⁷Ru, ^{99m}Tc, ¹¹⁰In, ¹¹¹In ¹¹³In ¹¹⁴In, ¹¹⁷Sn, ¹²⁰I, ¹²²Xe, ¹²³I ¹²⁴I, ¹²⁵I, ¹⁶⁶Ho, ¹⁶⁷Tm, ¹⁶⁹Yb, ¹⁹³Pt, ¹⁹⁵Pt, ²⁰¹Tl, ²⁰³Pb, ²⁴Na, ³²P, ³³P, ⁴⁷Sc, ⁵⁹Fe, ⁶⁷Cu, ⁷⁶As, ⁷⁷As, ⁹⁰Nb, ¹⁰³Ru, ¹⁰⁵Rh, ¹⁰⁹Pd, ¹¹¹Ag, ¹²¹Sn, ¹²⁷Te, ¹³¹I, ¹⁴⁰La, ¹⁴¹Ce, ¹⁴²Pr, ¹⁴³Pr ¹⁴⁴Pr, ¹⁴⁹Pm, ¹⁴⁹Tb, ¹⁵¹Pm, ¹⁵³Sm, ¹⁵⁹Gd, ¹⁶¹Tb, ¹⁶⁵Dy, ¹⁶⁶Dy, ¹⁶⁶Ho, ¹⁶⁹Er, ¹⁷²Tm, ¹⁷⁵Yb, ¹⁷⁷Lu, ¹⁸⁶Re, ¹⁸⁸Re, ¹⁹⁸Au, ¹⁹⁹Au, ²¹¹At, ²¹¹Bi, ²¹²Bi, ²¹²Pb, ²¹³Bi, ²¹⁴Bi, ²²³Ra, ²²⁴Ra, ²²⁵Ac, and ²²⁷Th,
and are optionally further substituted with a moiety selected from the group consisting of -Cl, -F, -Br, -I, -OR₃₇, -N(R₃₇)₂, -SO₃R₃₇, -PO₃(R₃₇)₂, -PO₄(R₃₇)₂, -NO₂,-CF₃, =O, =NR₃₇, and -SR₃₇, and optionally contain one or more heteroatoms selected from the group consisting of O, S, NR₃₇, P, and Si, wherein the N, S, and P atoms are optionally oxidized, wherein the N atoms are optionally quaternized.

In preferred embodiments, each Label is independently selected from the group consisting of -OR₃₇, -N(R₃₇)₂, -CF₃, -SR₃₇, S(=O)₂N(R₃₇)₂, OC(=O)R₃₇, SC(=O)R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, C₁-C₆ alkyl groups, C₂-C₆ alkenyl groups, C₂-C₆ alkynyl groups, C₆ aryl groups, C₂-C₆ heteroaryl groups, C₃-C₆ cycloalkyl groups, C₅-C₆ cycloalkenyl groups, C₈ cycloalkynyl groups, C₃-C₆ (cyclo)alkyl(hetero)aryl groups, C₃-C₆ (hetero)aryl(cyclo)alkyl, C₄-C₆ (cyclo)alkenyl(hetero)aryl groups, C₄-C₆ (hetero)aryl(cyclo)alkenyl groups, C₄-C₆ (cyclo)alkynyl(hetero)aryl groups, C₄-C₆ (hetero)aryl(cyclo)alkynyl groups, C₄-C₆ alkylcycloalkyl groups, and C₄-C₆ cycloalkylalkyl groups;
the R₃₇ groups, alkyl groups, alkenyl groups, alkynyl groups, aryl, heteroaryl, cycloalkyl groups, cycloalkenyl groups, cycloalkynyl groups, (cyclo)alkyl(hetero)aryl groups, (hetero)aryl(cyclo)alkyl groups, (cyclo)alkenyl(hetero)aryl groups, (hetero)aryl(cyclo)alkenyl groups, (cyclo)alkynyl(hetero)aryl groups, (hetero)aryl(cyclo)alkynyl groups, alkylcycloalkyl groups, cycloalkylalkyl groups comprise, are substituted with and/or chelating at least one isotope selected from the group consisting of ³H, ¹¹C, ¹³N, ¹⁵O, ¹⁸F, ¹⁹F, ⁴⁴Sc, ⁵¹Cr, ⁵²Fe, ⁵²Mn, ⁵⁵Co, ⁶⁰Cu, ⁶¹Cu, ⁶²Zn, ⁶²Cu, ⁶³Zn, ⁶⁴Cu, ⁶⁶Ga, ⁶⁷Ga, ⁶⁸Ga, ⁷⁰As, ⁷¹As, ⁷²As, ⁷⁴As, ⁷⁵Se, ⁷⁵Br, ⁷⁶Br, ⁷⁷Br, ^{8O}Br, ⁸²Br, ⁸²Rb, ⁸⁶Y, ⁸⁸Y, ⁹⁰Y, ⁸⁹Sr, ⁸⁹Zr, ⁹⁷Ru, ^{99m}Tc, ¹¹⁰In ¹¹¹In ¹¹³In ¹¹⁴In, ¹¹⁷Sn, ¹²⁰I, ¹²²Xe, ¹²³I ¹²⁴I, ¹²⁵I, ¹⁶⁶Ho, ¹⁶⁷Tm, ¹⁶⁹Yb, ¹⁹³Pt, ¹⁹⁵Pt, ²⁰¹Tl, ²⁰³Pb, ²⁴Na, ³²P, ³³P, ⁴⁷Sc, ⁵⁹Fe, ⁶⁷Cu, ⁷⁶As, ⁷⁷As, ⁹⁰Nb, ¹⁰³Ru, ¹⁰⁵Rh, ¹⁰⁹Pd, ¹¹¹Ag, ¹²¹Sn, ¹²⁷Te, ¹³¹I, ¹⁴⁰La, ¹⁴¹Ce, ¹⁴²Pr, ¹⁴³Pr ¹⁴⁴Pr, ¹⁴⁹Pm, ¹⁴⁹Tb, ¹⁵¹Pm, ¹⁵³Sm, ¹⁵⁹Gd, ¹⁶¹Tb, ¹⁶⁵Dy, ¹⁶⁶Dy, ¹⁶⁶Ho, ¹⁶⁹Er, ¹⁷²Tm, ¹⁷⁵Yb, ¹⁷⁷Lu, ¹⁸⁶Re, ¹⁸⁸Re, ¹⁹⁸Au, ¹⁹⁹Au, ²¹¹At, ²¹¹Bi, ²¹²Bi, ²¹²Pb, ²¹³Bi, ²¹⁴Bi, ²²³Ra, ²²⁴Ra,²²⁵Ac, and ²²⁷Th,
and are optionally further substituted with a moiety selected from the group consisting of -Cl, -F, -Br, -I, -OR₃₇, -N(R₃₇)₂, -SO₃R₃₇, -PO₃(R₃₇)₂, -PO₄(R₃₇)₂, -NO₂,-CF₃, =O, =NR₃₇, and -SR₃₇, and optionally contain one or more heteroatoms selected from the group consisting of O, S, NR₃₇, P, and Si, wherein the N, S, and P atoms are optionally oxidized, wherein the N atoms are optionally quaternized.

In a preferred embodiment the Label is derived from a prosthetic group. The person skilled in the art will understand that a prosthetic group is a precursor that can be radiolabeled with a radionuclide like ¹³¹I thus forming the Label.

In another preferred embodiment, the Label is selected from the group consisting of C₁-C₁₂ alkyl groups, C₂-C₁₂ alkenyl groups, C₂-C₁₂ alkynyl groups, C₆-C₁₂ aryl groups, C₂-C₁₂ heteroaryl groups, C₃-C₁₂ cycloalkyl groups, C₅-C₁₂ cycloalkenyl groups, C₁₂-C₁₂ cycloalkynyl groups, C₃-C₁₂ (cyclo)alkyl(hetero)aryl groups, C₃-C₁₂ (hetero)aryl(cyclo)alkyl, C₄-C₁₂ (cyclo)alkenyl(hetero)aryl groups, C₄-C₁₂ (hetero)aryl(cyclo)alkenyl groups, C₄-C₁₂ (cyclo)alkynyl(hetero)aryl groups, C₄-C₁₂ (hetero)aryl(cyclo)alkynyl groups, C₄-C₁₂ alkylcycloalkyl groups, and C₄-C₁₂ cycloalkylalkyl groups; said groups comprise, or are substituted with, at least one isotope selected from the group consisting of ³H, ¹¹C, ¹³N, ¹⁵O, ¹⁸F, ¹⁹F, ⁷⁵Br, ⁷⁶Br, ⁷⁷Br, ^{8O}Br, ⁸²Br, ¹²⁰I ¹²³I ¹²⁴I ¹²⁵I ³²P, ³³P, ¹³¹I and ²¹¹At;
and are optionally further substituted with a moiety selected from the group consisting of -Cl, -F, -Br, -I, -OR₃₇, -N(R₃₇)₂, -SO₃R₃₇, -PO₃(R₃₇)₂, -PO₄(R₃₇)₂, -NO₂,-CF₃, =O, =NR₃₇, and -SR₃₇, and optionally contain one or more heteroatoms selected from the group consisting of O, S, NR₃₇, P, and Si, wherein the N, S, and P atoms are optionally oxidized, wherein the N atoms are optionally quaternized.

In a preferred embodiment the Label comprises a chelating moiety.

In a preferred embodiment, the Label is a chelating moiety selected from the group consisting of conjugates of DTPA (diethylenetriaminepentaacetic acid), DOTA (1,4,7,10- tetraazacyclododecane-N,N',N",N"-tetraacetic acid), DOTAGA anhydride (2,2',2"-(10-(2,6-dioxotetrahydro-2H-pyran-3-yl)-1,4,7,10-tetraazacyclododecane-1,4,7-triyl)triacetic acid), NOTA (1,4,7-triazacyclononane-N,N',N"-triacetic acid), TETA (1,4,8,11-tetraazacyclotetradecane-N,N',N",N'-tetraacetic acid), OTTA (N1-(*p*-isothiocyanatobenzyl)-diethylenetriamine-N₁,N₂,N₃,N₃-tetraacetic acid), deferoxamine or DFO (N'-[5-[[4-[[5-(acetylhydroxyamino)pentyl]amino]-1,4-dioxobutyl]hydroxyamino]pentyl]-N-(5-aminopentyl)-N-hydroxybutanediamide), and the DFO derivative called DFO*, and HYNIC (hydrazinonicotinamide); and the chelating moiety chelates a metal selected from the group consisting of ⁴⁴Sc, ⁵¹Cr, ⁵²Fe, ⁵²Mn, ⁵⁵Co, ⁶⁰Cu, ⁶¹Cu, ⁶²Zn, ⁶²Cu, ⁶³Zn, ⁶⁴Cu, ⁶⁶Ga, ⁶⁷Ga, ⁶⁸Ga, ⁷⁰As, ⁷¹As, ⁷²As, ⁷⁴As, ⁷⁵Se, ⁸²Rb, ⁸⁶Y, ⁸⁸Y, ⁸⁹Sr, ⁸⁹Zr, ⁹⁷Ru, ^{99m}Tc, ¹¹⁰In, ¹¹¹In ¹¹³In, ¹¹⁴In, ¹¹⁷Sn, ¹²²Xe, ¹⁶⁶Ho, ¹⁶⁷Tm, ¹⁶⁹Yb, ¹⁹³Pt, ¹⁹⁵Pt, ²⁰¹Tl, ²⁰³Pb, ²⁴Na, ⁴⁷Sc, ⁵⁹Fe, ⁶⁷Cu, ⁷⁶As, ⁷⁷As, ⁸⁹Sr, ⁹⁰Nb, ⁹⁰Y, ¹⁰³Ru, ¹⁰⁵Rh, ¹⁰⁹Pd, ¹¹¹Ag ¹¹¹In ¹²¹Sn, ¹²⁷Te, ¹⁴⁰La, ¹⁴¹Ce, ¹⁴²Pr, ¹⁴³Pr, ¹⁴⁴Pr, ¹⁴⁹Pm, ¹⁴⁹Tb, ¹⁵¹Pm, ¹⁵³Sm, ¹⁵⁹Gd, ¹⁶¹Tb, ¹⁶⁵Dy, ¹⁶⁶Dy, ¹⁶⁶Ho, ¹⁶⁹Er, ¹⁷²Tm, ¹⁷⁵Yb, ¹⁷⁷Lu, ¹⁸⁶Re, ¹⁸⁸Re, ¹⁹⁸Au, ¹⁹⁹Au, ²¹¹At, ²¹¹Bi, ²¹²Bi, ²¹²Pb, ²¹³Bi, ²¹⁴Bi, ²²³Ra, ²²⁴Ra, ²²⁵Ac, and ²²⁷Th.

In a preferred embodiment the metal chelate comprises an acyclic derivative of ethylenediaminotetraacetic acid (EDTA) or diethylenediaminotetraacetic acid (DTPA): 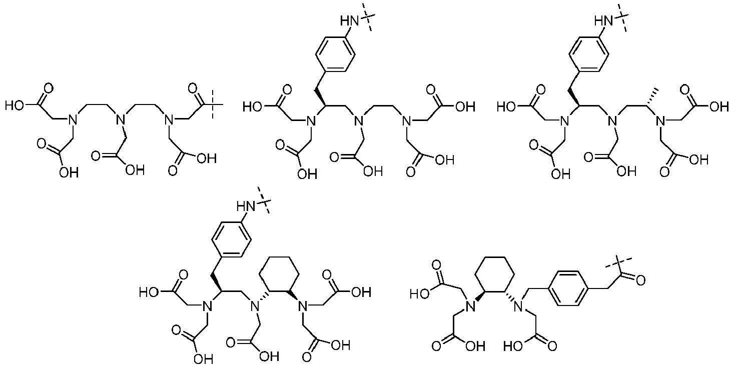 , wherein the dashed line denotes a bond to the rest of the molecule.

In another preferred embodiment the metal chelate comprises an acyclic chelator containing carboxy-pyridine groups: 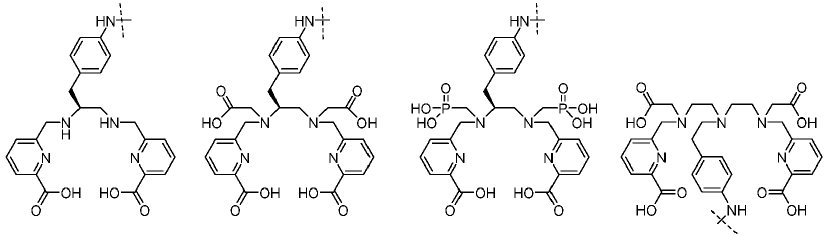 , wherein the dashed line denotes a bond to the rest of the molecule.

In another preferred embodiment the metal chelate comprises a cyclic derivative of 1,4,7,10-tetraazadodecane (cyclen): 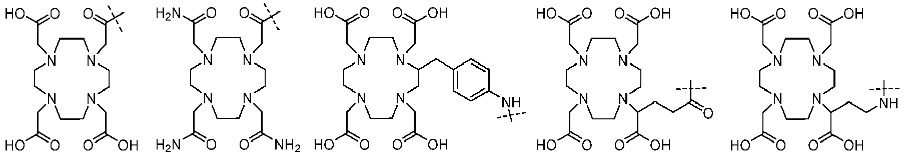 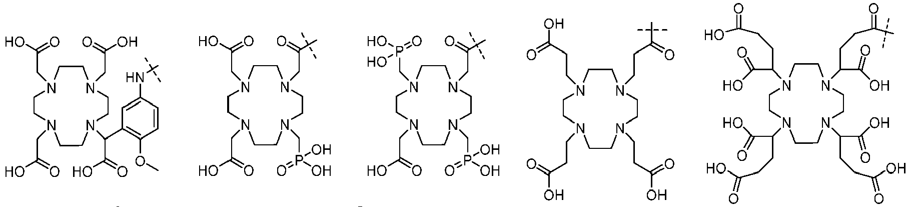 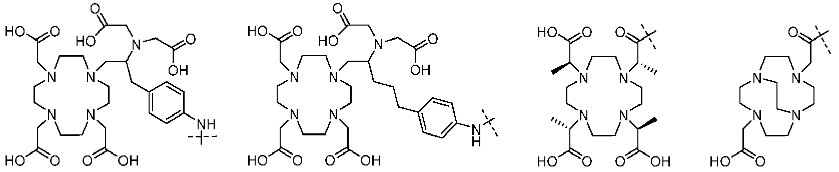 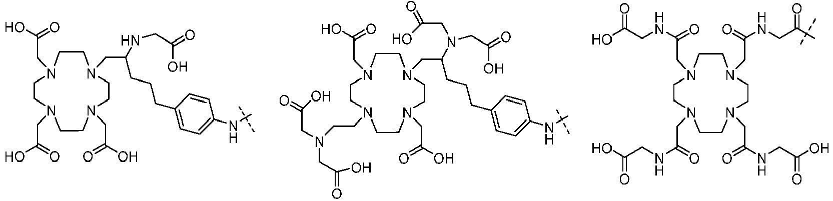 , wherein the dashed line denotes a bond to the rest of the molecule.

In another preferred embodiment the metal chelate comprises a derivative of 1,4,7-triazacyclononane (TACN): 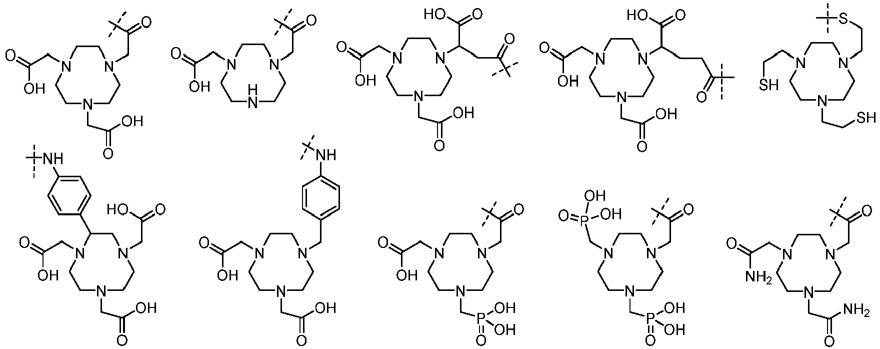 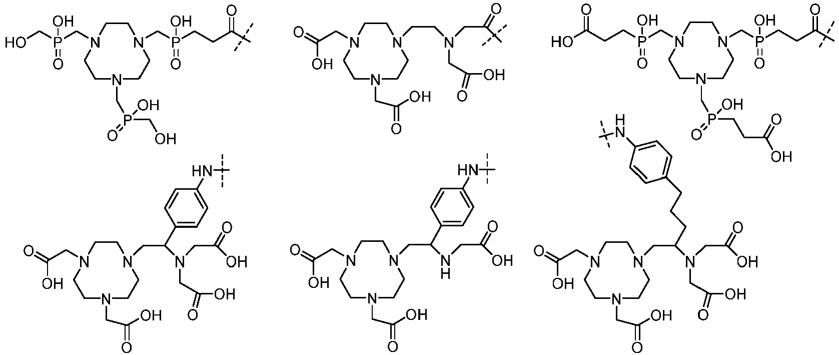 , wherein the dashed line denotes a bond to the rest of the molecule.

In yet another preferred embodiment the metal chelate comprises a macrocyclic chelator containing N and O heteroatoms: 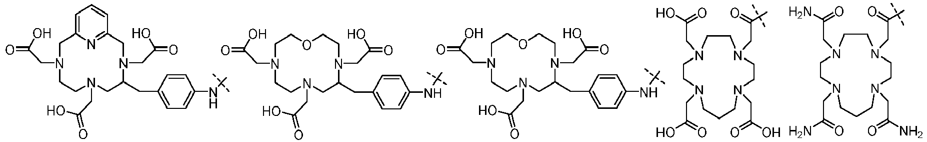 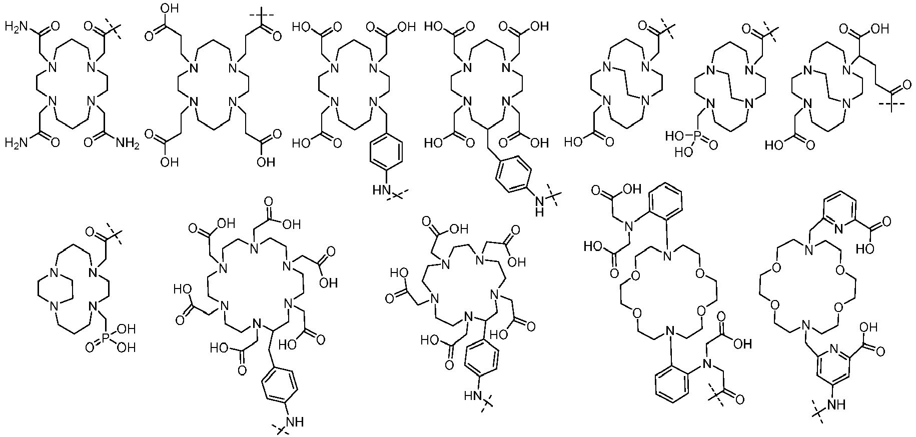 , wherein the dashed line denotes a bond to the rest of the molecule.

In another preferred embodiment the metal chelate comprises a derivative of the cryptand agent sarcophagine (Sar): 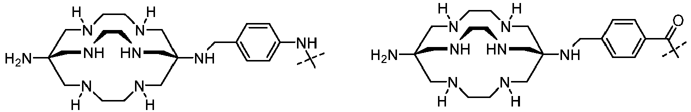 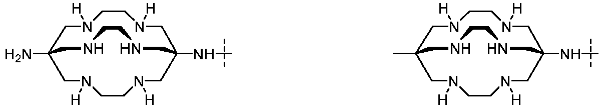 , wherein the dashed line denotes a bond to the rest of the molecule.

In another preferred embodiment the metal chelate comprises a linear or cyclic chelator containing hydroxamate groups: 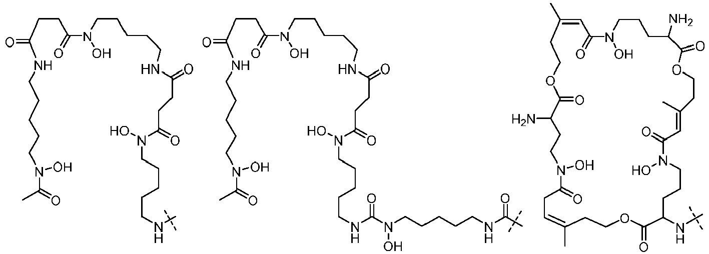 , wherein the dashed line denotes a bond to the rest of the molecule.

In yet another preferred embodiment the metal chelate comprises a linear or cyclic chelator containing 3-hydroxy-4-pyridinone (3,4-HOPO) groups: 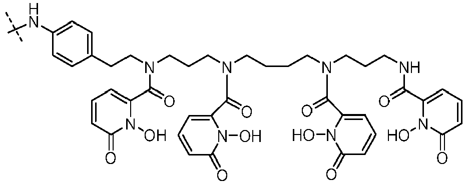 , wherein the dashed line denotes a bond to the rest of the molecule.

In another preferred embodiment the metal chelate comprises a linear or cyclic chelator containing N, S and P heteroatoms: 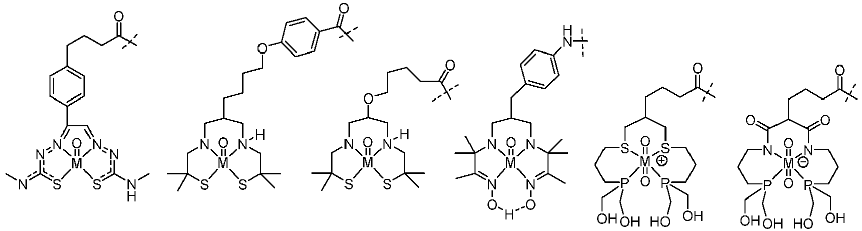 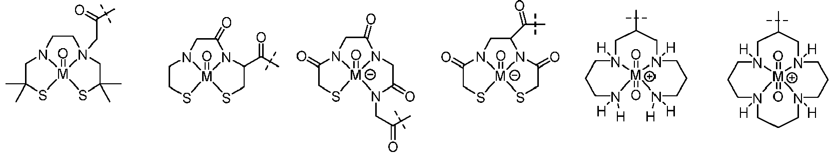 , wherein the dashed line denotes a bond to the rest of the molecule M denotes a radionuclide selected from the group consisting of ^{99m}Tc, ¹⁸⁶Re, and ¹⁸⁸Re.

In another preferred embodiment the metal chelate comprises glycine, serine, cysteine, lysine and alanine residues: 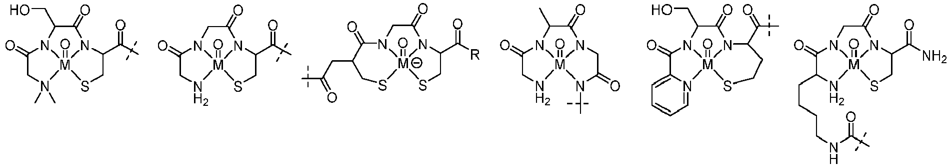 , wherein the dashed line denotes a bond to the rest of the molecule M denotes a radionuclide selected from the group consisting of ^{99m}Tc, ¹⁸⁶Re, and ¹⁸⁸Re.

In another preferred embodiment the metal chelate contains hydrazinonicotinic acid (HYNIC) and a co-ligand: 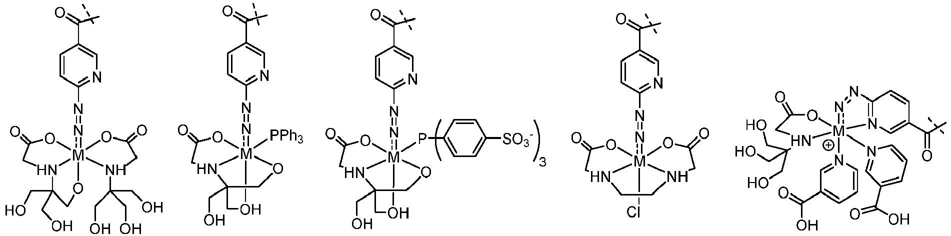 , wherein the dashed line denotes a bond to the rest of the molecule M denotes a radionuclide selected from the group consisting of ^{99m}Tc, ¹⁸⁶Re, and ¹⁸⁸Re.

In another preferred embodiment the chelate comprises carbonyl groups and a chelator containing N, O and S heteroatoms or a cyclopentadienyle: 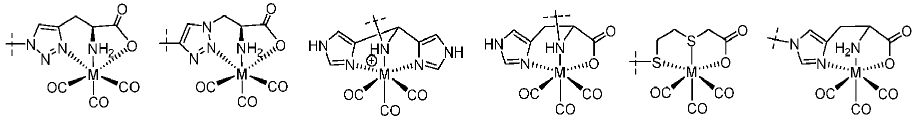 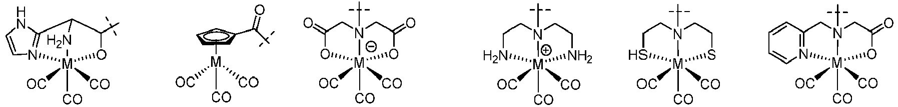 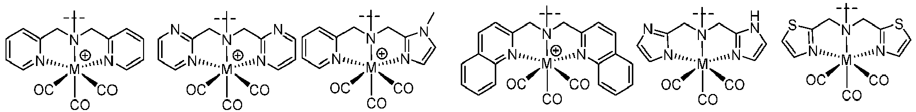 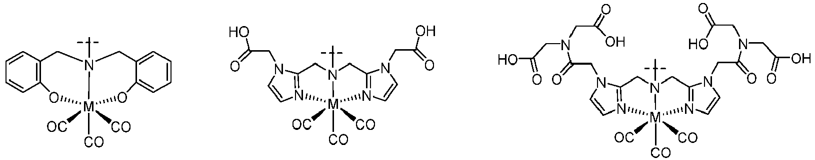 , wherein the dashed line denotes a bond to the rest of the molecule M denotes a radionuclide selected from the group consisting of ^{99m}Tc, ¹⁸⁶Re, and ¹⁸⁸Re.

In some preferred embodiments of the present invention the label contains ¹⁸F and can be produced by the skilled person on the basis of known synthesis routes using known labeled synthons or prosthetic groups. Several non limiting examples of ¹⁸F-containing labels are depicted below: 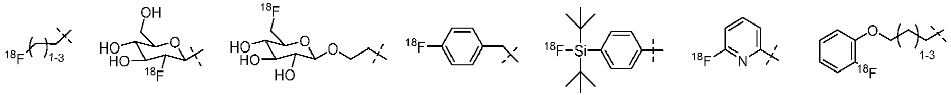 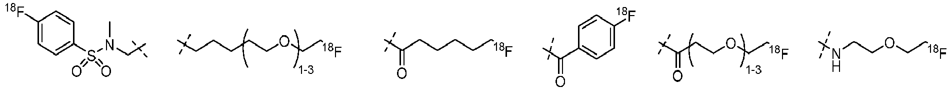 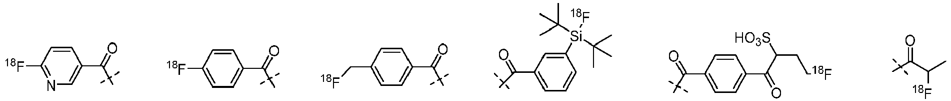 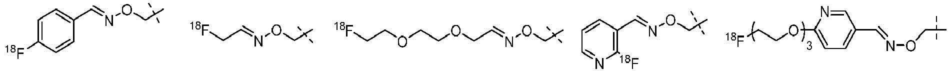 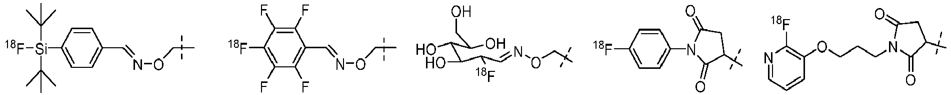 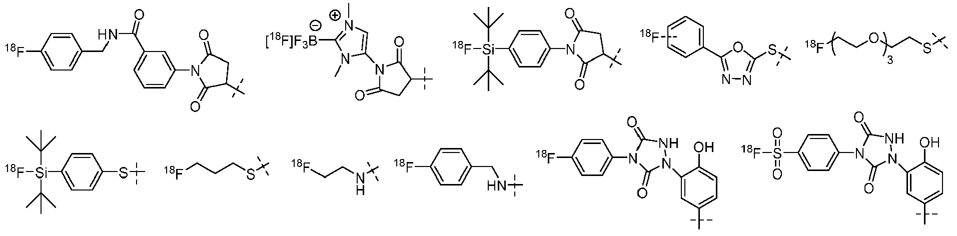 , wherein the dashed line denotes a bond to the rest of the molecule.

In preferred embodiments of the present invention the label contains at least one isotope selected from the group consisting of ¹²³I, ¹²⁴I, ¹²⁵I, ¹³¹I, and ²¹¹At; and is synthesized by the skilled person on the basis of known synthesis routes using prosthetic groups. Several preferred embodiments of such labels are depicted below: 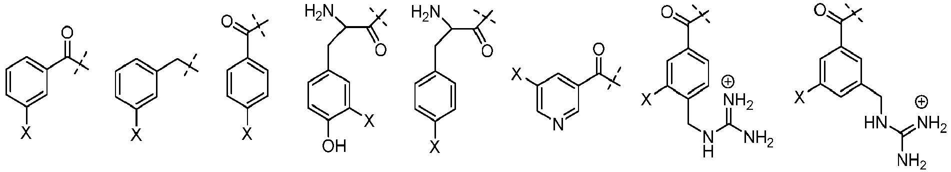 , wherein the dashed line denotes a bond to the rest of the molecule and X denotes ¹²³I, ¹²⁴I, ¹²⁵I, ¹³¹I or ²¹¹At.

In yet another preferred embodiment of the present invention the label contains at least one isotope selected from the group consisting of ¹²³I, ¹²⁴I, ¹²⁵I, ¹³¹I, and ²¹¹At;and is synthetized by the skilled person on the basis of known synthesis routes using a closo-decaborate(2-) group: 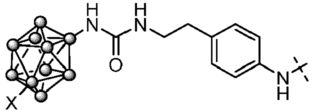 , wherein the dashed line denotes a bond to the rest of the molecule and X denotes ¹²³I, ¹²⁴I, ¹²⁵I, ¹³¹I or ²¹¹At.

### Administration Agent

The Administration Agent is an antibody. The Administration Agent can be any construct of which it is desired to modify it with a Label for radio-imaging or radiotherapy and of which it is desired to remove its imaging or radiotherapy label at a particular time after injection. This particularly is the case in the event of targeted imaging and radiotherapy to a site, such as a tumor, within the body of a subject, notably a human subject. The sole requirement is that it can be provided with a Trigger T^{R}, which is further linker to a Label. The precise linkage of the Trigger to the Administration Agent will depend on the molecular structure of both, but it should be noted that this does not normally present a particular challenge to the person skilled in the art, as many proven conjugation methods and linkage moieties for various biomolecules exist. The linkage can, optionally, be via a spacer such as a polyethylene glycol (PEG) chain.

Typically the Administration Agent can bind to a Primary Target, as defined herein. Said Primary Target can be a target to which a Targeting Agent binds or it can be a therapeutic target upon which a drug has its effect. In a preferred embodiment the Primary Target is a therapeutic target and the Targeting Agent is a drug and binds said Primary Target.

In preferred embodiments, the Administration Agent is a Targeting Agent as defined herein, insofar the Targeting Agent is described as an antibody.

In other preferred embodiments, the Administration Agent equals a Targeting Agent, and the Targeting Agent is radiolabeled with a therapeutic radioisotope in order to target therapeutic radiation to tissues expressing a Primary Target.

In other preferred embodiments, the Administration Agent equals a Targeting Agent, and the Targeting Agent is radiolabeled with a diagnostic radioisotope in order to image tissues expressing a Primary Target.

In preferred embodiments, the Administration Agent is an antibody that comprises an FcRn binding domain, more preferably an intact IgG antibody. Preferably, the Administration Agent is an intact antibody.

In other preferred embodiments, the Administration Agent is an antibody that comprises an albumin-binding moiety.

In other preferred embodiments the Administration Agent equals a drug. In other preferred embodiments the Administration Agent equals a drug and the drug is labeled using the presented invention for the purpose of imaging in vivo drug distribution. Drugs that can be used in an Administration Agent relevant to this invention are pharmaceutically active compounds which are antibodies.

In a preferred embodiment the pharmaceutically active compound or drug is selected from the group consisting of cytotoxins, antiproliferative/antitumor agents, antiviral agents, antibiotics, anti-inflammatory agents, chemosensitizing agents, radiosensitizing agents, immunomodulators, immunosuppressants, immunostimulants, anti-angiogenic factors, and enzyme inhibitors.

In other preferred embodiments the drug is designed to act in the central neural system, for example in the context of Alzheimer's disease and Parkinsons' disease, for example antibodies against beta-amyloid and Tau proteins.

Exemplary immunemodulators are antibodies against PD-L1, PD-1, LAG-3, OX40, TIGIT, TIM-3, B7H4, Vista, CTLA-4, APRIL, GITR, CD3, CD28, CD40, CD74, RIG, MDA-5, NLRP1, NLRP3, AIM2, IDO, MEK, cGAS, and CD25, NKG2A.

It will be understood that chemical modifications may also be made to the Administration Agent in order to make reactions of that compound more convenient for purposes of preparing conjugates of the invention.

In a preferred embodiment, the Administration Agent before conjugation to the remainder of the compound of Formula (1) comprises at least one moiety selected from the group consisting of -OH, -NHR', -CO₂H, -SH, -S-S-, -SCH₃-, -N₃, terminal alkynyl, terminal alkenyl, -C(O)R', C₈-C₁₂ (hetero)cycloalkynyl, nitrone, nitrile oxide, (imino)sydnone, isonitrille, and (oxa)norbornene, tetrazine, wherein R' equals R₃₇, said moiety used for conjugation to a moiety comprising the dienophile, the Label and R₃₂ so as to form the compound satisfying Formula (1), and comprising a C^{M2} or C^{X} moiety.

In preferred embodiments the Administration Agent is bound to the remainder of the compound of Formula (1) via a C^{M2} selected from the group consisting of amine, amide, thioamide, aminooxy, carbamate, thiocarbamate, urea, thiourea, sulfonamide, and sulfoncarbamate.

In preferred embodiments C^{M2} equals R₁₀ as defined herein.

In a preferred embodiment, when the Administration Agent is conjugated via -SH or -S-S-, then C^{M2} is selected from the group consisting of 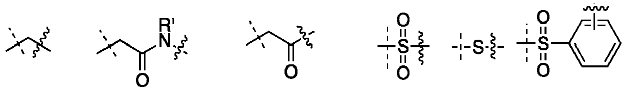 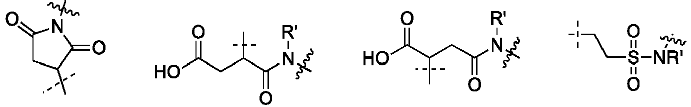 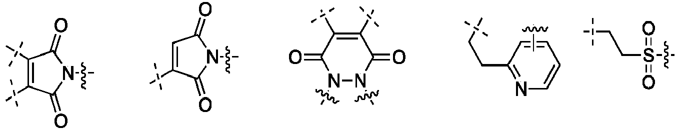 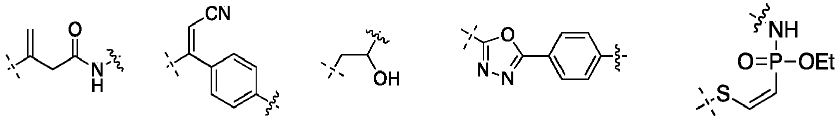 wherein the wiggly line denotes a bond to the remaining part of the molecule, and wherein the dashed line denotes a bond to the Administration Agent, wherein R' equals R₃₇ as defined herein.

In a preferred embodiment, when the Administration Agent is conjugated via -SCH₃- then C^{M2} preferably is: 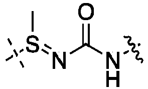 wherein the wiggly line denotes a bond to the remaining part of the molecule, and wherein the dashed line denotes a bond to the Administration Agent.

In a preferred embodiment, when the Administration Agent is conjugated via -NR'-, then C^{M2} is selected from the group consisting of 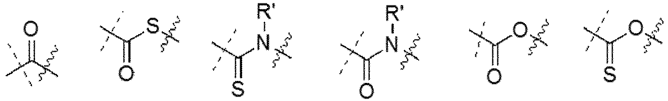 wherein R' equals R₃₇ as defined herein, wherein the wiggly line denotes a bond to the remaining part of the molecule, and wherein the dashed line denotes a bond to the Administration Agent.

In a preferred embodiment, when the Administration Agent is conjugated via -C- derived from a moiety that was -C(O)R' or -C(O)R'-, then C^{M2} is selected from the group consisting of 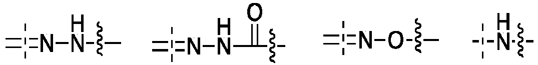 wherein the wiggly line denotes a bond to the remaining part of the molecule, and wherein the dashed line denotes a bond to the Administration Agent.

In a preferred embodiment, when the Administration Agent is conjugated via -C(O)- derived from a moiety that was -C(O)OH, then C^{M2} is selected from the group consisting of 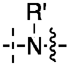 wherein R' equals R₃₇ as defined herein, wherein the wiggly line denotes a bond to the remaining part of the molecule, and wherein the dashed line denotes a bond to the Administration Agent.

In a preferred embodiment, when the Administration Agent is conjugated via -O-, then C^{M2} is selected from the group consisting of 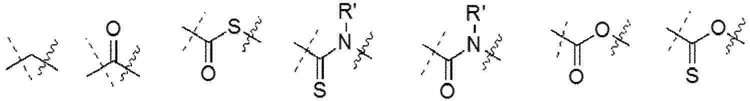 wherein R' equals R₃₇ as defined herein, wherein the wiggly line denotes a bond to the remaining part of the molecule, and wherein the dashed line denotes a bond to the Administration Agent.

In a preferred embodiment, when the Administration Agent is conjugated via -N₃ that was reacted with an R₃₂ that comprised an alkyne group, then the resulting C^{X} comprises a triazole ring, wherein each C^{X} is independently selected from the group consisting of 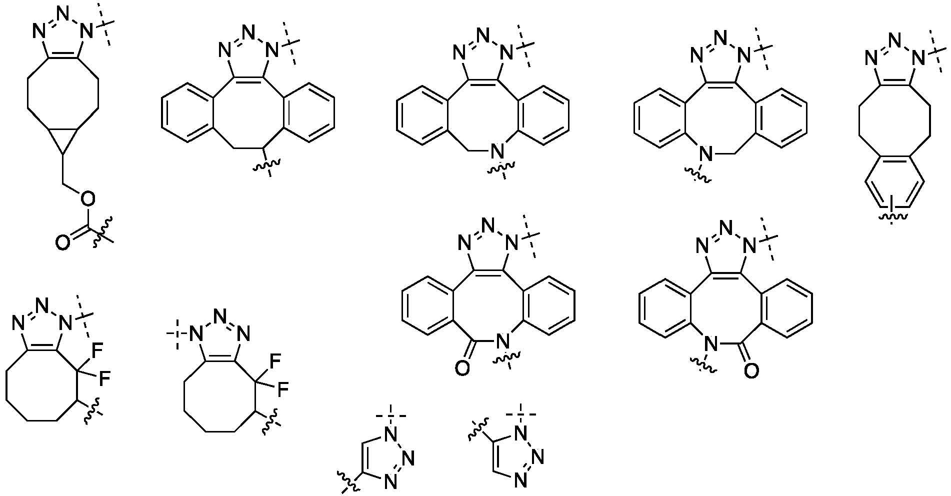 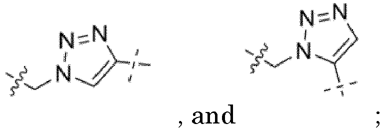 wherein the wiggly line denotes a bond to the remaining part of the molecule, and wherein the dashed line denotes a bond to the Administration Agent.

Preferably, the Administration Agent is modified with further moieties that equal Formula (1), except that these further moieties do not comprise an Administration Agent (as the first mentioned Administration Agent is already coupled to said moiety). In a preferred embodiment, the Administration Agent is coupled to further moieties as defined in this paragraph at 1 to 8 positions, more preferably from 1 to 6 positions, even more preferably at 1 to 4 positions.

### Further embodiments in relation to the compound of Formula (1)

In the compound of the invention the Administration Agent is an antibody.

In the compound of the invention the Label is a radiolabel, preferably a chelating moiety that chelates a radioisotope.

The skilled person is familiar with the fact that the dienophile activity is not necessarily dependent on the presence of all carbon atoms in the ring, since also heterocyclic monoalkenylene eight-membered rings are known to possess dienophile activity. Thus, in general, the invention is not limited to strictly *trans-*cyclooctene. The person skilled in organic chemistry will be aware that other eight-membered ring-based dienophiles exist, which comprise the same endocyclic double bond as the *trans*-cyclooctene, but which may have one or more heteroatoms elsewhere in the ring. I.e., the invention generally pertains to eight-membered non-aromatic cyclic alkenylene moieties, preferably a cyclooctene moiety, and more preferably a *trans*-cyclooctene moiety.

*Trans*-cyclooctene or E-cyclooctene derivatives are very suitable as Triggers, especially considering their high reactivity. Optionally, the *trans-*cyclooctene (TCO) moiety comprises at least two exocyclic bonds fixed in substantially the same plane, preferably as described in WO 2012/156919A1, and/or it optionally comprises at least one substituent in the axial position, and not the equatorial position. The person skilled in organic chemistry will understand that the term "fixed in substantially the same plane" refers to bonding theory according to which bonds are normally considered to be fixed in the same plane. Typical examples of such fixations in the same plane include double bonds and strained fused rings. E.g., the at least two exocyclic bonds can be the two bonds of a double bond to an oxygen (i.e. C=O). The at least two exocyclic bonds can also be single bonds on two adjacent carbon atoms, provided that these bonds together are part of a fused ring (i.e. fused to the TCO ring) that assumes a substantially flat structure, therewith fixing said two single bonds in substantially one and the same plane. Examples of the latter include strained rings such as cyclopropyl and cyclobutyl. Without wishing to be bound by theory, the inventors believe that the presence of at least two exocyclic bonds in the same plane will result in an at least partial flattening of the TCO ring, which can lead to higher reactivity in the IEDDA reaction. A background reference providing further guidance is WO 2012/153254. The at least two exocyclic bonds fixed in substantially the same plane are preferably as described on page 16 and further of WO 2012/156919 A1.

TCO moieties may consist of multiple isomers, also comprising the equatorial *vs*. axial positioning of substituents on the TCO. In this respect, reference is made to Whitham et al. J. Chem. Soc. (C), 1971, 883-896, describing the synthesis and characterization of the equatorial and axial isomers of *trans-*cyclo-oct-2-en-ol, identified as (1RS, 2RS) and (1SR, 2RS), respectively. In these isomers the OH substituent is either in the equatorial or axial position. Without wishing to be bound by theory, the inventors believe that the presence of an axial substituent increases the TCO ring strain resulting in higher reactivity in the IEDDA reaction. A background reference providing further guidance is WO 2012/049624.

Furthermore, in case of allylic substituents on the TCO in some embodiments it is preferred that these are positioned axially and not equatorially.

It should be noted that, depending on the choice of nomenclature, the TCO dienophile may also be denoted E-cyclooctene. With reference to the conventional nomenclature, it will be understood that, as a result of substitution on the cyclooctene ring, depending on the location and molecular weight of the substituent, the same cyclooctene isomer may formally become denoted as a Z-isomer. In the present invention, any substituted variants of the invention, whether or not formally "E" or "Z," or "cis" or "trans" isomers, will be considered derivatives of unsubstituted *trans*-cyclooctene, or unsubstituted E-cyclooctene. The terms "*trans*-cyclooctene" (TCO) as well as E-cyclooctene are used interchangeably and are maintained for all dienophiles according to the present invention, also in the event that substituents would formally require the opposite nomenclature. I.e., the invention relates to cyclooctene in which carbon atoms 1 and 6 as numbered below are in the E *(entgegen)* or *trans* position.

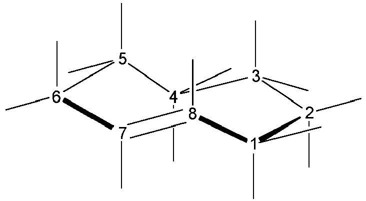

The dienophiles for use in the invention can be synthesized by the skilled person, on the basis of known synthesis routes to cyclooctenes and corresponding hetero atom(s)-containing rings. The skilled person further is aware of the wealth of cyclooctene derivatives that can be synthesized via the ring closing metathesis reaction using Grubbs catalysts. As mentioned above, the TCO possibly includes one or more heteroatoms in the ring. This is as such sufficiently accessible to the skilled person [e.g. WO2016025480], Reference is made, e.g., to the presence of a thioether in TCO: [Cere et al. J. Org. Chem. 1980, 45, 261]. Also, e.g., an - O-SiR₂-O moiety in TCO: [Prevost et al. J. Am. Chem. Soc. 2009, 131, 14182]. References to TCO syntheses wherein the allylic positioned leaving group (R₄₈) is an ether, ester, carbonate, carbamate or a thiocarbamate are: [Versteegen et al Angew. Chem. Int. Ed. 2018, 57, 10494], and [Steiger et al Chem Comm 2017, 53, 1378]. Exemplary TCOs include the following structures, indicated below with literature references. Where a cyclooctene derivative is depicted as a Z-cyclooctene it is conceived that this can be converted to the E-cyclooctene analog.

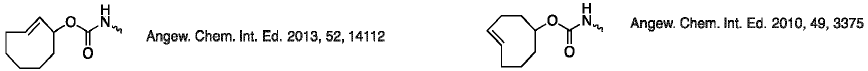

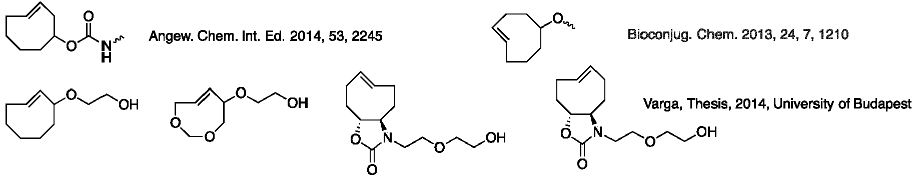

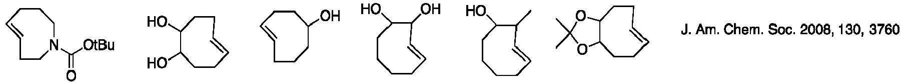

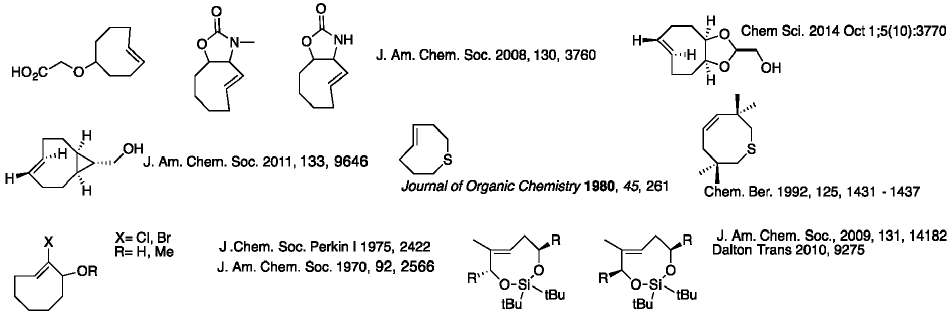

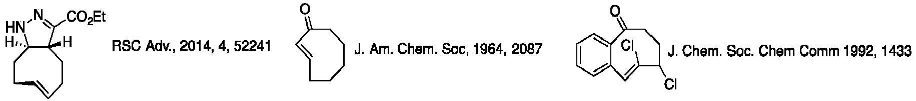

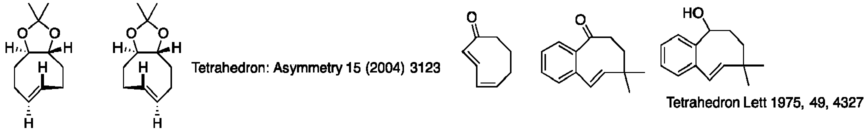

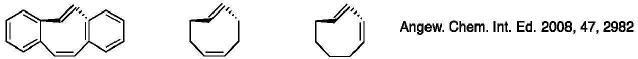

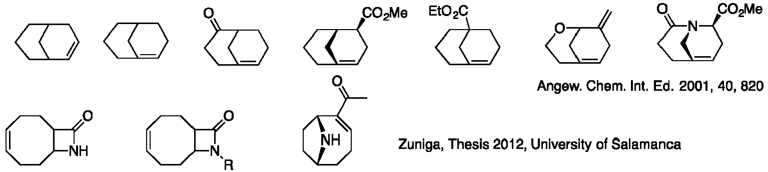

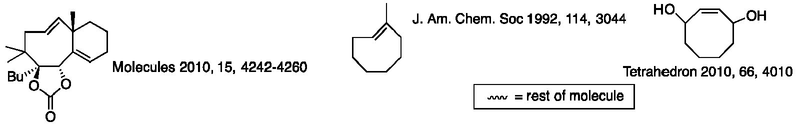

In a preferred embodiment, the compound of Formula (1) satisfies any one of the following structures:

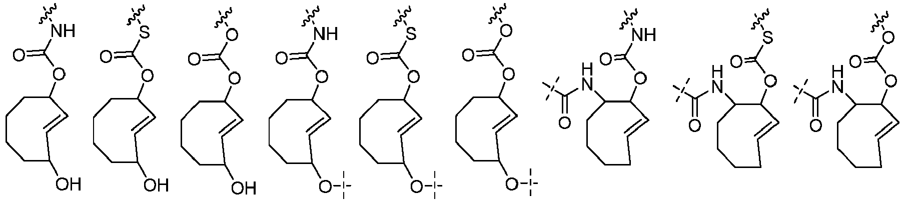

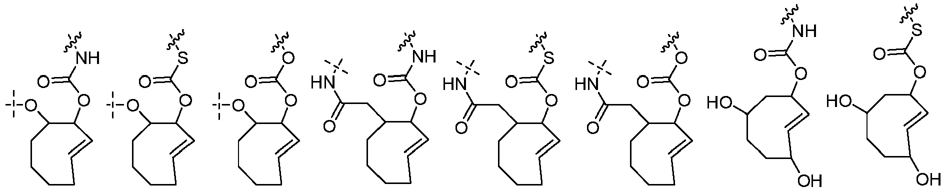

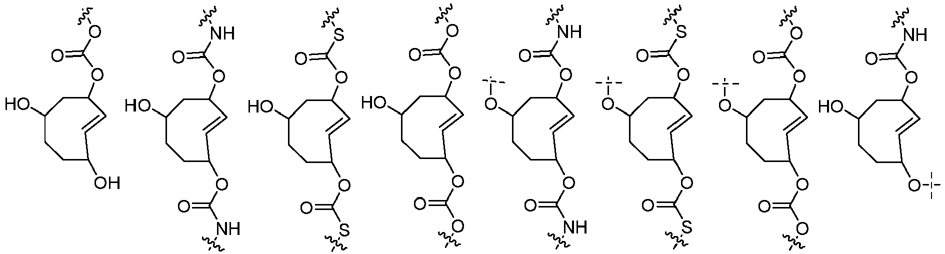

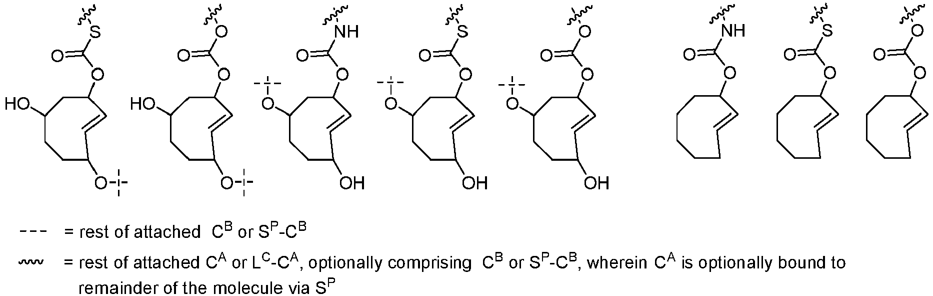

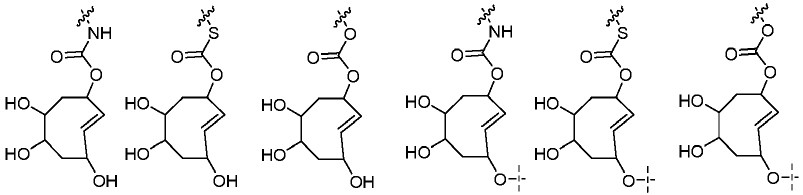

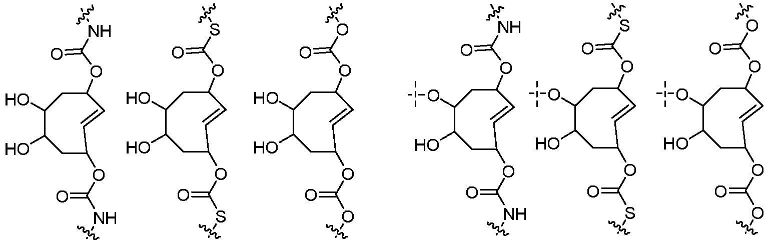

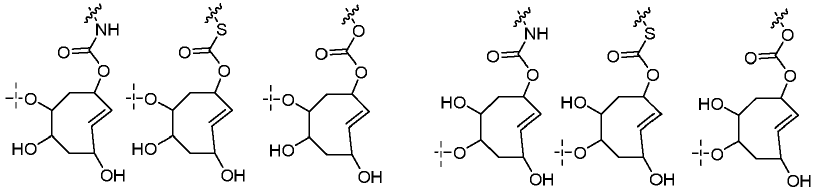

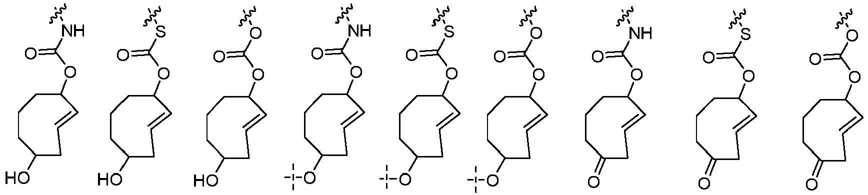

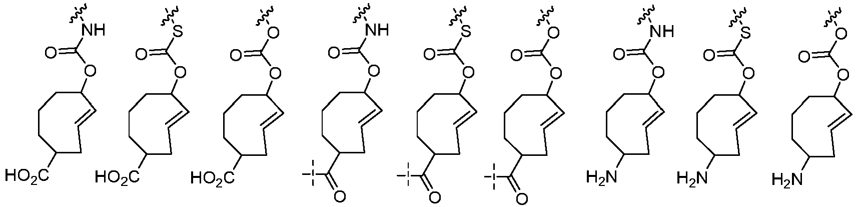

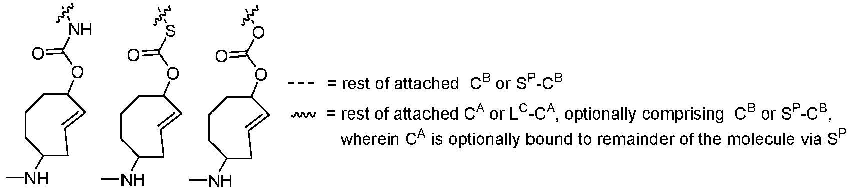

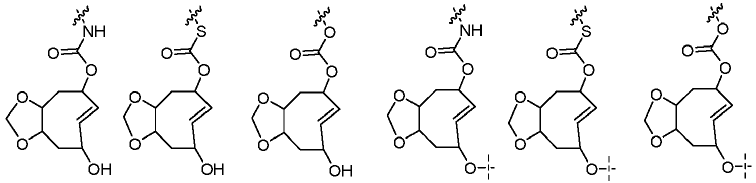

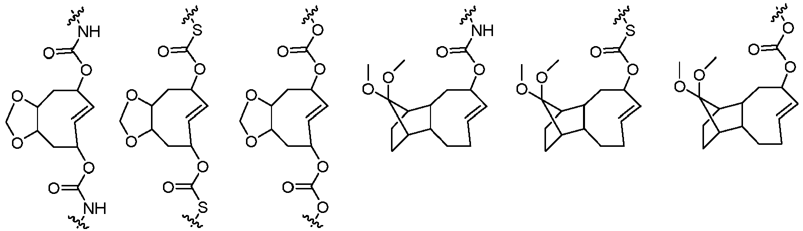

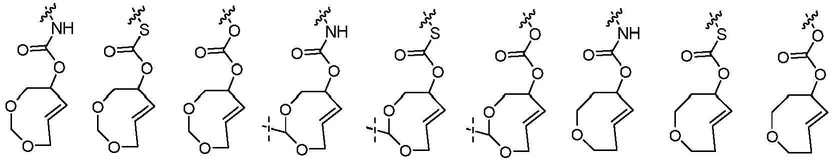

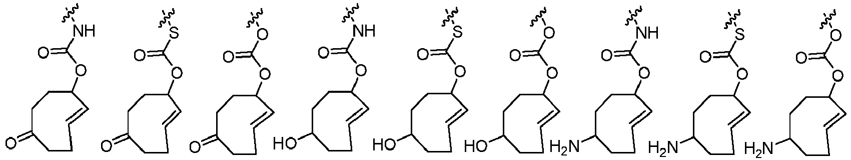

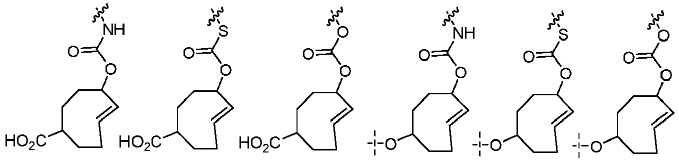

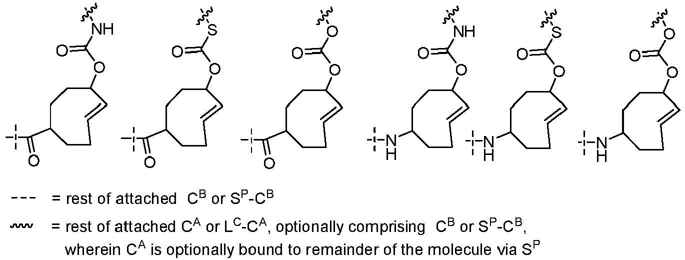

In a preferred embodiment, the compound of Formula (1) is any one of the racemic and enantiomerically pure compounds listed below: 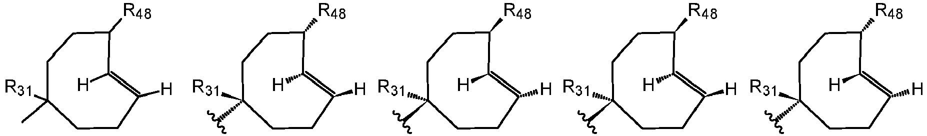 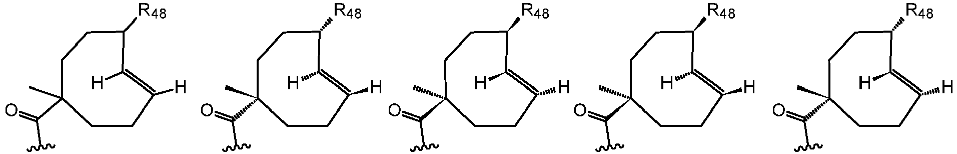 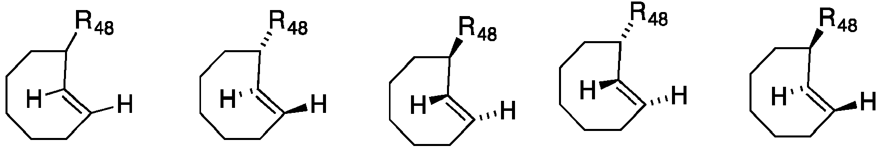 wherein the wiggly line indicates a bond to the remainder of the compound of Formula (1).

Especially preferred compounds of Formula (1) are the enantiomerically pure compounds listed below: 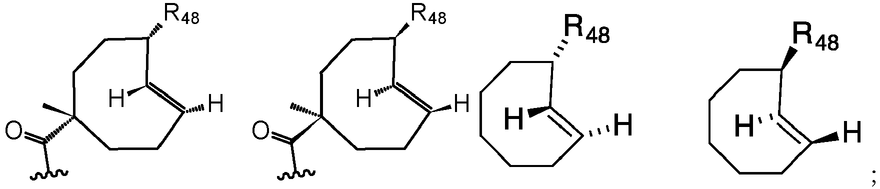 wherein the wiggly line indicates a bond to the remainder of the compound of Formula (1).

Other preferred compounds of Formula (1) are:

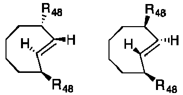

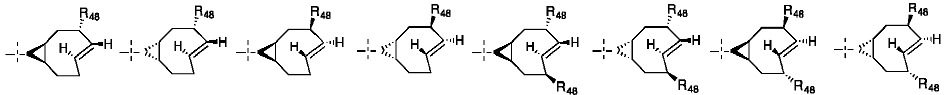

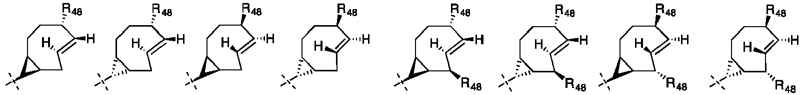

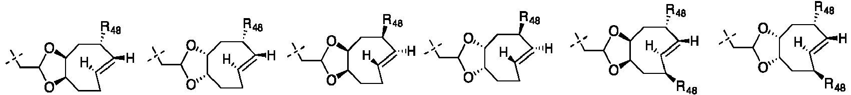

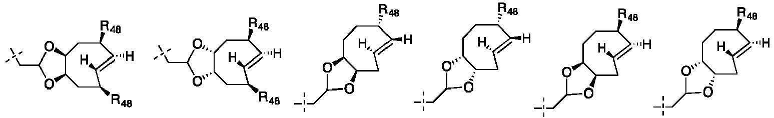

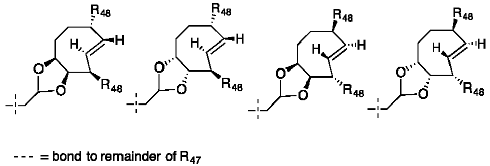

In preferred embodiments, L^{A} not being R₄₈, L^{B} not being R₄₈, the Label, the Administration Agent, R₉₈, and/or C^{C} are bound to the remainder of the compound of Formula (1) via a residue of R₃₂, or a moiety C^{M2} or C^{X} as defined herein, wherein preferably said residue of R₃₂ or a moiety C^{M2} or C^{X} equals or is comprised in a Spacer. A person skilled in the art will understand that "residue of R₃₂" means the conjugation reaction product of R₃₂ with another chemical group so as to form a conjugate, for example between C^{C} with the remainder of the compound according to Formula (1).

In preferred embodiments, L^{A} not being R₄₈, L^{B} not being R₄₈, the Label, the Administration Agent, R₉₈, and/or C^{C} are bound to the remainder of the molecule via C^{M2} as defined herein, wherein preferably C^{M2} equals or is comprised in a Spacer.

In yet other preferred embodiments, L^{A} not being R₄₈, L^{B} not being R₄₈, the Label, the Administration Agent, R₉₈, and/or C^{C} are bound to the remainder of the molecule via C^{X} as defined herein, wherein preferably C^{X} equals or is comprised in a Spacer.

In preferred embodiments, moiety C^{X}, C^{M2} and the said residue of R₃₂ are comprised in L^{A} not being R₄₈, L^{B} not being R₄₈, the Label, the Administration Agent, R₉₈, and/or C^{C}.

### C^{M2}

In preferred embodiments, C^{M2} is selected from the group consisting of amine, amide, thioamide, aminooxy, carbamate, thiocarbamate, urea, thiourea, ether, sulfonamide, and sulfoncarbamate. In preferred embodiments C^{M2} equals R₁₀.

In preferred embodiments, C^{M2} is: 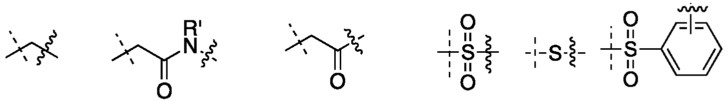 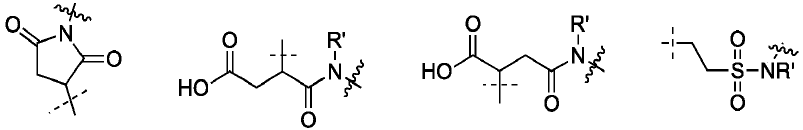 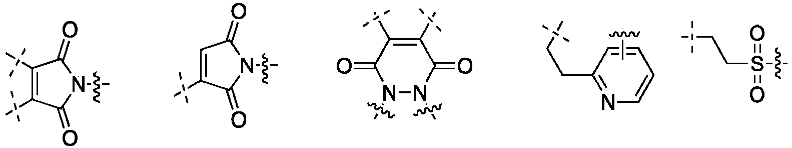 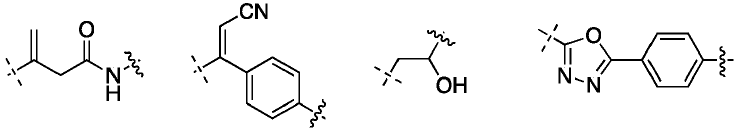 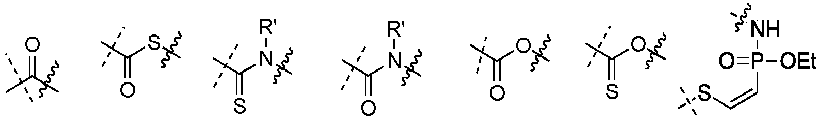 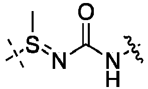 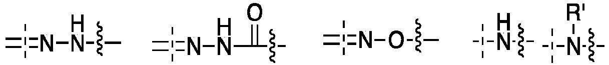 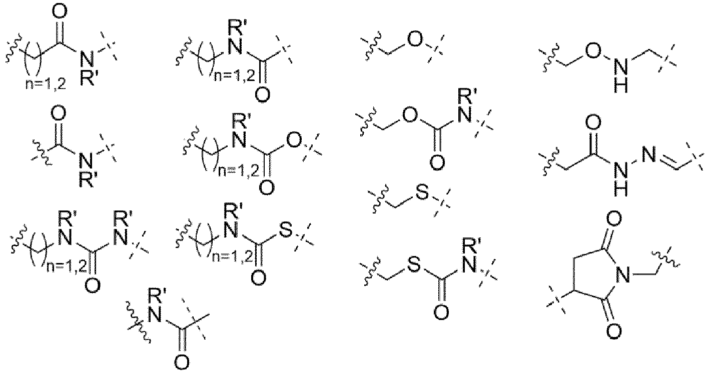 wherein R' equals R₃₇ as defined herein, wherein the dashed line denotes a bond to or towards L^{A} not being R₄₈, L^{B} not being R₄₈, the Label, the Administration Agent, S^{P}, L^{C}, R₉₈, or C^{C} and the wiggly line denotes a bond to the remaining part of the dienophile. In preferred embodiments the wiggly line denotes a bond to or towards L^{A} not being R₄₈, L^{B} not being R₄₈, the Label, the Administration Agent, S^{P}, L^{C}, R₉₈, or C^{C} and the dashed line denotes a bond to the remaining part of the dienophile.

### C^{X}

In preferred embodiments, C^{X} is: 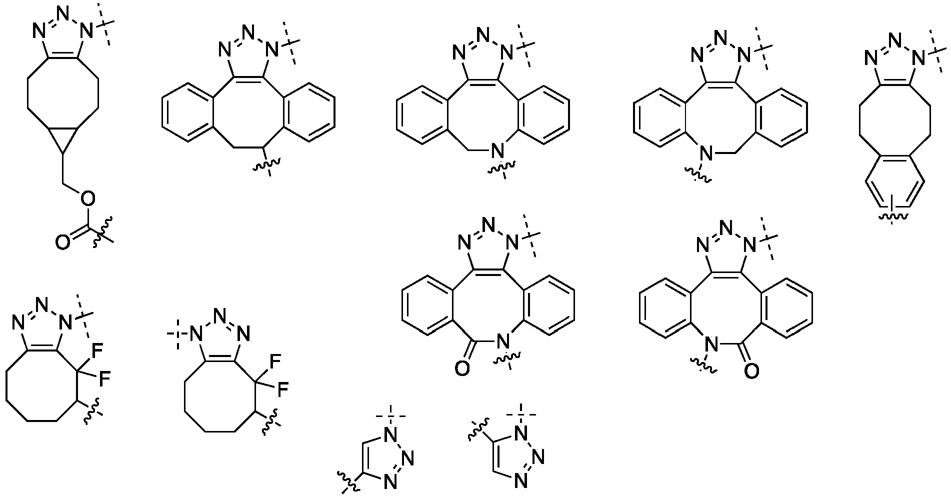 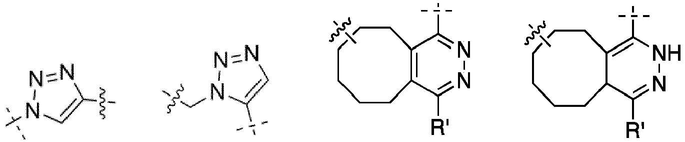 wherein the dashed line denotes a bond to or towards L^{A} not being R₄₈, L^{B} not being R₄₈, the Label, the Administration Agent, S^{P}, L^{C}, R₉₈, or C^{C} and the wiggly line denotes a bond to the remaining part of the dienophile. In preferred embodiments the wiggly line denotes a bond to or towards L^{A} not being R₄₈, L^{B} not being R₄₈, the Label, the Administration Agent, S^{P}, L^{C}, R₉₈, or C^{C} and the dashed line denotes a bond to the remaining part of the dienophile.

With reference to above schemes with examples of C^{M2} and C^{X}, in some embodiments it is preferred that when L^{A} not being R₄₈, L^{B} not being R₄₈, the Label, the Administration Agent, R₉₈, or C^{C} is a protein, such as an antibody, that the dashed line denotes a bond to or towards L^{A} not being R₄₈, L^{B} not being R₄₈, the Label, the Administration Agent, R₉₈, or C^{C}.

### Intermediates for the preparation of compounds of Formula (1)

Preferred intermediates to prepare compounds of Formula (1) of the invention are listed below. Particularly preferred intermediates from the below are enantiomerically pure compounds A-F, in particular A, D, E, F. A person skilled in the art will understand that compounds E and F still need to be isomerized to E-cyclooctenes, after which the enantiomer with the axial OH can be separated from the enantiomer with the equatorial OH as described by Rossin et al Bioconj.Chem., 2016 27(7):1697-1706.

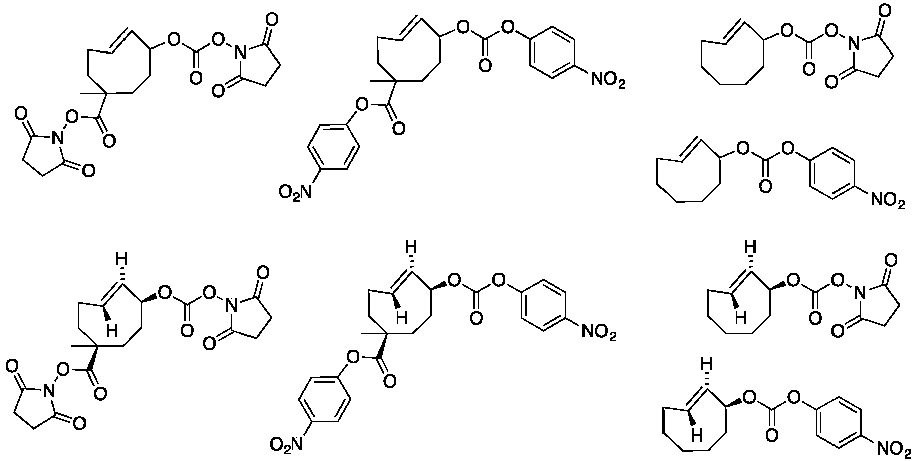

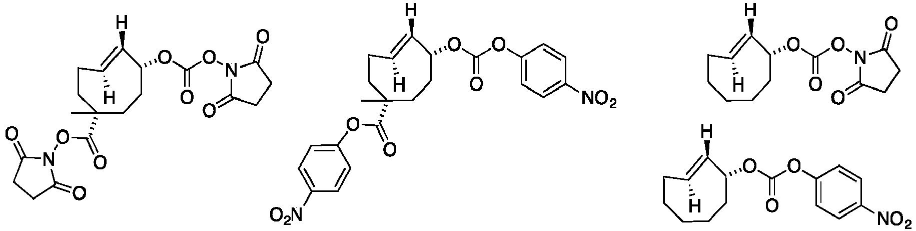

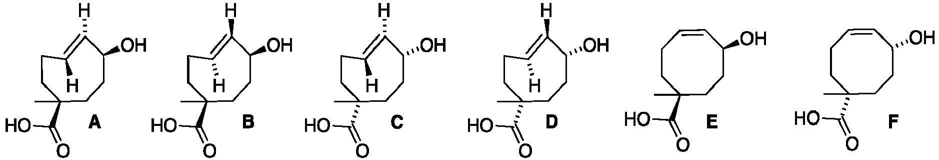

A general synthesis method of a TCO trigger and its corresponding compound of Formula (1)s is shown directly below. The synthesis method is as reported in Rossin et al Nature Communications 2018, 9, 1484 and Rossin et al Bioconj.Chem., 2016 27(7):1697-1706 with the exception of the conversion of D to F, which now is conducted by mixing D with hydroxide solution in methanol, followed by evaporation and reaction with iodomethane. Please note that for sake of clarity only one of the two enantiomers of E-K is shown. A person skilled in the art will understand that the enantiomers can be separated at various stages in the synthesis using established chiral resolution methods to obtain enantiomerically pure B, E, F, H, for example, such as chiral salts. 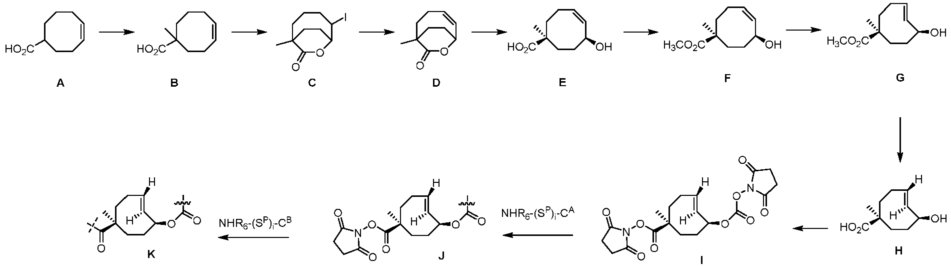 wherein the wiggly line denotes the bond to the remainder of R₄₈ and the dashed line denotes the bond to the remainder of the molecule.

### Cleaving Agent

The compound used to release one or more moieties R₄₈ from the structure of Formula (1) is herein referred to as "Cleaving Agent".

In a preferred embodiment, the combination of the invention comprises a Cleaving Agent with the proviso that when at least one R₄₈ in Formula (1) is L^{B}, then the Cleaving Agent does not comprise the Label of L^{B}; with the proviso that when at least one R₄₈ in Formula (1) is L^{A}, then the Cleaving Agent does not comprise the Administration Agent of L^{A};
wherein the Cleaving Agent is a diene.

In a preferred embodiment, the Cleaving Agent does not comprise a Label.

In a preferred embodiment, the Cleaving Agent does not comprise an Administration Agent.

In preferred embodiments wherein the Primary Target is an internalizing receptor and it is desired to selectively cleave the Label in blood and not at the Primary Target, the Cleaving Agent is preferentially designed to be cell impermeable. In said embodiment, wherein the Label is a chelate then the Cleaving Agent can be either internalizing or non-internalizing ,as the cleaved chelate cannot escape the target cell.

In preferred embodiments, wherein the Primary Target is a non internalizing receptor and it is desired to cleave the Label in blood and not at the Primary Target, the Cleaving Agent is preferentially designed to extravasate poorly into tissues and rapidly clear, to minimize reaction at the Primary Target while achieving cleavage in blood.

In a preferred embodiment, the combination of the invention is a kit.

In some embodiments, preferably, the Cleaving Agent has a molecular weight of at most 150 kDa, when at least one R₄₈ in Formula (1) is L^{A} that does not comprise a Label; more preferably at most 70 kDa, most preferably at most 1 kDa.

In other embodiments, preferably, the Cleaving Agent has a molecular weight of at least 300 kDa, to minimize extravasation.

In preferred embodiments the Cleaving Agent is cell-impermeable, to further increase the selective cleavage of a compound of Formula (1) present in non-target sites (e.g. blood).

In preferred embodiments, the properties of the Cleaving Agent (e.g. level of cell permeability and extravasation) are achieved by a suitably chosen R⁸⁷ group (vide infra), comprised in the Clearing Agent formulas below.

Preferably, the reaction of the Cleaving Agent with the Administration Agent present in a tissue of interest in vivo results in at least 20 % reduction of radioactivity, more preferably at least 40 %, more preferably at least 60 %, even more preferably at least 80 %.

In a preferred embodiment, the Cleaving Agent is a tetrazine. Tetrazines are dienes and are highly reactive towards dienophiles, especially the TCO constructs *(vide supra*)*.* The diene of the Cleaving Agent is selected so as to be capable of reacting with the dienophile, e.g. the TCO, by undergoing a Diels-Alder cycloaddition followed by a retro Diels-Alder reaction, giving the IEDDA adduct. This intermediate adduct then releases the Construct-A.

Synthesis routes to tetrazines in general are readily available to the skilled person, based on standard knowledge in the art. References to tetrazine synthesis routes include for example Lions et al, J. Org. Chem., 1965, 30, 318-319; Horwitz et al, J. Am. Chem. Soc., 1958, 80, 3155-3159; Hapiot et al, New J. Chem., 2004, 28, 387-392, Kaim et al, Z. Naturforsch., 1995, 50b, 123-127, Yang et al., Angew. Chem. 2012, 124, 5312 -5315; Mao et al., Angew. Chem. Int. Ed. 2019, 58, 1106-1109; Qu et al. Angew. Chem. Int. Ed. 2018, 57, 12057 -12061; Selvaraj et al., Tetrahedron Lett. 2014, 55, 4795-4797; Fan et al., Angew. Chem. Int. Ed. 2016, 55, 14046-14050.

Preferably, the Cleaving Agent is a tetrazine satisfying Formula (4) and preferably including pharmaceutically accepted salts thereof: 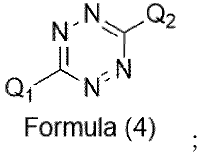 wherein
each moiety Q₁ and Q₂ is independently selected from the group consisting of hydrogen, -F, -Cl, -Br, -I, -OR₃₇, -N(R₃₇)₂, -SO₃, -PO₃⁻, -NO₂, -CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, - S(O)₂R₃₇, NR₃₇S(O)₂R₃₇, -ON(R₃₇)₂, ₋NR₃₇OR₃₇, alkyl groups, alkenyl groups, alkynyl groups, aryl groups, heteroaryl groups, cycloalkyl groups, cycloalkenyl groups, cycloalkynyl groups, (cyclo)alkyl(hetero)aryl groups, (hetero)aryl(cyclo)alkyl, (cyclo)alkenyl(hetero)aryl groups, (hetero)aryl(cyclo)alkenyl groups, (cyclo)alkynyl(hetero)aryl groups, (hetero)aryl(cyclo)alkynyl groups, alkylcycloalkyl groups, and cycloalkylalkyl groups.

In Formula (4), the Q₁ and Q₂ groups not being H, -F, -Cl, -Br, -I, -OH, - NH₂, -SO₃, -PO₃⁻, -NO₂, -CF₃, are optionally substituted, preferably with a moiety selected from the group consisting of -Cl, -F, -Br, -I, -OR₃₇, -N(R₃₇)₂, -SO₃R₃₇, - PO₃(R₃₇)₂, -PO₄(R₃₇)₂, -NO₂, -CF₃, =O, =NR₃₇, and -SR₃₇, and optionally contain one or more heteroatoms selected from the group consisting of O, S, NR₃₇, P, and Si, wherein the N, S, and P atoms are optionally oxidized, wherein the N atoms are optionally quaternized.

In Formula (4), the Q₁ and Q₂ groups are optionally bound to a polymer, a particle, a peptide, a peptoid, a dendrimer, a protein, an aptamer, a carbohydrate, an oligonucleotide, an oligosaccharide, a lipid, a steroid, a liposome, a micelle, a Targeting Agent T^{T}, a -(S^{P})_{D}-R⁸⁷, an albumin-binding moiety, and a chelating moiety; wherein D is 0 or 1.

In Formula (4), preferably, at least one of moieties Q₁ and Q₂ is not hydrogen.

In Formula (4), preferably each moiety Q₁ and Q₂ is independently selected from the group consisting of hydrogen, -F, -Cl, -Br, -I, -OR₃₇, -N(R₃₇)₂, -SO₃, -PO₃⁻, -NO₂, -CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, -S(O)₂R₃₇, NR₃₇S(O)₂R₃₇, -ON(R₃₇)₂, ₋NR₃₇OR₃₇, C₁-C₂₄ alkyl groups, C₂-C₂₄ alkenyl groups, C₂-C₂₄ alkynyl groups, C₆-C₂₄ aryl groups, C₂-C₂₄ heteroaryl groups, C₃-C₂₄ cycloalkyl groups, C₅-C₂₄ cycloalkenyl groups, C₁₂-C₂₄ cycloalkynyl groups, C₃-C₂₄ (cyclo)alkyl(hetero)aryl groups, C₃-C₂₄ (hetero)aryl(cyclo)alkyl, C₄-C₂₄ (cyclo)alkenyl(hetero)aryl groups, C₄-C₂₄ (hetero)aryl(cyclo)alkenyl groups, C₄-C₂₄ (cyclo)alkynyl(hetero)aryl groups, C₄-C₂₄ (hetero)aryl(cyclo)alkynyl groups, C₄-C₂₄ alkylcycloalkyl groups, and C₄-C₂₄ cycloalkylalkyl groups.

In preferred embodiments, the Q₁ and Q₂ groups not being hydrogen are not substituted.

In a preferred embodiment, Q₁ and Q₂ in Formula (4) are selected from the group of hydrogen, -F, -Cl, -Br, -I, -OR₃₇, -N(R₃₇)₂, -SO₃, -PO₃⁻, -NO₂, -CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, - S(O)₂R₃₇, NR₃₇S(O)₂R₃₇, -ON(R₃₇)₂, ₋NR₃₇OR₃₇, C₁-C₈ alkyl groups, C₂-C₈ alkenyl groups, C₂-C₈ alkynyl groups, C₆-C₁₂ aryl groups, C₂-C₁₂ heteroaryl groups, C₃-C₈ cycloalkyl groups, C₅-C₈ cycloalkenyl groups, C₃-C₁₂ alkyl(hetero)aryl groups, C₃-C₁₂ (hetero)arylalkyl groups, C₄-C₁₂ alkylcycloalkyl groups, C₄-C₁₂ cycloalkylalkyl groups, C₅-C₁₂ cycloalkyl(hetero)aryl groups and C₅-C₁₂ (hetero)arylcycloalkyl groups.

In a preferred embodiment, Q₁ and Q₂ in Formula (4) are selected from the group of hydrogen, -F, -Cl, -Br, -I, -OR₃₇, -N(R₃₇)₂, -SO₃, -PO₃⁻, -NO₂, -CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, - S(O)₂R₃₇, NR₃₇S(O)₂R₃₇, -ON(R₃₇)₂, ₋NR₃₇OR₃₇, C₁-C₄ alkyl groups, C₂-C₄ alkenyl groups, C₂-C₄ alkynyl groups, C₆-C₈ aryl groups, C₂-C₈ heteroaryl groups, C₃-C₆ cycloalkyl groups, C₅-C₆ cycloalkenyl groups, C₃-C₁₀ alkyl(hetero)aryl groups, C₃-C₁₀ (hetero)arylalkyl groups, C₄-C₁₀ alkylcycloalkyl groups, C₄-C₁₀ cycloalkylalkyl groups, C₅-C₁₀ cycloalkyl(hetero)aryl groups and C₅-C₁₀ (hetero)arylcycloalkyl groups.

In a preferred embodiment, Q₁ and Q₂ in Formula (4) are selected from the group of hydrogen, phenyl, 2-pyridyl, 3-pyridyl, 4-pyridyl, 2,6-pyrimidyl, 2,5-pyrimidyl, 3,5-pyrmidyl, and 2,4-pyrimidyl; and Q₁ and Q₂ not being hydrogen are optionally substituted with a moiety selected from the group consisting of -F, -Cl, -Br, -I, -OR₃₇, -N(R₃₇)₂, -SO₃, -PO₃⁻, -NO₂, -CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, -S(O)₂R₃₇, NR₃₇S(O)₂R₃₇, -ON(R₃₇)₂, -NR₃₇OR₃₇, C₁-C₂₄ alkyl groups, C₂-C₂₄ alkenyl groups, C₂-C₂₄ alkynyl groups, C₆-C₂₄ aryl groups, C₂-C₂₄ heteroaryl groups, C₃-C₂₄ cycloalkyl groups, C₅-C₂₄ cycloalkenyl groups, C₁₂-C₂₄ cycloalkynyl groups, C₃-C₂₄ (cyclo)alkyl(hetero)aryl groups, C₃-C₂₄ (hetero)aryl(cyclo)alkyl, C₄-C₂₄ (cyclo)alkenyl(hetero)aryl groups, C₄-C₂₄ (hetero)aryl(cyclo)alkenyl groups, C₄-C₂₄ (cyclo)alkynyl(hetero)aryl groups, C₄-C₂₄ (hetero)aryl(cyclo)alkynyl groups, C₄-C₂₄ alkylcycloalkyl groups, and C₄-C₂₄ cycloalkylalkyl groups; preferably with a moiety selected from the group consisting of -F, -Cl, -Br, -I, -OR₃₇, -N(R₃₇)₂, -SO₃, -PO₃⁻, -NO₂, -CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, - S(O)₂R₃₇, NR₃₇S(O)₂R₃₇, -ON(R₃₇)₂, ₋NR₃₇OR₃₇, C₁-C₈ alkyl groups, C₂-C₈ alkenyl groups, C₂-C₈ alkynyl groups, C₆-C₁₂ aryl groups, C₂-C₁₂ heteroaryl groups, C₃-C₈ cycloalkyl groups, C₅-C₈ cycloalkenyl groups, C₃-C₁₂ alkyl(hetero)aryl groups, C₃-C₁₂ (hetero)arylalkyl groups, C₄-C₁₂ alkylcycloalkyl groups, C₄-C₁₂ cycloalkylalkyl groups, C₅-C₁₂ cycloalkyl(hetero)aryl groups, and C₅-C₁₂ (hetero)arylcycloalkyl groups; more preferably with a moiety selected from the group consisting of -F, - Cl, -Br, -I, -OR₃₇, -N(R₃₇)₂, -SO₃, -PO₃⁻, -NO₂, -CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, -S(O)₂R₃₇, NR₃₇S(O)₂R₃₇, -ON(R₃₇)₂,. NR₃₇OR₃₇, C₁-C₄ alkyl groups, C₂-C₄ alkenyl groups, C₂-C₄ alkynyl groups, C₆-C₈ aryl groups, C₂-C₈ heteroaryl groups, C₃-C₆ cycloalkyl groups, C₅-C₆ cycloalkenyl groups, C₃-C₁₀ alkyl(hetero)aryl groups, C₃-C₁₀ (hetero)arylalkyl groups, C₄-C₁₀ alkylcycloalkyl groups, C₄-C₁₀ cycloalkylalkyl groups, C₅-C₁₀ cycloalkyl(hetero)aryl groups, and C₅-C₁₀ (hetero)arylcycloalkyl groups.

In a preferred embodiment, in Formula (4):
(a) Q₁ and Q₂ are selected from the group consisting of 2-pyridyl, 3-pyridyl, and 4-pyridyl;
(b) Q₁ is selected from the group consisting of 2,6-pyrimidyl, 2,5-pyrimidyl, 3,5-pyrmidyl, and 2,4-pyrimidyl; and Q₂ is (hetero)alkyl; or
(c) Q₁ is phenyl and Q₂ is hydrogen;
(d) Q₁ is alkyl and Q₂ is alkyl;
(e) Q₁ is phenyl and Q₂ is alkyl;
(f) Q₁ is phenyl and Q₂ is phenyl;
and in (a)-(f) all Q₁ and Q₂ not being hydrogen are optionally substituted as defined in the previous paragraph.

In a preferred embodiment, in Formula (4) the alkyl is a C₁-C₂₄ alkyl group, preferably a C₁-C₁₂ alkyl group, more preferably a C₁-C₆ (hetero)alkyl group.

In a preferred embodiment, in Formula (4) the (hetero)aryl is a C₆-C₂₄ aryl group, preferably a C₆-C₁₂ aryl, more preferably a phenyl.

In a preferred embodiment, in Formula (4) the (hetero)aryl is a C₂-C₂₄ heteroaryl, preferably a C₂-C₁₂ heteroaryl, more preferably a C₂-C₅ heteroaryl.

In a preferred embodiment, in Formula (4) the alkenyl is a C₂-C₂₄ alkenyl, preferably a C₂-C₁₂ alkenyl, more preferably a C₂-C₆ alkenyl.

In a preferred embodiment, in Formula (4) the alkynyl is a C₂-C₂₄ alkynyl, preferably a C₂-C₁₂ alkynyl, more preferably a C₂-C₆ alkynyl.

In a preferred embodiment, in Formula (4) the cycloalkyl is a C₃-C₂₄ cycloalkyl, preferably a C₃-C₁₂ cycloalkyl, more preferably a C₃-C₆ cycloalkyl.

In a preferred embodiment, in Formula (4) the cycloalkenyl, C₅-C₂₄ cycloalkenyl groups, preferably a C₅-C₁₂ cycloalkenyl, more preferably a C₅-C₆ cycloalkenyl.

In a preferred embodiment, in Formula (4) the cycloalkynyl is a C₆-C₂₄ cycloalkynyl, preferably a C₈-C₁₂ cycloalkynyl, more preferably a C₈ cycloalkyl.

In preferred embodiments, the Cleaving Agent can be a multimeric compound, comprising a plurality of dienes as defined herein. These multimeric compounds include but are not limited to biomolecules, proteins, peptides, peptoids, polymers, dendrimers, liposomes, micelles, particles, gels, polymer particles, or other polymeric constructs.

### Preferred tetrazines

### Formula (4a)

Preferred tetrazines are in accordance with Formula (4a), and preferably include pharmaceutically accepted salts thereof: 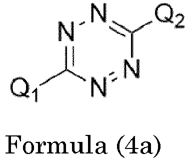 , wherein each moiety Q₁ and Q₂ is independently selected from the group consisting of hydrogen and moieties according to Formula (5): 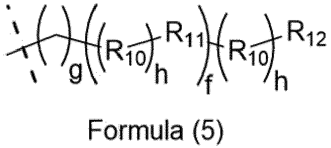 , wherein the dashed line indicates a bond to the remainder of the molecule, and wherein R₁₀, R₁₁, and R₁₂ are as defined herein.

As will be understood herein, in relation to Formula (5), the dashed line may indicate a bond to a tetrazine group of Formula (4a), another moiety according to Formula (5), or to the remainder of the compound according to Formulae (6)-(13) as defined below.

In a preferred embodiment, each f in Formula (5) is an integer independently selected from a range of from 0 to 24, preferably in a range of from 1 to 12, more preferably in a range of from 2 to 6, even more preferably from 1 to 3. In a preferred embodiment, f is 1. In other preferred embodiments f is an integer in the range from 12 to 24.

In a preferred embodiment, in Formula (5) g is an integer in a range of from 0 to 12, preferably in a range of from 1 to 6, more preferably in a range of from 2 to 4.

In a preferred embodiment, in Formula (5) each h is independently 0 or 1. In a preferred embodiment, g is 0, and f is 1. In a preferred embodiment, g is 1, and f is 1.

In case the compound according to the invention comprises more than one moiety satisfying Formula (5), each g, h, and f is independently selected. In a preferred embodiment, the moiety according to Formula (5) is optionally substituted with another independently selected moiety according to Formula (5). In another preferred embodiment, the moiety according to Formula (5) is not substituted with another independently selected moiety according to Formula (5). In a preferred embodiment, the moiety according to Formula (5) is a R⁸⁷, as defined further below.

In a preferred embodiment, the moiety according to Formula (5) satisfies molecules from Group R^{M} shown further below

It is preferred that at least one of moieties Q₁ and Q₂ in Formula (4a) is not hydrogen.

In preferred embodiments, Q₁ in Formula (4a) is selected from the group consisting of C₆-C₂₄ aryl, and C₂-C₂₄ heteroaryl, and is optionally further substituted with a moiety according to Formula (5), preferably not more than one moiety according to Formula (5).

In preferred embodiments, Q₁ in Formula (4a) is selected from the group consisting of C₆-C₂₄ aryl, and C₂-C₂₄ heteroaryl, and is optionally further substituted with a moiety according to Formula (5), preferably not more than one moiety according to Formula (5), and Q₂ in Formula (4a) is selected from the group consisting of C₆-C₂₄ aryl, and C₂-C₂₄ heteroaryl, and is optionally further substituted with a moiety according to Formula (5), preferably not more than one moiety according to Formula (5).

In preferred embodiments, Q₁ in Formula (4a) is selected from the group consisting of C₆ aryl, and C₃-C₅ heteroaryl, and is optionally further substituted with at least one moiety according to Formula (5), preferably not more than one moiety according to Formula (5). Herein, preferred heteroaryls are 2-pyridyl, 3-pyridyl, 4-pyridyl, 2,6-pyrimidyl, 3,5-pyrimidyl, 2,5-pyrimidyl, 2,4-pyrimidyl, 2,4 imidazyl, 2,5 imidazyl, phenyl, 2,3-pyrazyl, 3,4-pyrazyl, oxazol, isoxazol, thiazol, oxazoline, 2-pyrryl, 3-pyrryl, 2-thiophene, and 3-thiophene.

In preferred embodiments, Q₁ in Formula (4a) is C₃-C₅ heteroaryl, and is optionally further substituted with at least one moiety according to Formula (5), preferably not more than one moiety according to Formula (5), and Q₂ is C₃-C₅ heteroaryl, and is optionally further substituted with a moiety according to Formula (5), preferably not more than one moiety according to Formula (5). Herein, preferred heteroaryls are 2-pyridyl, 3-pyridyl, 4-pyridyl, 2,6-pyrimidyl, 3,5-pyrimidyl, 2,5-pyrimidyl, 2,4-pyrimidyl, 2,4 imidazyl, 2,5 imidazyl, phenyl, 2,3-pyrazyl, 3,4-pyrazyl, oxazol, isoxazol, thiazol, oxazoline, 2-pyrryl, 3-pyrryl, 2-thiophene, and 3-thiophene.

In preferred embodiments, Q₁ in Formula (4a) is C₃-C₅ heteroaryl, and is optionally further substituted with at least one moiety according to Formula (5), preferably not more than one moiety according to Formula (5), and Q₂ is H.

In preferred embodiments, Q₁ in Formula (4a) is a phenyl ring, and is optionally further substituted with at least one moiety according to Formula (5), preferably not more than one moiety according to Formula (5), and Q₂ is -H.

In preferred embodiments, Q₁ in Formula (4a) is a phenyl ring, and is optionally further substituted with at least one moiety according to Formula (5), preferably not more than one moiety according to Formula (5), and Q₂ is a phenyl ring, and is optionally further substituted with at least one moiety according to Formula (5), preferably not more than one moiety according to Formula (5).

In preferred embodiments, Q₁ in Formula (4a) is a phenyl ring, and is optionally further substituted with at least one moiety according to Formula (5), preferably not more than one moiety according to Formula (5), and Q₂ is selected from the group consisting of C₆ aryl, and C₃₋₅ heteroaryl, and is optionally further substituted with at least one moiety according to Formula (5), preferably not more than one moiety according to Formula (5).

In preferred embodiments, Q₁ in Formula (4a) is C₁-C₁₂ alkyl, and is optionally further substituted with at least one moiety according to Formula (5), preferably not more than one moiety according to Formula (5), and Q₂ selected from the group consisting of C₆ aryl, and C₃₋₅ heteroaryl, and is optionally further substituted with at least one moiety according to Formula (5), preferably not more than one moiety according to Formula (5).

In preferred embodiments, Q₁ in Formula (4a) is C₁-C₁₂ alkyl, and is optionally further substituted with at least one moiety according to Formula (5), preferably not more than one moiety according to Formula (5), and Q₂ in Formula (4a) is C₁-C₁₂ alkyl, and is optionally further substituted with at least one moiety according to Formula (5), preferably not more than one moiety according to Formula (5).

In preferred embodiments Q₂ equals Q₁.

### R₁₀

In preferred embodiments, each R₁₀ is independently selected from the group consisting of -O-, -S-, -SS-, -NR₄-, -N=N-, -C(O)-, -C(O)NR₄-, -OC(O)-, -C(O)O-, -OC(O)O-, -OC(O)NR₄-, -NR₄C(O)-, -NR₄C(O)O-, -NR₄C(O)NR₄-, -SC(O)-, -C(O)S-, -SC(O)O-, -OC(O)S-, -SC(O)NR₄-, -NR₄C(O)S-, -S(O)-, -S(O)₂-, -OS(O)₂-, -S(O₂)O-, -OS(O)₂O-, -OS(O)₂NR₄-, -NR₄S(O)₂O-, -C(O)NR₄S(O)₂NR₄-, -OC(O)NR₄S(O)₂NR₄-, -OS(O)-, -OS(O)O-, -OS(O)NR₄-, -ONR₄C(O)-, -ONR₄C(O)O-, -ONR₄C(O)NR₄-, -NR₄OC(O)-, -NR₄OC(O)O-, -NR₄OC(O)NR₄-, -ONR₄C(S)-, -ONR₄C(S)O-, -ONR₄C(S)NR₄-, -NR₄OC(S)-, -NR₄OC(S)O-, -NR₄OC(S)NR₄-, -OC(S)-, -C(S)O-, -OC(S)O-, -OC(S)NR₄-, -NR₄C(S)-, -NR₄C(S)O-, -SS(O)₂-, -S(O)₂S-, -OS(O₂)S-, -SS(O)₂O-, -NR₄OS(O)-, -NR₄OS(O)O-, -NR₄OS(O)NR₄-, -NR₄OS(O)₂-, -NR₄OS(O)₂O-, -NR₄OS(O)₂NR₄-, -ONR₄S(O)-, -ONR₄S(O)O-, -ONR₄S(O)NR₄-, -ONR₄S(O)₂O-, -ONR₄S(O)₂NR₄-, -ONR₄S(O)₂-, -OP(O)(R₄)₂-, -SP(O)(R₄)₂-, -NR₄P(O)(R₄)₂-, and combinations thereof, wherein R₄ is defined as described herein.

In preferred embodiments, each R₁₀ is independently selected from the group consisting of -O-, -S-, -SS-, -NR₄-, -N=N-, -C(O)-, -C(O)NR₄-, -OC(O)-, - C(O)O-, -OC(O)NR₄-, -NR₄C(O)-, -NR₄C(O)O-, -NR₄C(O)NR₄-, -SC(O)-, -C(O)S-, - SC(O)O-, -OC(O)S-, -SC(O)NR₄-, -NR₄C(O)S-, -S(O)-, -S(O)₂-, -C(O)NR₄S(O)₂NR₄-, -OC(O)NR₄S(O)₂NR₄-, -OC(S)-, -C(S)O-, -OC(S)O-, -OC(S)NR₄-, -NR₄C(S)-, - NR₄C(S)O-, and -SS(O)₂-.

### R₁₁

In preferred embodiments, each R₁₁ is independently selected from the group consisting of C₁-C₂₄ alkylene groups, C₂-C₂₄ alkenylene groups, C₂-C₂₄ alkynylene groups, C₆-C₂₄ arylene, C₂-C₂₄ heteroarylene, C₃-C₂₄ cycloalkylene groups, C₅-C₂₄ cycloalkenylene groups, and C₁₂-C₂₄ cycloalkynylene groups, which are optionally further substituted with one or more substituents selected from the group consisting of -Cl, -F, -Br, -I, -OH, -NH₂, -SO₃H, -PO₃H, -PO₄H₂, -NO₂, -CF₃, =O, =NR₃₆, -SR₃₆, C₁-C₂₄ alkyl groups, C₂-C₂₄ alkenyl groups, C₂-C₂₄ alkynyl groups, C₆-C₂₄ aryl groups, C₂-C₂₄ heteroaryl groups, C₃-C₂₄ cycloalkyl groups, C₅-C₂₄ cycloalkenyl groups, C₁₂-C₂₄ cycloalkynyl groups, C₃-C₂₄ alkyl(hetero)aryl groups, C₃-C₂₄ (hetero)arylalkyl groups, C₄-C₂₄ (hetero)arylalkenyl groups, C₄-C₂₄ (hetero)arylalkynyl groups, C₄-C₂₄ alkenyl(hetero)aryl groups, C₄-C₂₄ alkynyl(hetero)aryl groups, C₄-C₂₄ alkylcycloalkyl groups, C₆-C₂₄ alkylcycloalkenyl groups, C₁₃-C₂₄ alkylcycloalkynyl groups, C₄-C₂₄ cycloalkylalkyl groups, C₆-C₂₄ cycloalkenylalkyl groups, C₁₃-C₂₄ cycloalkynylalkyl groups, C₅-C₂₄ alkenylcycloalkyl groups, C₇-C₂₄ alkenylcycloalkenyl groups, C₁₄-C₂₄ alkenylcycloalkynyl groups, C₅-C₂₄ cycloalkylalkenyl groups, C₇-C₂₄ cycloalkenylalkenyl groups, C₁₄-C₂₄ cycloalkynylalkenyl groups, C₅-C₂₄ alkynylcycloalkyl groups, C₇-C₂₄ alkynylcycloalkenyl groups, C₁₄-C₂₄ alkynylcycloalkynyl groups, C₅-C₂₄ cycloalkylalkynyl groups, C₇-C₂₄ cycloalkenylalkynyl groups, C₁₄-C₂₄ cycloalkynylalkynyl groups, C₅-C₂₄ cycloalkyl(hetero)aryl groups, C₇-C₂₄ cycloalkenyl(hetero)aryl groups, C₁₄-C₂₄ cycloalkynyl(hetero)aryl groups, C₅-C₂₄ (hetero)arylcycloalkyl groups, C₇-C₂₄ (hetero)arylcycloalkenyl groups, and C₁₄-C₂₄ (hetero)arylcycloalkynyl groups, wherein the substituents optionally contain one or more heteroatoms selected from the group consisting of O, S, NR₃₆, P, and Si, wherein the N, S, and P atoms are optionally oxidized, wherein the N atoms are optionally quaternized; and wherein preferably the alkylene groups, alkenylene groups, alkynylene groups, cycloalkylene groups, cycloalkenylene groups, and cycloalkynylene groups optionally contain one or more heteroatoms selected from the group consisting of O, S, NR₃₆, P, and Si, wherein the N, S, and P atoms are optionally oxidized, wherein the N atoms are optionally quaternized.

In preferred embodiments, each R₁₁ is independently selected from the group consisting of C₁-C₁₂ alkylene groups, C₂-C₁₂ alkenylene groups, C₂-C₁₂ alkynylene groups, C₆-C₁₂ arylene, C₂-C₁₂ heteroarylene, C₃-C₁₂ cycloalkylene groups, C₅-C₁₂ cycloalkenylene groups, and C₁₂ cycloalkynylene groups; and wherein preferably the alkylene groups, alkenylene groups, alkynylene groups, cycloalkylene groups, cycloalkenylene groups, and cycloalkynylene groups optionally contain one or more heteroatoms selected from the group consisting of O, S, NR₃₆, P, and Si, wherein the N, S, and P atoms are optionally oxidized, wherein the N atoms are optionally quaternized.

In preferred embodiments, each R₁₁ is independently selected from the group consisting of C₁-C₆ alkylene groups, C₂-C₆ alkenylene groups, C₂-C₆ alkynylene groups, C₆-C₆ arylene, C₂-C₆ heteroarylene, C₃-C₆ cycloalkylene groups, and C₅-C₆ cycloalkenylene groups;
and wherein preferably the alkylene groups, alkenylene groups, alkynylene groups, cycloalkylene groups, cycloalkenylene groups, and cycloalkynylene groups optionally contain one or more heteroatoms selected from the group consisting of O, S, NR₃₆, P, and Si, wherein the N, S, and P atoms are optionally oxidized, wherein the N atoms are optionally quaternized.

In preferred embodiments, the R₁₁ groups are optionally further substituted with one or more substituents selected from the group consisting of - Cl, -F, -Br, -I, -OH, -NH₂, -SO₃H, -PO₃H, -PO₄H₂, -NO₂, -CF₃, =O, =NR₃₆, -SR₃₆, C₁-C₁₂ alkyl groups, C₂-C₁₂ alkenyl groups, C₂-C₁₂ alkynyl groups, C₆-C₁₂ aryl groups, C₂-C₁₂ heteroaryl groups, C₃-C₁₂ cycloalkyl groups, C₅-C₁₂ cycloalkenyl groups, C₁₂ cycloalkynyl groups, C₃-C₁₂ alkyl(hetero)aryl groups, C₃-C₁₂ (hetero)arylalkyl groups, C₄-C₁₂ (hetero)arylalkenyl groups, C₄-C₁₂ (hetero)arylalkynyl groups, C₄-C₁₂ alkenyl(hetero)aryl groups, C₄-C₁₂ alkynyl(hetero)aryl groups, C₄-C₁₂ alkylcycloalkyl groups, C₆-C₁₂ alkylcycloalkenyl groups, C₁₃-C₁₈ alkylcycloalkynyl groups, C₄-C₁₂ cycloalkylalkyl groups, C₆-C₁₂ cycloalkenylalkyl groups, C₁₃-C₁₈ cycloalkynylalkyl groups, C₅-C₁₂ alkenylcycloalkyl groups, C₇-C₁₂ alkenylcycloalkenyl groups, C₁₄-C₁₆ alkenylcycloalkynyl groups, C₅-C₁₂ cycloalkylalkenyl groups, C₇-C₁₂ cycloalkenylalkenyl groups, C₁₄-C₁₆ cycloalkynylalkenyl groups, C₅-C₁₂ alkynylcycloalkyl groups, C₇-C₁₂ alkynylcycloalkenyl groups, C₁₄-C₁₆ alkynylcycloalkynyl groups, C₅-C₁₂ cycloalkylalkynyl groups, C₇-C₁₂ cycloalkenylalkynyl groups, C₁₄-C₁₆ cycloalkynylalkynyl groups, C₅-C₁₂ cycloalkyl(hetero)aryl groups, C₇-C₁₂ cycloalkenyl(hetero)aryl groups, C₁₄-C₁₆ cycloalkynyl(hetero)aryl groups, C₅-C₁₂ (hetero)arylcycloalkyl groups, C₇-C₁₂ (hetero)arylcycloalkenyl groups, and C₁₄-C₁₆ (hetero)arylcycloalkynyl groups, wherein the substituents optionally contain one or more heteroatoms selected from the group consisting of O, S, NR₃₆, P, and Si, wherein the N, S, and P atoms are optionally oxidized, wherein the N atoms are optionally quaternized.

In preferred embodiments, the R₁₁ groups are optionally further substituted with one or more substituents selected from the group consisting of - Cl, -F, -Br, -I, -OH, -NH₂, -SO₃H, -PO₃H, -PO₄H₂, -NO₂, -CF₃, =O, =NR₃₆, -SR₃₆, C₁-C₆ alkyl groups, C₂-C₆ alkenyl groups, C₂-C₆ alkynyl groups, C₆ aryl groups, C₂-C₆ heteroaryl groups, C₃-C₆ cycloalkyl groups, C₅-C₆ cycloalkenyl groups, C₃-C₆ alkyl(hetero)aryl groups, C₃-C₆ (hetero)arylalkyl groups, C₄-C₆ (hetero)arylalkenyl groups, C₄-C₆ (hetero)arylalkynyl groups, C₄-C₆ alkenyl(hetero)aryl groups, C₄-C₆ alkynyl(hetero)aryl groups, C₄-C₆ alkylcycloalkyl groups, C₆ alkylcycloalkenyl groups, C₄-C₆ cycloalkylalkyl groups, C₆ cycloalkenylalkyl groups, C₅-C₆ alkenylcycloalkyl groups, C₇ alkenylcycloalkenyl groups, C₅-C₆ cycloalkylalkenyl groups, C₇ cycloalkenylalkenyl groups, C₅-C₆ alkynylcycloalkyl groups, C₇ alkynylcycloalkenyl groups, C₅-C₆ cycloalkylalkynyl groups, C₅-C₆ cycloalkyl(hetero)aryl groups, and C₅-C₆ (hetero)arylcycloalkyl groups, wherein the substituents optionally contain one or more heteroatoms selected from the group consisting of O, S, NR₃₆, P, and Si, wherein the N, S, and P atoms are optionally oxidized, wherein the N atoms are optionally quaternized.

In preferred embodiments, the R₁₁ groups are optionally further substituted with one or more substituents selected from the group consisting of - Cl, -F, -Br, -I, -OH, -NH₂, -SO₃H, -PO₃H, -PO₄H₂, -NO₂, -CF₃, =O, =NR₃₆, -SR₃₆, C₁-C₆ alkyl groups, C₂-C₆ alkenyl groups, C₂-C₆ alkynyl groups, C₆ aryl groups, C₂-C₆ heteroaryl groups, C₃-C₆ cycloalkyl groups, C₅-C₆ cycloalkenyl groups, C₃-C₇ alkyl(hetero)aryl groups, C₃-C₇ (hetero)arylalkyl groups, C₄-C₈ (hetero)arylalkenyl groups, C₄-C₈ (hetero)arylalkynyl groups, C₄-C₈ alkenyl(hetero)aryl groups, C₄-C₈ alkynyl(hetero)aryl groups, C₄-C₆ alkylcycloalkyl groups, C₆-C₇ alkylcycloalkenyl groups, C₄-C₆ cycloalkylalkyl groups, C₆-C₇ cycloalkenylalkyl groups, C₅-C₆ alkenylcycloalkyl groups, C₇-C₈ alkenylcycloalkenyl groups, C₅-C₆ cycloalkylalkenyl groups, C₇-C₈ cycloalkenylalkenyl groups, C₅-C₆ alkynylcycloalkyl groups, C₇-C₈ alkynylcycloalkenyl groups, C₅-C₆ cycloalkylalkynyl groups, C₅-C₉ cycloalkyl(hetero)aryl groups, and C₅-C₆ (hetero)arylcycloalkyl groups, wherein the substituents optionally contain one or more heteroatoms selected from the group consisting of O, S, NR₃₆, P, and Si, wherein the N, S, and P atoms are optionally oxidized, wherein the N atoms are optionally quaternized.

It is preferred that when f > 2, that R₁₁ is independently selected from the group consisting of C₁-C₆ alkylene groups, C₂-C₆ alkenylene groups, C₂-C₆ alkynylene groups, C₆-C₆ arylene, C₂-C₆ heteroarylene, C₃-C₆ cycloalkylene groups, and C₅-C₆ cycloalkenylene groups; and wherein preferably the alkylene groups, alkenylene groups, alkynylene groups, cycloalkylene groups, cycloalkenylene groups, and cycloalkynylene groups optionally contain one or more heteroatoms selected from the group consisting of O, S, NR₃₆, P, and Si, wherein the N, S, and P atoms are optionally oxidized, wherein the N atoms are optionally quaternized.

In a preferred embodiment, the R₁₁ substituents do not contain heteroatoms.

In a preferred embodiment, the R₁₁ groups are not substituted.

In another preferred embodiment, the R₁₁ groups do not contain heteroatoms.

### R12

R₁₂ is selected from the group consisting of -H, -OH, -NH₂, -N₃, -Cl, -Br, -F, -I, a polymer, a particle, a peptide, a peptoid, a dendrimer, a protein, a biomolecule, a carbohydrate, an oligonucleotide, an oligosaccharide, a lipid, a liposome, a micelle, an imaging moiety, a Targeting Agent T^{T}, a R⁸⁷, an albumin-binding moiety, and a chelating moiety.

Non-limiting examples of chelating moieties for use in R₁₂ are
DTPA (diethylenetriaminepentaacetic acid),
DOTA (1,4,7,10- tetraazacyclododecane-N,N',N",N"-tetraacetic acid),
NOTA (1,4,7-triazacyclononane-N,N',N"-triacetic acid),
TETA (1,4,8,11-tetraazacyclotetradecane-N,N',N",N'-tetraacetic acid),
OTTA (N1-(*p*-isothiocyanatobenzyl)-diethylenetriamine-N₁,N₂,N₃,N₃-tetraacetic acid), deferoxamine or DFO (N'-[5-[[4-[[5-(acetylhydroxyamino)pentyl]amino]-1,4-dioxobutyl]hydroxyamino]pentyl]-N-(5-aminopentyl)-N-hydroxybutanediamide) or HYNIC (hydrazinonicotinamide).

In a preferred embodiment, when R¹² is a polymer, a particle, a peptide, a peptoid, a dendrimer, a protein, a biomolecule, an oligonucleotide, an oligosaccharide, a lipid, a liposome, a micelle, a Targeting Agent T^{T}, or a R⁸⁷, then f is at most 2, preferably at most 1.

### Formulae (6), (7), (8), (9), (10), (11), (12), and (13)

In preferred embodiments of the invention the tetrazine is in accordance with any one of the Formulae (6), (7), (8), (9), (10), (11), (12), or (13): 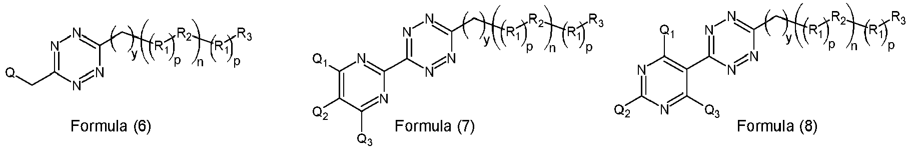 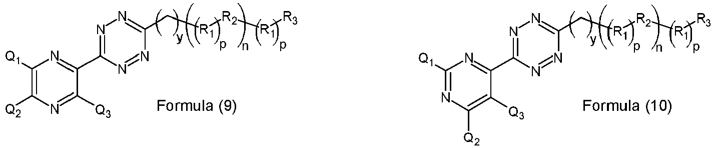 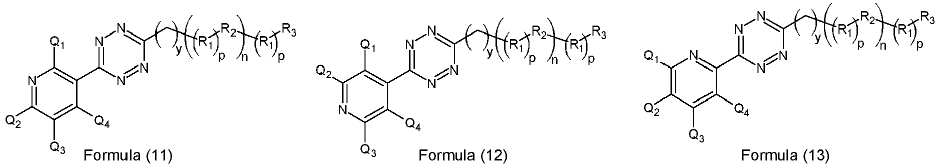 wherein each moiety Q, Q₁, Q₂, Q₃, and Q₄ is independently selected from the group consisting of hydrogen and moieties according to Formula (5) as defined herein; and wherein R₁, R₂, and R₃ are as defined herein.

In preferred embodiments, in the tetrazines according to any one of Formulae (6), (7), (8), (9), (10), (11), (12), and (13), at most one moiety selected from the group consisting of Q, Q₁, Q₂, Q₃, and Q₄ is hydrogen.

In preferred embodiments, in the tetrazines according to any one of Formulae (7), (8), (9), (10), (11), (12), and (13), at most two moieties selected from the group consisting of Q, Q₁, Q₂, Q₃, and Q₄ are hydrogen.

In preferred embodiments, in the tetrazines according to any one of Formulae (7), (8), (9), (10), (11), (12), and (13), at most three moieties selected from the group consisting of Q, Q₁, Q₂, Q₃, and Q₄ are hydrogen.

In preferred embodiments, in the tetrazines according to any one of Formulae (7), (8), (9), (10), (11), (12), and (13), all moieties selected from the group consisting of Q, _{Q 1}, Q₂, Q₃, and Q₄ are hydrogen.

In preferred embodiments, in the tetrazines according to any one of Formulae (7), (8), (9), (10), (11), (12), and (13), at most one moiety selected from the group consisting of Q, Q₁, Q₂, Q₃, and Q₄ is not hydrogen.

In preferred embodiments, in the tetrazines according to any one of Formulae (7), (8), (9), (10), (11), (12), and (13), at most two moieties selected from the group consisting of Q, Q₁, Q₂, Q₃, and Q₄ is not hydrogen.

### Molecular weight

Preferably, for all compounds disclosed herein comprising a group Q, Q₁, Q₂, Q₃, Q₄ or -(CH₂)_{y}-((R₁)ₚ-R₂)ₙ-(R₁)ₚ-R₃, at least one of these groups has a molecular weight in a range of from 100 Da to 3000 Da. Preferably, at least one of these groups has a molecular weight in a range of from 100 Da to 2000 Da. More preferably, at least one of these groups has a molecular weight in a range of from 100 Da to 1500 Da, even more preferably in a range of from 150 Da to 1500 Da. Even more preferably still, at least one of these groups has a molecular weight in a range of from 150 Da to 1000 Da, most preferably in a range of from 200 Da to 1000 Da.

Preferably, for all compounds disclosed herein, comprising a group Q, Q1, Q2, Q3, Q4 or -(CH₂)_{y}-((R₁)ₚ-R₂)ₙ-(R₁)ₚ-R₃, none of these groups has a molecular weight of more than 3000 Da, in particular in the case the Clearing Agent needs to efficiently extravasate into tissues.

### Group -(CH₂)_{y}-((R₁)ₚ-R₂)ₙ-(R₁)ₚ-R₃

In preferred embodiments, y is an integer in a range of from 1 to 12, preferably from 1 to 10, more preferably from 1 to 8, even more preferably from 2 to 6, most preferably from 2 to 4. In preferred embodiments, y is at least 2, preferably y is at least 3. In preferred embodiments, p is 0 or 1, wherein each p is independently selected. In preferred embodiments, each n is an integer independently selected from a range of from 0 to 24, preferably from 1 to 12, more preferably from 1 to 6, even more preferably from 1 to 3, most preferably n is 0 or 1. In preferred embodiments n is preferably an integer from 12 to 24. In preferred embodiments, n is 1.

In preferred embodiments, the entire group -((R₁)ₚ-R₂)ₙ-(R₁)ₚ-R₃ has a molecular weight in a range of from 100 Da to 3000 Da. Preferably, the entire group -((R₁)ₚ-R₂)ₙ-(R₁)ₚ-R₃ has a molecular weight in a range of from 100 Da to 2000 Da. More preferably, the entire group -((R₁)ₚ-R₂)ₙ-(R₁)ₚ-R₃ has a molecular weight in a range of from 100 Da to 1500 Da, even more preferably in a range of from 150 Da to 1500 Da. Even more preferably still, the entire group -((R₁)ₚ-R₂)ₙ-(R₁)ₚ-R₃ has a molecular weight in a range of from 150 Da to 1000 Da, most preferably in a range of from 200 Da to 1000 Da.

It is preferred that when n > 2, that R₂ is independently selected from the group consisting of C₁-C₆ alkylene groups, C₂-C₆ alkenylene groups, C₂-C₆ alkynylene groups, C₆-C₆ arylene, C₂-C₆ heteroarylene, C₃-C₆ cycloalkylene groups, and C₅-C₆ cycloalkenylene groups; and wherein preferably the alkylene groups, alkenylene groups, alkynylene groups, cycloalkylene groups, cycloalkenylene groups, and cycloalkynylene groups optionally contain one or more heteroatoms selected from the group consisting of O, S, NR₃₆, P, and Si, wherein the N, S, and P atoms are optionally oxidized, wherein the N atoms are optionally quaternized.

In preferred embodiments, the entire group -((R₁)ₚ-R₂)ₙ-(R₁)ₚ-R₃ satisfies molecules from Group R^{M} shown below.

### R^{M}:

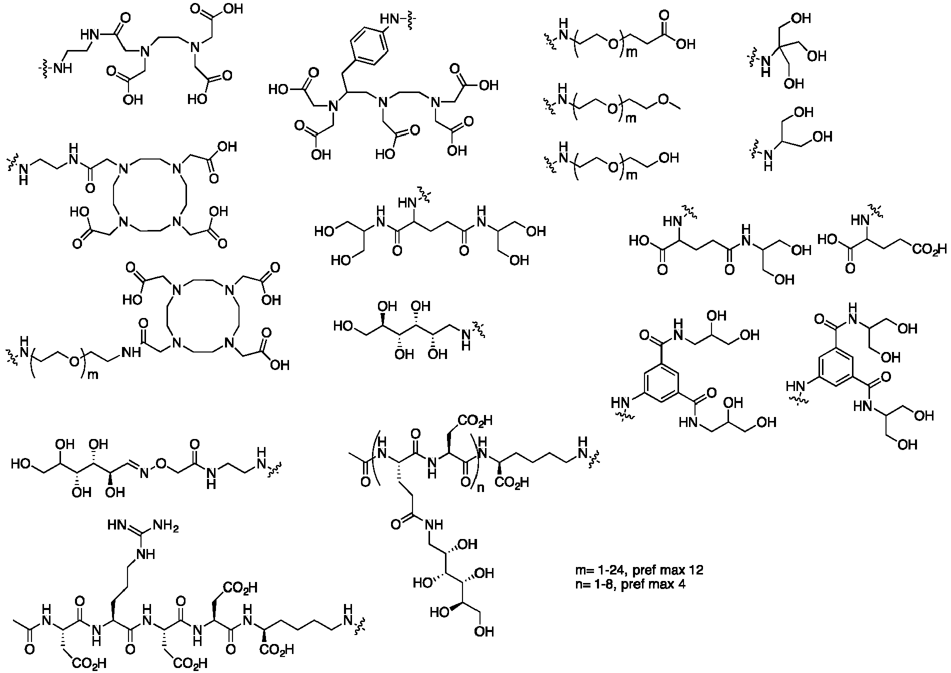

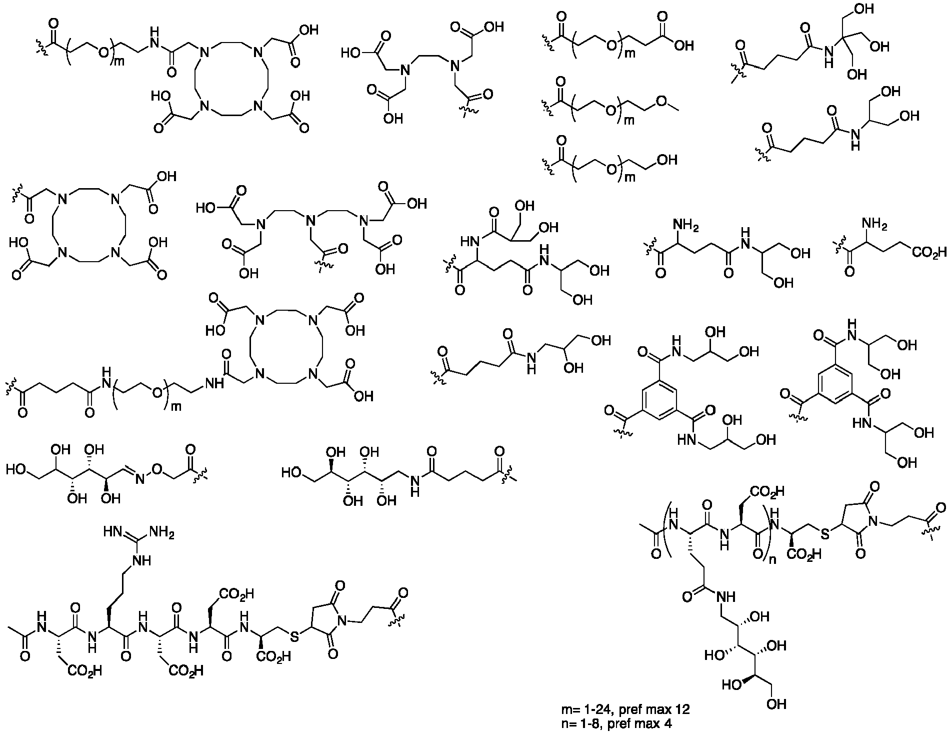

, wherein the wiggly line denotes a bond to a tetrazine group as disclosed herein or to a group R₁ or R₂.

In preferred embodiments, the group -((R₁)ₚ-R₂)ₙ-(R₁)ₚ-R₃ satisfies molecules from Group R^{M}, wherein it is understood that when n is more than 1, -((R₁)ₚ-R₂)ₙ-(R₁)ₚ-R₃ may be preceded by a group -((R₁)ₚ-R₂)- so as to form a group -((R₁)ₚ-R₂)-((R₁)ₚ-R₂)ₙ₋₁-(R₁)ₚ-R₃. It is understood that this follows from the definition of how to write out the repeating units, i.e. -((R₁)ₚ-R₂)₂- would first be written as -(R₁)ₚ-R₂-(R₁)ₚ-R₂- before R₁, p, and R₂ are independently selected.

### R_{1,} R₂, and R₃

R₁ is as defined for R₁₀. R₂ is as defined for R₁₁. R₃ is as defined for R₁₂.

### R₄

Preferably, each R₄ is independently selected from the group consisting of hydrogen, C₁-C₂₄ alkyl groups, C₂-C₂₄ alkenyl groups, C₂-C₂₄ alkynyl groups, C₆-C₂₄ aryl, C₂-C₂₄ heteroaryl, C₃-C₂₄ cycloalkyl groups, C₅-C₂₄ cycloalkenyl groups, and C₁₂-C₂₄ cycloalkynyl groups.

In a preferred embodiment, each R₄ is independently selected from the group consisting of hydrogen, C₁-C₁₂ alkyl groups, C₂-C₁₂ alkenyl groups, C₂-C₁₂ alkynyl groups, C₆-C₁₂ aryl, C₂-C₁₂ heteroaryl, C₃-C₁₂ cycloalkyl groups, C₅-C₁₂ cycloalkenyl groups, and C₁₂ cycloalkynyl groups.

In a preferred embodiment, each R₄ is independently selected from the group consisting of hydrogen, C₁-C₄ alkyl groups, C₂-C₄ alkenyl groups, C₂-C₄ alkynyl groups, C₆ aryl, C₂-C₆ heteroaryl, C₃-C₈ cycloalkyl groups, C₅-C₈ cycloalkenyl groups, and C₈ cycloalkynyl groups.

Preferably, the R₄ groups not being hydrogen, optionally contain one or more heteroatoms selected from the group consisting of O, S, NR₅, P, and Si, wherein the N, S, and P atoms are optionally oxidized, wherein the N atoms are optionally quaternized.

Preferably, the R₄ groups not being hydrogen, are optionally further substituted with one or more substituents selected from the group consisting of - Cl, -F, -Br, -I, -OH, -NH₂, -SO₃H, -PO₃H, -PO₄H₂, -NO₂, -CF₃, =O, =NR₅, -SR₅, C₁-C₂₄ alkyl groups, C₂-C₂₄ alkenyl groups, C₂-C₂₄ alkynyl groups, C₆-C₂₄ aryl groups, C₂-C₂₄ heteroaryl groups, C₃-C₂₄ cycloalkyl groups, C₅-C₂₄ cycloalkenyl groups, C₁₂-C₂₄ cycloalkynyl groups, C₃-C₂₄ alkyl(hetero)aryl groups, C₃-C₂₄ (hetero)arylalkyl groups, C₄-C₂₄ (hetero)arylalkenyl groups, C₄-C₂₄ (hetero)arylalkynyl groups, C₄-C₂₄ alkenyl(hetero)aryl groups, C₄-C₂₄ alkynyl(hetero)aryl groups, C₄-C₂₄ alkylcycloalkyl groups, C₆-C₂₄ alkylcycloalkenyl groups, C₁₃-C₂₄ alkylcycloalkynyl groups, C₄-C₂₄ cycloalkylalkyl groups, C₆-C₂₄ cycloalkenylalkyl groups, C₁₃-C₂₄ cycloalkynylalkyl groups, C₅-C₂₄ alkenylcycloalkyl groups, C₇-C₂₄ alkenylcycloalkenyl groups, C₁₄-C₂₄ alkenylcycloalkynyl groups, C₅-C₂₄ cycloalkylalkenyl groups, C₇-C₂₄ cycloalkenylalkenyl groups, C₁₄-C₂₄ cycloalkynylalkenyl groups, C₅-C₂₄ alkynylcycloalkyl groups, C₇-C₂₄ alkynylcycloalkenyl groups, C₁₄-C₂₄ alkynylcycloalkynyl groups, C₅-C₂₄ cycloalkylalkynyl groups, C₇-C₂₄ cycloalkenylalkynyl groups, C₁₄-C₂₄ cycloalkynylalkynyl groups, C₅-C₂₄ cycloalkyl(hetero)aryl groups, C₇-C₂₄ cycloalkenyl(hetero)aryl groups, C₁₄-C₂₄ cycloalkynyl(hetero)aryl groups, C₅-C₂₄ (hetero)arylcycloalkyl groups, C₇-C₂₄ (hetero)arylcycloalkenyl groups, and C₁₄-C₂₄ (hetero)arylcycloalkynyl groups; wherein the substituents optionally contain one or more heteroatoms selected from the group consisting of O, S, NR₅, P, and Si, wherein the N, S, and P atoms are optionally oxidized, wherein the N atoms are optionally quaternized.

Preferably, the R₄ groups not being hydrogen, are optionally further substituted with one or more substituents selected from the group consisting of - Cl, -F, -Br, -I, -OH, -NH₂, -SO₃H, -PO₃H, -PO₄H₂, -NO₂, -CF₃, =O, =NR₅, -SR₅, C₁-C₄ alkyl groups, C₂-C₄ alkenyl groups, C₂-C₄ alkynyl groups, C₆ aryl groups, C₂-C₄ heteroaryl groups, C₃-C₄ cycloalkyl groups, C₅-C₄ cycloalkenyl groups, C₁₂ cycloalkynyl groups, C₃-C₁₂ alkyl(hetero)aryl groups, C₃-C₁₂ (hetero)arylalkyl groups, C₄-C₁₂ (hetero)arylalkenyl groups, C₄-C₁₂ (hetero)arylalkynyl groups, C₄-C₁₂ alkenyl(hetero)aryl groups, C₄-C₁₂ alkynyl(hetero)aryl groups, C₄-C₁₂ alkylcycloalkyl groups, C₆-C₁₂ alkylcycloalkenyl groups, C₁₃-C₁₂ alkylcycloalkynyl groups, C₄-C₁₂ cycloalkylalkyl groups, C₆-C₁₂ cycloalkenylalkyl groups, C₁₃ cycloalkynylalkyl groups, C₅-C₁₂ alkenylcycloalkyl groups, C₇-C₁₂ alkenylcycloalkenyl groups, C₁₄ alkenylcycloalkynyl groups, C₅-C₁₂ cycloalkylalkenyl groups, C₇-C₁₂ cycloalkenylalkenyl groups, C₁₄ cycloalkynylalkenyl groups, C₅-C₁₂ alkynylcycloalkyl groups, C₇-C₁₂ alkynylcycloalkenyl groups, C₁₄-C₁₂ alkynylcycloalkynyl groups, C₅-C₁₂ cycloalkylalkynyl groups, C₇-C₁₂ cycloalkenylalkynyl groups, C₁₄ cycloalkynylalkynyl groups, C₅-C₁₂ cycloalkyl(hetero)aryl groups, C₇-C₁₂ cycloalkenyl(hetero)aryl groups, C₁₄ cycloalkynyl(hetero)aryl groups, C₅-C₁₂ (hetero)arylcycloalkyl groups, C₇-C₁₂ (hetero)arylcycloalkenyl groups, and C₁₄ (hetero)arylcycloalkynyl groups;
wherein the substituents optionally contain one or more heteroatoms selected from the group consisting of O, S, NR₅, P, and Si, wherein the N, S, and P atoms are optionally oxidized, wherein the N atoms are optionally quaternized.

In a preferred embodiment, the R₄ substituents do not contain heteroatoms. In a preferred embodiment, the R₄ groups are not substituted.

In another preferred embodiment, the R₄ groups do not contain heteroatoms.

### R5

Preferably, each R₅ is independently selected from the group consisting of hydrogen, C₁-C₈ alkyl groups, C₂-C₈ alkenyl groups, C₂-C₈ alkynyl groups, C₆-C₁₂ aryl, C₂-C₁₂ heteroaryl, C₃-C₈ cycloalkyl groups, C₅-C₈ cycloalkenyl groups, C₃-C₁₂ alkyl(hetero)aryl groups, C₃-C₁₂ (hetero)arylalkyl groups, C₄-C₁₂ alkylcycloalkyl groups, C₄-C₁₂ cycloalkylalkyl groups, C₅-C₁₂ cycloalkyl(hetero)aryl groups and C₅-C₁₂ (hetero)arylcycloalkyl groups, wherein the R₅ groups not being hydrogen are optionally substituted with a moiety selected from the group consisting of -Cl, -F, -Br, -I, -OH, -NH₂, -SO₃H, -PO₃H, -PO₄H₂, -NO₂, -CF₃, =O, =NH, and -SH, and optionally contain one or more heteroatoms selected from the group consisting of O, S, NH, P, and Si, wherein the N, S, and P atoms are optionally oxidized, wherein the N atoms are optionally quaternized.

In a preferred embodiment, the R₅ groups are not substituted.

In another preferred embodiment, the R₅ groups do not contain heteroatoms.

### Moieties Q, Q₁, Q₂, Q₃, Q₄

In preferred embodiments, g is an integer in a range of from 0 to 12, preferably from 0 to 10, more preferably from 0 to 8, even more preferably from 1 to 6, most preferably from 2 to 4. In other preferred embodiments g is 0. In case more than one moiety selected from the group consisting of Q, Q₁, Q₂, Q₃, and Q₄ within one compound satisfies Formula (5), each g is independently selected.

In preferred embodiments, h is 0 or 1. In case more than one moiety selected from the group consisting of Q, Q₁, Q₂, Q₃, and Q₄ within one compound satisfies Formula (5), each h is independently selected.

In preferred embodiments, each f belonging to a moiety Q, Q₁, Q₂, Q₃, or Q₄ is an integer independently selected from a range of from 0 to 24, preferably from 1 to 12, more preferably from 1 to 6, even more preferably from 1 to 3, most preferably f is 0 or 1. In preferred embodiments f is preferably an integer from 12 to 24. In other preferred embodiments, f is 1.

In preferred embodiments, the group -((R₁₀)ₕ-R₁₁)ₙ-(R₁₀)ₕ-R₁₂ satisfies molecules from Group R^{M} shown above.

In preferred embodiments, the group -((R₁₀)ₕ-R₁₁)ₙ-(R₁₀)ₕ-R₁₂ satisfies molecules from Group R^{M}, wherein it is understood that when n is more than 1, e.g. -((R₁₀)ₕ-R₁₁)ₙ₋₁-(R₁₀)ₕ-R₁₂ may be preceded by a group -(R₁₀)ₕ-R₁₁- so as to form a group -(R₁₀)ₕ-R₁₁-((R₁₀)ₕ-R₁₁)ₙ₋₁-(R₁₀)ₕ-R₁₂. It is understood that this follows from the definition of how to write out the repeating units, i.e. -((R₁₀)ₕ-R₁₁)₂- would first be written as -(R₁₀)ₕ-R₁₁-(R₁₀)ₕ-R₁₁- before R₁₀, h, and R₁₁ are independently selected.

### Formulae (14), (14a), (14b), (14c), (14d), (14e), and (14f)

In a preferred embodiment, the Cleaving Agent is a tetrazine satisfying Formula (14): 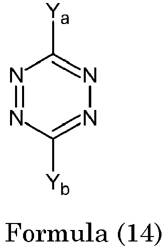 and preferably including pharmaceutically acceptable salts thereof, wherein, Yₐ is selected from the group consisting of Y₁, Y₂, Y₃, Y₄, Y₅ and Y₆: 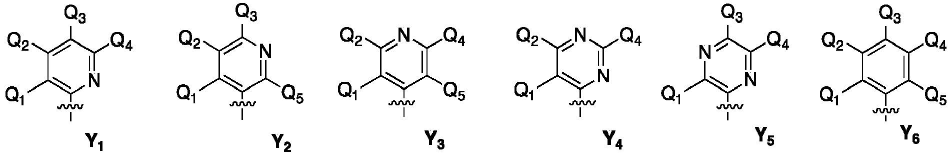 wherein, Y_{b} is selected from the group consisting of Y₁, Y₂, Y₃, Y₄, Y₅, Y₆, hydrogen, X₄₇, and -(S^{P})_{D}-R⁸⁷; wherein SP is a spacer, preferably as defined herein, wherein D is 0 or 1, preferably D is 0; wherein when Yₐ is Y₆, then Y_{b} is hydrogen, wherein each Q₁ and Q₅, are individually selected from the group consisting of X₄₅, hydrogen, X₄₇ and -(S^{P})_{D}-R⁸⁷; wherein each Q₂ and Q₄, are individually selected from the group consisting of X₄₆, hydrogen, X₄₇, and -(S^{P})_{D}-R⁸⁷; wherein each Q₃ is individually selected from the group consisting of hydrogen, X₄₇, and -(S^{P})_{D}-R⁸⁷; wherein preferably the compound of Formula (14) comprises at least one X₄₅ or X₄₆ group, wherein each X₄₅ individually is selected from the group consisting of N(X₅₀)₂, C(X₅₁)₂N(X₅₀)₂, NX₅₀C(O)X₅₁, NX₅₀C(S)X₅₁, OH, SH, C(O)OH, C(S)OH, C(O)SH, C(S)SH, NX₅₀C(O)OX₅₁, NX₅₀C(S)OX₅₁, NX₅₀C(O)SX₅₁, NX₅₀C(S)SX₅₁, NX₅₀C(O)N(X₅₁)₂, NX₅₀C(S)N(X₅₁)₂, NX₅₀SO₂X₅₁, NX₅₀SO₃X₅₁, NX₅₀OX₅₁, SO₃H, , SO₂N(X₅₁)₂, and PO₃H₂; wherein each X₄₆ individually is selected from the group consisting of N(X₅₀)₂, C(X₅₁)₂N(X₅₀)₂, NX₅₀C(O)X₅₁, NX₅₀C(S)X_{51,}, OH, SH, C(O)OH, C(S)OH, C(O)SH, C(S)SH, NX₅₀C(O)OX₅₁, NX₅₀C(S)OX₅₁, NX₅₀C(O)SX₅₁, NX₅₀C(S)SX₅₁, NX₅₀C(O)N(X₅₁)₂, NX₅₀C(S)N(X₅₁)₂, NX₅₀SO₂X₅₁, NX₅₀SO₃X₅₁, NX₅₀OX₅₁, SO₃H, and PO₃H₂; wherein each X₅₀ and X₅₁ individually is selected from the group consisting of hydrogen, X₄₈, and -(S^{P})_{D}-R⁸⁷; wherein each X₄₈ is preferably independently selected from the group consisting of hydrogen, C₁-C₄ alkyl groups, C₂-C₄ alkenyl groups, and C₄₋₆ (hetero)aryl groups; wherein for X₄₈ the alkyl groups, alkenyl groups, and (hetero)aryl groups are optionally substituted with a moiety selected from the group consisting of -Cl, -F, -Br, -I, -OH, -NH₂, =O, -SH, -SO₃H, -PO₃H, -PO₄H₂, and -NO₂; and optionally contain at most two heteroatoms selected from the group consisting of -O-, -S-, -NH-, -P-, and -Si-, wherein the N, S, and P atoms are optionally oxidized, wherein each X₄₇ is selected from the group consisting of -F,-Cl, -Br, -I, -OX₄₉, -N(X₄₉)₂, -SO₃, -PO₃⁻, -NO₂, -CF₃, -SX₄₉, S(=O)₂N(X₄₉)₂, OC(=O)X₄₉, SC(=O) X₄₉, OC(=S)X₄₉, SC(=S)X₄₉, NX₄₉C(=O)-X₄₉, NX₄₉C(=S)-X₄₉, NX₄₉C(=O)O-X₄₉, NX₄₉C(=S)O-X₄₉, NX₄₉C(=O)S-X₄₉, NX₄₉C(=S)S-X₄₉, OC(=O)N(X₄₉)₂, SC(=O)N(X₄₉)₂, OC(=S)N(X₄₉)₂, SC(=S)N(X₄₉)₂, NX₄₉C(=O)N(X₄₉)₂, NX₄₉C(=S)N(X₄₉)₂, C(=O)X₄₉, C(=S)X₄₉, C(=O)N(X₄₉)₂, C(=S)N(X₄₉)₂, C(=O)O-X₄₉, C(=O)S-X₄₉, C(=S)O-X₄₉, C(=S)S-X₄₉, -S(O)X₄₉, -S(O)₂X₄₉, NX₄₉S(O)₂X₄₉, -ON(X₄₉)₂, ₋NX₄₉OX₄₉, C₁-C₈ alkyl groups, C₂-C₈ alkenyl groups, C₂-C₈ alkynyl groups, C₆-C₁₂ aryl, C₂-C₁₂ heteroaryl, C₃-C₈ cycloalkyl groups, C₅-C₈ cycloalkenyl groups, C₃-C₁₂ alkyl(hetero)aryl groups, C₃-C₁₂ (hetero)arylalkyl groups, C₄-C₁₂ alkylcycloalkyl groups, C₄-C₁₂ cycloalkylalkyl groups, C₅-C₁₂ cycloalkyl(hetero)aryl groups and C₅-C₁₂ (hetero)arylcycloalkyl groups, wherein the alkyl groups, alkenyl groups, alkynyl groups, aryl, heteroaryl, cycloalkyl groups, cycloalkenyl groups, alkyl(hetero)aryl groups, (hetero)arylalkyl groups, alkylcycloalkyl groups, cycloalkylalkyl groups, cycloalkyl(hetero)aryl groups and (hetero)arylcycloalkyl groups are optionally substituted with a moiety selected from the group consisting of -Cl, -F, -Br, -I, -OX₄₉, -N(X₄₉)₂, -SO₃X₄₉,-PO₃(X₄₉)₂, -PO₄(X₄₉)₂, -NO₂, -CF₃, =O, =NX₄₉, and -SX₄₉, and optionally contain one or more heteroatoms selected from the group consisting of O, S, NX₄₉, P, and Si, wherein the N, S, and P atoms are optionally oxidized, wherein the N atoms are optionally quaternized; wherein X₄₉ is selected from the group consisting of hydrogen, C₁-C₈ alkyl groups, C₂-C₈ alkenyl groups, C₂-C₈ alkynyl groups, C₆-C₁₂ aryl, C₂-C₁₂ heteroaryl, C₃-C₈ cycloalkyl groups, C₅-C₈ cycloalkenyl groups, C₃-C₁₂ alkyl(hetero)aryl groups, C₃-C₁₂ (hetero)arylalkyl groups, C₄-C₁₂ alkylcycloalkyl groups, C₄-C₁₂ cycloalkylalkyl groups, C₅-C₁₂ cycloalkyl(hetero)aryl groups and C₅-C₁₂ (hetero)arylcycloalkyl groups, wherein the X₄₉ groups not being hydrogen are optionally substituted with a moiety selected from the group consisting of -Cl, -F, -Br, -I, -OH, -NH₂, -SO₃H, -PO₃H, -PO₄H₂, -NO₂, -CF₃, =O, =NH, and -SH, and optionally contain one or more heteroatoms selected from the group consisting of O, S, NH, P, and Si, wherein the N, S, and P atoms are optionally oxidized, wherein the N atoms are optionally quaternized; wherein for each individual Yₐ and Y_{b} preferably at most two, more preferably at most one of Q₁, Q₂, Q₃, Q₄, and Q₅ are said R⁸⁷; wherein the compound according to Formula (14) preferably comprises at most four R⁸⁷ moieties, more preferably at most two R⁸⁷ moieties, most preferably at most one R⁸⁷;wherein the compound according to Formula (14) preferably comprises at least one R⁸⁷; wherein preferably for each individual Yₐ and Y_{b} at most three, more preferably at most two of Q₁, Q₂, Q₃, Q₄, and Q₅ are not hydrogen; wherein preferably for each individual Yₐ and Y_{b} at most two of Q₁, Q₂, Q₃, Q₄, and Q₅ are X₄₅ or X₄₆, wherein preferably for each individual Yₐ and Y_{b} one of Q₁, Q₂, Q₄, and Q₅ is X₄₅ or X₄₆, wherein preferably both Yₐ and Y_{b} comprise at least one X₄₅ or X₄₆, wherein preferably both Yₐ and Y_{b} comprise one X₄₅ or X₄₆, wherein preferably both Yₐ and Y_{b} comprise one X₄₅ or X₄₆, wherein preferably the X₄₅ comprised in Yₐ is the same as the X₄₅ comprised in Y_{b}, and/or the X₄₆ comprised in Yₐ is the same as the X₄₆ comprised in Y_{b}, wherein preferably Yₐ and Y_{b} are both independently selected Y₁, or both independently selected Y₂, or both independently selected Y₃, or both independently selected Y₄, or both independently selected Y₅.

In a preferred embodiment, in Formula (14) or in any one of Formulae (14a)-(14f), when Q₁ is a X₄₇ or -(S^{P})_{D}-R⁸⁷, then for Q₁ the X₄₇ and the R⁸⁷ are not a group in accordance with the definition of X₄₅.

In a preferred embodiment, in Formula (14) or in any one of Formulae (14a)-(14f), when Q₅ is a X₄₇ or -(S^{P})_{D}-R⁸⁷, then for Q₅ the X₄₇ and the R⁸⁷ are not a group in accordance with the definition of X₄₅.

In a preferred embodiment, in Formula (14) or in any one of Formulae (14a)-(14f), when Q₂ is a X₄₇ or -(S^{P})_{D}-R⁸⁷, then for Q₂ the X₄₇ and the R⁸⁷ are not a group in accordance with the definition of X₄₆.

In a preferred embodiment, in Formula (14) or in any one of Formulae (14a)-(14f), when Q₄ is a X₄₇ or -(S^{P})_{D}-R⁸⁷, then for Q₄ the X₄₇ and the R⁸⁷ are not a group in accordance with the definition of X₄₆.

In preferred embodiments Yₐ equals Y_{b}. In preferred embodiments Yₐ is selected from Y₁, Y₂, Y₃, Y₄ or Y₅ and Y_{b} is hydrogen, X₄₇ or -(S^{P})_{D}-R⁸⁷. In preferred embodiments Yₐ is selected from Y₁, Y₂, Y₃, Y₄ or Y₅ and Y_{b} is hydrogen. In preferred embodiments the compound according to Formula (14) does not comprise a R⁸⁷. In preferred embodiments, X₅₀ is hydrogen. In preferred embodiments when X₄₅ or X₄₆ is N(X₅₀)₂, then one X₅₀ is hydrogen and one X₅₀ is X₄₈ or -(S^{P})_{D}-R⁸⁷. In preferred embodiments Formula (14) does not comprise X₄₆.

In preferred embodiments, the Cleaving Agent is a tetrazine satisfying any one of Formulae (14a)-(14f): 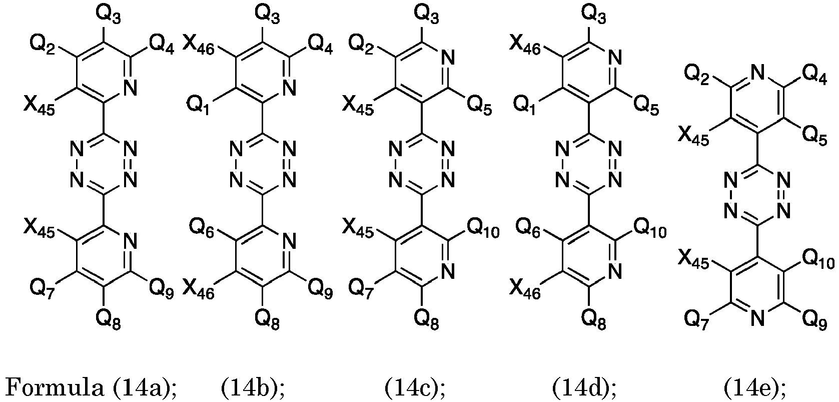 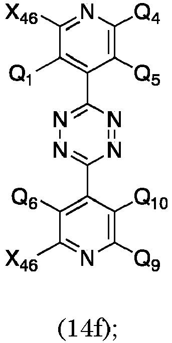
wherein Q₆ is as defined for Q₁ in Formula (14), Q₇ is as defined for Q₂ in Formula (14), Q₈ is as defined for Q₃ in Formula (14), Q₉ is as defined for Q₄ in Formula (14), and Q₁₀ is as defined for Q₅ in Formula (14),
wherein preferably at most two, more preferably at most one of Q₁, Q₂, Q₃, Q₄, and Q₅ are said -(S^{P})_{D}-R⁸⁷; wherein preferably at most two, more preferably at most one of Q₆, Q₇, Q₈, Q₉, and Q₁₀ are said -(S^{P})_{D}-R⁸⁷; wherein preferably the compound according to any one of Formulae (14a) to (14f) comprises at most four R⁸⁷ moieties, more preferably at most two R⁸⁷ moieties; wherein the compound according to Formulae (14a)-(14f) preferably comprises at least one R⁸⁷; wherein preferably at most six, more preferably at most four of Q₁, Q₂, Q₃, Q₄, Q₅,Q₆, Q₇, Q₈, Q₉, Q₁₀ are not hydrogen, wherein preferably the X₄₅ or X₄₆ groups are identical.

In a preferred embodiment, in Formulae (14b), (14b), and (14f) when Q₆ is a X₄₇ or -(S^{P})_{D}-R⁸⁷, then for Q₆ the X₄₇ and the R⁸⁷ are not a group in accordance with the definition of X₄₅.

In a preferred embodiment, in Formulae (14c)-(14f) when Q₁₀ is a X₄₇ or-(S^{P})_{D}-R⁸⁷, then for Q₁₀ the X₄₇ and the R⁸⁷ are not a group in accordance with the definition of X₄₅.

In a preferred embodiment, in Formulae (14a), (14c), and (14e) when Q₇ is a X₄₇ or -(S^{P})_{D}-R⁸⁷, then for Q₇ the X₄₇ and the R⁸⁷ are not a group in accordance with the definition of X₄₆.

In a preferred embodiment, in Formulae (14a), (14b), (14e), and (14f) when Q₉ is a X₄₇ or -(S^{P})_{D}-R⁸⁷, then for Q₉ the X₄₇ and the R⁸⁷ are not a group in accordance with the definition of X₄₆.

### X45

In a preferred embodiment, each X₄₅ individually is selected from the group consisting of N(X₅₀)₂, NX₅₀C(O)X₅₁, NX₅₀C(S)X₅₁, OH, SH, NX₅₀C(O)OX₅₁, NX₅₀C(S)OX₅₁, NX₅₀C(O)SX₅₁, NX₅₀C(S)SX₅₁, NX₅₀C(O)N(X₅₁)₂, NX₅₀C(S)N(X₅₁)₂, NX₅₀SO₂X₅₁, NX₅₀SO₃X₅₁, NX₅₀OX₅₁, and SO₂N(X₅₁)₂.

In a preferred embodiment, each X₄₅ individually is selected from the group consisting of N(X₅₀)₂, NX₅₀C(O)X₅₁, NX₅₀C(S)X₅₁, OH and SH.

In a preferred embodiment, each X₄₅ individually is selected from the group consisting of NX₅₀C(O)OX₅₁, NX₅₀C(S)OX₅₁, NX₅₀C(O)SX₅₁, NX₅₀C(S)SX₅₁, NX₅₀C(O)N(X₅₁)₂, NX₅₀C(S)N(X₅₁)₂, NX₅₀SO₂X₅₁, NX₅₀SO₃X₅₁, NX₅₀OX₅₁,

In a preferred embodiment, X₄₅ is selected from the group consisting of NHX₅₀, C(X₅₁)₂NH₂, CHX₅₁NH₂, CH₂N(X₅₀)₂, CH₂NHX₅₀, NHC(O)X₅₁, NHC(S)X₅₁, OH, and SH. In a preferred embodiment, X₄₅ is NHX₅₀. In a preferred embodiment, X₄₅ is C(X₅₁)₂NH₂. In a preferred embodiment, X₄₅ is CHX₅₁NH₂. In a preferred embodiment, X₄₅ is CH₂N(X₅₀)₂. In a preferred embodiment, X₄₅ is CH₂NHX₅₀.

In a preferred embodiment, X₄₅ is NH₂. In a preferred embodiment, X₄₅ is CH₂NH₂. In a preferred embodiment, X₄₅ is NHC(O)X₅₁. In a preferred embodiment, X₄₅ is NHC(S)X₅₁. In a preferred embodiment, X₄₅ is OH.

In a preferred embodiment, X₄₅ is SH. In a preferred embodiment, X₄₅ is SO₂NH₂.

### X₄6

In a preferred embodiment, X₄₆ is individually selected from the group consisting of N(X₅₀)₂, NX₅₀C(O)X₅₁, NX₅₀C(O)OX₅₁, andNX₅₀C(O)N(X₅₁)₂,. In a preferred embodiment, X₄₆ is selected from the group consisting of N(X₅₀)₂, and NX₅₀C(O)X₅₁,. In a preferred embodiment, X₄₆ is selected from the group consisting of NHX₅₀ and NHC(O)X₅₁. In a preferred embodiment, X₄₆ is NHX₅₀. In a preferred embodiment, X₄₆ is NH₂. In a preferred embodiment, X₄₆ is NHC(O)X₅₁.

### X₄₇

In a preferred embodiment, each X₄₇ is individually selected from the group consisting of F, -OH, -NH₂, -SO₃⁻, -NO₂, -CF₃, -SH, C₁-C₆ alkyl groups, C₆ aryl groups, C₄-C₅ heteroaryl groups, C₅-C₈ alkyl(hetero)aryl groups, C₅-C₈ (hetero)arylalkyl groups, C₄-C₈ alkylcycloalkyl groups, and C₄-C₈ cycloalkylalkyl groups. In a more preferred embodiment, each X₄₇ is individually selected from the group consisting of F, -SO₃; -NO₂, -CF₃, C₁-C₆ alkyl groups, C₆ aryl groups, C₄-C₅ heteroaryl groups, C₅-C₈ alkyl(hetero)aryl groups, C₅-C₈ (hetero)arylalkyl groups, C₄-C₈ alkylcycloalkyl groups, and C₄-C₈ cycloalkylalkyl groups.

In a preferred embodiment, the X₄₇ substituents do not contain heteroatoms.

In a preferred embodiment, the X₄₇ groups are not substituted.

In another preferred embodiment, the X₄₇ groups do not contain heteroatoms.

### X48

In a preferred embodiment, each X₄₈ is independently selected from the group consisting of hydrogen, C₁-C₄ alkyl groups, C₂-C₄ alkenyl groups, and C₄₋₆ (hetero)aryl groups. For X₄₈ the alkyl groups, alkenyl groups, and (hetero)aryl groups are optionally substituted with a moiety selected from the group consisting of -Cl, -F, -Br, -I, -OH, -NH₂, =O, -SH, -SO₃H, -PO₃H, -PO₄H₂ and -NO₂; and optionally contain at most two heteroatoms selected from the group consisting of -O-, -S-, -NH-, -P-, and -Si-, wherein the N, S, and P atoms are optionally oxidized. In a preferred embodiment, X₄₈ is C₁-C₄ alkyl.

In a preferred embodiment, the X₄₈ substituents do not contain heteroatoms.

In a preferred embodiment, the X₄₈ groups are not substituted.

In another preferred embodiment, the X₄₈ groups do not contain heteroatoms.

### X₄₉

In preferred embodiments, X₄₉ is selected from the group consisting of hydrogen, C₁-C₈ alkyl groups, C₂-C₈ alkenyl groups, C₂-C₈ alkynyl groups, C₆-C₁₂ aryl, C₂-C₁₂ heteroaryl, C₃-C₈ cycloalkyl groups, C₅-C₈ cycloalkenyl groups, C₃-C₁₂ alkyl(hetero)aryl groups, C₃-C₁₂ (hetero)arylalkyl groups, C₄-C₁₂ alkylcycloalkyl groups, C₄-C₁₂ cycloalkylalkyl groups, C₅-C₁₂ cycloalkyl(hetero)aryl groups and C₅-C₁₂ (hetero)arylcycloalkyl groups, wherein the X₄₉ groups not being hydrogen are optionally substituted with a moiety selected from the group consisting of -Cl, -F, -Br, -I, -OH, -NH₂, -SO₃H, -PO₃H, -PO₄H₂, -NO₂, -CF₃, =O, =NH, and -SH, and optionally contain one or more heteroatoms selected from the group consisting of O, S, NH, P, and Si, wherein the N, S, and P atoms are optionally oxidized, wherein the N atoms are optionally quaternized.

In preferred embodiments, X₄₉ is selected from the group consisting of hydrogen, C₁-C₄ alkyl groups, C₂-C₄ alkenyl groups, C₂-C₄ alkynyl groups, C₆-C₈ aryl, C₂-C₈ heteroaryl, C₃-C₆ cycloalkyl groups, C₅-C₆ cycloalkenyl groups, C₃-C₁₀ alkyl(hetero)aryl groups, C₃-C₁₀ (hetero)arylalkyl groups, C₄-C₈ alkylcycloalkyl groups, C₄-C₈ cycloalkylalkyl groups, C₅-C₁₀ cycloalkyl(hetero)aryl groups and C₅-C₁₀ (hetero)arylcycloalkyl groups, wherein the X₄₉ groups not being hydrogen are optionally substituted with a moiety selected from the group consisting of -Cl, -F, -Br, -I, -OH, -NH₂, -SO₃H, -PO₃H, -PO₄H₂, -NO₂, -CF₃, =O, =NH, and -SH, and optionally contain one or more heteroatoms selected from the group consisting of O, S, NH, P, and Si, wherein the N, S, and P atoms are optionally oxidized, wherein the N atoms are optionally quaternized. In a preferred embodiment, the X₄₉ substituents do not contain heteroatoms. In a preferred embodiment, the X₄₉ groups are not substituted. In another preferred embodiment, the X₄₉ groups do not contain heteroatoms.

### X₅₀

In a preferred embodiment, each X₅₀ is individually selected from the group consisting of hydrogen, X₄₈, and -(S^{P})_{D}-R⁸⁷. In a preferred embodiment, X₅₀ is X₄₈. In a preferred embodiment, X₅₀ is -(S^{P})_{D}-R⁸⁷. In a preferred embodiment, X₅₀ is H.

### X₅₁

In a preferred embodiment, each X₅₁ is individually selected from the group consisting of hydrogen, X₄₈, and -(S^{P})_{D}-R⁸⁷. In a preferred embodiment, X₅₁ is X₄₈. In a preferred embodiment, X₅₁ is -(S^{P})_{D}-R⁸⁷. In a preferred embodiment, X₅₁ is H.

### Q₁

In a preferred embodiment, in any one of Formulae (14)-(14f) Q₁ is selected from the group consisting of hydrogen, X₄₇, and -(S^{P})_{D}-R⁸⁷. In a preferred embodiment, Q₁ in any one of Formulae (14)-(14f) is hydrogen. In a preferred embodiment, Q₁ in any one of Formulae (14)-(14f) is X₄₇. In a preferred embodiment, Q₁ in any one of Formulae (14)-(14f) is a R⁸⁷, and preferably Q₂, Q₃, Q₄, Q₅, Q₆, Q₇, Q₈, Q₉, and Q₁₀ are X₄₅, X₄₆, or hydrogen.

### Q₂

In a preferred embodiment, Q₂ in any one of Formulae (14)-(14f) is selected from the group consisting of hydrogen X₄₇, and -(S^{P})_{D}-R⁸⁷. In a preferred embodiment, Q₂ in any one of Formulae (14)-(14f) is hydrogen. In a preferred embodiment, Q₂ is in any one of Formulae (14)-(14f) X₄₇. In a preferred embodiment, Q₂ in any one of Formulae (14)-(14f) is -(S^{P})_{D}-R⁸⁷, and preferably Q₁, Q₃, Q₄, Q₅, Q₆, Q₇, Q₈, Q₉, and Q₁₀ are X₄₅, X₄₆, or hydrogen.

### Q₃

In a preferred embodiment, Q₃ in any one of Formulae (14)-(14f) is selected from the group consisting of hydrogen X₄₇, and -(S^{P})_{D}-R⁸⁷. In a preferred embodiment, Q₃ in any one of Formulae (14)-(14f) is hydrogen. In a preferred embodiment, Q₃ in any one of Formulae (14)-(14f) is X₄₇. In a preferred embodiment, Q₃ in any one of Formulae (14)-(14f) is -(S^{P})_{D}-R⁸⁷, and preferably Q₁, Q₂, Q₄, Q₅, Q₆, Q₇, Q₈, Q₉, and Q₁₀ are X₄₅, X₄₆, or hydrogen.

### Q₄

In a preferred embodiment, Q₄ in any one of Formulae (14)-(14f) is selected from the group consisting of hydrogen X₄₇, and -(S^{P})_{D}-R⁸⁷. In a preferred embodiment, Q₄ in any one of Formulae (14)-(14f) is hydrogen. In a preferred embodiment, Q₄ in any one of Formulae (14)-(14f) is X₄₇. In a preferred embodiment, Q₄ in any one of Formulae (14)-(14f) is -(S^{P})_{D}-R⁸⁷, and preferably Q₁, Q₂, Q₃, Q₅, Q₆, Q₇, Q₈, Q₉ and Q₁₀ are X₄₅, X₄₆, or hydrogen.

### Q₅

In a preferred embodiment, Q₅ in any one of Formulae (14)-(14f) is selected from the group consisting of hydrogen X₄₇, and -(S^{P})_{D}-R⁸⁷. In a preferred embodiment, Q₅ in any one of Formulae (14)-(14f) is hydrogen. In a preferred embodiment, Q₅ in any one of Formulae (14)-(14f) is X₄₇. In a preferred embodiment, Q₅ is -(S^{P})_{D}-R⁸⁷, and preferably Q₁, Q₂, Q₃, Q₄, Q₆, Q₇, Q₈, Q₉ and Q₁₀ are X₄₅, X₄₆, or hydrogen.

### Q₆

In a preferred embodiment, Q₆ in any one of Formulae (14)-(14f) is selected from the group consisting of hydrogen X₄₇, and -(S^{P})_{D}-R⁸⁷. In a preferred embodiment, Q₆ in any one of Formulae (14)-(14f) is hydrogen. In a preferred embodiment, Q₆ in any one of Formulae (14)-(14f) is X₄₇. In a preferred embodiment, Q₆ in any one of Formulae (14)-(14f) is -(S^{P})_{D}-R⁸⁷, and preferably Q₁, Q₂, Q₃, Q₄, Q₅, Q₇, Q₈, Q₉ and Q₁₀ are X₄₅, X₄₆, or hydrogen.

### Q₇

In a preferred embodiment, Q₇ in any one of Formulae (14)-(14f) is selected from the group consisting of hydrogen X₄₇, and -(S^{P})_{D}-R⁸⁷. In a preferred embodiment, Q₇ in any one of Formulae (14)-(14f) is hydrogen. In a preferred embodiment, Q₇ in any one of Formulae (14)-(14f) is X₄₇. In a preferred embodiment, Q₇ in any one of Formulae (14)-(14f) is -(S^{P})_{D}-R⁸⁷, and preferably Q₁, Q₂, Q₃, Q₄, Q₅, Q₆, Q₈, Q₉ and Q₁₀ are X₄₅, X₄₆, or hydrogen.

### Q₈

In a preferred embodiment, Q₈ in any one of Formulae (14)-(14f) is selected from the group consisting of hydrogen X₄₇, and -(S^{P})_{D}-R⁸⁷. In a preferred embodiment, Q₈ in any one of Formulae (14)-(14f) is hydrogen. In a preferred embodiment, Q₈ in any one of Formulae (14)-(14f) is X₄₇. In a preferred embodiment, Q₈ in any one of Formulae (14)-(14f) is -(S^{P})_{D}-R⁸⁷, and preferably Q₁, Q₂, Q₃, Q₄, Q₅, Q₆, Q₇, Q₉ and Q₁₀ are X₄₅, X₄₆, or hydrogen.

### Q₉

In a preferred embodiment, Q₉ in any one of Formulae (14)-(14f) is selected from the group consisting of hydrogen X₄₇, and -(S^{P})_{D}-R⁸⁷. In a preferred embodiment, Q₉ in any one of Formulae (14)-(14f) is hydrogen. In a preferred embodiment, Q₉ in any one of Formulae (14)-(14f) is X₄₇. In a preferred embodiment, Q₉ in any one of Formulae (14)-(14f) is -(S^{P})_{D}-R⁸⁷, and preferably Q₁, Q₂, Q₃, Q₄, Q₅, Q₆, Q₇, Q₈ and Q₁₀ are X₄₅, X₄₆, or hydrogen.

### Q₁₀

In a preferred embodiment, Q₁₀ in any one of Formulae (14)-(14f) is selected from the group consisting of hydrogen X₄₇, and -(S^{P})_{D}-R⁸⁷. In a preferred embodiment, Q₁₀ in any one of Formulae (14)-(14f) is hydrogen. In a preferred embodiment, Q₁₀ in any one of Formulae (14)-(14f) is X₄₇. In a preferred embodiment, Q₁₀ in any one of Formulae (14)-(14f) is -(S^{P})_{D}-R⁸⁷, and preferably Q₁, Q₂, Q₃, Q₄, Q₅, Q₆, Q₇, Q₈ and Q₉ are X₄₅, X₄₆, or hydrogen.

### Formula (14a)

In a preferred embodiment, the compound according to the invention is a compound according to Formula (14a), wherein preferably, each individual X₄₅ and Q₂-Q₄, Q₇-Q₉ are as described herein.

In a preferred embodiment, the compound according to the invention is a compound according to Formula (14a), wherein both X₄₅ are the same and are selected from the group consisting of NH₂, NHC(O)X₅₁, NX₅₀C(O)OX₅₁, NX₅₀C(O)N(X₅₁)₂, NX₅₀C(S)N(X₅₁)₂, OH, and SH; and Q₂-Q₄, Q₇-Q₉ are hydrogen.

In a preferred embodiment, the compound according to the invention is a compound according to Formula (14a), wherein both X₄₅ are the same and are selected from the group consisting of NH₂, NHC(O)X₅₁, and OH; and Q₂-Q₄, Q₇-Q₉ are hydrogen.

### Formula (14b)

In a preferred embodiment, the compound according to the invention is a compound according to Formula (14b), wherein preferably, each individual X₄₆ and Q₁, Q₃-Q₄, Q₆, Q₈-Q₉ are as described herein.

In a preferred embodiment, the compound according to the invention is a compound according to Formula (14b), wherein both X₄₆ are the same and are NH₂ or NHC(O)X₅₁, and Q₁, Q₃-Q₄, Q₆, Q₈-Q₉ are hydrogen.

### Formula (14c)

In a preferred embodiment, the compound according to the invention is a compound according to Formula (14c), wherein preferably, each individual X₄₅ and Q₂-Q₃, Q₅, Q₇,Q₈, Q₁₀ are as described herein.

In a preferred embodiment, the compound according to the invention is a compound according to Formula (14c), wherein both X₄₅ are the same and are selected from the group consisting of NH₂, NHC(O)X₅₁, NX₅₀C(O)OX₅₁, NX₅₀C(O)N(X₅₁)₂, NX₅₀C(S)N(X₅₁)₂, OH, and SH; and Q₂-Q₃, Q₅, Q₇,Q₈, Q₁₀ are hydrogen.

In a preferred embodiment, the compound according to the invention is a compound according to Formula (14c), wherein both X₄₅ are the same and are NH₂, NHC(O)X₅₁, and OH, and Q₂-Q₃, Q₅, Q₇,Q₈, Q₁₀ are hydrogen.

### Formula (14d)

In a preferred embodiment, the compound according to the invention is a compound according to Formula (14d), wherein preferably each individual X₄₆ and Q₁, Q₃, Q₅, Q₆, Q₈, Q₁₀ are as described herein.

In a preferred embodiment, the compound according to the invention is a compound according to Formula (14d), wherein both X₄₆ are the same and are NH₂ or NHC(O)X₅₁, and Q₁, Q₃, Q₅, Q₆, Q₈, Q₁₀ are hydrogen.

### Formula (14e)

In a preferred embodiment, the compound according to the invention is a compound according to Formula (14e), wherein preferably each individual X₄₅ and Q₂, Q₄, Q₅, Q₇, Q₉, Q₁₀ are as described herein.

In a preferred embodiment, the compound according to the invention is a compound according to Formula (14e), wherein both X₄₅ are the same and are selected from the group consisting of NH₂, NHC(O)X₅₁, NX₅₀C(O)OX₅₁, NX₅₀C(O)N(X₅₁)₂, NX₅₀C(S)N(X₅₁)₂, OH, and SH; and Q₂, Q₄, Q₅, Q₇, Q₉, Q₁₀ are hydrogen.

In a preferred embodiment, the compound according to the invention is a compound according to Formula (14e), wherein both X₄₅ are the same and are NH₂, NHC(O)X₅₁, and OH, and Q₂, Q₄, Q₅, Q₇, Q₉, Q₁₀ are hydrogen.

### Formula (14f)

In a preferred embodiment, the compound according to the invention is a compound according to Formula (14f), wherein preferably each individual X₄₆ and Q₁, Q₄, Q₅, Q₆, Q₉, Q₁₀ are as described herein.

In a preferred embodiment, the compound according to the invention is a compound according to Formula (14f), wherein both X₄₆ are the same and are NH₂ or NHC(O)X₅₁, and Q₁, Q₄, Q₅, Q₆, Q₉, Q₁₀ are hydrogen.

In a preferred embodiment, the Cleaving Agent is according to Formula (14) and comprises at least one, preferably two, X₄₅ groups.

In a preferred embodiment, the Cleaving Agent is according to Formula (14a), (14c), or (14e).

### R87

Preferably, a R⁸⁷ in relation to the invention is a moiety that modulates the pharmacokinetics of a compound. Thus, in a preferred embodiment R⁸⁷ is a pharmacokinetics-modulating moiety (P^{K} moiety). The functions of the R⁸⁷ include, but are not limited to, one or more of delaying clearance of said compound, affecting the volume of distribution of said compound (e.g. reducing or increasing the volume of distribution), affecting the biodistribution of said compound, achieving spatial control over its reaction with the Trigger, affecting (more particularly avoiding) the metabolism of said compound, and/or affecting (more particularly avoiding) the (undesired) sticking or (undesired) uptake of said compound to tissues. The skilled person is well aware of such groups, and how to synthesize these.

In preferred embodiments, each R⁸⁷ is independently selected from the group consisting of organic molecules, inorganic molecules, organometallic molecules, resins, beads, glass, microparticles, nanoparticles, gels, surfaces, and cells. Preferably, R⁸⁷ is independently selected from the group consisting of organic molecules, and inorganic molecules.

In a preferred embodiment the R⁸⁷ serves to increase the blood circulation time, increasing reaction time with the Trigger.

In a preferred embodiment the R⁸⁷ serves modulate the pharmacokinetics of a reaction product between a dienophile of this invention and a diene as defined herein.

Without wishing to be bound by theory, it is believed that the function and performance of the tetrazine as defined herein in a bioorthogonal reaction is not significantly affected by the nature of the R⁸⁷.

In a preferred embodiment, each R⁸⁷ is individually selected from the group consisting of biomolecule, polymer, peptide, peptoid, dendrimer, protein, carbohydrate, oligonucleotide, oligosaccharide, aptamer, steroid, lipid, albumin, albumin-binding moiety, dye moiety, fluorescent moiety, imaging probe, and a Targeting Agent (T^{T}); and wherein the R⁸⁷ is optionally bound to the tetrazine via a Spacer (S^{P}). Typically, a suitable polymer as a R⁸⁷ is polyethyleneglycol (PEG). Such suitable PEG includes PEG with a number of repeating units in a range of from 2 to 4000, and PEG with a molecular weight in a range of from 200 Da to 100,000 Da.

In a preferred embodiment, the R⁸⁷ is a moiety according to Formula (5).

In a preferred embodiment, the R⁸⁷ is a moiety according to Formula (5), and is directly linked to the remainder of a compound according to any one of Formulae (14)-(14f), for example without a spacer S^{P} between the R⁸⁷ and the remainder of the moiety Yₐ or Y_{b} of Formula (14) or the pyridyl moiety of the compound according to any one of Formulae (14a)-(14f).

In a preferred embodiment, the R⁸⁷ is a moiety according to Formula (5), and is directly linked to the remainder of a, for example without a spacer S^{P} between the R⁸⁷ and the remainder of the moiety Yₐ or Y_{b} of any one of Formulae (4), (11), and (14); or the pyridyl moiety of the compound according to any one of Formulae (14a)-(14f), and if attached to an amine functionality of X₄₅ or X₄₆, z in Formula (5) is not 0.

In a preferred embodiment, the R⁸⁷ is linked to the remainder of a compound via a spacer S^{P} as defined herein.

In a preferred embodiment, the R⁸⁷ is linked to the remainder of a compound optionally via a spacer S^{P} as defined herein and each R⁸⁷ is individually selected from the group consisting of biomolecule, polymer, peptide, peptoid, dendrimer, protein, carbohydrate, oligonucleotide, oligosaccharide, lipid, micelle, liposomes, polymersome, particle, nanoparticle, microparticle, bead, gel, metal complex, organometallic moiety, albumin, albumin-binding moiety, dye moiety, fluorescent moiety, imaging probe, and a Targeting Agent (T^{T}).

In a preferred embodiment, one or multiple copies of the compound of the invention may be conjugated to a R⁸⁷ that is a membrane translocation moiety (e.g. adamantine, poly-lysine/arginine, TAT, human lactoferrin) to reach an intracellular Prodrug. Exemplary references regarding such moieties include:
Trends in Biochemical Sciences, 2015,. 40, 12, 749; J. Am. Chem. Soc. 2015, 137, 12153-12160; Pharmaceutical Research, 2007, 24, 11, 1977.

With respect to application in a cellular environment, such as in vivo, depending on the position of the Trigger-Construct (e.g. inside the cell or outside the cell) the Cleaving Agent is designed to be able to effectively reach this Trigger-Construct. Therefore, the Cleaving Agent can for example be tailored by varying its log P value, its reactivity or its charge, and this can optionally be achieved by the R⁸⁷.

In a preferred embodiment, the tetrazine compounds of the invention comprise an imaging moiety instead of a R⁸⁷. In other embodiments, the R⁸⁷ is or comprises an imaging moiety. In this preferred embodiment the imaging moiety is bound to the remainder of the compounds of the invention in the same way as the R⁸⁷. In this embodiment the R⁸⁷ equals an imaging moiety. In a preferred embodiment, the compounds of the invention can comprise one or more imaging moieties and one or more R⁸⁷ moieties.

In a preferred embodiment, the R⁸⁷ is or comprises an imaging moiety.

Preferred imaging moieties are radionuclide-chelates complexes, radiolabeled molecules (e.g. with ¹⁸F, ¹²⁴I), and fluorescent dyes.

In a preferred embodiment, the R⁸⁷ is an imaging moiety that comprises at least one ¹⁸F isotope.

In a preferred embodiment, the R⁸⁷ comprises a chelating moiety, preferably a chelating moiety as described herein.

In a preferred embodiment, the R⁸⁷ includes but is not limited to amino acids, nucleosides, nucleotides, carbohydrates, and biopolymer fragments, such as oligo- or polypeptides, oligo- or polypeptoids, or oligo- or polylactides, or oligo- or poly-carbohydrates, oligonucleotides, varying from 2 to 200, particularly 2 to 113, preferably 2 to 50, more preferably 2 to 24 and more preferably 2 to 12 repeating units.

According to one embodiment, the Cleaving Agent can be a multimeric compound, comprising a plurality of tetrazines. According to one preferred embodiment, the Cleaving Agent can be a multimeric compound, comprising a plurality of tetrazines bound to one R⁸⁷. These multimeric compounds can be but are not limited to biomolecules, peptide, peptoid, protein, oligonucleotide, oligosaccharide, polymersome, bead, gel, polymers, dendrimers, liposomes, micelles, particles, polymer particles, or other polymeric constructs.

In a preferred embodiment, the R⁸⁷ is a polymer. This includes linear or branched polyalkylene glycols such as polyethylene glycol (PEG) or polypropylene glycol (PPG) chains varying from 2 to 200, particularly 2 to 113, preferably 2 to 50, more preferably 2 to 24 and more preferably 2 to 12 repeating units. It is preferred that when polyalkylene glycols such as PEG and PPG polymers are only bound via one end of the polymer chain, that the other end is terminated with -OCH₃, -OCH₂CH₃, OCH₂CH₂CO₂H.

Other polymeric R⁸⁷ moieties are polymers and copolymers such as poly-(2-oxazoline, poly(N-(2-hydroxypropyl)methacrylamide) (HPMA), polylactic acid (PLA), polylactic-glycolic acid (PLGA), polyglutamic acid (PG), dextran, polyvinylpyrrolidone (PVP), poly(1-hydroxymethylethylene hydroxymethyl-formal (PHF). Other exemplary polymers are polysaccharides, glycopolysaccharides, glycolipids, polyglycoside, polyacetals, polyketals, polyamides, polyethers, polyesters. Examples of naturally occurring polysaccharides that can be used are cellulose, amylose, dextran, dextrin, levan, fucoidan, carraginan, inulin, pectin, amylopectin, glycogen, lixenan, agarose, hyaluronan, chondroitinsulfate, dermatansulfate, keratansulfate, alginic acid and heparin. In yet other exemplary embodiments, the polymer is a copolymer of a polyacetal/polyketal and a hydrophilic polymer selected from the group consisting of polyacrylates, polyvinyl polymers, polyesters, polyorthoesters, polyamides, oligopeptides, polypeptides and derivatives thereof. Exemplary preferred polymeric R⁸⁷ moieties are PEG, HPMA, PLA, PLGA, PVP, PHF, dextran, oligopeptides, and polypeptides.

In some aspects of the invention polymeric R⁸⁷ moieties have a molecular weight ranging from 2 to 200 kDa, from 2 to 100 kDa, from 2 to 80 kDa, from 2 to 60 kDa, from 2 to 40 kDa, from 2 to 20 kDa, from 3 to 15 kDa, from 5 to 10 kDa, from 500 dalton to 5 kDa.

Other exemplary R⁸⁷ moieties are dendrimers, such as polypropylene imine) (PPI) dendrimers, PAMAM dendrimers, and glycol based dendrimers.

In a preferred embodiment the R⁸⁷ equals Group R^{M}.

In a preferred embodiment the R⁸⁷ serves to increase the blood circulation time, increasing reaction time with the Trigger.

In a preferred embodiment the R⁸⁷ serves to modulate the pharmacokinetics of a reaction product between a dienophile and diene of this invention.

In a preferred embodiment, the Cleaving Agent is designed to be cell impermeable as a result of its log P value or its charge, and this can optionally be achieved by the R⁸⁷.

In embodiments where it is required that the Cleaving Agent has an extracellular volume of distribution it is preferred that the Log P of the Cleaving Agent is at most 2, preferably at most 1, more preferably at most 0, even more preferably at most -1.

In embodiments where it is required that the Cleaving Agent has an intracellular volume of distribution it is preferred that the Log P of the Cleaving Agent is at least -1, preferably at least 0, more preferably at least 1, even more preferably at least 2.

In embodiments where it is required that the Cleaving Agent has an extracellular volume of distribution it is preferred that the Agent has a negative net charge at pH 7.

In embodiments where it is required that the Cleaving Agent has an intracellular volume of distribution it is preferred that the Agent has a molecular weight of less than 1000 Da, preferably less than 500 Da.

In embodiments where it is required that the Cleaving Agent has an extracellular volume of distribution it is preferred that the Agent has a molecular weight of more than 500 Da, preferably more than 1 kDa, more preferably more than 2 kDa.

In embodiments where it is required that the Cleaving Agent has slow or inefficient extravasation from circulation into tissues it is preferred that the Agent has a molecular weight of more than 5 kDa, preferably more than 60 Da, more preferably more than 150 Da, even more preferably more than 500 kDa,

In preferred embodiments wherein the Cleaving Agent is not cell permeable, R⁸⁷ can be a protein or polymer,

In preferred embodiments the R⁸⁷ reduces the extravasation of the Cleaving Agent from blood into target tissue, by virtue of its large size and/or by the presence of a clearance-directing group (i.e. as R₉₈). For example, R⁸⁷ being a PLGA microparticle will allow efficient IEDDA reaction with Administration Agent in circulation but will hamper efficient extravasation of the Cleaving Agent into tumor tissue, and will result in rapid clearance by the liver. Likewise, R⁸⁷ being an albumin protein modified with ca. 10 galactose moieties (i.e. clearance-directing groups, as R₉₈) to ensure rapid uptake by the liver, affords efficient IEDDA reaction in blood with no or minimal in mimizing extravasation into tumor tissue. Reference is made to [Rossin et al J. Nucl. Med. 2013, 4, 11, 1989-1995]. Likewise R⁸⁷ can be a small moiety comprising a Clearance-directing group to favor IEDDA reaction in blood vs. in tumor tissue.

Conversely, if whole body extracellular Label cleavage is desired the R⁸⁷ can be a 20 or 40 kDa PEG or albumin or an albumin-binding moiety ensuring prolonged retention in circulation, optionally combined with EPR based targeting of tumor tissue.

In one embodiment the Administration Agent specifically binds or complexes with a cell surface molecule, such as a cell surface receptor or antigen, for a given cell population. Following specific binding or complexing with the receptor, the cell is permissive for uptake of the Administration Agent, which then internalizes into the cell. The subsequently administered Cleaving Agent will then enter the cell and cleave the Administration Agent, releasing the Effector Moiety inside the cell. In another embodiment the Administration Agent specifically binds or complexes with a cell surface molecule, such as a cell surface receptor or antigen, for a given cell population. Following specific binding or complexing the receptor, the cell is not permissive for uptake of the Administration Agent. The subsequently administered Cleaving Agent will then cleave the Administration Agent on the outside of the cell.

In an image cycling embodiment, centered on sequential imaging procedures of the same or different Primary Targets, it is preferred that the Cleaving Agent acts systemically (i.e. in the whole body). In other image cycling embodiments it is preferred that the Cleaving Agent comprises a R⁸⁷ that is a T^{T} and selectively cleaves the Label at the Primary Target being imaged.

### Targeting Agent T^{T}

In preferred embodiments, a Targeting Agent T^{T} is used to bind a Primary Target.

In preferred embodiments, the dienes of this invention are bound to a Targeting Agent T^{T}. A T^{T} bound to a diene can be advantageously used in relation to the invention, as a T^{T} may direct the diene to, or retain the diene at a location where the Label needs to be released from the compound of Formula (1). For example, to increase the target/non-target ratio of the Label, it may be preferred to direct the diene to, or retain the diene in the blood if that is not the target. Then, the Cleaving Agent will mainly release the Label in the blood, after which the Label is rapidly cleared. The Label at the target (e.g. a tumor) is not released, thus increasing the target/non-target ratio of the Label.

In preferred embodiments the T^{T} is used in affecting (more particularly avoiding) the metabolism of the diene, and/or affecting (more particularly avoiding) the (undesired) sticking or (undesired) uptake of the diene to tissues. In preferred embodiments, the T^{T} is an Administration Agent.

The skilled person is well aware of such groups, and how to synthesize these.

In a preferred embodiment, the T^{T} is selected from the group consisting of polymer, polymer particle, peptide, peptoid, dendrimer, protein, carbohydrate, oligonucleotide, oligosaccharide, lipid, liposome, albumin, albumin-binding moiety. Typically, a suitable polymer is polyethyleneglycol (PEG). Such suitable PEG includes PEG with a number of repeating units in a range of from 2 to 4000, and PEG with a molecular weight in a range of from 200 Da to 100,000 Da.

In a preferred embodiment, the T^{T} bound to the diene includes but is not limited to amino acids, nucleosides, nucleotides, carbohydrates, and biopolymer fragments, such as oligo- or polypeptides, oligo- or polypeptoids, or oligo- or polylactides, or oligo- or poly-carbohydrates, oligonucleotides, varying from 2 to 200, particularly 2 to 113, preferably 2 to 50, more preferably 2 to 24 and more preferably 2 to 12 repeating units.

In a preferred embodiment, the T^{T} bound to the diene is a polymer. This includes linear or branched polyalkylene glycols such as polyethylene glycol (PEG) or polypropylene glycol (PPG) chains varying from 2 to 200, particularly 2 to 113, preferably 2 to 50, more preferably 2 to 24 and more preferably 2 to 12 repeating units. It is preferred that when polyalkylene glycols such as PEG and PPG polymers are only bound via one end of the polymer chain, that the other end is terminated with -OCH₃, -OCH₂CH₃, OCH₂CH₂CO₂H.

Other suitable polymeric T^{T} Moieties include polymers and copolymers such as poly-(2-oxazoline), poly(N-(2-hydroxypropyl)methacrylamide) (HPMA), polylactic acid (PLA), polylactic-glycolic acid (PLGA), polyglutamic acid (PG), dextran, polyvinylpyrrolidone (PVP), poly(1-hydroxymethylethylene hydroxymethyl-formal (PHF). Other exemplary polymers are polysaccharides, glycopolysaccharides, glycolipids, polyglycoside, polyacetals, polyketals, polyamides, polyethers, polyesters. Examples of naturally occurring polysaccharides that can be used are cellulose, amylose, dextran, dextrin, levan, fucoidan, carraginan, inulin, pectin, amylopectin, glycogen, lixenan, agarose, hyaluronan, chondroitinsulfate, dermatansulfate, keratansulfate, alginic acid and heparin. In yet other exemplary embodiments, the polymer is a copolymer of a polyacetal/polyketal and a hydrophilic polymer selected from the group consisting of polyacrylates, polyvinyl polymers, polyesters, polyorthoesters, polyamides, oligopeptides, polypeptides and derivatives thereof. Exemplary preferred polymeric T^{T} moieties are PEG, HPMA, PLA, PLGA, PVP, PHF, dextran, oligopeptides, and polypeptides.

In some aspects of the invention polymeric T^{T} moieties have a molecular weight ranging from 2 to 200 kDa, from 2 to 100 kDa, from 2 to 80 kDa, from 2 to 60 kDa, from 2 to 40 kDa, from 2 to 20 kDa, from 3 to 15 kDa, from 5 to 10 kDa, from 500 dalton to 5 kDa.

Other exemplary T^{T} Moieties are dendrimers, such as polypropylene imine) (PPI) dendrimers, PAMAM dendrimers, and glycol based dendrimers.

In a preferred embodiment, the diene comprises an imaging moiety, for example a optical or fluorescent dye, or a radioactive isotope such as ¹⁸F. Preferably, the diene does not comprise a radionuclide.

It will be understood that chemical modifications may also be made to the tetrazine and the Targeting Agent, in order to make reactions of that compound more convenient for purposes of preparing conjugates of the invention.

In preferred embodiments, a Targeting Agent, T^{T}, binds to a Primary Target. In order to allow specific targeting of the above-listed Primary Targets, the Targeting Agent T^{T} can comprise compounds including but not limited to antibodies, antibody derivatives, antibody fragments, antibody (fragment) fusions (e.g. bi-specific and tri-specific mAb fragments or derivatives), proteins, peptides, e.g. octreotide and derivatives, VIP, MSH, LHRH, chemotactic peptides, cell penetrating peptide, membrane translocation moiety, bombesin, elastin, peptide mimetics, organic compounds, inorganic compounds, carbohydrates, monosaccharides, oligosacharides, polysaccharides, oligonucleotides, aptamers, viruses, whole cells, phage, drugs, polymers, liposomes, chemotherapeutic agents, receptor agonists and antagonists, cytokines, hormones, steroids, toxins. Examples of organic compounds envisaged within the context of the present invention are, or are derived from, dyes, compounds targeting CAIX and PSMA, estrogens, e.g. estradiol, androgens, progestins, corticosteroids, methotrexate, folic acid, and cholesterol.

According to a particular embodiment of the present invention, the Primary Target is a receptor and a Targeting Agent is employed, which is capable of specific binding to the Primary Target. Suitable Targeting Agents include but are not limited to, the ligand of such a receptor or a part thereof which still binds to the receptor, e.g. a receptor binding peptide in the case of receptor binding protein ligands. Other examples of Targeting Agents of protein nature include insulin, transferrin, fibrinogen-gamma fragment, thrombospondin, claudin, apolipoprotein E, Affibody molecules such as for example ABY-025, Ankyrin repeat proteins, ankyrin-like repeat proteins, interferons, e.g. alpha, beta, and gamma interferon, interleukins, lymphokines, colony stimulating factors and protein growth factor, such as tumor growth factor, e.g. alpha, beta tumor growth factor, platelet-derived growth factor (PDGF), uPAR targeting protein, apolipoprotein, LDL, annexin V, endostatin, and angiostatin. Alternative examples of targeting agents include DNA, RNA, PNA and LNA which are e.g. complementary to the Primary Target.

Examples of peptides as targeting agents include LHRH receptor targeting peptides, EC-1 peptide, RGD peptides, HER2-targeting peptides, PSMA targeting peptides, somatostatin-targeting peptides, bombesin. Other examples of targeting agents include lipocalins, such as anticalins. One particular embodiment uses Affibodies^{™} and multimers and derivatives.

In a preferred embodiment the T^{T} is selected from antibodies and antibody derivatives such as antibody fragments, fragment fusions, proteins, peptides, peptide mimetics, organic molecules, dyes, fluoresencent molecules, and enzyme substrates.

In a preferred embodiment the T^{T} being an organic molecule has a molecular weight of less than 2000 Da, more preferably less than 1500 Da, more preferably less than 1000 Da, even more preferably less than 500 Da.

In another preferred embodiment the T^{T} is selected from antibody fragments, fragment fusions, and other antibody derivatives that do not contain a Fc domain.

In another embodiment the T^{T} is a polymer. Typical polymers used in this embodiment include but are not limited to polyethyleneglycol (PEG), poly(N-(2-hydroxypropyl)methacrylamide) (HPMA), polylactic acid (PLA), polylactic-glycolic acid (PLGA), polyglutamic acid (PG), polyvinylpyrrolidone (PVP), poly(1-hydroxymethylethylene hydroxymethyl-formal (PHF). Other examples are copolymers of a polyacetal/polyketal and a hydrophilic polymer selected from the group consisting of polyacrylates, polyvinyl polymers, polyesters, polyorthoesters, polyamides, oligopeptides, polypeptides and derivatives thereof. Other examples are oligopeptides, polypeptides, glycopolysaccharides, and polysaccharides such as dextran and hyaluronan, In addition reference is made to [G. Pasut, F.M. Veronese, Prog. Polym. Sci. 2007, 32, 933-961]. In preferred embodiments the T^{T} being a polymer accumulates at the Primary Target by virtue of the EPR effect.

According to a further particular embodiment of the invention, the Primary Target and Targeting Agent are selected so as to result in the specific or increased targeting of a tissue or disease, such as cancer, an inflammation, an infection, a cardiovascular disease, e.g. thrombus, atherosclerotic lesion, hypoxic site, e.g. stroke, tumor, cardiovascular disorder, brain disorder, apoptosis, angiogenesis, an organ, and reporter gene/enzyme. This can be achieved by selecting Primary Targets with tissue-, cell- or disease- specific expression. For example, the CC49 antibody targets TAG72, the expression of which is limited in normal tissues, but receptors are overexpressed in various solid tumor cell types.

As used herein, a T^{T} that "specifically binds or complexes with" or "targets" a cell surface molecule, an extracellular matrix target, or another target, preferentially associates with the target via intermolecular forces. For example, the ligand can preferentially associate with the target with a dissociation constant (K_{d} or K_{D}) of less than 50 nM, less than 5 nM, or less than 500 pM.

In another embodiment the targeting agent T^{T} localizes in the target tissue by means of the EPR effect. An exemplary T^{T} for use in with the EPR effect is a polymer.

In preferred embodiments the targeting agent T^{T} localizes or has retention in a particular system, tissue, or organ in the body, for example, blood circulation, lymphatic system, the nervous system, the digestion system, RES system, or organs such as the heart or kidney. For example, microparticles will localize in the liver, large hydrophilic polymers will have retention in circulation. Likewise, use of an albumin binding moiety as T^{T} will result in prolonged retention in circulation.

In preferred embodiments, T^{T} is used to modify the pharmacokinetics of the moiety it is attached to. This can include, but is not limited to, delaying the blood clearance of said moiety, affecting the volume of distribution of said moiety (e.g. reducing or increasing the volume of distribution), affecting the metabolism of said moiety, and/or affecting (preferably avoiding) the sticking or uptake of said moiety to non-target tissues. Exemplary T^{T}'s in this regard are polymer, peptide, peptoid, dendrimer, protein, carbohydrate, oligonucleotide, oligosaccharide, lipid, liposome, micelle, nanoparticle, microparticle, albumin, albumin-binding moiety, and small to medium sized organic molecules such as steroids and dyes. Typically, a suitable polymer is polyethyleneglycol (PEG) or polypropyleneglycol (PPG). Such suitable PEG includes PEG with a number of repeating units in a range of from 2 to 4000, and PEG with a molecular weight in a range of from 200 Da to 100,000 Da.

In preferred embodiments, when a T^{T} is an Administration Agent, it equals C^{B}.

### R₃₂

R₃₂ is a conjugation moiety, which is a chemical group that can be used for binding, conjugation or coupling to *e.g.* a Label, Administration Agent, Construct C, R₉₈, L^{A}, L^{B}, S^{L}, S^{P}, and L^{C}. The person skilled in the art is aware of the myriad of strategies that are available for the chemoselective or -unselective or enzymatic coupling or conjugation of one molecule or construct to another.

In preferred embodiments, R₃₂ is a moiety that allows conjugation to a protein comprising natural and/or non-natural amino acids. Moieties suitable for conjugation are known to the skilled person. Conjugation strategies are for example found in [O. Boutureira, G.J.L. Bernardes, Chem. Rev., 2015, 115, 2174-2195].

In particularly favourable embodiments, R₃₂ is selected from the group consisting of N-maleimidyl groups, halogenated N-alkylamido groups, sulfonyloxy N-alkylamido groups, vinyl sulfone groups, activated carboxylic acids, benzenesulfonyl halides, ester groups, carbonate groups, sulfonyl halide groups, thiol groups or derivatives thereof, C₂₋₆ alkenyl groups, C₂₋₆ alkynyl groups, C₇₋₁₈ cycloalkynyl groups, C₅₋₁₈ heterocycloalkynyl groups, bicyclo[6.1.0]non-4-yn-9-yl] groups, C₃₋₁₂ cycloalkenyl groups, azido groups, phosphine groups, nitrile oxide groups, nitrone groups, nitrile imine groups, isonitrile groups, diazo groups, ketone groups, (O-alkyl)hydroxylamino groups, hydrazine groups, halogenated N-maleimidyl groups , aryloxymaleimides, dithiophenolmaleimides, bromo- and dibromopyridazinediones, 2,5-dibromohexanediamide groups, alkynone groups, 3-arylpropiolonitrile groups, 1,1-bis(sulfonylmethyl)-methylcarbonyl groups or elimination derivatives thereof, carbonyl halide groups, allenamide groups, 1,2-quinone groups, isocyanate groups, isothiocyanate groups, aldehyde groups, triazine groups, squaric acids, 2-imino-2-methoxyethyl groups, (oxa)norbornene groups, (imino)sydnones, methylsulfonyl phenyloxadiazole groups, aminooxy groups, 2-amino benzamidoxime groups, ethynylphosphonamidates, groups reactive in the Pictet- Spengler ligation and hydrazino- Pictet- Spengler (HIPS) ligation, DNA intercalator, tetrazine groups, and photocrosslinkers.

In preferred embodiments, R₃₂ is an N-maleimidyl group connected to the remaining part of the compound according to Formula (20) via the N atom of the N-maleimidyl group. In other preferred embodiments R₃₂ is selected from the group consisting of, hydroxyl groups, amine groups, halogens, vinyl pyridine groups, disulfide groups, pyridyl disulfide groups, sulfonyloxy groups, mercaptoacetamide groups, anhydride groups, sulfonylated hydroxyacetamido groups, sulfonyl chlorides, thiosemicarbazone, hydrazine carboxylate, and arylhydrazide.

In other embodiments R₃₂ is group that can be connected to another group by means of an enzyme, for example sortase or Tubulin tyrosine ligase.

### R'

In preferred embodiments, each R' is independently selected from the group consisting of hydrogen, C₁-C₆ alkylene groups, C₂-C₆ alkenylene groups, C₂-C₆ alkynylene groups, C₆ arylene, C₄-C₅ heteroarylene, C₃-C₆ cycloalkylene groups, C₅-C₈ cycloalkenylene groups, C₅-C₁₂ alkyl(hetero)arylene groups, C₅-C₁₂ (hetero)arylalkylene groups, C₄-C₁₂ alkylcycloalkylene groups, and C₄-C₁₂ cycloalkylalkylene groups.

In preferred embodiments, each R' is independently selected from the group consisting of hydrogen, C₁-C₄ alkylene groups, C₂-C₄ alkenylene groups, C₂-C₄ alkynylene groups, C₆ arylene, C₄-C₅ heteroarylene, C₃-C₆ cycloalkylene groups, C₅-C₈ cycloalkenylene groups, C₅-C₈ alkyl(hetero)arylene groups, C₅-C₈ (hetero)arylalkylene groups, C₄-C₁₂ alkylcycloalkylene groups, and C₄-C₈ cycloalkylalkylene groups.

Unless stated otherwise, for R' the alkylene groups, alkenylene groups, alkynylene groups, (hetero)arylene groups, cycloalkylene groups, cycloalkenylene groups, alkyl(hetero)arylene groups, (hetero)arylalkylene groups, alkylcycloalkylene groups, cycloalkylalkylene groups are optionally substituted with a moiety selected from the group consisting of -Cl, -F, -Br, -I, -OH, -NH₂, =O, -SH, -SO₃H, -PO₃H, -PO₄H₂, -NO₂, and optionally contain one or more heteroatoms selected from the group consisting of -O-, -S-, -NH-, -P-, and -Si, wherein the N, S, and P atoms are optionally oxidized.

### Medical use and non-therapeutic methods

In the following, the references to methods of treatment by therapy or in vivo diagnosis methods are to be interpreted as references to compounds and medicaments of the present invention for use in those methods.

### Therapeutic use

In one aspect, the invention relates to a compound, a combination, or a kit of the invention for use as a medicament. Alternatively, the kits of the invention are used in a method for treating or imaging patients, said method comprising administering the compounds comprised in the kits of the invention to a subject.

In another aspect, the invention pertains to a compound, a combination, or a kit of the invention for use in the treatment of a disease, preferably cancer, in a subject, preferably a human. Preferably, the treatment is radiotherapy.

Preferably, the radiotherapy comprises the steps of
(a) administering a compound according to Formula (1) as defined herein, to the subject;
(b) administering a Cleaving Agent as defined herein, to said subject;
(c) irradiating the compound according to Formula (1) present in the subject.

The disclosure also pertains to a method of treatment in a subject as defined herein, said method comprising the steps (a)-(c) as defined in the preceding paragraph. Preferably, the method is for treating cancer in said subject.

In a preferred embodiment, step (a) is carried out first, secondly step (b) is carried out, and then step (c). In that embodiment, preferably step (b) is carried out after waiting a sufficient amount of time after step (a), so that a significant part, preferably at least 10%, more preferably at least 50 % of what is maximally achievable, of the dose of a compound according to Formula (1) has reached the target. Preferably, the Cleaving Agent and/or its dose are chosen in this embodiment so as to ensure that compound according to Formula (1) that has reached the target is not cleaved in significant amounts, preferably less than 30 % , more preferably less than 10%. In such a way, in step (b) the target-to-background ratio of the radionuclide can be optimized before irradiation in step (c). Preferably, in this embodiment a further step (b) is carried out after step (c) so as to quickly reduce the amount of radionuclides in the subject after irradiation.

In another embodiment, step (a) is carried out first, secondly step (c) is carried out, and then step (b). In this way, the amount of radionuclides in the subject can be quickly reduced after irradiation to minimize the radiation dose received by non-target tissues.

In another embodiment, step (b) is carried out first, allowing the Cleaving Agent to accumulate in non-target tissue that needs protecting from the radiation; secondly step (a) is carried out, affording the targeting of the radiation to target tissue, while the Label is cleaved and removed in non-target tissue by the pre-localized Cleaving Agent; and then step (c) takes place.

Likewise, the Cleaving Agent can be locally administered, i.e. directly injected, into tissues that need protecting, as opposed to a systemic intravenous administration.

One prominent application of the invention, is radioimmunotherapy targeted to an internalizing cancer receptor such as HER2. In this approach the HER2-targeting antibody Trastuzumab (Tmab) is modified with, for example three TCO-chelate constructs as shown below. The TCO-chelate construct (NHS-TCO-DOTA) comprises an active ester for lysine conjugation, the TCO linker, and a DOTA chelate for ¹¹⁷Lu labeling, a therapeutic beta emitter. Following ¹¹⁷Lu- labeling of Tmab and intravenous (i.v.) injection, the ¹¹⁷Lu-Tmab is allowed to circulate, bind the HER2 target on the breast cancer or ovarian cancer sites, allowed time to internalize (ca 2 days) after which the tetrazine comprising Cleaving Agent is intravenously injected, which cleaves ¹¹⁷Lu-DOTA construct from the freely circulating Tmab, resulting in rapid clearance of ¹¹⁷Lu-DOTA construct via the kidney, and a vast reduction of radiation dose to the bone marrow. Typically the Cleaving Agent is hydrophilic and as a result non cell permeable and will therefore not cleave the ¹¹⁷Lu-Tmab inside the target cell. However, even if the Cleaving Agent is cell permeable and cleaves ¹¹⁷Lu-Tmab inside the target cell, typically the released ¹¹⁷Lu-DOTA will remain trapped inside the cell.

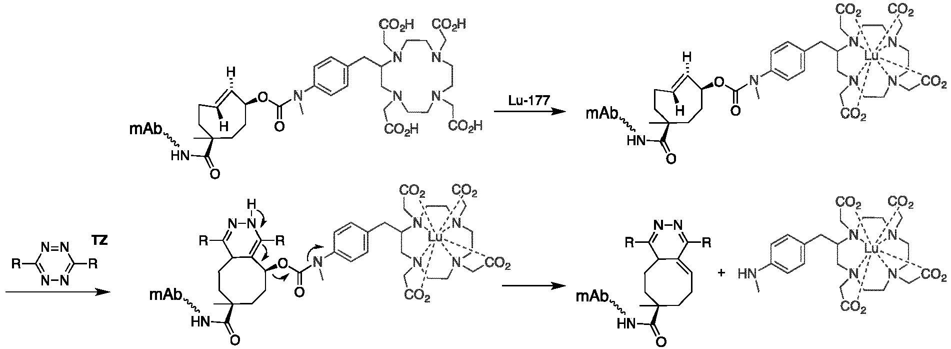

Also non-internalizing cancer receptors can be used as Primary Targets in above examples if the Cleaving Agent is designed to have a low or slow uptake in the tumor. For example, the Cleaving Agent can comprise a biodegradable PLGA particle core of ca 500 nm diameter, modified with tetrazine moieties. Such a particle will exhibit rapid clearance from blood by the liver, ensuring that it can only react with TCO containing constructs in circulation, and does not accumulate in the tumor, as previously shown for tetrazine clearing agents [Rossin et al., J. Nucl. Med. 2013, 4, 11, 1989-1995; and WO2012085789A1]. For imaging applications, slow accumulation at the target site may be acceptable, in which case the tetrazine can be modified with an albumin binding moiety, or a protein or a polymer such as a PEG, maximizing its retention in circulation, and thereby facilitating its reaction with TCO constructs in circulation.

In one other preferred embodiment of this invention, the administration of the compound of Formula (1) followed by the Cleaving agent allows to tune the blood circulation and excretion pathway of the released Label.

In the context of ¹¹⁷Lu-Tmab RIT , after cell internalization a Cleaving Agent comprising, for example, a tetrazine functionalized with a short polyethylene glycol (PEG) polymer is injected and binds the TCO Trigger, resulting in the cleavage of the bond between the TCO and the antibody. Subsequently the ¹⁷⁷Lu-DOTA chelate carrying the PEG is released in circulation and due to the PEG it clears via the kidneys.

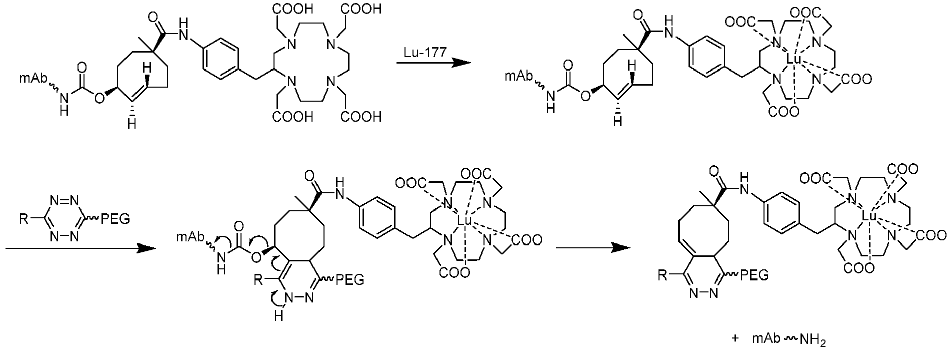

In a similar approach, instead of a linear or branched polymer the Cleaving Agent carries one or more functional groups that influence the clearance pathway of the released moiety. For instance, a Cleaving Agent comprising a tetrazine and several galactose groups will produce a released moiety that binds the Ashwell receptor in hepatocytes, therefore resulting in fast hepatobiliary clearance of the label.

In another preferred embodiment of this invention, the Administration Agent is an antibody fragment carrying an imageable or therapeutic radiometal chelate that accumulates specifically in a tumor or another diseased tissue and internalizes into target cells, but is also non-specifically retained in non-target organs such as, but not restricted to, kidneys, salivary glands and lacrimal glands. In this approach the Label (e.g. ¹⁷⁷Lu or ²²⁵Ac labeled DOTA or ⁸⁹Zr labeled DFO) is linked to the targeting moiety via a TCO trigger. After i.v. injection of the compound of Formula (1) and after such Agent has accumulated in the diseased tissue and internalized into target cells (e.g. 4 to 24h post-injection), the patient in administered a Cleaving Agent intravenously. The Cleaving Agent specifically binds the TCO trigger on the Administration Agent in non-target organs and releases the Label thus inducing radioactivity wash out from these organs via the urine. This reduces the radioactive dose delivered to non-target organs thus increasing the therapeutic index of Administration Agents object of this invention and reducing the chance of toxic side effects for the patient in nuclear imaging procedures.

In another preferred embodiment of this invention the compound of Formula (1) is functionalized on both allylic positions. The compound of Formula (1)is injected i.v. in an animal or human subject and is followed, after a suitable time (e.g. 2 days), by i.v. injection of the Cleaving Agent which specifically reacts with the Trigger resulting in the release of at least one of the two moieties conjugated in TCO allylic position. This approach allows the very fast and complete release of a small fragment (e.g. a Label) that will rapidly clear from non target tissues, preferentially via the kidney, regardless of the pyridazine tautomer (either the 1,4 or 2,5) formed after reaction of the TCO Trigger and the tetrazine Cleaving Agent. For very fast and complete release it is preferred that the tetrazine is according to Formula (14), with Yₐ being Y₁, Y₃, or Y₅ with Q₁ being X₄₅, preferably X₄₅ being OH, and Y_{b} being identical to Yₐ or H. One non limiting example of this approach is shown below for a ¹⁷⁷Lu-labeled mAb

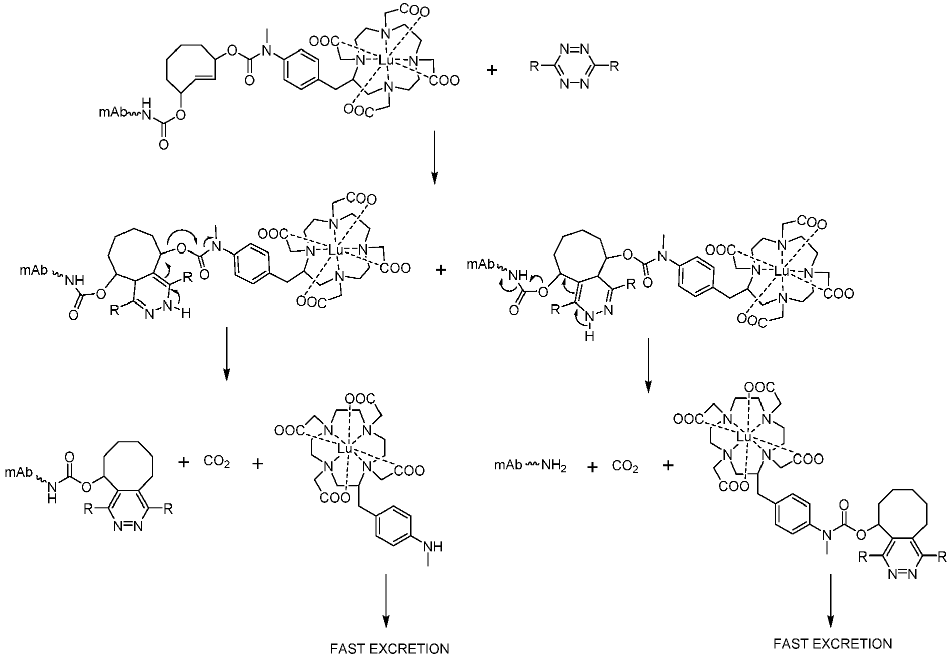

In yet another preferred embodiment of this invention the compound of Formula (1) comprises a TCO Trigger conjugated Targeting Agent and to an imaging or therapeutic moiety (Label) via a self-immolative linker. The compound of Formula (1) is injected i.v. in an animal or human subject, it accumulates at the target site and, after a suitable time (e.g. one or two days) is followed by i.v. injection of a tetrazine Cleaving Agent. The tetrazine reacts with the Trigger resulting in an intermediate that rearranges electronically resulting in the fragmentation of the linker and the detachment of the Label from the Targeting Agent. As a result the Label, being a small molecule, rapidly clears from the subject's circulation, preferentially via the kidney. A non limiting example of this invention is shown below

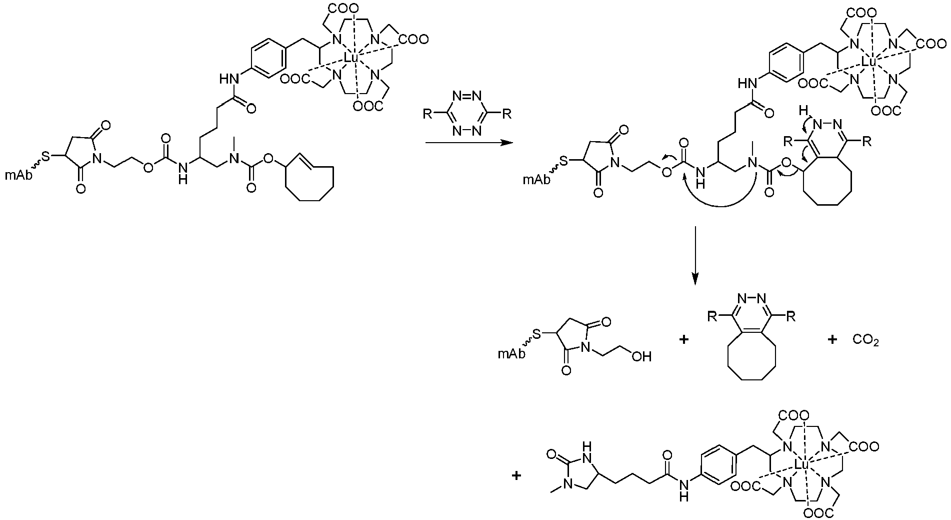

In one other preferred embodiment of this invention the compound of Formula (1) comprises an imageable or therapeutic moiety bound via a TCO Trigger to a Targeting Agent that binds a specific receptor or molecule present both at a disease site and in circulation due to shedding. Non limiting examples of shedding targets are the carcinoembryonic antigen (CEA), the prostate specific antigen (PSA), and the tumor necrosis factor α (TNF-α) receptor. In the presence of target shedding, Administration Agent binding to circulating target is detrimental as it causes loss of image contrast and/or toxic side effects in the subject of the medical intervention. With the approach of this invention, the compound of Formula (1) is injected i.v. in an animal or human subject and binds to its target at the disease site and in circulation. After a suitable time (one or two days) the subject is injected the tetrazine Cleaving Agent i.v. which is designed to specifically react with the TCO Trigger on the circulating (bound) Administration Agent. Upon reaction between the tetrazine and the TCO, the imageable or therapeutic moiety is released from the Administration Agent and it clears rapidly from circulation.

In one preferred embodiment, the invention is used to reduce the kidney dose instead of the bone marrow dose. In this embodiment, the Administration Agent being an intact IgG antibody is labeled with ²²⁵Ac and allowed to bind its Primary Target, such as HER2, PSMA. ²²⁵Ac has a chain of daughter isotopes, and upon the 1^{st} decay of ²²⁵Ac, the nuclide is separated from the DOTA and the Administration Agent resulting in renal uptake of the free daughter ²²¹Fr. Timely injection of the Cleaving Agent results cleavage in blood and rapid elimination of DOTA-²²⁵Ac via the kidneys and reduced ²²¹Fr radiation dose to the kidneys.

In another embodiment, the Primary Target is a receptor on a blood cancer cell, i.e. CD33 on AML cells, and the Administration Agent is antiCD33 mAb, and the Label is DOTA-²²⁵Ac. After CD33 binding and internalization, the DOTA-²²⁵Ac Label of the freely circulating mAb in cleaved to reduce the radiation toxicity to kidneys, and also the bone marrow, liver, and spleen.

Furthermore, the disclosure pertains to a diagnostic method comprising the steps of
(a) administering a compound according to Formula (1) as defined herein, to a subject, preferably a human;
(b) administering a Cleaving Agent as defined herein, to said subject;
(c) imaging the compound according to Formula (1) present in the subject to collect data;
(d) comparing said data to standard values;
(e) finding a significant deviation from said standard values during comparison;
(f) attributing the significant deviation to a particular clinical picture, preferably to cancer.

### Diagnostic method

The invention also pertains to a compound of Formula (1) as defined herein, a combination as defined herein, or a kit as defined herein for use in a diagnostic method comprising the steps of
(a) administering a compound according to Formula (1) as defined herein, to a subject, preferably a human;
(b) administering a Cleaving Agent as defined herein, to said subject;
(c) imaging the compound according to Formula (1) present in the subject to collect data;
(d) comparing said data to standard values;
(e) finding a significant deviation from said standard values during comparison;
(f) attributing the significant deviation to a particular clinical picture, preferably to cancer.

### Non-therapeutic method

The invention also pertains to a non-therapeutic method for imaging a compound of Formula (1) as defined herein, in a subject as defined herein, preferably a human, said non-therapeutic method comprising the steps of
(a) administering a compound according to Formula (1) as defined herein, to the subject;
(b) administering a Cleaving Agent as defined herein, to said subject;
(c) imaging the compound according to Formula (1) present in the subject.

In a preferred embodiment, step (a) is carried out first, secondly step (b) is carried out, and then step (c). In that embodiment, preferably step (b) is carried out after waiting a sufficient amount of time after step (a), so that a significant part, preferably at least 10%, more preferably at least 50 % of what is maximally achievable, of the of the initial dose of a compound according to Formula (1) has reached the target. Preferably, the Cleaving Agent and/or its dose are chosen in this embodiment so as to ensure that compound according to Formula (1) that has reached the target is not cleaved in significant amounts, preferably less than 30 %, more preferably less than 10%. In such a way, in step (b) the target-to-background ratio of the radionuclide can be optimized before imaging in step (c).

Preferably, in this embodiment a further step (b) is carried out after step (c) so as to quickly reduce the amount of radionuclides in the subject after imaging.

In another embodiment, step (a) is carried out first, secondly step (c) is carried out, and then step (b). In this way, the amount of radionuclides in the subject can be quickly reduced after imaging, to reduce whole body radiation dose and / or optionally to allow for another imaging procedure (image cycling).

In another embodiment, step (b) is carried out first, allowing the Cleaving Agent to accumulate in non-target tissue that needs protecting from the radiation or that would otherwise obscure imaging of the Primary Target; secondly step (a) is carried out, affording the targeting of the radiation to target tissue, while the Label is cleaved and removed in non-target tissue by the pre-localized Cleaving Agent; and then step (c) takes place.

Likewise, the Cleaving Agent can be locally administered, i.e. directly injected, into selected non-target tissues, as opposed to a systemic i.v. administration.

The invention can be used to improve radioimmunoimaging of HER2 with Trastuzumab (Tmab). In this approach Tmab is modified with for example 2 TCO-DFO chelate constructs, as shown below, wherein the Tmab is conjugated via thiol maleimide chemistry. Following ⁸⁹Zr-labeling of Tmab and intravenous (i.v.) injection, the ⁸⁹Zr -Tmab is allowed to circulate, bind the HER2 target on the breast cancer or ovarian cancer sites, allowed time to internalize (ca 2 days) after which the tetrazine comprising Cleaving Agent is i.v. injected, which cleaves the ⁸⁹Zr -DFO label from the freely circulating Tmab, resulting in rapid clearance of ⁸⁹Zr -DFO construct via the kidney, and a vast improvement tumor-blood ratio in imaging of the target.

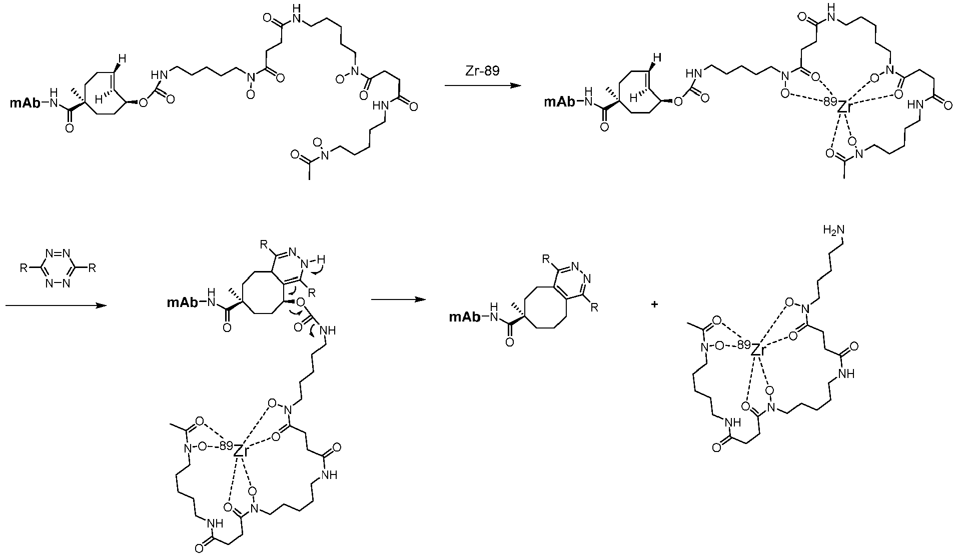

In a similar approach as with the above HER2 imaging example, the invention can be used in companion imaging of antibody drugs that are being developed to cross the blood brain barrier (BBB) to treat, for example, Alzheimer's disease. In such an approach the therapeutic antibody can be modified with an additional domain which binds to the transferrin receptor, resulting in crossing of the BBB. As only a minor amount will cross the BBB and bind to its Primary Target, and as a large amount will still freely circulate in blood, conventional imaging approaches (e.g. by labeling the antibody with ⁸⁹Zr) is hampered by very poor target-non-target (T-NT) ratios. Conjugation of the cleavable DFO-TCO-maleimide construct shown above to the anti-Alzheimer antibody, labeling with⁸⁹Zr, i.v. injection, followed by some time for target uptake will allow the cleavage of freely circulating ⁸⁹Zr-antibody at desired time points, making the circulating antibody essentially invisible, while retaining the ⁸⁹Zr-signal at the target site, boosting T-NT ratios. This approach also allows discriminating between ⁸⁹Zr-antibody that has crossed the BBB and the portion that has bound in the brain but has not crossed the BBB, or where the BBB is impaired. In this embodiment, the diene is preferably designed such that it does extravasate, but does not significantly permeate the BBB. In another preferred embodiment, the diene is designed such that it does not extravasate into other tissues.

### Administration

When administering the compound of Formula (1) and the Cleaving Agent to a subject, such as an animal or human, in preferred embodiments the compound of Formula (1) is administered first. It will take a certain time period before the the compound of Formula (1) has reached the Primary Target, and optionally internalized in the cell or crossed the blood brain barrier. This time period may differ from one application to the other and may be for example minutes or hours.

After the time period of choice has elapsed, the Cleaving Agent is administered, which reacts with the the compound of Formula (1) to decouple the Administration Agent and the Label, preferably in the non-target tissues. In some preferred embodiments, the time interval between the administration of the compound of Formula (1) and the Cleaving Agent is between 10 minutes and 4 weeks. In some preferred embodiments, the time interval between the administration of the compound of Formula (1) and the Cleaving Agent is between 1 hour and 2 weeks, preferably between 1 and 168 hours, more preferably between 1 and 120 hours, even more preferably between 1 and 96 hours, more preferably between 3 and 72 hours, more preferably still between 4 and 48 hours, and most preferably between 5 and 24 hours.

The compounds and the combinations of the invention can be administered via different routes including but not limited to intravenous or subcutaneous injection, intraperitoneal, local injection, oral administration, rectal administration and inhalation. Formulations suitable for these different types of administrations are known to the skilled person. Compounds of Formula (1) or Cleaving Agents according to the invention can be administered together with a pharmaceutically acceptable carrier. A suitable pharmaceutical carrier as used herein relates to a carrier suitable for medical or veterinary purposes, not being toxic or otherwise unacceptable. Such carriers are well known in the art and include for example saline, buffered saline, dextrose, water, glycerol, ethanol, and combinations thereof. The formulation should suit the mode of administration.

It will be understood that the chemical entities administered, viz. the compound of Formula (1) and the Cleaving Agent, can be in a modified form that does not alter the chemical functionality of said chemical entity, such as salts, hydrates, or solvates thereof.

In preferred embodiments, the Cleaving Agent, preferably comprising a Targeting Agent, is administered first, and thereafter the compound of Formula (1) is administered.

Preferably, in embodiments where the compound of Formula (1) and the Cleaving Agent are administered approximately simultaneously, they are administered via different routes.

### Subject

As used herein, "subject" means any animal, preferably a mammal, most preferably a human. The term "mammal" as used herein, encompasses any mammal. Examples of mammals include, but are not limited to, cows, horses, sheep, pigs, cats, dogs, mice, rats, rabbits, guinea pigs, non-human primates (NHPs) such as monkeys or apes, humans, etc., more preferably a human.

### Non-therapeutic use

The invention also pertains to a use of a compound of Formula (1) as defined herein, a combination as defined herein, or a kit as defined herein, for imaging in a subject, preferably a human.

### Examples

### General methods

All reagents, chemicals, materials and solvents were obtained from commercial sources and were used as received, including nitrile starting compounds that not have been described. All solvents were of AR quality. Sodium [¹²⁵I]iodide, [¹¹¹In]Indium and [¹⁷⁷Lu]lutetium chloride, and [⁸⁹Zr]zirconium oxalate solutions were purchased from PerkinElmer, Curium and IDB. Zeba desalting spin columns (7 and 40 kDa MW cut-off, 0.5 mL) and Slide-A-Lyzer dialysis cassettes (20 kDa MW cut-off) were purchased from Pierce Protein Research (Thermo Fisher Scientific). Mouse plasma was purchased from Innovative Research. 29-Amino-3,6,9,12,15,18,21,24,27-nonaoxanonacosan-1-ol was purchased from PurePEG. 3,6-Dimethyl-1,2,4,5-tetrazine and (*E*)-cyclooct-2-en-1-yl (4-nitrophenyl) carbonate were prepared according to literature procedures [Versteegen et al., Angew. Chem. Int. Ed. 2013, 52, 14112-14116]. Analytical thin layer chromatography was performed on Kieselgel F-254 precoated silica plates. Column chromatography was carried out on Screening Devices B.V. silica gel (flash: 40-63 pm mesh and normal: 60-200 pm mesh). ¹H-NMR and ¹³C-NMR spectra were recorded on a Bruker Avance III HD (400 MHz for ¹H-NMR and 100 MHz for ¹³C-NMR) spectrometer at 298 K. Chemical shifts are reported in ppm downfield from TMS at rt. Abbreviations used for splitting patterns are s = singlet, d = doublet, dd = double doublet, t = triplet, q = quartet, m = multiplet and br = broad. HPLC-PDA/MS was performed using a Shimadzu LC-10 AD VP series HPLC coupled to a diode array detector (Finnigan Surveyor PDA Plus detector, Thermo Electron Corporation) and an Ion-Trap (LCQ Fleet, Thermo Scientific). HPLC-analyses were performed using a Alltech Alltima HP C₁₈ 3p column using an injection volume of 1-4 µL, a flow rate of 0.2 mL min⁻¹ and typically a gradient (5 % to 100 % in 10 min, held at 100 % for a further 3 min) of MeCN in H₂O (both containing 0.1 % formic acid) at 298 K. Size exclusion chromatography (SEC) was performed on an Akta system (GE Healthcare Life Science) equipped with a Superdex200 column. Radio-HPLC was performed on an Agilent 1100 system, equipped with a Gabi radioactive detector (Raytest). The samples were loaded on an Alltima C18 column (4.6 × 150mm, 5p), which was eluted at 1 mL min-1 with a linear gradient of water (A) and acetonitrile (B) containing 0.1% v/v% TFA (4 min at 3% B followed by an increase to 90% B in 15 min). Radio-ITLC was performed on ITLC-SG strips (Varian Inc.) eluted with 200 mM EDTA in saline solution (¹¹¹In/¹⁷⁷Lu) or in 0.1M citrate pH 6.0 (⁸⁹Zr). In these conditions the radiolabeled products remain at the base while unbound radionuclide migrates with a R_{f} of 0.7-0.9. SDS-PAGE was performed on a Mini-PROTEAN Tetra Cell system using 4-20% precast Mini-PROTEAN TGX gels and Precision Plus Protein All Blue Prestained protein standards (BioRad Laboratories). The radioactivity distribution on ITLC strips and SDS-PAGE gels was monitored with a Typhoon FLA 7000 phosphor imager (GE Healthcare Life Science) using the AIDA software.

### Example 1: Synthesis of conjugatable TCO-DOTA constructs

### Compound 1.1 (axial isomer) was prepared according to a published method

### [Rossin et al., Bioconjugate Chem. 2016, 27, 1697-1706]

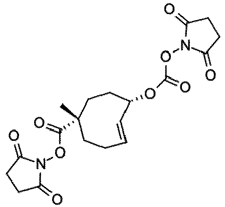

### NHS-TCO-PEG4-DOTAGA (1.2)

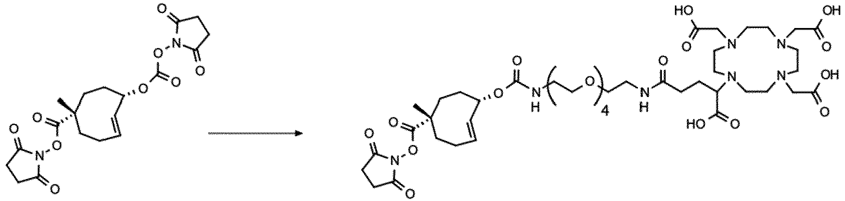

A solution of 4-[(14-amino-3,6,9,12-tetraoxatetradecan-1-yl)carbamoyl]-2-[4,7,10-tris(carboxymethyl)-1,4,7,10-tetraazacyclododecan-1-yl]butanoic acid HCl salt (18.0 mg, 25.9 µmol) and DiPEA (50.2 µL, 288 µmol) in H₂O was added to a solution of **1.1** (12.2 mg, 28.8 µmol) in acetonitrile and the solution was stirred for 5 min. The reaction mixture was acidified followed by preparative RP-HPLC purification using an elution gradient of 5 % to 90 % MeCN in H₂O (both containing 0.1 % TFA) to yield compound **1.2** (10.0 mg, 9.98 µg, 39%) after lyophilization as a fluffy white powder. ¹H NMR (400 MHz, CD₃OD): δ 5.92-5.99 (m, 1H), 5.72 (d, J = 16.8 Hz, 1H), 5.12 (bs, 1H), 4.08-4.25 (m, 2H), 4.07-3.96 (m, 1H) 3.78-3.94 (bs, 2H), 3.61-3.67 (m, 11H), 3.53-3.57 (m, 6H), 3.35 (m, 2H), 3.10-3.25 (bs, 4H), 2.90-3.05 (m, 2H), 2.78-2.85 (s, 4H) 2.62-2.72 (m, 1H), 2.50-2.59 (m, 1H), 2.24-2.40 (m, 2H), 2.13-2.20 (m, 10H), 2.02-2.10 (3H), 1.90-2.02 (m, 3H), 1.30 (s, 3H), 1.27 (bs, 2H) ppm. ESI-MS: m/z calc for C₄₄H₇₁N₇O₁₉ 1001.48; Obs. [M+H]⁺ 1002.40, [M+2H]⁺ 501.36.

### Mal-PEG2-TCO-PEG4-DOTAGA (1.3)

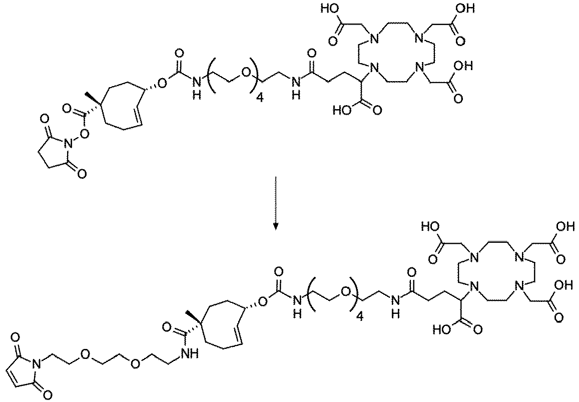

Commercially available tert-butyl N-(2-{2-[2-(2,5-dioxo-2,5-dihydro-1H-pyrrol-1-yl)ethoxy]ethoxy}ethyl)carbamate (3.3 mg, 9.98 µmol) was treated with CHCl₃:TFA (1:1) for 20 min at rt before removal of the volatiles. The resulting species was dissolved in CH₂Cl₂ and added drop wise to a solution of **1.2** (10.0 mg, 9.98 µmol) and DiPEA (17.4 µL, 99.8 µmol) in CH₂Cl₂ and the reaction was stirred for 15 min at rt. Following acidification and removal of the volatiles, preparative RP-HPLC purification using an elution gradient of 5 % to 90 % MeCN in H₂O (both containing 0.1 % TFA) yielded compound **1.3** after lyophilization as a fluffy white powder.

### NHS-TCO-C2-DOTAGA (1.4)

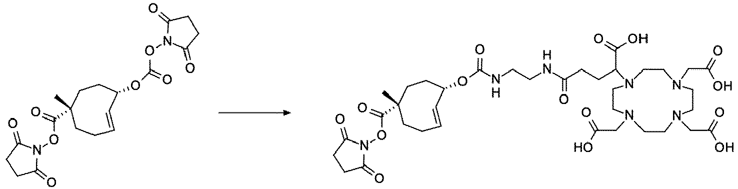

A solution of 4-[(2-aminoethyl)carbamoyl]-2-[4,7,10-tris(carboxymethyl)-1,4,7,10-tetraazacyclododecan-1-yl]butanoic acid (13.4 mg, 25.9 µmol) and DiPEA (50.2 µL, 288 µmol) in H₂O was added to a solution of **1.1** (12.2 mg, 28.8 µmol) in acetonitrile and the solution was stirred for 5 min. Acidification of the reaction mixture was followed by preparative RP-HPLC purification using an elution gradient of 5 % to 90 % MeCN in H₂O (both containing 0.1 % TFA) to yield compound **1.4** (5.0 mg, 6.1 µg, 23%) after lyophilization as a fluffy white powder. ESI-MS: m/z calc for C₃₆H₅₅N₇O₁₅ 825.38; Obs. [M+H]⁺ 826.24, Obs. [M+2H]⁺ 413.24.

### 2-[({[(1S,2E,6S)-6-{[(2,5-Dioxopyrrolidin-1-yl)oxy]carbonyl}-6-methylcyclooct-2-en-1-yl] oxy}carbonyl)(methyl)amino] acetic acid (1.5)

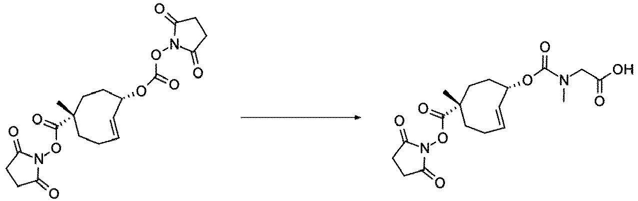

A solution of sarcosine (5.8 mg, 64.9 µmol) and DiPEA (20.6 µL, 118 µmol) in H₂O was added to a solution of **1.1** (25 mg, 59 µmol) in MeCN and the mixture was acidified after 5 min. Following removal of MeCN in vacuo, dilution with H₂O and extraction with CHCl₃ (3x20 mL), followed by drying over Na₂SO₄ and concentration yielded **1.5** in ca. 90% purity (20 mg, 50.7 µmol, 86%). ESI-MS: m/z calc for C₁₈H₂₄N₂O₈ 396.15; Obs. [M-H]- 395.16, Obs. [2M-H]- 791.12.

### NHS-TCO-Sar-C2-DOTAGA (1.6)

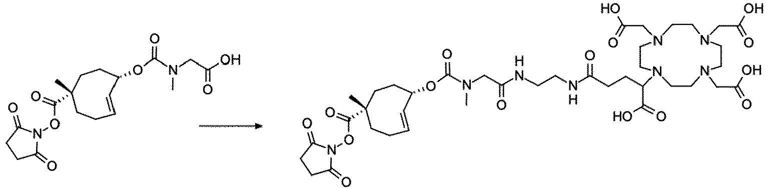

PyBOP (10 mg, 19.2 µmol) and DiPEA (14 µL, 80.4 µmol) were added to a solution of **1.5** (12 mg, ca. 27 µmol) in MeCN and the reaction mixture was stirred for 10 min. A solution of 4-[(2-aminoethyl)carbamoyl]-2-[4,7,10-tris(carboxymethyl)-1,4,7,10-tetraazacyclododecan-1-yl]butanoic acid (10.4 mg, 20.1 µmol) and DiPEA (21 µL, 120.6 µmol) in H₂O was added and the solution was stirred for 5 min. Acidification of the reaction mixture was followed by preparative RP-HPLC purification using an elution gradient of 5 % to 90 % MeCN in H₂O (both containing 0.1 % TFA) to yield compound **1.6** (12.5 mg, 13.9 µg, 73%) after lyophilization as a fluffy white powder. ESI-MS: m/z calc for C₃₉H₆₀N₈O₁₆ 896.41; Obs. [M+H]⁺ 897.48, Obs. [M+2H]⁺ 448.96.

### NHS-TCO-Sar-PEG4-DOTAGA (1.7)

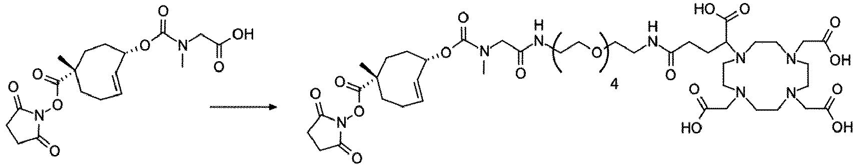

PyBOP (12.7 mg, 24.3 µmol) and DiPEA (14 µL, 80.4 µmol) were added to a solution of **1.5** (12 mg, ca. 27 µmol) in MeCN and the reaction mixture was stirred for 10 min. A solution of 4-[(14-amino-3,6,9,12-tetraoxatetradecan-1-yl)carbamoyl]-2-[4,7,10-tris(carboxymethyl)-1,4,7,10-tetraazacyclododecan-1-yl]butanoic acid HCl salt (18.8 mg, 27 µmol) and DiPEA (21 µL, 120.6 µmol) in H₂O was added and the solution was stirred for 5 min. Acidification of the reaction mixture was followed by preparative RP-HPLC purification using an elution gradient of 5 % to 90 % MeCN in H₂O (both containing 0.1 % TFA) to yield compound **1.7** (19.5 mg, 18.2 µg, 75%) after lyophilization as a fluffy white powder. ESI-MS: m/z calc for C₄₇H₇₆N₈O₂₀ 1072.52; Obs. [M+H]⁺ 1073.68, Obs. [M+2H]⁺ 537.08.

### Mal-PEG9-TCO-Sar-PEG4-DOTAGA (1.8)

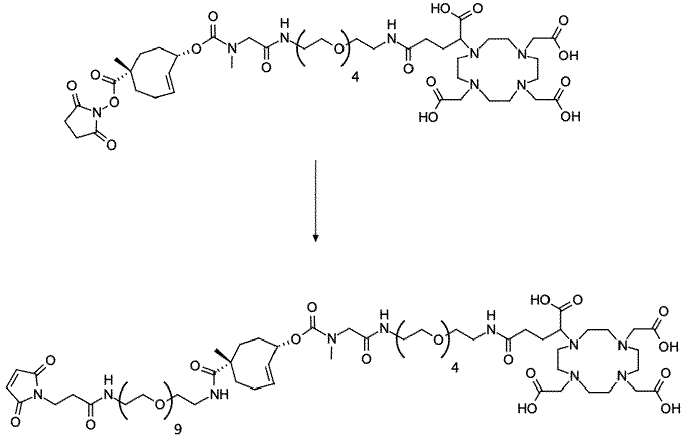

Compound **1.7** (15 mg, 14.0 µmol) was treated with commercially available N-(29-amino-3,6,9,12,15,18,21,24,27-nonaoxanonacosan-1-yl)-3-(2,5-dioxo-2,5-dihydro-1H-pyrrol-1-yl)propanamide TFA salt (12.2 mg, 16.9 µmol) and DiPEA (17 µL, 98.1 µmol) in DMF for 6 days at rt. Acidification and concentration of the reaction mixture was followed by preparative RP-HPLC purification using an elution gradient of 5 % to 90 % MeCN in H₂O (both containing 0.1 % TFA) to yield compound **1.8** (3.5 mg, 2.2 µg, 16%) after lyophilization as a sticky oil. ESI-MS: m/z calc for C₇₀H₁₂₀N₁₀O₂₉ 1564.82; Obs. [M+H]⁺ 1565.80 Obs. [M-H]- 1564.32.

### NHS-TCO-PEG4-BCN (1.9)

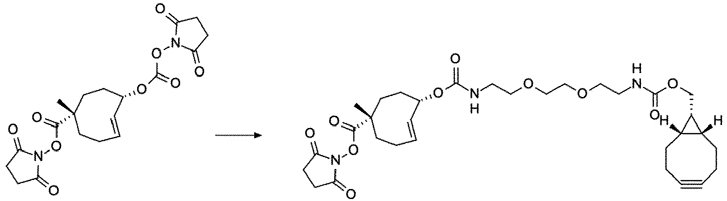

Compound **1.1** (35 mg, 83 µmol) was treated with commercially available [(1R,8S,9R)-bicyclo[6.1.0]non-4-yn-9-yl]methyl N-{2-[2-(2-aminoethoxy)ethoxy]ethyl}carbamate (29.9 mg, 92.1 µmol) and DiPEA (28.9 µL, 166 µmol) in CH₂Cl₂ for 45 min prior to concentration. Compound **1.9** was isolated as a white fluffy powder after preparative RP-HPLC purification using an elution gradient of 5 % to 90 % MeCN in H₂O (both containing 0.1 % TFA) (32 mg, 50.7 µg, 61%). ESI-MS: m/z calc for C₃₂H₄₅N₃O₁₀ 631.31; Obs. [M+H]⁺ 632.00, Obs. [2M+H₂O+H]⁺ 1279.84.

### 4-{[2-(2-Azidoacetamido)ethyl]carbamoyl}-2-[4,7,10-tris(carboxymethyl)-1,4,7,10-tetraazacyclododecan-1-yl]butanoic acid (1.10)

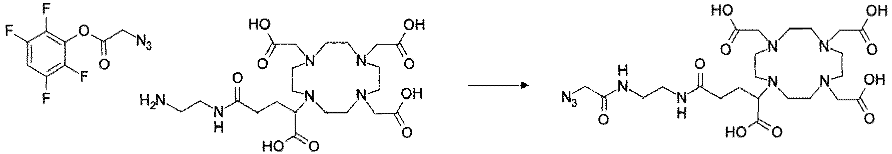

A mixture of 4-[(2-aminoethyl)carbamoyl]-2-[4,7,10-tris(carboxymethyl)-1,4,7,10-tetraazacyclododecan-1-yl]butanoic acid (24.8 mg, 47.8 µmol), 2,3,5,6-tetrafluorophenyl 2-azidoacetate (26.5 mg, 106.4 µmol) and DiPEA (50.4 µL, 289.3 µmol in MeCN/H₂O was stirred for 30 min at rt. Following acidification and concentration, preparative RP-HPLC purification of 1/3^{rd} of the material using an elution gradient of 2 % to 10 % MeCN in H₂O (both containing 0.1 % TFA) yielded compound **1.10** (6.4 mg, 10.6 µg, 67%) after lyophilization as a white fluffy powder. ESI-MS: m/z calc for C₂₃H₃₉N₉O₁₀ 601.28; Obs. [M+H]⁺ 602.32, Obs. [2M+H]⁺ 301.60.

### 4-({14-[2-(Methylamino)acetamido]-3,6,9,12-tetraoxatetradecan-1-yl}carbamoyl)-2-[4,7,10-tris(carboxymethyl)-1,4,7,10-tetraazacyclododecan-1-yl]butanoic acid TFA salt (1.11)

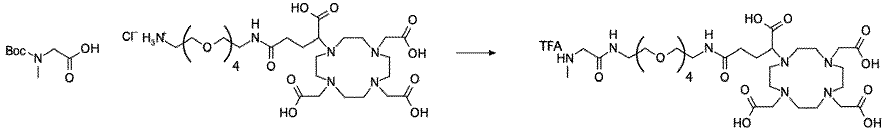

Commercially available 2-{[(tert-butoxy)carbonyl](methyl)amino}acetic acid (6.0 mg, 31.6 µmol) was treated with PyBOP (7.1 mg, 13.7 µmol) and DiPEA (7.5 µL, 43.1 µL) for 15 min in MeCN at rt. A solution of 4-[(14-amino-3,6,9,12-tetraoxatetradecan-1-yl)carbamoyl]-2-[4,7,10-tris(carboxymethyl)-1,4,7,10-tetraazacyclododecan-1-yl]butanoic acid HCl salt (10 mg, 14.4 µmol) and DiPEA (15.1 µL, 86.4 µmol) in H₂O was added and the mixture was stirred for 30 min. Following concentration, the residue was treated with CH₂Cl₂/TFA (1:1) for 30 min. Preparative RP-HPLC purification using an elution gradient of 2 % to 10 % MeCN in H₂O (both containing 0.1 % TFA) yielded compound **1.11** (6.8 mg, 7.7 µg, 56%) after lyophilization. ESI-MS: m/z calc for C₃₂H₅₉N₇O₁₄ 765.41; Obs. 766.60 [M+H]⁺, Obs. [M+2H]⁺ 383.88.

### NHS-TCO-PEG22-CO₂H (1.12)

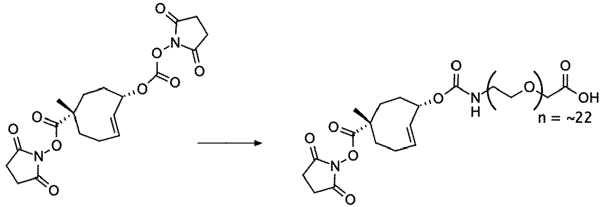

Compound **1.1** (15 mg, 35.1 µmol ) was treated with commercially available 68-amino-3,6,9,12,15,18,21,24,27,30,33,36,39,42,45,48,51,54,57,60,63,66-docosaoxaoctahexacontanoic acid (Avg MW: 1100, n=~22, 39.1 mg, 35.5 µmol) and DiPEA (18.6 µL, 106.7 µmol) in CH₂Cl₂ for 3 h. Following concentration, preparative RP-HPLC purification using an elution gradient of 5 % to 95 % MeCN in H₂O (both containing 0.1 % TFA) yielded compound **1.12** (30 mg, 22.2 µg, 63%) after lyophilization as a sticky gum.

### NHS-TCO-PEG22-C2-DOTAGA (1.13)

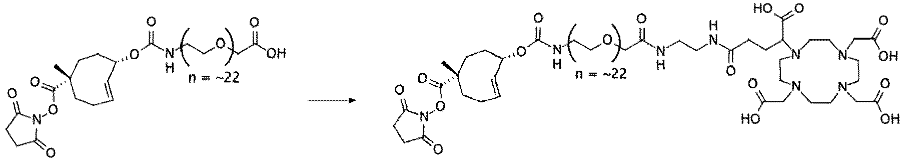

PyBOP (11.1 mg, 17.9 µmol) and DiPEA (14.9 µL, 86.9 µmol) were added to a solution of **1.12** (30 mg, ca. 22.2 µmol) in DMSO and the reaction mixture was stirred for 15 min. A solution of 4-[(2-aminoethyl)carbamoyl]-2-[4,7,10-tris(carboxymethyl)-1,4,7,10-tetraazacyclododecan-1-yl]butanoic acid (13.3 mg, 21.5 µmol) and DiPEA (22.3 µL, 128.9 µmol) in H₂O was added and the solution was stirred for 5 min. Acidification of the reaction mixture was followed by preparative RP-HPLC purification using an elution gradient of 5 % to 90 % MeCN in H₂O (both containing 0.1 % TFA) to yield compound **1.13** (6 mg, 3.2 µg, 15%) after lyophilization as a sticky gum.

### NHS-TCO-DFO (1.14)

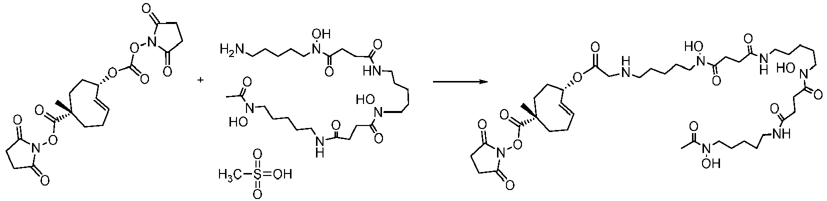

A solution of commercially available desferrioxamine mesylate salt (29.9 mg, 42.6µmol) and DiPEA (7.4 µL, 42.6 µmol) in DMSO was added to a solution of **1.1** (15 mg, 35.5 µmol) in DMSO and the solution was stirred for 4 h. The reaction mixture was acidified followed by preparative RP-HPLC purification using an elution gradient of 5% to 90% MeCN in H₂O (both containing 0.1 % TFA) to yield compound **1.14** (25.7 mg, 82%) after lyophilization as a fluffy white powder. ESI-MS: m/z calc for C₄₁H₆₇N₇O₁₄ 867.46; Obs [M+H]⁺ 868.8. ¹H NMR (400 MHz, CDCl3) δ 7.22 (s, 2H), 6.52 (s, 1H), 5.96-5.72 (m, 2H), 5.60 (d, 1H), 5.17 (d, 1H), 3.78 (t, 3H), 3.64 (s, 21H), 3.57 (d, 4H), 3.45-3.35 (m, 2H), 3.20 (s, 6H), 2.82 (s, 4H), 2.70-2.53 (m, 7H), 2.34-2.05 (m, 7H) ppm.

### NHS-TCO-Sar-DFO (1.15)

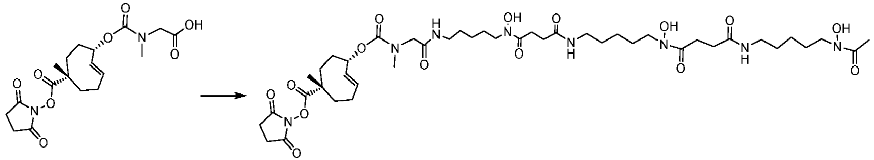

PyBOP (15.7 mg, 30.2 µmol) and DiPEA (10.5 µL, 60.4 µmol) were added to a solution of **1.5** (12 mg, 30.2 µmol) in DMSO and the reaction mixture was stirred for 10 min. A solution of desferrioxamine mesylate salt (21.8 mg, 33.2 µmol) and DiPEA (10.5 µL, 60.4 µmol) in DMSO was added and the solution was stirred for 3 h. Acidification of the reaction mixture was followed by preparative RP-HPLC purification using an elution gradient of 5% to 90% MeCN in H₂O (both containing 0.1 % TFA) to yield compound **1.15** (18.2 mg, 64 %) after lyophilization as a fluffy white powder. ESI-MS: m/z calc for C₄₃H₇₀N₈O₁₅ 938.5; Obs. [M+H]⁺ 939.2.

### NHS-TCO-Sar-PEG4-CO₂H (1.16)

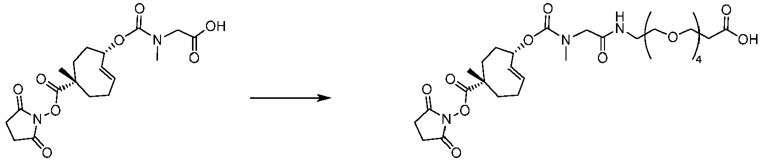

PyBOP (15.7 mg, 30.2 µmol) and DiPEA (10.5 µL, 60.4 µmol) were added to a solution of **1.5** (12 mg, 30.2 µmol) in DMF and the reaction mixture was stirred for 10 min. A solution of commercially available amino-PEG4-acid (9.6 mg, 36.2 µmol) and DiPEA (10.5 µL, 60.4 µmol) in DMF was added and the solution was stirred for 3 h. Acidification of the reaction mixture was followed by preparative RP-HPLC purification using an elution gradient of 5 % to 90 % MeCN in H₂O (both containing 0.1 % TFA) to yield compound **1.16** (12 mg, 62 %) after lyophilization as a sticky oil. ESI-MS: m/z calc for C₂₈H₄₃N₃O₁₃ 643.3; Obs. [M+H]⁺ 644.6.

### NHS-TCO-Sar-PEG4-DFO (1.17)

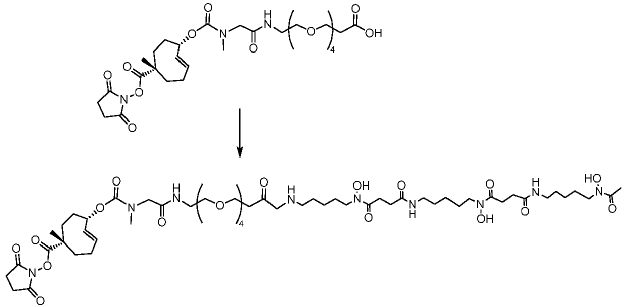

PyBOP (8.5 mg, 16.3 µmol) and DiPEA (5.6 µL, 32.6 µmol) were added to a solution of **1.16** (10.5 mg, 16.3 µmol) in DMSO and the reaction mixture was stirred for 10 min. A solution of desferrioxamine mesylate salt (11.6 mg, 17.9 µmol) and DiPEA (5.6 µL, 32.6 µmol) in DMSO was added and the solution was stirred for 3 h. Acidification of the reaction mixture was followed by preparative RP-HPLC purification using an elution gradient of 5 % to 90 % MeCN in H₂O (both containing 0.1 % TFA) to yield compound **1.17** (13.2 mg, 68 %) after lyophilization as a fluffy white powder. ESI-MS: m/z calc for C₅₄H₉₁N₉O₂₀ 896.41; Obs. [M+H]⁺1186.37, Obs. [M+2H]+ 1187.5.

### Mal-PEG9-TCO-DFO (1.18)

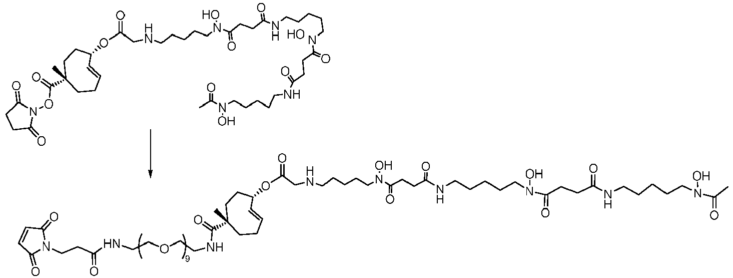

Compound **1.14** (15 mg, 17.3 µmol) was treated with commercially available mal-amido-PEG9-amine TFA salt (11.5 mg, 19 µmol) and DiPEA (11.9 µL, 69.2 µmol) in DMF for 6 days at rt. Acidification and concentration of the reaction mixture was followed by preparative RP-HPLC purification using an elution gradient of 5 % to 90 % MeCN in H₂O (both containing 0.1 % TFA) to yield compound **1.18** (6.6 mg, 28%) after lyophilization as a sticky oil. ESI-MS: m/z calc for C₆₄H₁₁₁N₉O₂₃ 1564.82; Obs. [M+H]⁺ 1565.9.

### (1R,4E)-Cyclooct-4-en-1-yl 2,5-dioxopyrrolidin-1-yl carbonate (1.19)

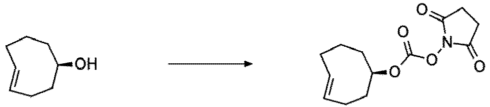

(1R,4E)-Cyclooct-4-en-1-ol (TCO-5-OH, axial isomer) (0.20 g, 1.6 mmol) was treated with DSC (0.81 g, 3.16 mmol), DMAP (0.22 g, 1.8 mmol) and DiPEA (1.4 mL, 4.9 mmol) in dry MeCN for 16 h at rt. The reaction mixture was partitioned between EtOAc and 2M HCl followed by washing of the organics with saturated NaHCOs. After drying over Na₂SO₄ and concentration, compound **1.19** was isolated after silica gel column chromatography (EtOAc / heptanes, 1:9 to 3:7). Yield: 36% (0.15 g, 0.56 mmol).

### 3-({[(1R,4E)-Cyclooct-4-en-1-yloxy]carbonyl}amino)-2-sulfopropanoic acid (1.20)

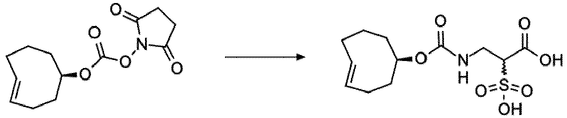

**1.19** (0.15g, 0.56 mmol) in MeCN was added to 3-amino-2-sulfopropanoic acid (0.14 g, 0.84 mmol) in sat. NaHCOs. After stirring for 1 h, the MeCN was removed in vacuo. Following acidification, preparative HPLC (a H₂O/MeCN gradient with 0.1% TFA) and lyophilization, **1.20** was isolated in 74 % yield (0.13 g, 0.42 mmol). ESI-MS: m/z calc for C₁₂H₁₉NO₇S 321.09; Obs. [M-H]⁺ 320.20.

### 1-({2-[2-(2-Aminoethoxy)ethoxy|ethyl}carbamoyl)-2-({[(1R,4E)-cyclooct-4-en-1-yloxy]carbonyl}amino)ethane-1-sulfonic acid (1.21)

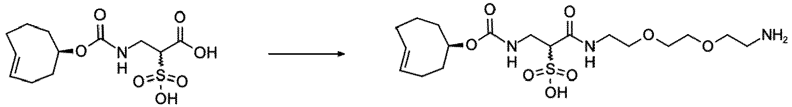

PyBOP (0.28 g, 0.54 mmol) was added to a solution of **1.20** (0.13 g, 0.42 mmol) and amino-PEG₂-amine (2.47 g, 16.6 mmol) in MeCN and the reaction mixture was stirred for 1 h at rt. Following acidification with aqueous citric acid, preparative HPLC (a H₂O/MeCN gradient with 0.1% TFA) and lyophilization, **1.21** was isolated in 71% yield (0.17 g, 0.30 mmol) as stick solid. ESI-MS: m/z calc for C₁₈H₃₃N₃O₈S 451.20; Obs. [M+H]⁺ 452.12.

### Maleimide-TCO-TCO (1.22)

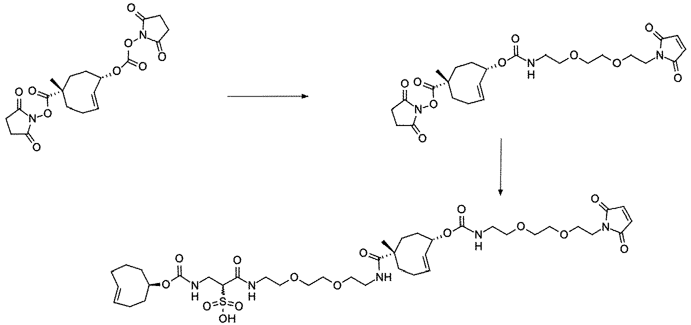

**1.1** (10 mg, 22.6 µmol) was treated with the TFA salt of 1-{2-[2-(2-aminoethoxy) ethoxy] ethyl}-2,5-dihydro-1H-pyrrole-2,5-dione (7.7 mg, 22.6 µmol), and DiPEA (11.9 µL, 67.8 µmol) in DMSO for 3 h. **1.21** (15.3 mg, 27.1 µmol) and DiPEA (11.9 µL, 67.8 µmol) were added and the reaction mixture was stirred for 16 h at rt. Preparative HPLC (a H₂O/MeCN gradient with 0.1% TFA) and lyophilization, yielding **1.22** in 9.3 % yield (1.8 mg, 2.1 µmol). ESI-MS: m/z calc for C₃₉H₆₁N₅O₁₅S 871.39; Obs. [M+H]⁺ 872.16.

### Ethyl 2-hydroxy-2-[4-(methylamino)phenyl]acetate (1.23)

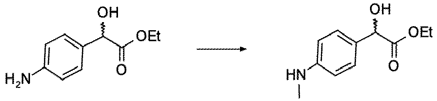

Ethyl 2-(4-aminophenyl)-2-hydroxyacetate (1.5 g, 7.7 mmol) was treated with K₂CO₃ (5.3 g, 38.4 mmol) and MeI (0.6 mL, 9.6 mmol) under N₂ for 30 min in DMF (10 mL). H₂O (80 mL) was added followed by extraction with EtOAc (3×80 mL). The combined organics were washed with brine (2×80 mL), dried over MgSO₄ and concentrated. Compound **1.23** was isolated after silica gel column chromatography (EtOAc / pentanes, 2:8 to 3:7). Yield: 32% (0.51 g, 2.4 mmol). ESI-MS: m/z calc for C₁₁H₁₅NO₃ 209.11; Obs. [M+H]⁺ 210.08. ¹H NMR (400 MHz, CD₃OD): δ 7.23 (d, J = 8.4 Hz, 2H), 6.60 (d, J = 8.6 Hz, 2H), 5.06 (s, 1H), 4.32-4.24 (m, 1H), 4.22-4.14 (m, 1H), 2.85 (s, 3H), 1.25 (t, J = 7.14 Hz) ppm.

### 2-Hydroxy-2-[4-(methylamino)phenyl]acetic acid (1.24)

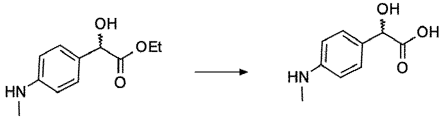

10M NaOH (1 mL, 10 mmol) in H₂O was added to **1.23** (0.48 g, 2.3 mmol) in THF followed by stirring for 2 h at 40°C. Following acidification, preparative HPLC (a H₂O/MeCN gradient with 0.1% TFA) and lyophilization, the TFA salt of **1.24** was isolated in 78% yield (0.52g, 1.76 mmol). ¹H NMR (400 MHz, CD₃OD): δ 7.51 (d, J = 8.4 Hz, 2H), 7.39 (d, J = 8.6 Hz, 2H), 5.24 (s, 1H), 2.96 (s, 3H) ppm. NOESY (400 MHz, D₂O) confirmed the location of the methyl group to be at the anilinic amine.

### 2-[4-({[(1R,2E)-Cyclooct-2-en-1-yloxy]carbonyl}(methyl)amino) phenyl]-2-hydroxyacetic acid (1.25)

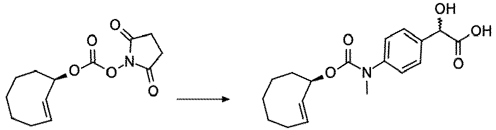

### (1R,2E)-Cyclooct-2-en-1-yl 2,5-dioxopyrrolidin-1-yl carbonate (the axial isomer)

(0.11 g, 0.41 mmol) was treated with **1.24**, DiPEA (0.29 mL, 1.65 mmol), and HOBt.H₂O (31.5 mg, 0.21 mmol) for 3 days in DMF at rt. Following preparative HPLC (a H₂O/MeCN gradient with 0.1% TFA) and lyophilization, **1.25** was isolated in 66% yield (91 mg, 0.27 mol). ESI-MS: m/z calc for C₁₈H₂₃NO₅ 333.16; Obs. [M-H]⁺ 332.12. ¹H NMR (400 MHz, CDCl₃): δ 7.42 (d, J = 8.4 Hz, 2H), 7.26 (d, J = 8.6 Hz, 2H), 6.04 (bs, 1H), 5.48 (d, J = 16.0 Hz, 1H), 5.40 (bs, 1H), 5.18 (bs, 1H), 3.32 (s, 3H), 2.40 (m, 1H), 1.90-2.12 (m, 3H), 1.83 (m, 1H), 1.55-1.70 (m, 2H), 1.26-1.43 (m, 2H), 0.71-0.83 (m, 1H) ppm.

### (1R,2E)-Cyclooct-2-en-1-yl N-(4-{[(2-{2-[2-(2,5-dioxo-2,5-dihydro-1H-pyrrol-1-yl)ethoxy]ethoxy}ethyl)carbamoyl] (hydroxy)methyl}phenyl)-N-methylcarbamate (1.26)

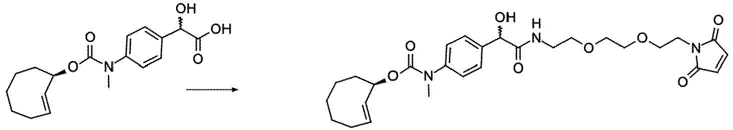

The TFA salt of 1-{2-[2-(2-aminoethoxy)ethoxy]ethyl}-2,5-dihydro-1H-pyrrole-2,5-dione (46.2 mg, 135.0 µmol), PyBOP (73.2 mg, 148.5 µmol), and DiPEA (117.6 µL, 675 µmol) were added to **1.25** (50 mg, ca. 135 µmol) in MeCN/CH₂Cl₂ followed by stirring for 15 min. Compound **1.26** was isolated after silica gel column chromatography (CH₂Cl₂ / MeOH, 98:2 to 97:3). Yield: 68% (50 mg, 92 µmol). ESI-MS: m/z calc for C₂₈H₃₇N₃O₈ 543.26; Obs. [M-H]⁺ 542.48. ¹H NMR (400 MHz, CDCl₃): δ 7.57 (t, J = 5.6 Hz, 1H), 7.42 (d, J = 8.4 Hz, 2H), 7.23 (d, J = 8.4 Hz, 2H), 6.71 (s, 2H), 6.60 (bs, 1H), 5.48 (d, J = 15.8 Hz, 1H), 5.33 (bs, 1H), 5.03 (bs, 1H), 3.70-3.74 (m, 6H), 3.61-3.66 (m, 4H), 3.57-3.60 (m, 2H), 3.50-3.55 (m, 4H), 3.40-3.42 (m, 2H), 3.28 (s, 3H), 2.38 (bs, 1H), 1.9-2.05 (m, 3H), 1.77-1.85 (m, 1H), 1.52-1.69 (m, 4H), 1.26-1.43 (m, 2H), 0.80-0.87 (m, 1H) ppm.

### TCO-Mandelic-(PEG2-Mal)-(PEG4-DOTAGA) (1.27)

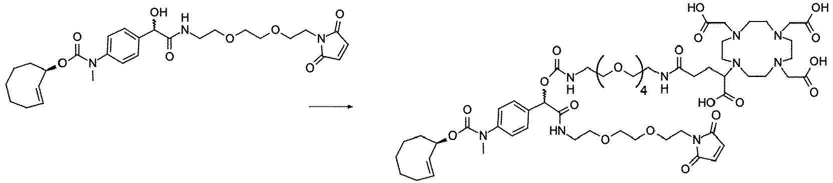

**1.26** is treated with DSC (1.5 eq.) and DiPEA (3 eq.) in dry DMSO for 4 days at rt followed by the addition of 4-[(14-amino-3,6,9,12-tetraoxatetradecan-1-yl)carbamoyl]-2-[4,7,10-tris(carboxymethyl)-1,4,7,10-tetraazacyclododecan-1-yl]butanoic acid HCl salt (2.5 eq) and DiPEA (7 eq) and stirring for 1 h at rt. Following acidification, preparative HPLC (a H₂O/MeCN gradient with 0.1% TFA) and lyophilization yields the named compound.

### DOTAGA-PEG4-TCO-PEG2-Mal (1.28)

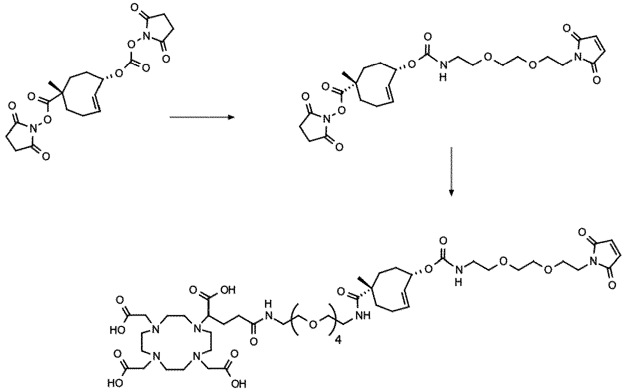

**1.1** (1.0 eq) is treated with the TFA salt of 1-{2-[2-(2-aminoethoxy) ethoxy] ethyl}-2,5-dihydro-1H-pyrrole-2,5-dione (1.1 eq), and DiPEA (3 eq.) in DMSO for 3 h. 2,2',2"-(10-(1-amino-19-carboxy-16-oxo-3,6,9,12-tetraoxa-15-azanonadecan-19-yl)-1,4,7,10-tetraazacyclododecane-1,4,7-triyl)triacetic acid hydrochloride (1.1 eq) and DiPEA (3 eq) is added and the reaction mixture is stirred for 16 h at rt. Preparative HPLC (a H₂O/MeCN gradient with 0.1% TFA) and lyophilization, yields the named compound.

### Mal-PEG9-TCO-Sar-PEG4-DFO (1.29)

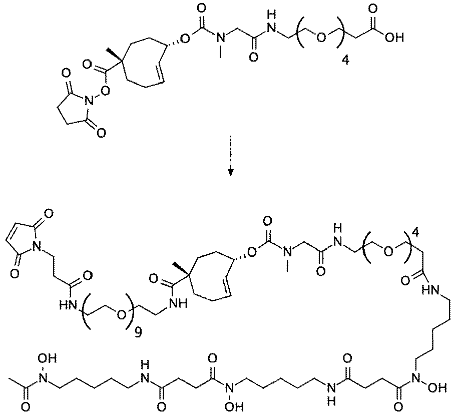

**1.16** is treated with PyBOP (1.1 eq) and DiPEA (3 eq) in dry DMSO for 15 min prior to addition of desferrioxamine mesylate salt (1.1 eq) and additional DiPEA (3 eq). After 1 h, and *N*-(29-amino-3,6,9,12,15,18,21,24,27-nonaoxanonacosyl)-3-(2,5-dioxo-2,5-dihydro-1*H*-pyrrol-1-yl)propanamide TFA salt (1.1 eq) is added and the reaction mixture is stirred for 16 h. Acidification, preparative HPLC (a H₂O/MeCN gradient with 0.1% TFA) and lyophilization, yields the named compound.

### NHS-TCO-PEG4-DOTA (1.30)

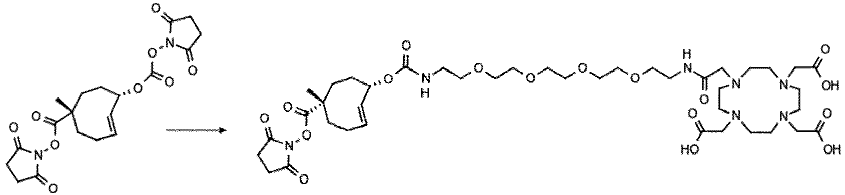

A solution of 2-(4-{[(14-amino-3,6,9,12-tetraoxatetradecan-1-yl)carbamoyl] methyl}-7, 10-bis(carboxymethyl)-1,4,7,10-tetraazacyclododecan-1-yl)acetic acid (1.1 eq.) and DiPEA (6 eq) in MQ-H₂O is added to a solution of **1.1** (1.0 eq) in MeCN and the reaction mixture is stirred for 5 min prior to acidification and prep purification to yield the title compound after lyophilization.

### NHS-TCO-PEG2-HOPO (1.31)

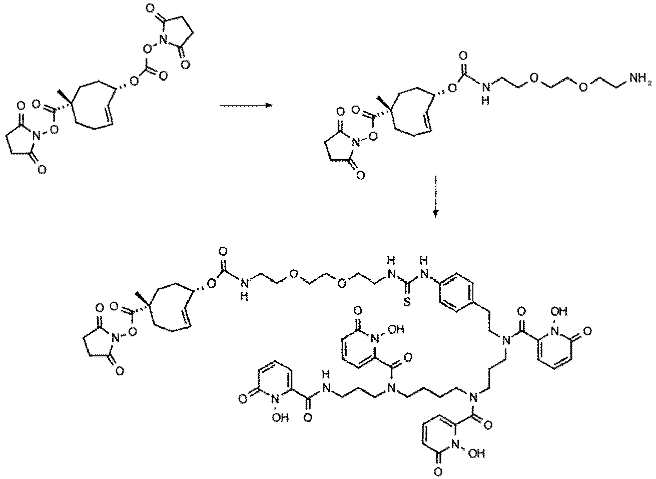

**1.1** (1 eq) is treated with amino-PEG₂-NH₂ (1 eq.) and DiPEA (2 eq) in MeCN for 5 min at rt prior to acidification and prep purification to yield the intermediate compound (TFA salt) after lyophilization. The intermediate is dissolved and treated with 1-hydroxy-N-{4-[1-(1-hydroxy-6-oxo-1,6-dihydropyridin-2-yl)-N-{3-[1-(1-hydroxy-6-oxo-1,6-dihydropyridin-2-yl)-N-[2-(4-isothio cyanatophenyl)ethyl] formamido]propyl}formamido]butyl}-N-{3-[(1-hydroxy-6-oxo-1,6-dihydropyridin-2-yl)formamido]propyl}-6-oxo-1,6-dihydropyridine-2-carboxamide [Bhupathiraju et al., Org Biomol Chem 2019, 17, 6866-6871] (1.1 eq) and DiPEA (3 eq) in DMSO for 18 h prior to acidification and prep purification to yield the title compound after lyophilization.

### Example 2: Synthesis of tetrazine Cleaving Agents

Tetrazines **2.15** and **2.16** where purchased from commercial sources. Tetrazines **2.13**, **2.14**, **2.17**, **2.18** (10 kDa dextrane), and **2.19** were prepared according to literature procedures [Versteegen et al., Angew Chem Int Ed 2013, 53, 14112; Rossin et al. Bioconjug Chem 2016, 27, 1697; Fan et al., Angew Chem Int Ed 2016, 55, 14046], while **2.20**, **2.21**, and **2.22** were prepared as reported in WO2019212356 (therein: compounds 2.12, 4.12, 4.28).

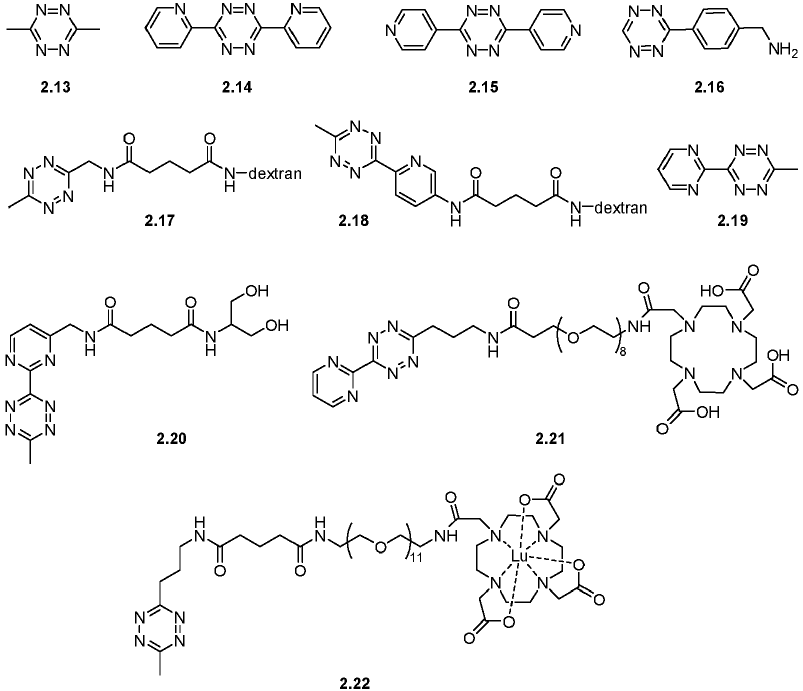

### 2,2'-(1,2,4,5-Tetrazine-3,6-diyl)bis(pyridin-3-ol) (2.1)

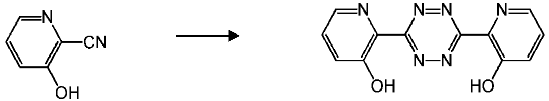

3-Hydroxypicolinonitrile (100 mg, 0.82 mmol) and hydrazine hydrate (280 µL, 4.9 mmol, 6 eq) were stirred at 90°C for 2 h. Ethanol (4 mL) was added and the suspension was stirred at rt for 5 min. The suspension was filtrated and the solid was washed with ethanol (5×2 mL). Drying of the solid in vacuo yielded pure intermediate [2H]-TZ (59 mg, 0.22 mmol, 54 %) as a yellow solid. The [2H]-TZ was suspended in acetic acid (6 mL) and NaNO₂ (75 mg, 1.1 mmol, 5 eq) in water (500 µL) was added dropwise. The suspension was stirred at rt for 1 h during which a clear red solution was obtained and, eventually, a red precipitate arose. Chloroform and water (both 40 mL) were added and the layers were separated. The aqueous layer was extracted with chloroform (2×20 mL) and the combined organic layers were dried using Na₂SO₄. After filtration, the filtrate was evaporated to dryness yielding pure **2.1** (55 mg, 0.21 mmol, 50 % overall) as a red solid. ¹H-NMR (DMSO-d6): δ = 10.74 (br s, 2H, O*H*), 8.38 (m, 2H, Ar*H*), 7.57 (m, 4H, Ar*H*) ppm. ¹³C-NMR (DMSO-d6): *δ* = 164.3, 154.3, 141.4, 137.5, 127.5, 125.3 ppm. ESI-MS: *m*/*z* Calc. for C₁₂H₈N₆O₂ 268.07; Obs. [M+H]⁺ 269.17, [M+Na]⁺ 291.25.

### 4,4'-(1,2,4,5-Tetrazine-3,6-diyl)bis(pyridin-3-ol) (2.2)

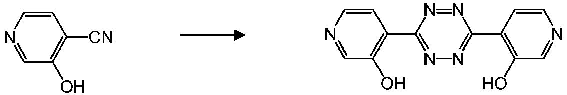

3-Hydroxyisonicotinonitrile (62 mg, 0.52 mmol), Zn(OTf)₂ (10 mg, 28 µmol, 0.05 eq), ethanol (80 µL) and hydrazine hydrate (300 µL, 5.3 mmol, 10 eq) were stirred at 60°C for 21 h. After removal of the volatiles in vacuo the solid was stirred in methanol (4 mL) for 5 min at rt. The suspension was filtrated, the solid was washed with methanol (5×2 mL) and dried in vacuo yielding impure intermediate [2H]-TZ as an orange solid. The [2H]-TZ was stirred in acetic acid (4 mL) and NaNO₂ (32 mg, 0.45 mmol) in water (200 µL) was added dropwise causing an immediate color change from orange to red. After stirring at rt for 1 h the suspension was filtrated, the solid was washed with ethanol, water and ethanol (all 5×2 mL) and dried in vacuo. This yielded **2.2** (10 mg, 37 µmol, 14 % overall) as a red solid. ¹H-NMR (DMSO-d6): *δ* = 10.89 (br, 2H, O*H*), 8.56 (s, 2H, Ar*H*), 8.37 (d, 2H, Ar*H*), 8.04 (d, 2H, ArH) ppm. ESI-MS: *m*/*z* Calc. for C₁₂H₈N₆O₂ 268.07; Obs. [M+H]⁺ 269.17.

### 2,2'-(1,2,4,5-tetrazine-3,6-diyl)bis(pyridin-3-amine) (2.3)

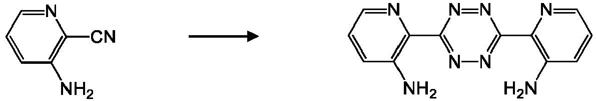

3-Aminopicolinonitrile (125 mg, 1.0 mmol) and hydrazine hydrate (300 µL, 5.0 mmol, 5 eq) were stirred at 100°C for 20 h. Cold water (2 mL) was added and the suspension was stirred at rt for 5 min. Filtration, washing of the solid with cold water and cold ethanol (both 5×2 mL) and drying in vacuo yielded the intermediate [2H]-TZ (38 mg, 0.14 mmol, 27 %) as an orange solid. To the [2H]-TZ and PhI(OAc)₂ (75 mg, 0.23 mmol, 1.6 eq) dichloromethane (1 mL) was added and the suspension was stirred at rt for 3 h. In time a color change occurred from orange to red. The suspension was filtrated, the solid was washed with dichloromethane (5×1 mL) and dried in vacuo yielding pure **2.3** (31 mg, 0.12 mmol, 22 % overall) as a red solid. ¹H-NMR (DMSO-d6): *δ* = 8.13 (dd, 2H, Ar*H*), 7.36 (2dd, 4H, Ar*H*), 6.98 (br s, 4H, N*H*₂) ppm. ¹³C-NMR (DMSO-d6): δ = 162.8, 146.6, 138.3, 129.5, 126.9, 124.5 ppm. ESI-MS: *m*/*z* Calc. for C₁₂H₁₀N₈ 266.10; Obs. [M+H]⁺ 267.08, [2M+H]⁺ 532.92, [2M+Na]⁺ 555.00.

### N,N'-(2,2'-(1,2,4,5-Tetrazine-3,6-diyl)bis(pyridine-3,2-diyl))diacetamide (2.4)

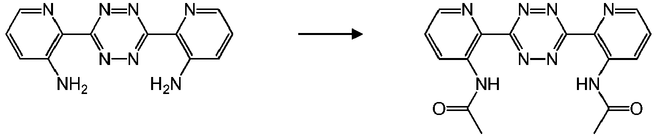

**2.3** (12 mg, 45 µmol) was suspended in acetic anhydride (0.5 mL) and the suspension was heated at 50°C for 3 days. The mixture was precipitated in diethyl ether (6 mL) and the solution was decanted. The solid was washed with diethyl ether (2 mL) and the solution was decanted, after which the wash step was repeated. Next, the solid was triturated with water (2 mL), the mixture was centrifuged at 12.7 krpm for 1 min and the solution was decanted. The solid was subsequently dissolved in methanol (1 mL), after which non-dissolved impurities were removed by filtration. The filtrate was evaporated to dryness and the obtained residue was triturated with water (2 mL). After centrifugation at 12.7 krpm for 1 min and decantation, the solid was dried in vacuo yielding **2.4** (0.75 mg, 2.1 µmol, 5 %) as a purple-red solid. ESI-MS: *m*/*z* Calc. for C₁₆H₁₄N₈O₂ 350.12; Obs. [M+H]⁺ 351.17, [2M+Na]⁺ 722.92.

### 4,4'-(1,2,4,5-tetrazine-3,6-diyl)bis(pyridin-3-amine) (2.5)

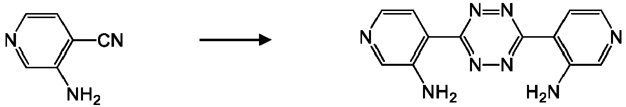

3-Aminoisonicotinonitrile (200 mg, 1.6 mmol), S (26 mg, 0.8 mmol, 0.5 eq) and hydrazine hydrate (460 µL, 8.1 mmol, 5 eq) were stirred at 100°C for 16 h. Cold water (2 mL) was added and the suspension was stirred at rt for 5 min. The suspension was filtrated and the solid was washed with cold water and cold ethanol (both 5×4 mL). Trituration in ethanol (20 mL) at 50°C followed by filtration, washing of the solid with ethanol (5×4 mL) and drying in vacuo yielded the intermediate [2H]-TZ (144 mg, 0.54 mmol, 67 %) as an orange solid. The [2H]-TZ was stirred in DMSO (5 mL) at 40°C while bubbling through O₂. In time a color change occurred from orange to red. After stirring for 16 h the reaction mixture was added dropwise to water (70 mL) and the resulting suspension was filtrated. The solid was washed with water, ethanol and dichloromethane (all 5×4 mL) and dried in vacuo yielding pure **2.5** (130 mg, 0.49 mmol, 61 % overall) as a red solid. ¹H-NMR (DMSO-d6): *δ* = 8.40 (br s, 2H, Ar*H*), 8.20 (d, 2H, Ar*H*), 7.95 (br d, 2H, Ar*H*), 7.16 (br s, 4H, N*H*₂) ppm. ¹³C-NMR (DMSO-d6): *δ* = 162.7, 144.3 (br), 141.3, 135.9, 120.8 (br), 115.9 ppm. ESI-MS: *m*/*z* Calc. for C₁₂H₁₀N₈ 266.10; Obs. [M+H]⁺ 267.17.

### N,N'-(4,4'-(1,2,4,5-Tetrazine-3,6-diyl)bis(pyridine-4,3-diyl))diacetamide (2.6)

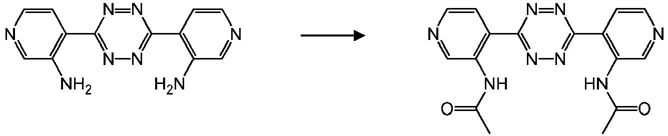

**2.5** (22 mg, 83 µmol) was suspended in acetic anhydride (0.5 mL) and the suspension was heated at 50°C for 3 days. The mixture was precipitated in diethyl ether (6 mL) and the solution was decanted. Next, the solid was triturated with water (2 mL), the mixture was centrifuged at 12.7 krpm for 1 min and the solution was decanted. This trituration-centrifugation procedure was repeated using methanol and 2-propanol (both 2 mL). The resulting solid was dried in vacuo yielding pure **2.6** (12 mg, 34 µmol, 41 %) as a purple-red solid. ¹H-NMR (DMSO-d6): *δ* = 10.35 (s, 2H, N*H*), 9.01 (s, 2H, ArH), 8.67 (d, 2H, Ar*H*), 8.02 (d, 2H, Ar*H*), 2.00 (s, 6H, C*H*₃) ppm. ¹³C-NMR (DMSO-d6): *δ* = 168.7, 164.1, 146.4, 146.1, 132.4, 131.4, 123.7, 23.3 ppm. ESI-MS: *m*/*z* Calc. for C₁₆H₁₄N₈O₂ 350.12; Obs. [M+H]⁺ 351.17.

### 5,5'-((2,2'-(1,2,4,5-Tetrazine-3,6-diyl)bis(pyridine-3,2-diyl))bis(azanediyl))bis(5-oxopentanoic acid) (2.7)

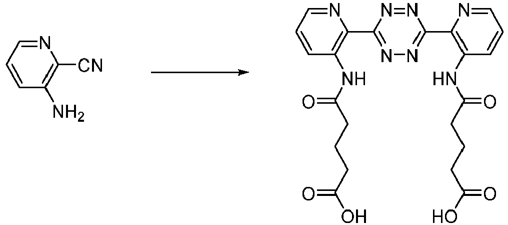

3-Aminopicolinonitrile (0.62 g, 5.2 mmol), 3-mercaptopropionic acid (0.46 mL, 5.2 mmol, 1 eq) and hydrazine hydrate (1.8 mL, 32 mmol, 6 eq) were stirred at 90°C for 18 h. Cold water (2 mL) was added and the suspension was stirred at rt for 5 min. Filtration, washing of the solid with cold water and cold ethanol (both 5×2 mL) and drying in vacuo yielded the intermediate [2H]-TZ (0.66 g, 2.4 mmol, 94%) as an orange solid. [2H]-TZ (200 mg, 0.75 mmol) and glutaric anhydride (0.9 g, 7.5 mmol, 10 eq) were suspended in dry THF (3 mL). Upon stirring at 52°C the suspension cleared and the mixture was stirred at 52°C for 19 h. The obtained suspension was filtrated and the resulting solid was washed with diethyl ether (3×4 mL). The solid was transferred to a round-bottom flask and the solvent was removed in vacuo. The compound was suspended in acetic acid (8 mL) and concentrated nitric acid (6 drops) was added dropwise (CAUTION: toxic fumes). The initially orange suspension turned red in seconds and the mixture was stirred at rt for 30 min. The suspension was filtrated and the resulting solid was washed with acetic acid (3×4 mL) and diethyl ether (3×4 mL). The solid was dried in vacuo at 35°C for 2 h yielding pure **2.7** (327 mg, 0.66 mmol, 89%) as a red solid. ¹H-NMR (DMSO-d6): *δ* = 10.15 (s, 2H, N*H*), 8.65 (dd, 2H, Ar*H*), 8.32 (dd, 2H, Ar*H*), 7.71 (dd, 2H, Ar*H*), 2.27 (t, 4H, C*H*₂), 2.22 (t, 4H, C*H*₂), 1.70 (qn, 4H, CH₂C*H*₂CH₂) ppm. ¹³C-NMR (DMSO-d6): *δ* = 174.1, 171.0, 164.5, 145.8, 142.2, 134.5, 132.0, 126.1, 35.0, 32.8, 20.1 ppm. ESI-MS: *m*/*z* Calc. for C₂₂H₂₂N₈O₆ 494.17; Obs. [M+H]⁺ 495.33, λₘₐₓ = 240, 328 and 525 nm.

### N¹,N^{1'}-(2,2'-(1,2,4,5-Tetrazine-3,6-diyl)bis(pyridine-3,2-diyl))bis(N⁵-(29-hydroxy-3,6,9,12,15,18,21,24,27-nonaoxanonacosyl)glutaramide) (2.8)

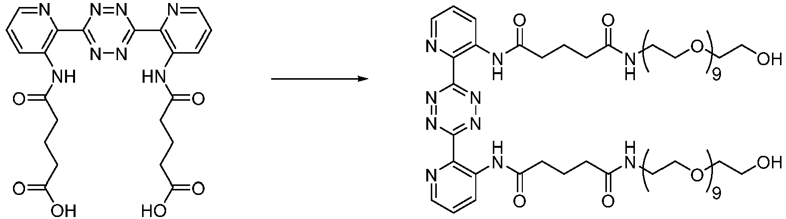

**2.7** (32.7 mg, 66 µmοl) and 29-amino-3,6,9, 12, 15, 18,21,24,27-nonaoxanonacosan-1-ol (64.1 mg, 0.14 mmol, 2.1 eq) were suspended in DMF (0.6 mL). A solution of PyBOP (92 mg, 0.17 mmol, 2.6 eq) in DMF (0.5 mL) and N-methylmorpholine (44 µL, 0.40 mmol, 6 eq) were added. The mixture was stirred at rt for 2 h during which the suspension eventually cleared. Column chromatography (flash SiO₂) using an elution gradient of 8% to 16% methanol in chloroform yielded impure product. The compound was dissolved in chloroform (60 mL, with some added methanol) and washed with water / brine 1:1 (30 mL). The organic layer was dried with Na₂SO₄, the mixture was filtrated and the solution was evaporated to dryness. This yielded pure **2.8** (57 mg, 41 µmοl, 63%) as a red sticky solid. ¹H-NMR (DMSO-d6): *δ* = 10.16 (s, 2H, ArN*H*), 8.65 (dd, 2H, Ar*H*), 8.33 (dd, 2H, ArH), 7.79 (t, 2H, N*H*CH₂), 7.71 (dd, 2H, Ar*H*), 4.54 (t, 2H, O*H*), 3.53-3.33 (m, 76H, OC*H*₂), 3.16 (m, 4H, NHC*H*₂), 2.23 (t, 4H, C*H*₂), 2.07 (t, 4H, C*H*₂), 1.70 (qn, 4H, CH₂C*H*₂CH₂) ppm. ESI-MS: *m*/*z* Calc. for C₆₂H₁₀₄N₁₀O₂₄ 1372.72; Obs. [2M+H]⁺ 687.58, [M+H]⁺ 1373.83, λₘₐₓ = 242 and 524 nm.

### 5,5'-((4,4'-(1,2,4,5-Tetrazine-3,6-diyl)bis(pyridine-4,3-diyl))bis(azanediyl))bis(5-oxopentanoic acid) (2.9)

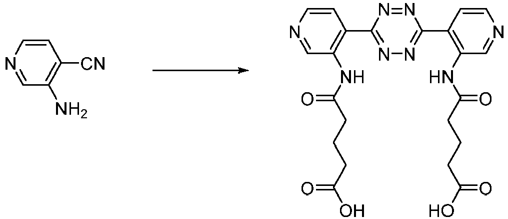

3-Aminoisonicotinonitrile (200 mg, 1.6 mmol), S (26 mg, 0.8 mmol, 0.5 eq) and hydrazine hydrate (0.46 mL, 8.1 mmol, 5 eq) were stirred at 100°C for 16 h. Cold water (2 mL) was added and the suspension was stirred at rt for 5 min. The suspension was filtrated and the solid was washed with cold water and cold ethanol (both 5×4 mL). Trituration of the solid in ethanol (20 mL) at 50°C for 15 min was followed by filtration. The solid was washed with ethanol (5×4 mL) and dried in vacuo yielding the intermediate [2H]-TZ (144 mg, 0.54 mmol, 67%) as an orange solid. The [2H]-TZ (200 mg, 0.75 mmol) and glutaric anhydride (0.9 g, 7.5 mmol, 10 eq) were suspended in dry THF (2 mL) and the mixture was stirred under an Ar atmosphere at 52°C for 14 days. In time, the reaction mixture turned into a paste and additional THF was occasionally added to ensure that stirring continued. After ¹H-NMR confirmed close to full conversion the paste was transferred to a round-bottom flask and the solvent was removed in vacuo. The compound was suspended in acetic acid (20 mL) and concentrated nitric acid (0.75 mL) was added dropwise (CAUTION: toxic fumes). The initially orange suspension turned red in seconds and the mixture was stirred at rt for 30 min. The suspension was filtrated and the resulting solid was washed with acetic acid (3×4 mL). The solid was thoroughly broken up using a spatula, washed with diethyl ether (3×4 mL) and dried in vacuo at 30°C for 1 h. The dry solid was powdered using a spatula and stirred in methanol (6 mL) at rt for 30 min. The suspension was filtrated and the resulting solid was washed with methanol (2×8 mL) and diethyl ether (5×8 mL). The solid was dried in vacuo at 30°C for 1 h yielding **2.9** (313 mg, 0.63 mmol, 85%) as a pink solid. ¹H-NMR (DMSO-d6): *δ* = 10.41 (s, 2H, N*H*), 9.05 (s, 2H, Ar*H*), 8.67 (d, 2H, Ar*H*), 8.04 (d, 2H, Ar*H*), 2.33 (t, 4H, C*H*₂), 2.24 (t, 4H, C*H*₂), 1.72 (qn, 4H, CH₂C*H*₂CH₂) ppm. ¹³C-NMR (DMSO-d6): *δ* = 174.1, 171.2, 164.0, 145.1, 144.8, 132.9, 131.9, 124.3, 35.0, 32.8, 20.1 ppm. ESI-MS: *m*/*z* Calc. for C₂₂H₂₂N₈O₆ 494.17; Obs. [M+H]⁺ 495.33, λₘₐₓ = 240, 328 and 525 nm.

### N¹,N^{1'}-(4,4'-(1,2,4,5-Tetrazine-3,6-diyl)bis(pyridine-4,3-diyl))bis(N⁵-(29-hydroxy-3,6,9,12,15,18,21,24,27-nonaoxanonacosyl)glutaramide) (2.10)

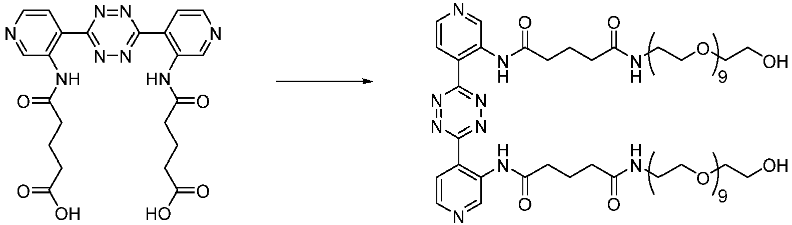

**2.9** (27.5 mg, 56 µmol) and 29-amino-3,6,9, 12, 15, 18,21,24,27-nonaoxanonacosan-1-ol (53.5 mg, 0.12 mmol, 2.1 eq) were suspended in DMF (0.5 mL). A solution of PyBOP (75 mg, 0.14 mmol, 2.5 eq) in DMF (0.4 mL) and N-methylmorpholine (37 µL, 0.33 mmol, 6 eq) were added. The mixture was stirred at rt for 90 min during which the suspension eventually cleared. Column chromatography (flash SiO₂) using an elution gradient of 10% to 20% methanol in chloroform yielded impure product. The compound was dissolved in chloroform (40 mL, with some added methanol) and washed with water / brine 1:1 (25 mL). The organic layer was dried with Na₂SO₄, the mixture was filtrated and the solution was evaporated to dryness. This yielded pure **2.10** (36 mg, 56 µmοl, 47%) as a red sticky solid. ¹H-NMR (DMSO-d6): δ = 10.35 (s, 2H, ArN*H*), 9.05 (s, 2H, Ar*H*), 8.66 (d, 2H, Ar*H*), 8.02 (d, 2H, Ar*H*), 7.80 (t, 2H, N*H*CH₂), 4.54 (t, 2H, O*H*), 3.53-3.34 (m, 76H, OC*H*₂), 3.17 (q, 4H, N*H*CH₂), 2.29 (t, 4H, C*H*₂), 2.09 (t, 4H, C*H*₂), 1.72 (qn, 4H, CH₂C*H*₂CH₂) ppm. ¹³C-NMR (CDCl₃): *δ* = 172.56, 171.54, 163.89, 145.73, 145.29, 133.81, 128.16, 122.44, 72.35, 70.5 - 69.7 (m), 61.50, 39.18, 36.56, 34.95, 21.30 ppm. ESI-MS: *m*/*z* Calc. for C₆₂H₁₀₄N₁₀O₂₄ 1372.72; Obs. [2M+H]⁺ 687.75, [M+H]⁺ 1373.75, λₘₐₓ = 237 and 526 nm.

### Tetrazine-bis-GalNAc (with clearance directing groups) (2.11)

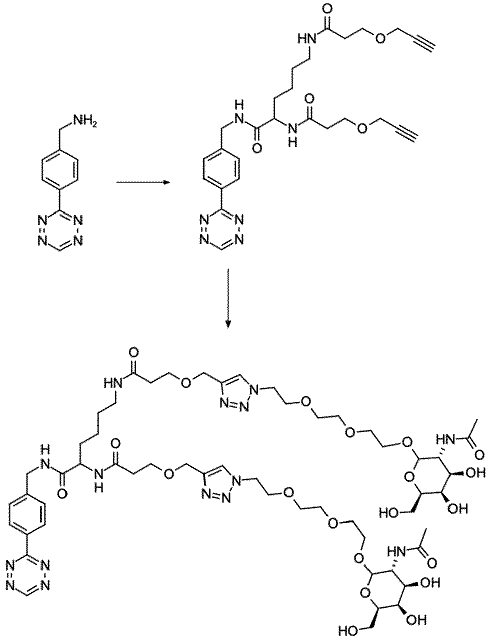

1-[4-(1,2,4,5-Tetrazin-3-yl)phenyl]methanamine (15.9 mg, 65.0 µmol) was treated with 2,6-bis[3-(prop-2-yn-1-yloxy)propanamido]hexanoic acid (23.8 mg, 65.0), HBTU (24.7 mg, 65.1 µmol) and TEA (13.6 µL, 97.6 µmol) in DMF for 4 h followed by partitioning with CH₂Cl₂ and H₂O. The organic phase was washed with 0.1M HCl, 0.1 M NaOH, H₂O, and brine prior to drying over Na₂SO₄ and concentration. The tetrazine-bis-alkyn intermediate (5 mg, 9 µmol) was treated with N-[(3R,4R,5R,6R)-2-{2-[2-(2-azidoethoxy)ethoxy] ethoxy}-4,5-dihydroxy-6-(hydroxymethyl)oxan-3-yl]acetamide (9 mg, 23 µmol) TEA (1 µL, 9 µmol) and CuI (3 mg, 18 µmol) in DMF/H₂O for two hours at rt. Preparative HPLC (a H₂O/MeCN gradient with 0.1% TFA) and lyophilization gave **2.11** in 40% yield (4.7 mg, 3.6 µmol). ESI-MS: m/z calc for C₅₅H₈₅N₁₅O₂₁ 1291.60; Obs [M+H]⁺ 1292.12, [M+2H]⁺ 646.64.

### Synthesis of tetrazine-albumin-galactose Cleaving Agent (with clearance directing groups) (2.12)

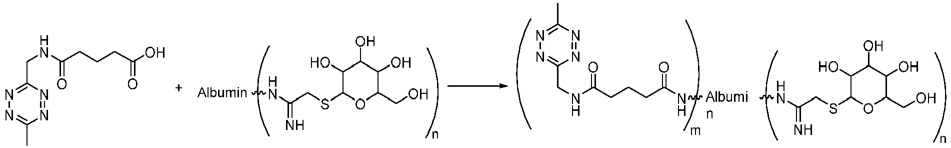

5-(((6-Methyl-1,2,4,5-tetrazin-3-yl)methyl)amino)-5-oxopentanoic acid [Bioconjug Chem 2016, 27, 1697-1706] (0.22 mg; 7.56 µmol) was activated with EDC and s-NHS in water following the protocol provided by the EDC and s-NHS manufacturer (Thermo Fisher) and then purified through a desalting cartridge. The solution was then added to 3 mg of a mouse albumin-galactose conjugate (prepared as reported in Rossin et al., J Nucl Med 2013, 54, 1989-1995) in PBS at pH 9.0 (750 ml total). The mixture was incubated at rt for 30 min and then it was transferred into an Amicon Ultra-15 centrifugal filter (30 kDa MW cut-off) where product **2.12** was washed extensively with water. After freeze-drying, the albumin modification grade was determined by MALDI-TOF analysis. MSA: [M+H]⁺ = 65941 m/z; MSA-Gal: [M+H]⁺ = 69302 m/z; TZ-MSA-Gal: [M+H]⁺ = 72001 m/z, corresponding to 14.2 galactose and 12.2 tetrazine moieties per albumin molecule.

### Example 3: Antibody conjugation and radiolabeling

The mAbs CC49, rituximab, cetuximab, trastuzumab, and girentuximab were functionalized with **1.2**, **1.4**, **1.6**, **1.7**, **1.14**, **1.15** and **1.17** using standard lysine conjugation procedures. Briefly, mAb solutions in PBS (4 mg/mL final concentration) were added with 15-20 eq of the NHS-derivative (dissolved at 10 mg/mL in DMSO) and the pH was adjusted to 9 with 1M sodium carbonate. The reaction mixtures were incubated at rt in the dark for 2h then the products were purified by dialysis (20 kDa MW cut-off membranes) in chelex-treated 0.25M NH₄OAc pH 5.5. The same mAbs were also functionalized with **1.8** and **1.18** using two different procedures: 1) partial reduction of the mAb hinge with TCEP (3 eq, 2h at rt) in 25 mM borate buffer pH 8.0 (containing 1 mM DTPA) or 2) lysine functionalization with SATA (4 eq) followed by deprotection with hydroxylamine, according to the protocol provided by the SATA manufacturer (Thermo Fisher Scientific). In both cases, the obtained free -SH groups were then reacted with **1.8** or **1.18** (20 eq with respect to mAb) overnight at +4°C, and the mAb conjugates were purified by dialysis in chelex-treated PBS. After dialysis, all mAb conjugates were characterized by SEC and SDS-PAGE and an average of 2-3 TCO groups were measured per mAb using a tetrazine titration, as previously published [Rossin et al., Angew Chem Int Ed 2010, 49, 3375-3378]. The mAbs functionalized with TCO-DOTA derivatives (typically 50-100 µg) were radiolabeled with ¹¹¹In and ¹⁷⁷Lu (typically 5-10 MBq) in 0.25 NH₄OAc pH 5.5 (also in presence of 0.5M MES buffer pH 5.5 for ¹¹¹In) at 37°C for 1h in the dark, obtaining 50-70% labeling yields as confirmed by radio-ITLC. The mAbs functionalized with TCO-DFO derivatives (typically 50-100 µg) were radiolabeled with ⁸⁹Zr following established protocols [Vosjan et al., Nature Protocols 2010, 5, 739-743], obtaining 80-90% labeling yields as confirmed by radio-ITLC. After a 5 min DTPA challenge, all radiolabeled mAbs were purified using desalting cartridges (Zeba spin columns, 40 kDa MW cut-off) and were then analyzed by SEC and SDS-PAGE, confirming >95% radiochemical purity.

### Example 4: CC49 conjugation with Trigger 1.22, radiolabeling with I-125 and triggered 1-125 label release in vitro

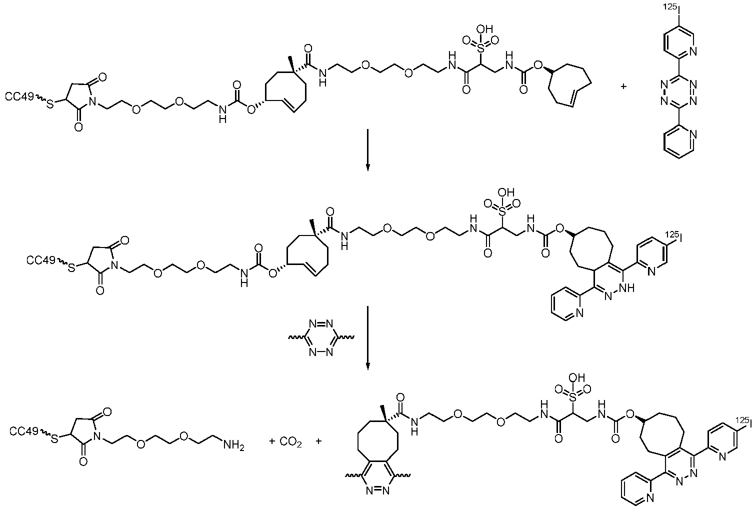

Maleimide-TCO-TCO **1.21** comprises two TCO moieties that can be selectively manipulated, as the TCO tag on the outside is ca 20-fold more reactive than the TCO linker, and will preferentially react with any tetrazine at sub-equimolar conditions. This property was used for the efficient iodination of a mAb via a cleavable Trigger. CC49 was conjugated with **1.22** using TCEP, as described in Example 3, and the conjugate was labeled via IEDDA conjugation on the outer TCO tag using a previously described ¹²⁵I-labeled tetrazine [Albu et al., Bioconjug Chem 2016, 27, 207-216] in PBS at 37°C for 1h. The crude labeling mixture was purified using a desalting cartridge (Zeba spin column, 40 kDa MW cut-off). The ¹²⁵I-labeled CC49 conjugate was subsequently incubated with Cleaving Agent **2.1** for 2h in PBS at 37°C, yielding 88.6% label release (Table 1), as confirmed by SEC analysis.

### Example 5: in vitro Label release from mAb-TCO-chelate conjugates

mAb conjugates radiolabeled with ¹¹¹In, ¹⁷⁷Lu, ⁸⁹Zr, or ¹²⁵I (>95% radiochemical purity at t=0) were reacted with a range of Cleaving Agents and the Label release was quantified. mAb conjugate (ca. 10 µg) was incubated with an excess (ca. 30 eq) of Cleaving Agent or without Cleaving Agent in 100 µL PBS or 50% mouse plasma at 37°C. After 15-24 h incubation the mixtures were analyzed by SEC and the amount of released Label was quantified from the radioactivity elution profile. The results of the experiments are summarized in Table 1, demonstrating efficient Label cleavage for a range of different mAbs (and conjugation methods), radionuclides, Label designs Trigger designs and Cleaving Agents, in physiological conditions.

**Table 1: Summary of in vitro Label release from radiolabeled mAb-conjugates after 15-24 h incubation with various Cleaving Agents.**

| **TCO derivative** | **mAb** | **radionuclide** | **Cleaving Agent** | **release (%)** |
|---|---|---|---|---|
| **1.2** | trastuzumab | ¹¹¹In | **2.1** | 78.2, 88.1^{a} |
| | | | **2.10** | 53.4, 52.2^{a} |
| | | | - | 5.3, 7.4^{a} |
| | CC49 | | **2.1** | 80.3 |
| | girentuximab | | | 83.2 |
| | rituximab | | | 77.9 |
| | cetuximab | | | 85.7 |
| **1.4** | trastuzumab | ¹¹¹In | **2.1** | 81.8^{a} |
| | | | **2.2** | 69.5 |
| | | | **2.10** | 57.9 |
| | | | **2.13** | 62.9^{a} |
| | | | **-** | 6.8, 7.1^{a} |
| | | ¹⁷⁷Lu | **2.13** | 69.5 |
| **1.6** | trastuzumab | ¹¹¹In | **2.1** | 83.6, 68.9^{a} |
| **1.7** | trastuzumab | ¹¹¹In | **2.1** | 78.9, 63.4^{a} |
| **1.8** | trastuzumab | ¹¹¹In | **2.1** | 86.4, 77.0^{a} |
| | | | **2.2** | 81.6 |
| | | | **2.4** | 54.5^{a} |
| | | | **2.7** | 55.3^{a} |
| | | | **2.8** | 79.9, 60.6^{a} |
| | | | **2.9** | 67.0^{a} |
| | | | **2.10** | 82.4, 82.4^{a} |
| | | | **2.11** | 67.2 |
| | | | **2.12** | 78.3 |
| | | | **2.13** | 71.4 |
| | | | **2.14** | 51.5 |
| | | | **2.16** | 64.5 |
| | | | **2.17** | 53.3 |
| | | | **2.18** | 55.3 |
| | | | **2.19** | 69.1 |
| | | | **2.20** | 67.2 |
| | | | **2.21** | 72.0 |
| | | | **2.22** | 79.0 |
| | | | - | 5.8, 6.6^{a} |
| | | ¹⁷⁷Lu | **2.10** | 81.2 |
| | | | **2.13** | 88.9 |
| | | | **2.22** | 78.9 |
| | CC49 | ¹¹¹In | **2.10** | 79.9 |
| | girentuximab | | | 83.6 |
| | rituximab | | | 82.5 |
| | cetuximab | | | 84.9 |
| **1.14** | CC49 | ⁸⁹Zr | **2.1** | 53.0 |
| | | | **2.2** | 60.3 |
| | | | **2.10** | 45.2 |
| | | | **2.13** | 53.6 |
| | | | - | 5.5 |
| **1.15** | CC49 | | **2.1** | 62.3 |
| | | | **2.2** | 65.8 |
| | | | **2.10** | 50.0 |
| | | | **2.13** | 66.3 |
| | | | - | 7.3 |
| **1.17** | CC49 | | **2.1** | 88.0, 81.3^{a} |
| | | | **2.2** | 87.2 |
| | | | **2.10** | 50.0 |
| | | | **2.13** | 72.3 |
| | | | - | 4.2, 7.5^{a} |
| | trastuzumab | | **2.1** | 88.9 |
| | | | **2.2** | 61.4 |
| | | | **2.13** | 84.4 |
| | | | - | 6.1 |
| **1.22** | CC49 | ¹²⁵I | **2.1** | 88.6^{b} |

| | | | | |
|---|---|---|---|---|
| ^{a} 50% mouse plasma in PBS; ^{b} 2h incubation in PBS at 37°C. | | | | |

### Example 6: Peptide conjugation, radiolabeling and Label cleavage in vitro (Reference Example)

Exendin (K₄₀-Exendin-3 TFA salt, 57.8 nmol) was dissolved in dry DMSO (230 µL), added with DIPEA (15 eq) and compound **1.2** (1.0 eq) and the mixture was incubated for 2 h at rt in the dark. After incubation, the reaction mixture was diluted with chelex-treated water (10 mL) and the product was extracted using two Oasis HLB cartridges and subsequently eluted from the cartridges with 2×1 mL EtOH. The solvent was evaporated under a stream of N₂ at 45°C, the residue was reconstituted in chelex-treated water. The exendin-**1.2** conjugate (5 µg) was labeled with ¹¹¹In (12 MBq) in 0.5M MES buffer pH 5.5 at 60°C for 10 min in the dark. After DTPA challenge (5 min), RP-HPLC analysis confirmed the formation of one major radioactive species with Rt consistent with peptide. Aliquots of the labeled peptide conjugate solution were then diluted with PBS and reacted with an excess (ca. 10 eq) of tetrazines **2.1**, **2.3**, **2.14**, and **2.15** or without Cleaving Agent. After overnight incubation at 37°C, RP-HPLC showed the formation of a more hydrophilic radioactive species (Table 2), which was confirmed to be ¹¹¹In-labeled NH₂-PEG4-DOTAGA, signifying cleavage of the TCO Trigger. The same species was not found when ¹¹¹In-exendin-**1.2** was incubated overnight in PBS without a tetrazine.

**Table 2: Label release (%) from ¹¹¹In-labeled exendin-1.2 after ca 15h incubation with various Cleaving Agents.**

| **Cleaving Agent** | **release (%)** |
|---|---|
| **2.1** | 85.4 |
| **2.3** | 70.0 |
| **2.14** | 63.0 |
| **2.15** | 54.0 |
| - | 1.0 |

### Example 7: In vivo stability of the TCO Trigger

In two separate experiments, female Balb/C nude mice were injected with ¹¹¹In-labeled trastuzumab-**1.8** conjugate (ca. 0.5 mg/kg, ca. 1 MBq) and whole blood was withdrawn by heart puncture 24 or 48 h post-mAb injection. Plasma was isolated from blood and analyzed by SEC to confirm the presence of intact mAb. Plasma aliquots were then reacted ex vivo with an excess of Cleaving Agent **2.10**. After overnight incubation at 37°C, the solutions were again analyzed by SEC and the fragment (i.e. Label) release % was compared to that achieved in PBS immediately before injection (t=0). In both cases, the release yield was the same as achieved at t=0, demonstrating close to 100% retention of TCO reactivity towards tetrazine after up to 48 h circulation in vivo.

The same procedure was applied to the plasma obtained from mice that were injected ⁸⁹Zr-labeled trastuzumab-**1.17** and that were euthanized 1 and 4 days post-mAb injection affording an extrapolated in vivo deactivation half-life of 12 days.

### Example 8: In vivo evaluation of mAb-TCO-Label conjugates in normal mice

In a preliminary dose finding experiment, two groups of female Balb/C nude mice were i.v. injected a trastuzumab-**1.2** conjugate labeled with ¹¹¹In (ca. 5 mg/kg, ca. 1 MBq) followed 1h later by Cleaving Agent **2.1** (ca. 8.4 µmol/kg) or vehicle. Blood samples were withdrawn 5 min before and at various times after Cleaving Agent administration and the radioactivity was measured in a gamma-counter. In the mice that received the radiolabeled mAb-TCO conjugate followed by vehicle the radioactivity levels in blood showed a modest ca. 13% decrease in 1h, from 30.3±1.2 to 26.2±1.7 %ID/g. On the contrary, the radioactivity circulating in mice that received the Cleaving Agent showed a rapid ca. 30% decrease from 5 min before (30.5±1.6 %ID/g) to 60 min after **2.7** injection (20.0±1.1 %ID/g). This enhanced radioactivity clearance signifies the reaction between the TCO trigger and the Cleaving Agent in circulation followed by release of the Label, which eliminates rapidly from blood. A higher Cleaving Agent / mAb-conjugate dose ratio was chosen for follow-up experiments to increase Label release. The ¹¹¹In-trastuzumab-**1.8** conjugate (ca. 0.5 mg/kg) was administered in 2 more groups of mice followed by **2.10** (ca. 33.5 µmol/kg) either 1h or 24h post-mAb injection. In both cases, Cleaving Agent administration resulted in ca. 70% reduction in blood radioactivity 24h later (Figure 2) with respect to control mice. Also, lower levels of radioactivity were found in all non-target organs and tissues (Table 3) including kidney, demonstrating that the released Label is not preferentially retained in kidney and eliminates from the body through the urine.

**Table 3: ¹¹¹In biodistribution in mice injected with ¹¹¹In-trastuzumab-1.8 followed by 2.10 (or vehicle) 1h or 24h post-mAb injection and euthanized 24h later. Data presented as % injected dose per gram (or * per organ) with SD (n=4).**

| **organ** | **1h post-mAb** | | **24h post-mAb** | |
|---|---|---|---|---|
| | **Cleaving Agent** | **vehicle** | **Cleaving Agent** | **vehicle** |
| **Blood** | 3.61±0.44 | 12.29±2.08 | 2.91±0.53 | 8.94±1.13 |
| **Heart** | 0.87±0.10 | 5.12±1.15 | 0.93±0.09 | 2.13±0.27 |
| **Lung** | 1.94±0.43 | 6.11±0.82 | 2.13±0.41 | 5.18±0.20 |
| **Liver** | 3.35±0.10 | 6.91±0.84 | 4.68±1.11 | 5.57±1.08 |
| **Spleen** | 1.19±0.16 | 3.09±0.75 | 1.89±0.17 | 2.78±0.18 |
| **Kidney L** | 5.39±0.84 | 8.38±1.52 | 4.75±0.45 | 6.02±0.55 |
| **Muscle** | 0.27±0.02 | 0.74±0.11 | 0.29±0.05 | 0.74±0.06 |
| **Bone** | 0.44±0.03 | 1.22±0.41 | 0.65±0.03 | 1.12±0.13 |
| **Brain** | 0.13±0.08 | 0.35±0.14 | 0.11±0.02 | 0.22±0.05 |
| **Sm. Intestine*** | 0.61±0.04 | 1.76±0.22 | 1.04±0.21 | 1.96±0.24 |
| **Lg. intestine*** | 0.34±0.07 | 1.35±0.22 | 0.56±0.14 | 1.12±0.38 |

### Example 9: In vivo evaluation of trastuzumab-TCO-Label conjugates in tumor-bearing mice

Female Balb/C mice were injected ca 1 million LS174T colon carcinoma cells or 10 million BT-474 breast cancer cells s.c. in the flank. When the tumors became palpable, the mice were injected ¹¹¹In-trastuzumab-1.8 (ca. 0.5 mg/kg, ca 1 MBq; n=3). One group of LS174T-bearing mice and one of BT-474 were then administered Cleaving Agent **2.10** (ca. 33.5 µmol/kg) 48h post-injection and were euthanized 24 h later. Control groups were euthanized 72h post-mAb injection. In control mice, sustained ¹¹¹In-trastuzumab-1.8 circulation in blood was observed 72h post mAb injection (10-15 %ID/g) and high radioactivity uptake was found in BT-474 tumors (43.3±5.6 %ID/g) and in LS174T tumors (16.4±1.0). In mice treated with **2.10** somewhat lower radioactivity uptake was found in tumors, most likely due to the fraction of residual cell-surface bound trastuzumab. However, the Label cleavage in blood resulted in much lower amounts of radioactivity in blood (2-3% ID/g) and non-target tissues, therefore affording a significantly improved tumor-to-organ ratios in both tumor models (Table 4).

**Table 4: Tumor-to-nontarget ratios in LS174T and BT-474 tumor-bearing mice treated with ¹¹¹In-trastuzumab-1.8 followed by Cleaving Agent 2.10 and controls.**

| | **LS174T** | | **BT-474** | |
|---|---|---|---|---|
| | **control** | **Cleaving Agent** | **control** | **Cleaving Agent** |
| **Tumor/Blood** | 2.1 | 3.7 | 2.8 | 12.0 |
| **Tumor/Heart** | 6.9 | 10.1 | 10.8 | 13.0 |
| **Tumor/Muscle** | 23.3 | 27.1 | 37.6 | 88.1 |

**Example 10: Evaluation of ⁸⁹Zr-labeled CC49-1.17 in tumor-bearing** mice Two groups of mice bearing s.c. LS174T colon carcinoma xenografts in the hindlimb were injected ⁸⁹Zr-labeled non-internalizing CC49-**1.17** (ca. 0.5 mg/kg, ca. 0.4 MBq) followed 48h later by non-extravasting Cleaving Agent **2.12** (50 µg/mouse) or vehicle, and were euthanized 6h post **2.12** injection. As expected, in mice that were administered the mAb conjugate followed by vehicle a high ⁸⁹Zr uptake was found in tumor (47.3 ± 5.2 %ID/g) but elevated radioactivity retention was found also in blood (14.9 ± 1.8 %ID/g) and non-target organs. On the contrary, in mice that received **2.12** the ⁸⁹Zr levels were significantly diminished in blood (3.4 ± 1.2 %ID/g) as well as in other non-target organs, while the uptake in tumor was mostly retained (41.7 ±7.7 %ID/g). As a result, significantly improved tumor-to-nontarget ratios can be achieved (Table 5).

**Table 5: Tumor-to-nontarget tissues ratio calculated in mice pre-treated with ⁸⁹Zr-CC49-1.17 followed by Cleaving Agent 2.12 or vehicle 48h later and euthanized 54h post-mAb injection.**

| | **vehicle** | **2.12** |
|---|---|---|
| **Tumor/Blood** | 3.2 | 12.3 |
| **Tumor/Liver** | 4.8 | 12.8 |
| **Tumor/Kidney** | 19.5 | 28.3 |
| **Tumor/Muscle** | 33.4 | 55.6 |

## Claims

1. A compound satisfying Formula (1): 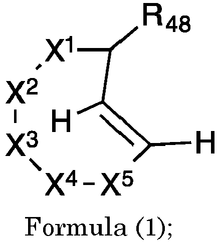
and pharmaceutically acceptable salts thereof, wherein
the compound of Formula (1) comprises at least one Label and at least one Administration Agent;
the Label is a moiety comprising a radionuclide;
the Administration Agent is an antibody, wherein the term "antibody" refers to an antibody, an antibody fragment which is at least a portion of the variable region of the immunoglobulin that binds to its target, or an antibody mimetic;
each X¹, X², X³, X⁴ is independently selected from the group consisting of -C(R₄₇)₂-, -NR₃₇-, -C(O)-, -O-, such that at most two of X¹, X², X³, X⁴ are not -C(R₄₇)₂-, and with the proviso that no sets consisting of adjacent atoms are present selected from the group consisting of -O-O-, -O-N-, -C(O)-O-, N-N-, and -C(O)-C(O)-;
X⁵ is -C(R₄₇)₂- or -CHR₄₈, preferably X⁵ is -C(R₄₇)₂-;
each R₄₈ is independently selected from the group consisting of -L^{B}, and -L^{A}; preferably R₄₈ is -L^{B};
R₄₈ is bound to the remainder of the compound of Formula (1) via a part of R₄₈ that is -O-, -S-, -OC(O)-, -OC(S)-, -SC(O)-, or -SC(S)-;
L^{B} is a moiety satisfying Formula (2): 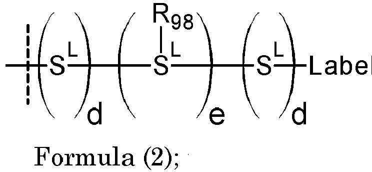 wherein
the dashed line denotes a bond to the remainder of the compound of Formula (1);
S^{L} is a linker, which optionally is a self-immolative linker L^{C};
each R₉₈ individually is the Label or a clearance-directing group, wherein a clearance-directing group is a moiety that directs a compound to an excretory organ;
each d independently is 0 or 1;
e is an integer in a range of from 0 to 4, preferably e is 0;
L^{A} is a moiety satisfying Formula (3): 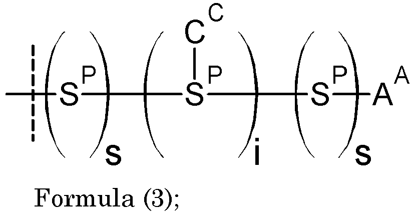 wherein
the dashed line denotes a bond to the remainder of the compound of Formula (1); each s is independently 0 or 1; preferably each s is 0;
i is an integer in a range of from 0 to 4, preferably 0 or 1, most preferably 0; each S^{P} independently is a spacer, which optionally is a self-immolative linker L^{C}; A^{A} denotes the Administration Agent;
C^{C} denotes a Construct-C, wherein each Construct-C is independently selected from the group consisting of the Label and the Administration Agent; preferably the compound of Formula (1) comprises at most one C^{C};
provided that L^{A} only comprises both the Label and the Administration Agent when L^{A} is R₄₈;
provided that if L^{A} being R₄₈ comprises both the Label and the Administration Agent, then the S^{p} linked to said Label and said Administration Agent is a self-immolative linker, wherein said Label and said Administration Agent will be uncoupled after reaction of the compound of Formula (1) with a Cleaving Agent due to the self-immolative character of the linker, wherein the Cleaving Agent is a diene;
each R₄₇ is independently selected from the group consisting of hydrogen, -L^{B}, -L^{A}, -(S^{p})ᵢ-C^{C}, -F, -Cl, -Br, -I, -OR₃₇, -N(R₃₇)₂, -SOs, -PO₃-, -NO₂, - CF₃, -SR₃₇, S(=O)₂N(R₃₇)₂, OC(=O)R₃₇, SC(=O) R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, -S(O)₂R₃₇, NR₃₇S(O)₂R₃₇, -ON(R₃₇)₂, ₋NR₃₇OR₃₇, C₁-C₂₄ alkyl groups, C₂-C₂₄ alkenyl groups, C₂-C₂₄ alkynyl groups, C₆-C₂₄ aryl groups, C₂-C₂₄ heteroaryl groups, C₃-C₂₄ cycloalkyl groups, C₅-C₂₄ cycloalkenyl groups, C₁₂-C₂₄ cycloalkynyl groups, C₃-C₂₄ (cyclo)alkyl(hetero)aryl groups, C₃-C₂₄ (hetero)aryl(cyclo)alkyl, C₄-C₂₄ (cyclo)alkenyl(hetero)aryl groups, C₄-C₂₄ (hetero)aryl(cyclo)alkenyl groups, C₄-C₂₄ (cyclo)alkynyl(hetero)aryl groups, C₄-C₂₄ (hetero)aryl(cyclo)alkynyl groups, C₄-C₂₄ alkylcycloalkyl groups, and C₄-C₂₄ cycloalkylalkyl groups; wherein i is an integer in a range of from 0 to 4, preferably i is an integer ranging from 0 to 1,
wherein the alkyl groups, alkenyl groups, alkynyl groups, aryl, heteroaryl, cycloalkyl groups, cycloalkenyl groups, cycloalkynyl groups, (cyclo)alkyl(hetero)aryl groups, (hetero)aryl(cyclo)alkyl groups, (cyclo)alkenyl(hetero)aryl groups, (hetero)aryl(cyclo)alkenyl groups, (cyclo)alkynyl(hetero)aryl groups, (hetero)aryl(cyclo)alkynyl groups, alkylcycloalkyl groups, cycloalkylalkyl groups are optionally substituted with a moiety selected from the group consisting of -Cl, -F, -Br, -I, -OR₃₇, -N(R₃₇)₂, - SO₃R₃₇, -PO₃(R₃₇)₂, -PO₄(R₃₇)₂, -NO₂, -CF₃, =O, =NR₃₇, and -SR₃₇, and optionally contain one or more heteroatoms selected from the group consisting of O, S, NR₃₇, P, and Si, wherein the N, S, and P atoms are optionally oxidized, wherein the N atoms are optionally quaternized;
two R₄₇ and/or R₃₇ are optionally comprised in a ring,
two R₄₇ and/or R₃₇ are optionally comprised in a ring so as to form a ring fused to the eight membered trans-ring of Formula (1);
each R₃₇ is independently selected from the group consisting of hydrogen, -L^{B}, - L^{A}, -(S^{P})ᵢ-C^{C}, C₁-C₂₄ alkyl groups, C₂-C₂₄ alkenyl groups, C₂-C₂₄ alkynyl groups, C₆-C₂₄ aryl groups, C₂-C₂₄ heteroaryl groups, C₃-C₂₄ cycloalkyl groups, C₅-C₂₄ cycloalkenyl groups, C₁₂-C₂₄ cycloalkynyl groups, C₃-C₂₄ (cyclo)alkyl(hetero)aryl groups, C₃-C₂₄ (hetero)aryl(cyclo)alkyl, C₄-C₂₄ (cyclo)alkenyl(hetero)aryl groups, C₄-C₂₄ (hetero)aryl(cyclo)alkenyl groups, C₄-C₂₄ (cyclo)alkynyl(hetero)aryl groups, C₄-C₂₄ (hetero)aryl(cyclo)alkynyl groups, C₄-C₂₄ alkylcycloalkyl groups, and C₄-C₂₄ cycloalkylalkyl groups; wherein i is an integer in a range of from 0 to 4, preferably i is 1;
the R₃₇ groups not being hydrogen are optionally substituted with a moiety selected from the group consisting of -Cl, -F, -Br, -I, -OH, -NH₂, -SO₃H, -PO₃H - PO₄H₂, -NO₂, -CF₃, =O, =NH, and -SH, and optionally contain one or more heteroatoms selected from the group consisting of O, S, NH, P, and Si, wherein the N, S, and P atoms are optionally oxidized, wherein the N atoms are optionally quaternized.

2. The compound according to claim 1, wherein the radionuclide is selected from the group consisting of ³H, ¹¹C, ¹³N, ¹⁵O, ¹⁸F, ¹⁹F, ⁵¹Cr, ⁵²Fe, ⁵²Mn, ⁵⁵Co, ⁶⁰Cu, ⁶¹Cu, ⁶²Zn, ⁶²Cu, ⁶³Zn, ⁶⁴Cu, ⁶⁶Ga, ⁶⁷Ga, ⁶⁸Ga, ⁷⁰As, ⁷¹As, ⁷²As, ⁷⁴As, ⁷⁵Se, ⁷⁵Br, ⁷⁶Br, ⁷⁷Br, ^{8O}Br, ⁸²Br, ⁸²Rb, ⁸⁶Y, ⁸⁸Y, ⁹⁰Y, ⁸⁹Sr, ⁸⁹Zr, ⁹⁷Ru, ^{99m}Tc, ¹¹⁰In, ¹¹¹In, ¹¹³In, ¹¹⁴In, ¹¹⁷Sn, ¹²⁰I, ¹²²Xe, ¹²³I, ¹²⁴I, ¹²⁵I, ¹⁶⁶Ho, ¹⁶⁷Tm, ¹⁶⁹Yb, ¹⁹³Pt, ¹⁹⁵Pt, ²⁰¹Tl, ²⁰³Pb, ²⁴Na, ³²P, ³³P, ⁴⁷Sc, ⁵⁹Fe, ⁶⁷Cu, ⁷⁶As, ⁷⁷As, ⁸⁹Br, ⁸²Br, ⁸⁹Sr, ⁹⁰Nb, ⁹⁰Y, ¹⁰³Ru, ¹⁰⁵Rh, ¹⁰⁹Pd, ¹¹¹Ag, ¹¹¹In ¹²¹Sn, ¹²⁷Te, ¹³¹I, ¹⁴⁰La, ¹⁴¹Ce, ¹⁴²Pr, ¹⁴³Pr, ¹⁴⁴Pr, ¹⁴⁹Pm, ¹⁴⁹Tb, ¹⁵¹Pm, ¹³³Sm, ¹⁵⁹Gd, ¹⁶¹Tb, ¹⁶⁵Dy, ¹⁶⁶Dy, ¹⁶⁶Ho, ¹⁶⁹Er, ¹⁷²Tm, ¹⁷⁵Yb, ¹⁷⁷Lu, ¹⁸⁶Re, ¹⁸⁸Re, ¹⁹⁸Au, ¹⁹⁹Au, ²¹¹At, ²¹¹Bi, ²¹²Bi, ²¹²Pb, ²¹³Bi, ²¹⁴Bi, ²²³Ra, ²²⁴Ra, ²²⁵Ac and ²²⁷Th.

3. The compound according to any one of the preceding claims, wherein the Label is a chelating moiety that chelates the radionuclide.

4. The compound according to any one of the preceding claims, wherein X¹, X², X³, and X⁴ are -C(R₄₇)₂-, and preferably at most four R₄₇, more preferably at most two R₄₇, are not H.

5. The compound according to any one of the preceding claims, which comprises at most one Label and at most one Administration Agent.

6. A combination comprising the compound according to any one of the preceding claims, and a Cleaving Agent, with the proviso that when at least one R₄₈ in Formula (1) comprises a Label, then the Cleaving Agent does not comprise the same Label as R₄₈; with the proviso that when at least one R₄₈ in Formula (1) comprises an Administration Agent, then the Cleaving Agent does not comprise the same Administration Agent as R₄₈;
wherein the Cleaving Agent is a diene.

7. The combination according to claim 6, wherein the diene is a tetrazine, preferably a tetrazine satisfying Formula (4) and preferably including pharmaceutically accepted salts thereof: 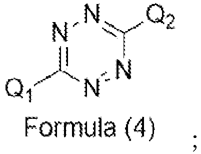 wherein
each moiety Q₁ and Q₂ is independently selected from the group consisting of hydrogen, -F, -Cl, -Br, -I, -OR₃₇, -N(R₃₇)₂, -SO₃, -PO₃⁻, -NO₂, -CF₃, -SR₃₇, S(=O)₂N(R₃₇)₂, OC(=O)R₃₇, SC(=O)R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, - S(O)₂R₃₇, NR₃₇S(O)₂R₃₇, -ON(R₃₇)₂, .NR₃₇OR₃₇, alkyl groups, alkenyl groups, alkynyl groups, aryl groups, heteroaryl groups, cycloalkyl groups, cycloalkenyl groups, cycloalkynyl groups, (cyclo)alkyl(hetero)aryl groups, (hetero)aryl(cyclo)alkyl, (cyclo)alkenyl(hetero)aryl groups, (hetero)aryl(cyclo)alkenyl groups, (cyclo)alkynyl(hetero)aryl groups, (hetero)aryl(cyclo)alkynyl groups, alkylcycloalkyl groups, and cycloalkylalkyl groups;
wherein the Q₁ and Q₂ groups not being H, -F, -Cl, -Br, -I, -OH, -NH₂, -SO₃, -PO₃⁻, -NO₂, -CF₃, are optionally substituted, preferably with a moiety selected from the group consisting of -Cl, -F, -Br, -I, -OR₃₇, -N(R₃₇)₂, -SO₃R₃₇, -PO₃(R₃₇)₂, -PO₄(R₃₇)₂, - NO₂, -CF₃, =O, =NR₃₇, and -SR₃₇, and optionally contain one or more heteroatoms selected from the group consisting of O, S, NR₃₇, P, and Si, wherein the N, S, and P atoms are optionally oxidized, wherein the N atoms are optionally quaternized, wherein the Q₁ and Q₂ groups are optionally bound to a polymer, a particle, a peptide, a peptoid, a dendrimer, a protein, an aptamer, a carbohydrate, an oligonucleotide, an oligosaccharide, a lipid, a steroid, a liposome, a Targeting Agent T^{T}, -(S^{P})_{D}-R⁸⁷, an albumin-binding moiety, and a chelating moiety;
wherein D is 0 or 1;
S^{P} is a spacer;
each R⁸⁷ is independently selected from the group consisting of organic molecules, inorganic molecules, organometallic molecules, resins, beads, glass, microparticles, nanoparticles, gels, surfaces, and cells;
and at least one of moieties Q₁ and Q₂ is not hydrogen.

8. The combination according to claim 7, wherein Q₁ and Q₂ are selected from the group of hydrogen, C₁-C₈ alkyl, phenyl, 2-pyridyl, 3-pyridyl, 4-pyridyl, 2,6-pyrimidyl, 2,5-pyrimidyl, 3,5-pyrimidyl, and 2,4-pyrimidyl; and Q₁ and Q₂ not being hydrogen are optionally substituted as defined in claim 7.

9. The combination according to any one of claims 7 to 8, wherein in Formula (4):
(a) Q₁ and Q₂ are selected from the group consisting of 2-pyridyl, 3-pyridyl, and 4-pyridyl;
(b) Q₁ is selected from the group consisting of 2,6-pyrimidyl, 2,5-pyrimidyl, 3,5-pyrmidyl, and 2,4-pyrimidyl; and Q₂ is (hetero)alkyl; or
(c) Q₁ is phenyl and Q₂ is hydrogen;
and in (a)-(c) all Q₁ and Q₂ not being hydrogen are optionally substituted as defined in claim 7.

10. The compound according to any one of claims 1 to 5, or the combination according to any one of claims 6 to 9 for use as a medicament.

11. The compound according to any one of claims 1 to 5, or the combination according to any one of claims 6 to 9, for use in the treatment of a disease, preferably cancer, in a subject, preferably a human, wherein the treatment is radiotherapy.

12. The compound according to any one of claims 1 to 5, or the combination according to any one of claims 6 to 9, for use in a diagnostic method comprising the steps of
(a) administering a compound according to Formula (1) as defined in any one of claims 1 to 5, to a subject, preferably a human;
(b) administering a Cleaving Agent as defined in any one of claims 6 to 9, to said subject;
(c) imaging the compound according to Formula (1) present in the subject to collect data;
(d) comparing said data to standard values;
(e) finding a significant deviation from said standard values during comparison;
(f) attributing the significant deviation to a particular clinical picture, preferably to cancer.

13. A non-therapeutic method for imaging a compound according to any one of claims 1 to 5 in a subject, preferably a human, wherein the following steps have been performed on the subject:
(a) administering a compound according to Formula (1) as defined in any one of claims 1 to 5, to the subject;
(b) administering a Cleaving Agent as defined in any one of claims 6 to 9, to said subject;
wherein the non-therapeutic method comprises the step of:
(c) imaging the compound according to Formula (1) present in the subject.

14. Non-therapeutic use of a compound according to any one of claims 1 to 5, or a combination according to any one of claims 6 to 9, for imaging in a subject, preferably a human.

## Patentansprüche

1. Verbindung, entsprechend Formel (1): 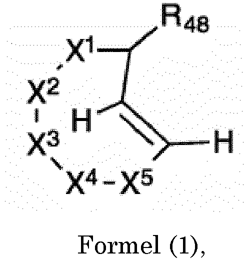
und pharmazeutisch akzeptable Salze davon, wobei
die Verbindung der Formel (1) mindestens einen Marker und mindestens ein Verabreichungsmittel umfasst;
der Marker ein Anteil ist, umfassend ein Radionuklid;
das Verabreichungsmittel ein Antikörper ist, wobei der Begriff "Antikörper" sich auf einen Antikörper, ein Antikörperfragment, das mindestens ein Abschnitt der variablen Region des Immunoglobulins ist, der an sein Ziel bindet, oder ein Antikörpermimetikum bezieht;
jedes X¹, X², X³, X⁴ unabhängig ausgewählt ist aus der Gruppe bestehend aus -C(R₄₇)₂-, -NR₃₇-, -C(O)-, -O-, so dass höchstens zwei von X¹, X², X³, X⁴ nicht -C(R₄₇)₂- sind, und mit der Maßgabe, dass keine Sätze, die aus benachbarten Atomen bestehen, vorliegen, ausgewählt aus der Gruppe bestehend aus -O-O-, -O-N-, -C(O)-O-, N-N-, und -C(O)-C(O)-;
X⁵-C(R₄₇)₂- oder -CHR₄₈ ist, vorzugsweise X⁵-C(R₄₇)₂- ist;
jedes R₄₈ unabhängig ausgewählt ist aus der Gruppe bestehend aus - L^{B}, und -L^{A}; vorzugsweise R₄₈ -L^{B} ist;
R₄₈ an den Rest der Verbindung von Formel (1) mittels eines Teils von R₄₈, der -O-, -S-, -OC(O)-, -OC(S)-, -SC(O)-, oder -SC(S)- ist, gebunden ist;
L^{B} ein Anteil ist, entsprechend Formel (2): 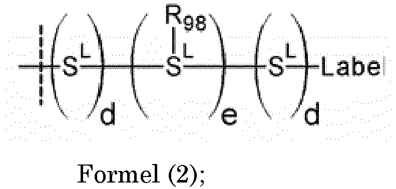 wobei
die Strichlinie eine Bindung zu dem Rest der Verbindung der Formel (1) bezeichnet;
S^{L} ein Linker ist, der optional ein selbstimmolativer Linker L^{C} ist; jedes R₉₈ individuell der Marker oder eine zur Clearance lenkende Gruppe ist, wobei eine zur Clearance lenkende Gruppe ein Anteil ist, der eine Verbindung zu einem Ausscheidungsorgan lenkt; jedes d unabhängig 0 oder 1 ist;
e eine ganze Zahl in einem Bereich von von 0 bis 4 ist, vorzugsweise e 0 ist;
L^{A} ein Anteil ist, entsprechend Formel (3): 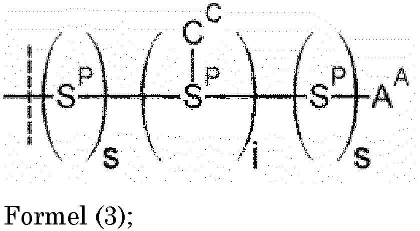 wobei
die Strichlinie eine Bindung zu dem Rest der Verbindung der Formel (1) bezeichnet;
jedes s unabhängig 0 oder 1 ist; vorzugsweise jedes s 0 ist;
i eine ganze Zahl in einem Bereich von von 0 bis 4, vorzugsweise 0 oder 1, am meisten bevorzugt 0 ist;
jedes S^{P} unabhängig ein Abstandshalter ist, der optional ein selbstimmolativer Linker L^{C} ist;
A^{A} das Verabreichungsmittel bezeichnet;
C^{C} ein Konstrukt-C bezeichnet, wobei jedes Konstrukt-C unabhängig ausgewählt ist aus der Gruppe bestehend aus dem Marker und dem Verabreichungsmittel; vorzugsweise die Verbindung der Formel (1) höchstens ein C^{C} umfasst;
vorausgesetzt, dass L^{A} nur sowohl den Marker als auch das Verabreichungsmittel umfasst, wenn L^{A} R₄₈ ist;
vorausgesetzt, dass, wenn L^{A} R₄₈ ist und sowohl den Marker als auch das Verabreichungsmittel umfasst, das S^{P}, das mit dem Marker und dem Verabreichungsmittel verknüpft ist, ein selbstimmolativer Linker ist, wobei der Marker und das Verabreichungsmittel nach einer Reaktion der Verbindung von Formel (1) mit einem Spaltmittel aufgrund der selbstimmolativen Beschaffenheit des Linkers abgekoppelt werden können, wobei das Spaltmittel ein Dien ist;
jedes R₄₇ unabhängig ausgewählt ist aus der Gruppe bestehend aus
Wasserstoff, -L^{B}, -L^{A}, -(S^{P})ᵢ-C^{C}, -F, -Cl, -Br, -I, -OR₃₇, -N(R₃₇)₂, -SO₃, - PO₃-, -NO₂, -CF₃, -SR₃₇, S(=O)₂N(R₃₇)₂, OC(=O)R₃₇, SC(=O)R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, -C(=S)N(R₃₇)₂, C(=O)O-R₃₇, C(=O)S-R₃₇, C(=S)O-R₃₇, C(=S)S-R₃₇, S(O)R₃₇, NR₃₇S(O)₂R₃₇, -ON(R₃₇)₂, NR₃₇OR₃₇, C₁-C₂₄-Alkylgruppen, C₂-C₂₄-Alkenylgruppen, C₂-C₂₄-Alkinylgruppen, C₆-C₂₄-Arylgruppen, C₂-C₂₄-Heteroarylgruppen, C₃-C₂₄-Cycloalkylgruppen, C₅-C₂₄-Cycloalkenylgruppen, C₁₂-C₂₄-Cycloalkinylgruppen, C₃-C₂₄-(Cyclo)alkyl(hetero)arylgruppen, C₃-C₂₄-(Hetero)aryl(cyclo)alkyl, C₄-C₂₄-(Cyclo)alkenyl(hetero)arylgruppen, C₄-C₂₄-(Hetero)aryl(cyclo)alkenylgruppen, C₄-C₂₄-(Cyclo)alkinyl(hetero)arylgruppen, C₄-C₂₄-(Hetero)aryl(cyclo)alkinylgruppen, C₄-C₂₄-Alkylcycloalkylgruppen, und C₄-C₂₄-Cycloalkylalkylgruppen; wobei i eine ganze Zahl in einem Bereich von 0 bis 4 ist, vorzugsweise wobei i eine ganze Zahl ist im Bereich von 0 bis 1,
wobei die Alkylgruppen, Alkenylgruppen, Alkinylgruppen, Aryl-, Heteroaryl-, Cycloalkylgruppen, Cycloalkenylgruppen, Cycloalkinylgruppen, (Cyclo)alkyl(hetero)arylgruppen, (Hetero)aryl(cyclo)alkylgruppen, (Cyclo)alkenyl(hetero)arylgruppen, (Hetero)aryl(cyclo)alkenylgruppen, (Cyclo)alkinyl(hetero)arylgruppen, (Hetero)aryl(cyclo)alkinylgruppen, Alkylcycloalkylgruppen, Cycloalkylalkylgruppen optional mit einem Anteil substituiert sind, ausgewählt aus der Gruppe bestehend aus -Cl, -F, -Br, -I, - OR₃₇, -N(R₃₇)₂, -SO₃R₃₇, -PO₃(R₃₇)₂, -PO₄(R₃₇)₂, -NO₂, -CF₃, =O, =NR₃₇, und - SR₃₇, und optional ein oder mehrere Heteroatome enthalten, ausgewählt aus der Gruppe bestehend aus O, S, NR₃₇, P, und Si, wobei die N-, S-, und P-Atome optional oxidiert sind,
wobei die N-Atome optional quaternisiert sind;
zwei R₄₇ und/oder R₃₇ optional in einem Ring umfasst sind,
zwei R₄₇ und/oder R₃₇ optional in einem Ring umfasst sind, um einen Ring zu bilden, der an den achtgliedrigen trans-Ring der Formel (1) anelliert ist;
jedes R₃₇ unabhängig ausgewählt ist aus der Gruppe bestehend aus Wasserstoff, -L^{B}, -L^{A}, -(S^{P})ᵢ-C^{C}, C₁-C₂₄-Alkylgruppen, C₂-C₂₄-Alkenylgruppen, C₂-C₂₄-Alkinylgruppen, C₆-C₂₄-Arylgruppen, C₂-C₂₄-Heteroarylgruppen, C₃-C₂₄-Cycloalkylgruppen, C₅-C₂₄-Cycloalkenylgruppen, C₁₂-C₂₄-Cycloalkinylgruppen, C₃-C₂₄-(Cyclo)alkyl(hetero)arylgruppen, C₃-C₂₄-(Hetero)aryl(cyclo)alkyl, C₄-C₂₄-(Cyclo)alkenyl(hetero)arylgruppen, C₄-C₂₄-(Hetero)aryl(cyclo)alkenylgruppen, C₄-C₂₄-(Cyclo)alkinyl(hetero)arylgruppen, C₄-C₂₄-(Hetero)aryl(cyclo)alkinylgruppen, C₄-C₂₄-Alkylcycloalkylgruppen und C₄-C₂₄-Cycloalkylalkylgruppen; wobei i eine ganze Zahl in einem Bereich von von 0 bis 4 ist, vorzugsweise i 1 ist;
die R₃₇-Gruppen, die nicht Wasserstoff sind, optional mit einem Anteil substituiert sind, ausgewählt aus der Gruppe bestehend aus -Cl, -F, - Br, -I, -OH, -NH₂, -SO₃H, -PO₃H, -PO₄H₂, -NO₂, -CF₃, =O, =NH, und -SH, und optional ein oder mehrere Heteroatome enthalten, ausgewählt aus der Gruppe bestehend aus O, S, NH, P und Si, wobei die N-, S- und P-Atome optional oxidiert sind, wobei die N-Atome optional quaternisiert sind.

2. Verbindung nach Anspruch 1, wobei das Radionuklid ausgewählt ist aus der Gruppe bestehend aus ³H, ¹¹C, ¹³N, ¹⁵O, ¹⁸F, ¹⁹F, ⁵¹Cr, ⁵²Fe, ⁵²Mn, ⁵⁵Co, ⁶⁰Cu, ⁶¹Cu, ⁶²Zn, ⁶²Cu, ⁶³Zn, ⁶⁴Cu, ⁶⁶Ga, ⁶⁷Ga, ⁶⁸Ga, ⁷⁹As, ⁷¹As, ⁷²As, ⁷⁴As, ⁷⁵Se, ⁷⁵Br, ⁷⁶Br, ⁷⁷Br, ⁸⁰Br, ⁸²Br, ⁸²Rb, ⁸⁶Y, ⁸⁸Y, ⁹⁰Y, ⁸⁹Sr, ⁸⁹Zr, ⁹⁷Ru, ^{99m}Tc, ¹¹⁰In, ¹¹¹In, ¹¹³In, ¹¹⁴In, ¹¹⁷Sn, ¹²⁰I, ¹²²Xe, ¹²³I, ¹²⁴I, ¹²⁵I, ¹⁶⁶Ho, ¹⁶⁷Tm, ¹⁶⁹Yb, ¹⁹³Pt, ¹⁹⁵Pt, ²⁰¹Tl, ²⁹³Pb, ²⁴Na, ³²P, ³³P, ⁴⁷Sc, ⁵⁹Fe, ⁶⁷Cu, ⁷⁶As, ⁷⁷As, ⁸⁰Br, ⁸²Br, ⁸⁹Sr, ⁹⁰Nb, ⁹⁰Y, ¹⁰³Ru, ¹⁰⁵Rh, ¹⁰⁹Pd, ¹¹¹Ag, ¹¹¹In, ¹²¹Sn, ¹²⁷Te, ¹³¹I, ¹⁴⁹La, ¹⁴¹Ce, ¹⁴²Pr, ¹⁴³Pr, ¹⁴⁴Pr, ¹⁴⁹Pm, ¹⁴⁹Tb, ¹⁵¹Pm, ¹⁵³Sm, ¹⁵⁹Gd, ¹⁶¹Tb, ¹⁶⁵Dy, ¹⁶⁶Dy, ¹⁶⁶Ho, ¹⁶⁹Er, ¹⁷²Tm, ¹⁷⁵Yb, ¹⁷⁷Lu, ¹⁸⁶Re, ¹⁸⁸Re, ¹⁹⁸Au, ¹⁹⁹Au, ²¹¹At, ²¹¹Bi, ²¹²Bi, ²¹²Pb, ²¹³Bi, ²¹⁴Bi, ²²³Ra, ²²⁴Ra, ²²⁵Ac und ²²⁷Th.

3. Verbindung nach einem der vorhergehenden Ansprüche, wobei der Marker ein chelatbildender Anteil ist, der das Radionuklid chelatiert.

4. Verbindung nach einem der vorhergehenden Ansprüche, wobei X¹, X², X³, und X⁴ -C(R₄₇)₂- sind, und vorzugsweise höchstens vier R₄₇, mehr bevorzugt höchstens zwei R4₇ nicht H sind.

5. Verbindung nach einem der vorhergehenden Ansprüche, die höchstens einen Marker und höchstens ein Verabreichungsmittel umfasst.

6. Kombination, umfassend die Verbindung nach einem der vorhergehenden Ansprüche, und ein Spaltmittel, mit der Maßgabe, dass, wenn mindestens ein R₄₈ in Formel (1) einen Marker umfasst, das Spaltmittel nicht denselben Marker wie R₄₈ umfasst; mit der Maßgabe, dass, wenn mindestens ein R₄₈ in Formel (1) ein Verabreichungsmittel umfasst, das Spaltmittel nicht dasselbe Verabreichungsmittel wie R₄₈ umfasst;
wobei das Spaltmittel ein Dien ist.

7. Kombination nach Anspruch 6, wobei das Dien ein Tetrazin, vorzugsweise ein Tetrazin, entsprechend Formel (4), ist, und vorzugsweise einschließend pharmazeutisch akzeptierte Salze davon: 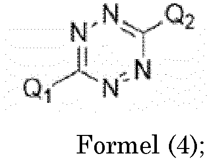 wobei
jeder Anteil Q₁ und Q₂ unabhängig ausgewählt ist aus der Gruppe bestehend aus Wasserstoff, -F, -Cl, -Br, -I, -OR₃₇, -N(R₃₇)₂, -SO₃, -PO₃-, -NO₂, -CF₃, -SR₃₇, S(=O)₂N(R₃₇)₂, OC(=O)R₃₇, SC(=O)R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, -S(O)₂R₃₇, NR₃₇S(O)₂R₃₇, -ON(R₃₇)₂, NR₃₇OR₃₇, Alkylgruppen, Alkenylgruppen, Alkinylgruppen, Arylgruppen, Heteroarylgruppen, Cycloalkylgruppen, Cycloalkenylgruppen, Cycloalkinylgruppen, (Cyclo)alkyl(hetero)arylgruppen, (Hetero)aryl(cyclo)alkyl-, (Cyclo)alkenyl(hetero)arylgruppen, (Hetero)aryl(cyclo)alkenylgruppen, (Cyclo)alkinyl(hetero)arylgruppen, (Hetero)aryl(cyclo)alkinylgruppen, Alkylcycloalkylgruppen, und Cycloalkylalkylgruppen;
wobei die Q₁- und Q₂-Gruppen, die nicht H, -F, -Cl, -Br, -I, -OH, -NH₂, -SO₃, -PO₃-, -NO₂, -CF₃ sind, optional substituiert sind, vorzugsweise mit einem Anteil, ausgewählt aus der Gruppe bestehend aus -Cl, -F, -Br, -I, - OR₃₇, -N(R₃₇)₂, -SO₃R₃₇, -PO₃(R₃₇)₂, -PO₄(R₃₇)₂, -NO₂, -CF₃, =O, =NR₃₇, und - SR₃₇, und optional ein oder mehrere Heteroatome enthalten, ausgewählt aus der Gruppe bestehend aus O, S, NR₃₇, P, und Si, wobei die N-, S- und P-Atome optional oxidiert sind, wobei die N-Atome optional quaternisiert sind, wobei die Q₁- und Q₂-Gruppen optional an ein Polymer, ein Partikel, ein Peptid, ein Peptoid, ein Dendrimer, ein Protein, ein Aptamer, ein Kohlenhydrat, ein Oligonukleotid, ein Oligosaccharid, ein Lipid, ein Steroid, ein Liposom, ein Targetierungsmittel T^{T}, -(S^{P})_{D}-R⁸⁷, einen albuminbindenden Anteil und einen chelatbildenden Anteil gebunden sind;
wobei D 0 oder 1 ist;
S^{P} ein Abstandshalter ist;
jedes R₈₇ unabhängig ausgewählt ist aus der Gruppe bestehend aus organischen Molekülen, anorganischen Molekülen, organometallischen Molekülen, Harzen, Beads, Glas, Mikropartikeln, Nanopartikeln, Gelen, Oberflächen, und Zellen;
und mindestens einer der Anteile Q₁ und Q₂ nicht Wasserstoff ist.

8. Kombination nach Anspruch 7, wobei Q₁ und Q₂ ausgewählt sind aus der Gruppe von Wasserstoff, C₁-C₈-Alkyl, Phenyl, 2-Pyridyl, 3-Pyridyl, 4-Pyridyl, 2,6-Pyrimidyl, 2,5-Pyrimidyl, 3,5-Pyrimidyl, und 2,4-Pyrimidyl und Q₁ und Q₂, die nicht Wasserstoff sind, optional wie in Anspruch 7 definiert substituiert sind.

9. Kombination nach einem der Ansprüche 7 bis 8, wobei in Formel (4):
(a) Q₁ und Q₂ ausgewählt sind aus der Gruppe bestehend aus 2-Pyridyl, 3-Pyridyl, und 4-Pyridyl;
(b) Q₁ ausgewählt sind aus der Gruppe bestehend aus 2,6-Pyrimidyl, 2,5-Pyrimidyl, 3,5-Pyrimidyl, und 2,4-Pyrimidyl; und Q₂ (Hetero)alkyl ist; oder
(c) Q₁ Phenyl ist und Q₂ Wasserstoff ist;
und in (a)-(c) alle Q₁ und Q₂, die nicht Wasserstoff sind, optional wie in Anspruch 7 definiert substituiert sind.

10. Verbindung nach einem der Ansprüche 1 bis 5, oder die Kombination nach einem der Ansprüche 6 bis 9 zur Verwendung als ein Arzneimittel.

11. Verbindung nach einem der Ansprüche 1 bis 5, oder die Kombination nach einem der Ansprüche 6 bis 9 zur Verwendung bei der Behandlung einer Krankheit, vorzugsweise Krebs, in einem Subjekt, vorzugsweise einem Menschen, wobei die Behandlung Radiotherapie ist.

12. Verbindung nach einem der Ansprüche 1 bis 5, oder die Kombination nach einem der Ansprüche 6 bis 9 zur Verwendung in einem Diagnoseverfahren, umfassend die Schritte von:
(a) Verabreichen einer Verbindung gemäß der Formel (1) wie in einem der Ansprüche 1 bis 5 definiert an ein Subjekt, vorzugsweise einen Menschen;
(b) Verabreichen eines Spaltmittels wie in einem der Ansprüche 6 bis 9 definiert an das Subjekt;
(c) Abbilden der Verbindung gemäß der Formel (1), die in dem Probanden vorliegt, um Daten zu sammeln;
(d) Vergleichen der Daten mit Standardwerten;
(e) Finden einer signifikanten Abweichung von den Standardwerten während des Vergleichs;
(f) Zuschreiben der signifikanten Abweichung einem bestimmten klinischen Bild, vorzugsweise Krebs.

13. Nichttherapeutisches Verfahren zum Abbilden einer Verbindung nach einem der Ansprüche 1 bis 5 in einem Subjekt, vorzugsweise einem Menschen, wobei die folgenden Schritte an dem Probanden durchgeführt werden:
(a) Verabreichen einer Verbindung gemäß der Formel (1) wie in einem der Ansprüche 1 bis 5 definiert an das Subjekt;
(b) Verabreichen eines Spaltmittels wie in einem der Ansprüche 6 bis 9 definiert an das Subjekt;
wobei das nichttherapeutische Verfahren den Schritt umfasst von:
(c) Abbilden der Verbindung gemäß der Formel (1), die in dem Subjekt vorliegt.

14. Nichttherapeutische Verwendung einer Verbindung nach einem der Ansprüche 1 bis 5 oder einer Kombination nach einem der Ansprüche 6 bis 9 zum Abbilden in einem Subjekt, vorzugsweise einem Menschen.

## Revendications

1. Composé répondant à la formule (I) : 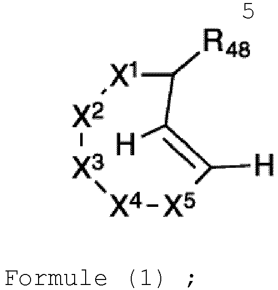
et sels pharmaceutiquement acceptables de celui-ci, où
le composé de formule (1) comprend au moins un marqueur et au moins un agent d'administration ;
le marqueur est une fraction comprenant un radionucléide ;
l'agent d'administration est un anticorps, où le terme « anticorps » désigne un anticorps, un fragment d'anticorps qui est au moins une partie de la région variable de l'immunoglobuline qui se lie à sa cible, ou un mimétique d'anticorps ;
chaque X¹, X², X³, X⁴ est choisi indépendamment dans le groupe consistant en -C(R₄₇)₂-, -NR₃₇-, -C(O)-, -O-, de telle sorte qu'au maximum deux parmi X¹, X², X³, X⁴ ne sont pas -C(R₄₇)₂-, et à condition qu'aucun ensemble se composant d'atomes adjacents ne soit présent choisi dans le groupe consistant en -O-O-, -O-N-, -C(O)-O-, N-N-, et -C(O)-C(O)- ;
X⁵ est -C(R₄₇)₂- ou -CHR₄₈, de préférence X⁵ est - C(R₄₇)₂- ;
chaque R₄₈ est choisi indépendamment dans le groupe consistant en -L^{B}, et -L^{A} ; de préférence R₄₈ est -L^{B} ;
R₄₈ est lié au restant du composé de formule (I) par le biais d'une partie de R₄₈ qui est -O-, -S-, -OC(O)-, -OC(S)-, -SC(O)- ou -SC(S)- ;
L^{B} est une fraction répondant à la formule (2) : 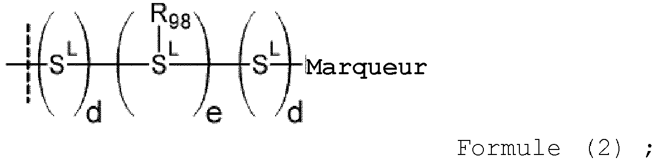 dans laquelle
la ligne en pointillés désigne une liaison au restant du composé de formule (1) ;
S^{L} est un lieur qui est éventuellement un lieur auto-immolable L^{C} ;
chaque R₉₈ individuellement est le marqueur ou un groupe dirigeant la clairance, où un groupe dirigeant la clairance est une fraction qui dirige un composé vers un organe excrétoire ;
chaque d indépendamment est 0 ou 1 ;
e est un entier dans une plage allant de 0 à 4, de préférence e est 0 ;
L^{A} est une fraction répondant à la formule (3) : 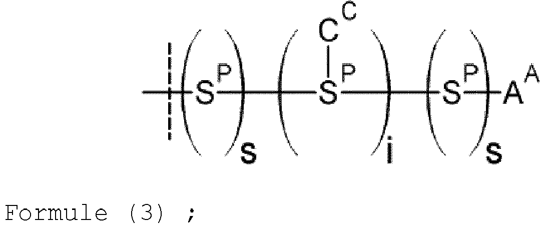 dans laquelle
la ligne en pointillés désigne une liaison au restant du composé de formule (1) ;
chaque s est indépendamment 0 ou 1 ; de préférence chaque s est 0 ;
i est un entier dans une plage allant de 0 à 4, de préférence 0 ou 1, de manière la mieux préférée 0 ;
chaque S^{P} indépendamment est un écarteur, qui est éventuellement un lieur auto-immolable L^{C} ;
A^{A} désigne l'agent d'administration ;
C^{C} désigne une construction-C, où chaque construction-C est choisie indépendamment dans le groupe consistant en le marqueur et l'agent d'administration ; de préférence le composé de formule (1) comprend au maximum un C^{C} ;
à condition que L^{A} seulement comprenne à la fois le marqueur et l'agent d'administration lorsque L^{A} est R₄₈ ;
à condition que si L^{A} qui est R₄₈ comprend à la fois le marqueur et l'agent d'administration, alors S^{P} lié audit marqueur et audit agent d'administration est un lieur auto-immolable, où ledit marqueur et ledit agent d'administration seront découplés après la réaction du composé de formule (1) avec un agent de clivage grâce au caractère auto-immolable du lieur, où l'agent de clivage est un diène ;
chaque R₄₇ est choisi indépendamment dans le groupe consistant en
hydrogène, -L^{B}, -L^{A}, -(S^{P})ᵢ-C^{C}, -F, -Cl, -Br, -I, - OR₃₇, -N(R₃₇)₂, -SO₃, -PO₃-, -NO₂, -CF₃, -SR₃₇, S(=O)₂N(R₃₇)₂, OC(=O)R₃₇, SC(=O)R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, -S(O)₂R₃₇, NR₃₇S(O)₂R₃₇, - ON(R₃₇)₂, -NR₃₇OR₃₇, groupes alkyle en C₁-C₂₄, groupes alcényle en C₂-C₂₄, groupes alcynyle en C₂-C₂₄, groupes aryle en C₆-C₂₄, groupes hétéroaryle en C₂-C₂₄, groupes cycloalkyle en C₃-C₂₄, groupes cycloalcényle en C₅-C₂₄, groupes cycloalcynyle en C₁₂-C₂₄, groupes (cycloalkyle en C₃-C₂₄)hétéroaryle, groupes hétéroarylcycloalkyle en C₃-C₂₄, groupes (cycloalcényle en C₄-C₂₄)hétéroaryle, groupes hétéroarylcycloalkyle en C₄-C₂₄, groupes (cycloalcynyle en C₄-C₂₄)hétéroaryle, groupes hétéroarylcycloalcynyle en C₄-C₂₄, groupes alkylcycloalkyle en C₄-C₂₄, et groupes cycloalkylalkyle en C₄-C₂₄ ; où i est un entier dans une plage allant de 0 à 4, de préférence i est un entier allant de 0 à 1,
où les groupes alkyle, groupes alcényle, groupes alcynyle, groupes aryle, hétéroaryle, cycloalkyle, groupes cycloalcényle, groupes cycloalcynyle, groupes cycloalkylhétéroaryle, groupes hétéroarylcycloalkyle, groupes cycloalcénylhétéroaryle, groupes hétéroarylcycloalcényle, groupes cycloalcynylhétéroaryle, groupes hétéroarylcycloalcynyle, groupes alkylcycloalkyle, groupes cycloalkylalkyle sont éventuellement substitués par une fraction choisie dans le groupe consistant en - Cl, -F, -Br, -I, -OR₃₇, -N(R₃₇)₂, -SO₃R₃₇, - PO₃(R₃₇)₂ , - PO₄(R₃₇)₂ , -NO₂ , -CF₃, =O, =NR₃₇, et -SR₃₇, et contiennent éventuellement un ou plusieurs hétéroatomes choisis dans le groupe consistant en O, S, NR₃₇, P et Si, où les atomes de N, S et P sont éventuellement oxydés, où les atomes de N sont éventuellement quaternisés ;
deux R₄₇ et/ou R₃₇ sont éventuellement compris dans un cycle,
deux R₄₇ et/ou R₃₇ sont éventuellement compris dans un cycle de manière à former un cycle fusionné au cycle trans à huit chaînons de formule (1) ;
chaque R₃₇ est choisi indépendamment dans le groupe consistant en hydrogène, -L^{B}, -L^{A}, -(S^{P})ᵢ-C^{C}, groupes alkyle en C₁-C₂₄, groupes alcényle en C₂-C₂₄, groupes alcynyle en C₂-C₂₄, groupes aryle en C₆-C₂₄, groupes hétéroaryle en C₂-C₂₄, groupes cycloalkyle en C₃-C₂₄, groupes cycloalcényle en C₅-C₂₄, groupes cycloalcynyle en C₁₂-C₂₄, groupes (cycloalkyle en C₃-C₂₄) hétéroaryle, groupes hétéroarylcycloalkyle en C₃-C₂₄, groupes (cycloalcényle en C₄-C₂₄) hétéroaryle, groupes hétéroarylcycloalkyle en C₄-C₂₄, groupes (cycloalcynyle en C₄-C₂₄)hétéroaryle, groupes hétéroarylcycloalcynyle en C₄-C₂₄, groupes alkylcycloalkyle en C₄-C₂₄, et groupes cycloalkylalkyle en C₄-C₂₄ ; où i est un entier dans une plage allant de 0 à 4, de préférence i est 1 ;
les groupes R₃₇ qui ne sont pas hydrogène sont éventuellement substitués par une fraction choisie dans le groupe consistant en -Cl, -F, -Br, -I, -OH, -NH₂, - SO₃H, -PO₃H , -PO₄H₂, -NO₂, -CF₃, =O, =NH, et -SH, et contiennent éventuellement un ou plusieurs hétéroatomes choisis dans le groupe consistant en O, S, NH, P et Si, où les atomes de N, S et P sont éventuellement oxydés, où les atomes de N sont éventuellement quaternisés.

2. Composé selon la revendication 1, dans lequel le radionucléide est choisi dans le groupe consistant en ³H, ²¹C, ¹³N, ¹⁵O, ¹⁸F, ¹⁹F, ⁵¹Cr, ⁵²Fe, ⁵²Mn, ⁵⁵Co, ⁶⁰Cu, ⁶¹Cu, ⁶²Zn, ⁶²Cu, ⁶³Zn, ⁶⁴Cu, ⁶⁶Ga, ⁶⁷Ga, ⁶⁸Ga, ⁷⁰As, ⁷¹As, ⁷²As, ⁷⁴As, ⁷⁵Se, ⁷⁵Br, ⁷⁶Br, ⁷⁷Br, ⁸⁰Br, ⁸²Br, ⁸²Rb, ⁸⁶Y, ⁸⁸Y, ⁹⁰Y, ⁸⁹Sr, ⁸⁹Zr, ⁹⁷Ru, ^{99m}Tc, ¹¹⁰In, ²¹¹In, ¹¹³In, ¹¹⁴In, ¹¹⁷Sn, ¹²⁰I, ¹²²Xe, ¹²³I, ¹²⁴I, ¹²⁵I, ¹⁶⁶Ho, ¹⁶⁷Tm, ¹⁶⁹Yb, ¹⁹³Pt, ¹⁹⁵Pt, ²⁰¹Tl, ²⁰³Pb, ²⁴Na, ³²P, ³³P, ⁴⁷Sc, ⁵⁹Fe, ⁶⁷Cu, ⁷⁶As, ⁷⁷As, ⁸⁰Br, ⁸²Br, ⁸⁹Sr, ⁹⁰Nb, ⁹⁰Y, ¹⁰³Ru, ¹⁰⁵Rh, ¹⁰⁹Pd, ²¹¹Ag, ²¹¹In, ¹²¹Sn, ¹²⁷Te, ¹³¹I, ¹⁴⁰La, ¹⁴¹Ce, ¹⁴²Pr, ¹⁴³Pr, ¹⁴⁴Pr, ¹⁴⁹Pm, ¹⁴⁹Tb, ¹⁵¹Pm, ¹⁵³Sm, ¹⁵⁹Gd, ¹⁶¹Tb, ¹⁶⁵Dy, ¹⁶⁶Dy, ¹⁶⁶Ho, ¹⁶⁹Er, ¹⁷²Tm, ¹⁷⁵Yb, ¹⁷⁷Lu, ¹⁸⁶Re, ¹⁸⁸Re, ¹⁹⁸Au, ¹⁹⁹Au, ²¹¹At, ²¹¹Bi, ²¹²Bi, ²¹²Pb, ²¹³Bi, ²¹⁴Bi, ²²³Ra, ²²⁴Ra, ²²⁵Ac et ²²⁷Th.

3. Composé selon l'une quelconque des revendications précédentes, dans lequel le marqueur est une fraction chélatrice qui chélate le radionucléide.

4. Composé selon l'une quelconque des revendications précédentes, dans lequel X¹, X², X³, et X⁴ sont -C(R₄₇)₂-, et de préférence au maximum quatre R₄₇, de manière mieux préférée au maximum deux R₄₇, ne sont pas H.

5. Composé selon l'une quelconque des revendications précédentes, qui comprend au maximum un marqueur et au maximum un agent d'administration.

6. Combinaison comprenant le composé selon l'une quelconque des revendications précédentes, et un agent de clivage, à condition que, lorsqu'au moins un R₄₈ dans la formule (1) comprend un marqueur, alors l'agent de clivage ne comprend pas le même marqueur que R₄₈ ; à la condition que, lorsqu'au moins un R₄₈ dans la formule (1) comprend un agent d'administration, alors l'agent de clivage ne comprend pas le même agent d'administration que R₄₈ ; où l'agent de clivage est un diène.

7. Combinaison selon la revendication 6, dans laquelle le diène est une tétrazine, de préférence une tétrazine répondant à la formule (4) et de préférence comprenant des sels pharmaceutiquement acceptés de celle-ci : 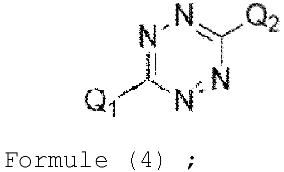 dans laquelle
chaque fraction Q₁ et Q₂ est choisie indépendamment dans le groupe consistant en hydrogène, -F, -Cl, -Br, - I, -OR₃₇, -N(R₃₇)₂, -SO₃, -PO₃-, -NO₂, -CF₃, -SR₃₇, S(=O)₂N(R₃₇)₂, OC(=O)R₃₇, SC(=O)R₃₇, OC(=S)R₃₇, SC(=S)R₃₇, NR₃₇C(=O)-R₃₇, NR₃₇C(=S)-R₃₇, NR₃₇C(=O)O-R₃₇, NR₃₇C(=S)O-R₃₇, NR₃₇C(=O)S-R₃₇, NR₃₇C(=S)S-R₃₇, OC(=O)N(R₃₇)₂, SC(=O)N(R₃₇)₂, OC(=S)N(R₃₇)₂, SC(=S)N(R₃₇)₂, NR₃₇C(=O)N(R₃₇)₂, NR₃₇C(=S)N(R₃₇)₂, C(=O)R₃₇, C(=S)R₃₇, C(=O)N(R₃₇)₂, C(=S)N(R₃₇)₂, C(=O)O-R₃₇, C(=O)S-R₃₇, C(=S)O-R₃₇, C(=S)S-R₃₇, S(O)R₃₇, -S(O)₂R₃₇, NR₃₇S(O)₂R₃₇, -ON(R₃₇)₂, -NR₃₇OR₃₇, groupes alkyle, groupes alcényle, groupes alcynyle , groupes aryle, groupes hétéroaryle, groupes cycloalkyle, groupes cycloalcényle, groupes cycloalcynyle, groupes cycloalkylhétéroaryle, groupes hétéroarylcycloalkyle, groupes cycloalcénylhétéroaryle, groupes hétéroarylcycloalkyle, groupes cycloalcynylhétéroaryle, groupes hétéroarylcycloalcynyle, groupes alkylcycloalkyle, et groupes cycloalkylalkyle ;
dans laquelle les groupes Q₁ et Q₂ qui ne sont pas H, -F, -Cl, -Br, -I, -OH, -NH₂, -SO₃, -PO₃, -NO₂, -CF₃, sont éventuellement substitués, de préférence par une fraction choisie dans le groupe consistant en -Cl, -F, -Br, -I, -OR₃₇, -N(R₃₇)₂, -SO₃R₃₇, -PO₃(R₃₇)₂ , -PO₄(R₃₇)₂ , -NO₂ , -CF₃, =O, =NR₃₇, et -SR₃₇, et contiennent éventuellement un ou plusieurs hétéroatomes choisis dans le groupe consistant en O, S, NR₃₇, P et Si, où les atomes de N, S et P sont éventuellement oxydés, où les atomes de N sont éventuellement quaternisés ;
dans laquelle les groupes Q₁ et Q₂ sont éventuellement liés à un polymère, une particule, un peptide, un peptoïde, un dendrimère, une protéine, un aptamère, un hydrate de carbone, un oligonucléotide, un oligosaccharide, un lipide, un stéroïde, un liposome, un agent de ciblage T^{T}, -(S^{P})_{D}-R⁸⁷, une fraction liant l'albumine, et une fraction chélatrice ;
dans laquelle D est 0 ou 1,
S^{P} est un écarteur ;
chaque R⁸⁷ est choisi indépendamment dans le groupe consistant en molécules organiques, molécules inorganiques, molécules organométalliques, résines, billes, verre, microparticules, nanoparticules, gels, surfaces, et cellules ;
et au moins une des fractions Q₁ et Q₂ n'est pas hydrogène.

8. Combinaison selon la revendication 7, dans laquelle Q₁ et Q₂ sont choisis dans le groupe des hydrogène, alkyle en C₁-C₈, phényle, 2-pyridyle, 3-pyridyle, 4-pyridyle, 2,6-pyrimidyle, 2,5-pyrimidyle, 3,5-pyrimidyle, et 2,4-pyrimidyle ; et Q₁ et Q₂ qui ne sont pas hydrogène sont éventuellement substitués tel que cela est défini dans la revendication 7.

9. Combinaison selon l'une quelconque des revendications 7 à 8, dans laquelle, dans la formule (4) :
(a) Q₁ et Q₂ sont choisis dans le groupe consistant en 2-pyridyle, 3-pyridyle et 4-pyridyle ;
(b) Q₁ est choisi dans le groupe consistant en 2,6-pyrimidyle, 2,5-pyrimidyle, 3,5-pyrimidyle, et 2,4-pyrimidyle ; et Q₂ est hétéroalkyle ; ou
(c) Q₁ est phényle et Q₂ est hydrogène ;
et dans (a) à (c), tous les Q₁ et Q₂ qui ne sont pas hydrogène sont éventuellement substitués tel que cela est défini dans la revendication 7.

10. Composé selon l'une quelconque des revendications 1 à 5, ou combinaison selon l'une quelconque des revendications 6 à 9 pour une utilisation en tant que médicament.

11. Composé selon l'une quelconque des revendications 1 à 5, ou combinaison selon l'une quelconque des revendications 6 à 9 pour une utilisation dans le traitement d'une maladie, de préférence un cancer, chez un sujet, de préférence un être humain, dans lequel le traitement est la radiothérapie.

12. Composé selon l'une quelconque des revendications 1 à 5, ou combinaison selon l'une quelconque des revendications 6 à 9 pour une utilisation dans une méthode de diagnostic comprenant les étapes de :
(a) administration d'un composé selon la formule (1) tel que défini selon l'une quelconque des revendications 1 à 5, à un sujet, de préférence un être humain ;
(b) administration d'un agent de clivage tel que défini selon l'une quelconque des revendications 6 à 9, audit sujet ;
(c) imagerie du composé selon la formule (1) présent chez le sujet pour collecter des données ;
(d) comparaison desdites données à des valeurs standards ;
(e) découverte d'un écart significatif par rapport aux valeurs standards au cours de la comparaison ;
(f) attribution de l'écart significatif à un tableau clinique particulier, de préférence à un cancer.

13. Méthode non thérapeutique pour l'imagerie d'un composé selon l'une quelconque des revendications 1 à 5 chez un sujet, de préférence un être humain, dans laquelle les étapes suivantes ont été réalisées sur le sujet :
(a) administration d'un composé selon la formule (1) tel que défini selon l'une quelconque des revendications 1 à 5, au sujet ;
(b) administration d'un agent de clivage tel que défini selon l'une quelconque des revendications 6 à 9, audit sujet ;
la méthode non thérapeutique comprenant l'étape de :
(c) imagerie du composé selon la formule (1) présent chez le sujet.

14. Utilisation non thérapeutique d'un composé selon l'une quelconque des revendications 1 à 5, ou d'une combinaison selon l'une quelconque des revendications 6 à 9, pour l'imagerie chez un sujet, de préférence un être humain.
